(19) Europäisches Patentamt
European Patent Office
Office européen des brevets

(11) EP 2 274 429 B1

(12) **EUROPEAN PATENT SPECIFICATION**

(45) Date of publication and mention of the grant of the patent:
**22.10.2014 Bulletin 2014/43**

(21) Application number: **09728214.9**

(22) Date of filing: **03.04.2009**

(51) Int Cl.:
*C12N 15/62* (2006.01) *C12N 9/00* (2006.01)

(86) International application number:
**PCT/US2009/039373**

(87) International publication number:
**WO 2009/124225 (08.10.2009 Gazette 2009/41)**

(54) **MULTIZYMES**

MULTIZYME

MULTIZYMES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **03.04.2008 US 41956**

(43) Date of publication of application:
**19.01.2011 Bulletin 2011/03**

(73) Proprietors:
- **E. I. du Pont de Nemours and Company Wilmington, DE 19898 (US)**
- **Pioneer Hi-Bred International, Inc. Des Moines, IA 50309 (US)**

(72) Inventors:
- **DAMUDE, Howard, Glenn Hockessin DE 19707 (US)**
- **GUTTERIDGE, Steven Wilmington DE 19810 (US)**
- **KINNEY, Anthony, J. Wilmington DE 19809 (US)**
- **LASSNER, Michael, W. Urbandale IA 50323 (US)**
- **SIEHL, Daniel, L. Menlo Park CA 94025 (US)**

(74) Representative: **Dzieglewska, Hanna Eva Dehns St Bride's House 10 Salisbury Square London EC4Y 8JD (GB)**

(56) References cited:
**WO-A-02/057465 WO-A-2008/124048**

- **MEYER A ET AL: "Biosynthesis of docosahexaenoic acid in Euglena gracilis: Biochemical and molecular evidence for the involvement of a DELTA4-fatty acyl group desaturase" BIOCHEMISTRY, AMERICAN CHEMICAL SOCIETY, EASTON, PA.; US, vol. 42, no. 32, 19 August 2003 (2003-08-19), pages 9779-9788, XP002298344 ISSN: 0006-2960 cited in the application**
- **CHIRALA SUBRAHMANYAM S ET AL: "Animal fatty acid synthase: Functional mapping and cloning and expression of the domain I constituent activities" PROCEEDINGS OF THE NATIONAL ACADEMY OF SCIENCES OF USA, NATIONAL ACADEMY OF SCIENCE, WASHINGTON, DC, US, vol. 94, no. 11, 1 January 1997 (1997-01-01), pages 5588-5593, XP002491682 ISSN: 0027-8424**
- **MEYER A ET AL: "NOVEL FATTY ACID ELONGASES AND THEIR USE FOR THE RECONSTITUTION OF DOCOSAHEXAENOIC ACID BIOSYNTHESIS" JOURNAL OF LIPID RESEARCH, BETHESDA, MD, US, vol. 45, no. 10, 1 October 2004 (2004-10-01), pages 1899-1909, XP009046591 ISSN: 0022-2275**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 274 429 B1

SEQUENCE LISTING

<110> E. I. duPont de Nemours & Company, Inc. Damude, Howard Gutteridge, Steve Kinney, Anthony Lassner, Michael Siehl, Dan

<120> MULTIZYMES

<130> BB1662 PCT

<150> 61/041956 <151> 2008-04-03

<160> 50

<170> PatentIn version 3.4

<210> 1
<211> 277
<212> PRT
<213> Pavlova sp. CCMP459

<400> 1

```
Met Met Leu Ala Ala Gly Tyr Leu Leu Val Leu Ser Ala Ala Arg Gln
1               5                   10                  15

Ser Phe Gln Gln Asp Ile Asp Asn Pro Asn Gly Ala Tyr Ser Thr Ser
            20                  25                  30

Trp Thr Gly Leu Pro Ile Val Met Ser Val Val Tyr Leu Ser Gly Val
        35                  40                  45

Phe Gly Leu Thr Lys Tyr Phe Glu Asn Arg Lys Pro Met Thr Gly Leu
    50                  55                  60

Lys Asp Tyr Met Phe Thr Tyr Asn Leu Tyr Gln Val Ile Ile Asn Val
65                  70                  75                  80

Trp Cys Val Val Ala Phe Leu Leu Glu Val Arg Arg Ala Gly Met Ser
                85                  90                  95

Leu Ile Gly Asn Lys Val Asp Leu Gly Pro Asn Ser Phe Arg Leu Gly
            100                 105                 110

Phe Val Thr Trp Val His Tyr Asn Asn Lys Tyr Val Glu Leu Leu Asp
        115                 120                 125

Thr Leu Trp Met Val Leu Arg Lys Lys Thr Gln Gln Val Ser Phe Leu
    130                 135                 140
```

```
His Val Tyr His His Val Leu Leu Met Trp Ala Trp Phe Val Val Val
145                 150                 155                 160

Lys Leu Gly Asn Gly Gly Asp Ala Tyr Phe Gly Gly Leu Met Asn Ser
                165                 170                 175

Ile Ile His Val Met Met Tyr Ser Tyr Tyr Thr Met Ala Leu Leu Gly
            180                 185                 190

Trp Ser Cys Pro Trp Lys Arg Tyr Leu Thr Gln Ala Gln Leu Val Gln
        195                 200                 205

Phe Cys Ile Cys Leu Ala His Ser Thr Trp Ala Ala Val Thr Gly Ala
    210                 215                 220

Tyr Pro Trp Arg Ile Cys Leu Val Glu Val Trp Val Met Val Ser Met
225                 230                 235                 240

Leu Val Leu Phe Thr Arg Phe Tyr Arg Gln Ala Tyr Ala Lys Glu Ala
                245                 250                 255

Lys Ala Lys Glu Ala Lys Lys Leu Ala Gln Glu Ala Ser Gln Ala Lys
            260                 265                 270

Ala Val Lys Ala Glu
        275
```

<210> 2
<211> 897
<212> DNA
<213> Euglena gracilis

<400> 2

```
atggcggata gcccagtcat caacctcagc accatgtgga aacccctttc actgatggga      60
catgtctgga agcaggcaca acaggagggc agcatttcgg cctatgctga ttctgttcgg     120
attcctctca ttatgtccgt tttatactta tcaatgatct tcgtggggtg ccgctggatg     180
aagaaccgtg agccctttga gatcaaaaca tacatgtttg cgtataacct gtatcagacc     240
ttgatgaacc tttgcatcgt gttgggattc ttgtaccagg tgcatgccac tgggatgcgc     300
ttttggggaa gtggtgtcga ccgaagcccg aaaggtttgg gcattggctt cttcatttat     360
gcccactacc acaacaagta tgtggaatat tttgatacac tttttatggt gctgcgaaag     420
aagaacaacc agatttcttt ccttcacgtg tatcatcatg ccctgttgac atgggcttgg     480
tttgctgttg tgtatttcgc acctggaggt gatggctggt ttggagcttg ctacaattct     540
```

```
tccatccatg tcctgatgta ctcttactac ttgcttgcaa cttttggcat cagttgccca    600

tggaagaaga tcttgacaca gctccagatg gttcagttct gtttctgttt tacacattcc    660

atttatgtgt ggatttgcgg gtcagagatc tacccacggc ctctgactgc tttgcagtcg    720

ttcgtgatgg tcaatatgtt ggtgctgttt ggcaatttct atgtcaagca atactcccaa    780

aagaacggca agccggagaa cggagccacc cctgagaacg gagcgaagcc gcaaccttgc    840

gagaacggca cggtggaaaa gcgagaggcg ccccgatctg tcggcatggg acgctga       897
```

<210> 3
<211> 298
<212> PRT
<213> Euglena gracilis

<400> 3

```
Met Ala Asp Ser Pro Val Ile Asn Leu Ser Thr Met Trp Lys Pro Leu
1               5                   10                  15

Ser Leu Met Gly His Val Trp Lys Gln Ala Gln Gln Glu Gly Ser Ile
            20                  25                  30

Ser Ala Tyr Ala Asp Ser Val Arg Ile Pro Leu Ile Met Ser Val Leu
        35                  40                  45

Tyr Leu Ser Met Ile Phe Val Gly Cys Arg Trp Met Lys Asn Arg Glu
    50                  55                  60

Pro Phe Glu Ile Lys Thr Tyr Met Phe Ala Tyr Asn Leu Tyr Gln Thr
65                  70                  75                  80

Leu Met Asn Leu Cys Ile Val Leu Gly Phe Leu Tyr Gln Val His Ala
                85                  90                  95

Thr Gly Met Arg Phe Trp Gly Ser Gly Val Asp Arg Ser Pro Lys Gly
            100                 105                 110

Leu Gly Ile Gly Phe Phe Ile Tyr Ala His Tyr His Asn Lys Tyr Val
        115                 120                 125

Glu Tyr Phe Asp Thr Leu Phe Met Val Leu Arg Lys Lys Asn Asn Gln
    130                 135                 140

Ile Ser Phe Leu His Val Tyr His His Ala Leu Leu Thr Trp Ala Trp
145                 150                 155                 160
```

```
Phe Ala Val Val Tyr Phe Ala Pro Gly Gly Asp Gly Trp Phe Gly Ala
                165                 170                 175

Cys Tyr Asn Ser Ser Ile His Val Leu Met Tyr Ser Tyr Tyr Leu Leu
            180                 185                 190

Ala Thr Phe Gly Ile Ser Cys Pro Trp Lys Lys Ile Leu Thr Gln Leu
        195                 200                 205

Gln Met Val Gln Phe Cys Phe Cys Phe Thr His Ser Ile Tyr Val Trp
    210                 215                 220

Ile Cys Gly Ser Glu Ile Tyr Pro Arg Pro Leu Thr Ala Leu Gln Ser
225                 230                 235                 240

Phe Val Met Val Asn Met Leu Val Leu Phe Gly Asn Phe Tyr Val Lys
                245                 250                 255

Gln Tyr Ser Gln Lys Asn Gly Lys Pro Glu Asn Gly Ala Thr Pro Glu
            260                 265                 270

Asn Gly Ala Lys Pro Gln Pro Cys Glu Asn Gly Thr Val Glu Lys Arg
        275                 280                 285

Glu Ala Pro Arg Ser Val Gly Met Gly Arg
    290                 295
```

<210> 4
<211> 2382
<212> DNA
<213> Euglena gracilis

<400> 4

```
atggcggata gcccagtcat caacctcagc accatgtgga aacccctttc actgatggct     60
ttggaccttg ccgttttggg acatgtctgg aagcaggcac aacaggaggg cagcatttcg    120
gcctatgctg attctgtttg gactcctctc attatgtccg gtttatactt atcaatgatc    180
ttcgtggggt gccgctggat gaagaaccgt gaaccctttg agatcaaaac atacatgttt    240
gcgtataacc tgtatcagac cttgatgaac ctttgcatcg tgttgggatt cttgtaccag    300
gtgcatgcca ctgggatgcg cttttgggga agtggtgtcg accgaagccc aaaaggtttg    360
ggcattggct tcttcattta tgcccactac cacaacaagt atgtggaata ttttgataca    420
ctttttatgg tgctgcgaaa gaagaacaac cagatttctt tccttcacgt gtatcatcat    480
gccctgttga catgggcttg gtttgctgtt gtgtatttcg cacctggagg tgatggctgg    540
tttggagctt gctacaattc ttccatccat gtcctgatgt actcttacta cttgcttgca    600
```

```
acttttggca tcagttgccc atggaagaag atcttgacac agctccagat ggttcaattc     660
tgtttctgtt ttacacattc catttatgtg tggatttgcg ggtcagagat ctacccacgg     720
cctctgactg ctttgcagtc gttcgtgatg gtcaatatgt tggtgctgtt tggcaatttc     780
tatgtcaagc aatactccca aaagaacggc aagccggaga acggagccac ccctgagaac     840
ggagcgaagc cgcaaccttg cgagaacggc acggtggaaa agcgagagaa tgacaccgcc     900
aacgttcggc ccgcccgtcc agctggactc ccgccggcca cgtactacga ctccctggca     960
gtgtcggggc agggcaagga gcggctgttc accaccgatg aggtgaggcg gcacatcctc    1020
cccaccgatg gctggctgac gtgccacgaa ggagtctacg atgtcactga tttccttgcc    1080
aagcaccctg gtggcggtgt catcacgctg ggccttggaa gggactgcac aatcctcgtc    1140
gagtcatacc accctgctgg gcgcccggac aaggtgatgg agaagtaccg cattggtacg    1200
ctgcaggacc ccaagacgtt ctatgcttgg ggagagtccg atttctaccc tgagttgaag    1260
cgccgggccc ttgcaaggct gaaggaggct ggtcaggcgc ggcgcggcgg ccttggggtg    1320
aaggccctcc tggtgctcac cctcttcttc gtgtcgtggt acatgtgggt ggcccacaag    1380
tccttcctct gggccgccgt ctggggcttc gccggctccc acgtcgggct gagcatccag    1440
cacgacggca accacggcgc gttcagccgc agcacactgg tgaaccgcct ggcggggtgg    1500
ggcatggact tgatcggcgc gtcgtcaacg gtgtgggagt accagcacgt catcggccac    1560
caccagtaca ccaacctcgt gtcggacacg ctattcagtc tgcctgagaa cgatccggac    1620
gtcttctcca gctacccgct gatgcgcatg cacccggata cggcgtggca gccgcaccac    1680
cgcttccagc acctgttcgc gttcccactg ttcgccctga tgacaatcag caaggtgctg    1740
accagcgatt tcgctgtctg cctcagcatg aagaaggggt ccatcgactg ctcctccagg    1800
ctcgtcccac tggaggggca gctgctgttc tggggggcca agctggcgaa cttcctgttg    1860
cagattgtgt tgccatgcta cctccacggg acagctatgg gcctggccct cttctctgtt    1920
gcccaccttg tgtcggggga gtacctcgcg atctgcttca tcatcaacca catcagcgag    1980
tcttgtgagt ttatgaatac aagctttcaa accgccgccc ggaggacaga gatgcttcag    2040
gcagcccatc aggcagcgga ggccaagaag gtgaagccca cccctccacc gaacgattgg    2100
gctgtgacac aggtccaatg ctgcgtgaat tggagatcag gtggcgtgtt ggccaatcac    2160
ctctctggag gcttgaacca ccagatcgag catcatctgt tccccagcat ctcgcatgcc    2220
aactacccca tcatcgcccg tgttgtgaag gaggtgtgcg aggagtatgg gttgccgtac    2280
aagaactacg tcacgttctg ggatgcagtc tgtggcatgg ttcagcacct ccggttgatg    2340
ggtgctccac cggtgccaac gaacggggac aaaaagtcat aa                       2382
```

<210> 5
<211> 793
<212> PRT
<213> Euglena gracilis

<400> 5

```
Met Ala Asp Ser Pro Val Ile Asn Leu Ser Thr Met Trp Lys Pro Leu
1               5                   10                  15

Ser Leu Met Ala Leu Asp Leu Ala Val Leu Gly His Val Trp Lys Gln
            20                  25                  30

Ala Gln Gln Glu Gly Ser Ile Ser Ala Tyr Ala Asp Ser Val Trp Thr
        35                  40                  45

Pro Leu Ile Met Ser Gly Leu Tyr Leu Ser Met Ile Phe Val Gly Cys
    50                  55                  60

Arg Trp Met Lys Asn Arg Glu Pro Phe Glu Ile Lys Thr Tyr Met Phe
65                  70                  75                  80

Ala Tyr Asn Leu Tyr Gln Thr Leu Met Asn Leu Cys Ile Val Leu Gly
                85                  90                  95

Phe Leu Tyr Gln Val His Ala Thr Gly Met Arg Phe Trp Gly Ser Gly
            100                 105                 110

Val Asp Arg Ser Pro Lys Gly Leu Gly Ile Gly Phe Phe Ile Tyr Ala
        115                 120                 125

His Tyr His Asn Lys Tyr Val Glu Tyr Phe Asp Thr Leu Phe Met Val
    130                 135                 140

Leu Arg Lys Lys Asn Asn Gln Ile Ser Phe Leu His Val Tyr His His
145                 150                 155                 160

Ala Leu Leu Thr Trp Ala Trp Phe Ala Val Val Tyr Phe Ala Pro Gly
                165                 170                 175

Gly Asp Gly Trp Phe Gly Ala Cys Tyr Asn Ser Ser Ile His Val Leu
            180                 185                 190

Met Tyr Ser Tyr Tyr Leu Leu Ala Thr Phe Gly Ile Ser Cys Pro Trp
        195                 200                 205

Lys Lys Ile Leu Thr Gln Leu Gln Met Val Gln Phe Cys Phe Cys Phe
```

```
    210                 215                 220

Thr His Ser Ile Tyr Val Trp Ile Cys Gly Ser Glu Ile Tyr Pro Arg
225                 230                 235                 240

Pro Leu Thr Ala Leu Gln Ser Phe Val Met Val Asn Met Leu Val Leu
                245                 250                 255

Phe Gly Asn Phe Tyr Val Lys Gln Tyr Ser Gln Lys Asn Gly Lys Pro
            260                 265                 270

Glu Asn Gly Ala Thr Pro Glu Asn Gly Ala Lys Pro Gln Pro Cys Glu
        275                 280                 285

Asn Gly Thr Val Glu Lys Arg Glu Asn Asp Thr Ala Asn Val Arg Pro
    290                 295                 300

Ala Arg Pro Ala Gly Leu Pro Pro Ala Thr Tyr Tyr Asp Ser Leu Ala
305                 310                 315                 320

Val Ser Gly Gln Gly Lys Glu Arg Leu Phe Thr Thr Asp Glu Val Arg
                325                 330                 335

Arg His Ile Leu Pro Thr Asp Gly Trp Leu Thr Cys His Glu Gly Val
            340                 345                 350

Tyr Asp Val Thr Asp Phe Leu Ala Lys His Pro Gly Gly Gly Val Ile
        355                 360                 365

Thr Leu Gly Leu Gly Arg Asp Cys Thr Ile Leu Val Glu Ser Tyr His
    370                 375                 380

Pro Ala Gly Arg Pro Asp Lys Val Met Glu Lys Tyr Arg Ile Gly Thr
385                 390                 395                 400

Leu Gln Asp Pro Lys Thr Phe Tyr Ala Trp Gly Glu Ser Asp Phe Tyr
                405                 410                 415

Pro Glu Leu Lys Arg Arg Ala Leu Ala Arg Leu Lys Glu Ala Gly Gln
            420                 425                 430

Ala Arg Arg Gly Gly Leu Gly Val Lys Ala Leu Leu Val Leu Thr Leu
        435                 440                 445

Phe Phe Val Ser Trp Tyr Met Trp Val Ala His Lys Ser Phe Leu Trp
    450                 455                 460
```

```
Ala Ala Val Trp Gly Phe Ala Gly Ser His Val Gly Leu Ser Ile Gln
465                 470                 475                 480

His Asp Gly Asn His Gly Ala Phe Ser Arg Ser Thr Leu Val Asn Arg
                485                 490                 495

Leu Ala Gly Trp Gly Met Asp Leu Ile Gly Ala Ser Ser Thr Val Trp
            500                 505                 510

Glu Tyr Gln His Val Ile Gly His His Gln Tyr Thr Asn Leu Val Ser
        515                 520                 525

Asp Thr Leu Phe Ser Leu Pro Glu Asn Asp Pro Asp Val Phe Ser Ser
    530                 535                 540

Tyr Pro Leu Met Arg Met His Pro Asp Thr Ala Trp Gln Pro His His
545                 550                 555                 560

Arg Phe Gln His Leu Phe Ala Phe Pro Leu Phe Ala Leu Met Thr Ile
                565                 570                 575

Ser Lys Val Leu Thr Ser Asp Phe Ala Val Cys Leu Ser Met Lys Lys
            580                 585                 590

Gly Ser Ile Asp Cys Ser Ser Arg Leu Val Pro Leu Glu Gly Gln Leu
        595                 600                 605

Leu Phe Trp Gly Ala Lys Leu Ala Asn Phe Leu Leu Gln Ile Val Leu
    610                 615                 620

Pro Cys Tyr Leu His Gly Thr Ala Met Gly Leu Ala Leu Phe Ser Val
625                 630                 635                 640

Ala His Leu Val Ser Gly Glu Tyr Leu Ala Ile Cys Phe Ile Ile Asn
                645                 650                 655

His Ile Ser Glu Ser Cys Glu Phe Met Asn Thr Ser Phe Gln Thr Ala
            660                 665                 670

Ala Arg Arg Thr Glu Met Leu Gln Ala Ala His Gln Ala Ala Glu Ala
        675                 680                 685

Lys Lys Val Lys Pro Thr Pro Pro Pro Asn Asp Trp Ala Val Thr Gln
    690                 695                 700
```

Val Gln Cys Cys Val Asn Trp Arg Ser Gly Gly Val Leu Ala Asn His
705 710 715 720

Leu Ser Gly Gly Leu Asn His Gln Ile Glu His His Leu Phe Pro Ser
725 730 735

Ile Ser His Ala Asn Tyr Pro Ile Ile Ala Arg Val Val Lys Glu Val
740 745 750

Cys Glu Glu Tyr Gly Leu Pro Tyr Lys Asn Tyr Val Thr Phe Trp Asp
755 760 765

Ala Val Cys Gly Met Val Gln His Leu Arg Leu Met Gly Ala Pro Pro
770 775 780

Val Pro Thr Asn Gly Asp Lys Lys Ser
785 790

<210> 6
<211> 541
<212> PRT
<213> Euglena gracilis

<220>
<221> MISC_FEATURE
<222> (1)..(541)
<223> NCBI Accession No. AAQ19605 (GI 33466346), locus AAQ19605, CDS AY278558

<400> 6

Met Leu Val Leu Phe Gly Asn Phe Tyr Val Lys Gln Tyr Ser Gln Lys
1 5 10 15

Asn Gly Lys Pro Glu Asn Gly Ala Thr Pro Glu Asn Gly Ala Lys Pro
20 25 30

Gln Pro Cys Glu Asn Gly Thr Val Glu Lys Arg Glu Asn Asp Thr Ala
35 40 45

Asn Val Arg Pro Thr Arg Pro Ala Gly Pro Pro Pro Ala Thr Tyr Tyr
50 55 60

Asp Ser Leu Ala Val Ser Gly Gln Gly Lys Glu Arg Leu Phe Thr Thr
65 70 75 80

Asp Glu Val Arg Arg His Ile Leu Pro Thr Asp Gly Trp Leu Thr Cys
85 90 95

```
His Glu Gly Val Tyr Asp Val Thr Asp Phe Leu Ala Lys His Pro Gly
            100                 105                 110

Gly Gly Val Ile Thr Leu Gly Leu Gly Arg Asp Cys Thr Ile Leu Ile
        115                 120                 125

Glu Ser Tyr His Pro Ala Gly Arg Pro Asp Lys Val Met Glu Lys Tyr
    130                 135                 140

Arg Ile Gly Thr Leu Gln Asp Pro Lys Thr Phe Tyr Ala Trp Gly Glu
145                 150                 155                 160

Ser Asp Phe Tyr Pro Glu Leu Lys Arg Arg Ala Leu Ala Arg Leu Lys
                165                 170                 175

Glu Ala Gly Gln Ala Arg Arg Gly Gly Leu Gly Val Lys Ala Leu Leu
            180                 185                 190

Val Leu Thr Leu Phe Phe Val Ser Trp Tyr Met Trp Val Ala His Lys
        195                 200                 205

Ser Phe Leu Trp Ala Ala Val Trp Gly Phe Ala Gly Ser His Val Gly
    210                 215                 220

Leu Ser Ile Gln His Asp Gly Asn His Gly Ala Phe Ser Arg Asn Thr
225                 230                 235                 240

Leu Val Asn Arg Leu Ala Gly Trp Gly Met Asp Leu Ile Gly Ala Ser
                245                 250                 255

Ser Thr Val Trp Glu Tyr Gln His Val Ile Gly His His Gln Tyr Thr
            260                 265                 270

Asn Leu Val Ser Asp Thr Leu Phe Ser Leu Pro Glu Asn Asp Pro Asp
        275                 280                 285

Val Phe Ser Ser Tyr Pro Leu Met Arg Met His Pro Asp Thr Ala Trp
    290                 295                 300

Gln Pro His His Arg Phe Gln His Leu Phe Ala Phe Pro Leu Phe Ala
305                 310                 315                 320

Leu Met Thr Ile Ser Lys Val Leu Thr Ser Asp Phe Ala Val Cys Leu
                325                 330                 335
```

```
Ser Met Lys Lys Gly Ser Ile Asp Cys Ser Ser Arg Leu Val Pro Leu
            340                 345                 350

Glu Gly Gln Leu Leu Phe Trp Gly Ala Lys Leu Ala Asn Phe Leu Leu
        355                 360                 365

Gln Ile Val Leu Pro Cys Tyr Leu His Gly Thr Ala Met Gly Leu Ala
    370                 375                 380

Leu Phe Ser Val Ala His Leu Val Ser Gly Glu Tyr Leu Ala Ile Cys
385                 390                 395                 400

Phe Ile Ile Asn His Ile Ser Glu Ser Cys Glu Phe Met Asn Thr Ser
                405                 410                 415

Phe Gln Thr Ala Ala Arg Arg Thr Glu Met Leu Gln Ala Ala His Gln
            420                 425                 430

Ala Ala Glu Ala Lys Lys Val Lys Pro Thr Pro Pro Pro Asn Asp Trp
        435                 440                 445

Ala Val Thr Gln Val Gln Cys Cys Val Asn Trp Arg Ser Gly Gly Val
    450                 455                 460

Leu Ala Asn His Leu Ser Gly Gly Leu Asn His Gln Ile Glu His His
465                 470                 475                 480

Leu Phe Pro Ser Ile Ser His Ala Asn Tyr Pro Thr Ile Ala Pro Val
                485                 490                 495

Val Lys Glu Val Cys Glu Glu Tyr Gly Leu Pro Tyr Lys Asn Tyr Val
            500                 505                 510

Thr Phe Trp Asp Ala Val Cys Gly Met Val Gln His Leu Arg Leu Met
        515                 520                 525

Gly Ala Pro Pro Val Pro Thr Asn Gly Asp Lys Lys Ser
    530                 535                 540
```

<210> 7
<211> 2382
<212> DNA
<213> Euglena gracilis

<400> 7

atggcggata gcccagtcat caacctcagc accatgtgga aacccctttc actgatggct 60

```
ttggaccttg ccattttggg acatgtctgg aagcaggcac aacaggaggg cagcatttcg    120
gcctatgctg attctgtttg gactcctctc attatgtccg ttttatactt atcaatgatc    180
ttcgtggggt gccgctggat gaagaaccgt gaaccctttg agatcaaaac atacatgttt    240
gcgtataacc tgtatcagac cttgatgaac ctttgcatcg tgttgggatt cttgtaccag    300
gtgcatgcca ctgggatgcg cttttgggga agtggtgtcg accgaagccc gaaaggtttg    360
ggcattggct tcttcattta tgcccactac cacaacaagt atgtggaata ttttgataca    420
ctttttatgg tgctgcgaaa gaagaacaac cagatttctt tccttcacgt gtatcatcat    480
gccctgttga catgggcttg gtttgctgtt gtgtatttcg cacctggagg tgatggctgg    540
tttggagctt gctacaattc ttccatccat gtcctgatgt actcttacta cttgcttgca    600
acttttggca tcagttgccc atggaagaag atcttgacac agctccagat ggttcaattc    660
tgtttctgtt ttacacattc catttatgtg tggatttgcg ggtcagagat ctacccacgg    720
cctctgactg ctttgcagtc gttcgtgatg gtcaatatgt tggtgctgtt tggcaatttc    780
tatgtcaagc aatactccca aaagaacggc aagccggaga acggagccac ccctgagaac    840
ggagcgaagc cgcaaccttg cgagaacggc acggtggaaa agcgagagaa tgacaccgcc    900
aacgttcggc ccacccgtcc agctggaccc ccgccggcca cgtactacga ctccctggca    960
gtgtcggggc agggcaagga gcggctgttc accaccgatg aggtgaggcg gcacatcctc   1020
cccaccgatg gctggctgac gtgccacgaa ggagtctacg atgtcactga tttccttgcc   1080
aagcaccctg gtggcggtgt catcacgctg ggccttggaa gggactgcac aatcctcatc   1140
gagtcatacc accctgctgg gcgcccggac aaggtgatgg agaagtaccg cattggtacg   1200
ctgcaggacc ccaagacgtt ctatgcttgg ggagagtccg atttctaccc tgagttgaag   1260
cgccgggccc ttgcaaggct gaaggaggct ggtcaggcgc ggcgcggcgg ccttggggtg   1320
aaggccctcc tggtgctcac cctcttcttc gtgtcgtggt acatgtgggt ggcccacaag   1380
tccttcctct gggccgccgt ctggggcttc gccggctccc acgtcgggct gagcatccag   1440
cacgatggca accacggcgc gttcagccgc aacacactgg tgaaccgcct ggcggggtgg   1500
ggcatggact tgatcggcgc gtcgtccacg gtgtgggagt accagcacgt catcggccac   1560
caccagtaca ccaacctcgt gtcggacacg ctattcagtc tgcctgagaa cgatccggac   1620
gtcttctcca gctacccgct gatgcgcatg cacccggata cggcgtggca gccgcaccac   1680
cgcttccagc acctgttcgc gttcccactg ttcgccctga tgacaatcag caaggtgctg   1740
accagcgatt tcgctgtctg cctcagcatg aagaaggggt ccatcgactg ctcctccagg   1800
ctcgtcccac tggaggggca gctgctgttc tgggggggcca agctggcgaa cttcctgttg   1860
cagattgtgt tgccatgcta cctccacggg acagctatgg gcctggccct cttctctgtt   1920
```

```
gctcaccttg tgtcgggggga gtacctcgcg atctgcttca tcatcaacca catcagcgag   1980

tcttgtgagt ttatgaatac aagctttcaa accgccgccc ggaggacaga gatgcttcag   2040

gcagcacatc aggcagcgga ggccaagaag gtgaagccca cccctccacc gaacgattgg   2100

gctgtgacac aggtccaatg ctgcgtgaat tggagatcag gtggcgtgtt ggccaatcac   2160

ctctctggag gcttgaacca ccagatcgag catcatctgt tccccagcat ctcgcatgcc   2220

aactacccca ccatcgcccc tgttgtgaag gaggtgtgcg aggagtacgg gttgccgtac   2280

aagaattacg tcacgttctg ggatgcagtc tgtggcatgg ttcagcacct ccggttgatg   2340

ggtgctccac cggtgccaac gaacggggac aaaaagtcat aa                       2382
```

<210> 8
<211> 793
<212> PRT
<213> Euglena gracilis

<400> 8

```
Met Ala Asp Ser Pro Val Ile Asn Leu Ser Thr Met Trp Lys Pro Leu
1               5                   10                  15

Ser Leu Met Ala Leu Asp Leu Ala Ile Leu Gly His Val Trp Lys Gln
            20                  25                  30

Ala Gln Gln Glu Gly Ser Ile Ser Ala Tyr Ala Asp Ser Val Trp Thr
        35                  40                  45

Pro Leu Ile Met Ser Val Leu Tyr Leu Ser Met Ile Phe Val Gly Cys
    50                  55                  60

Arg Trp Met Lys Asn Arg Glu Pro Phe Glu Ile Lys Thr Tyr Met Phe
65                  70                  75                  80

Ala Tyr Asn Leu Tyr Gln Thr Leu Met Asn Leu Cys Ile Val Leu Gly
                85                  90                  95

Phe Leu Tyr Gln Val His Ala Thr Gly Met Arg Phe Trp Gly Ser Gly
            100                 105                 110

Val Asp Arg Ser Pro Lys Gly Leu Gly Ile Gly Phe Phe Ile Tyr Ala
        115                 120                 125

His Tyr His Asn Lys Tyr Val Glu Tyr Phe Asp Thr Leu Phe Met Val
    130                 135                 140
```

```
Leu Arg Lys Lys Asn Asn Gln Ile Ser Phe Leu His Val Tyr His His
145                 150                 155                 160

Ala Leu Leu Thr Trp Ala Trp Phe Ala Val Val Tyr Phe Ala Pro Gly
                165                 170                 175

Gly Asp Gly Trp Phe Gly Ala Cys Tyr Asn Ser Ser Ile His Val Leu
            180                 185                 190

Met Tyr Ser Tyr Tyr Leu Leu Ala Thr Phe Gly Ile Ser Cys Pro Trp
        195                 200                 205

Lys Lys Ile Leu Thr Gln Leu Gln Met Val Gln Phe Cys Phe Cys Phe
    210                 215                 220

Thr His Ser Ile Tyr Val Trp Ile Cys Gly Ser Glu Ile Tyr Pro Arg
225                 230                 235                 240

Pro Leu Thr Ala Leu Gln Ser Phe Val Met Val Asn Met Leu Val Leu
                245                 250                 255

Phe Gly Asn Phe Tyr Val Lys Gln Tyr Ser Gln Lys Asn Gly Lys Pro
            260                 265                 270

Glu Asn Gly Ala Thr Pro Glu Asn Gly Ala Lys Pro Gln Pro Cys Glu
        275                 280                 285

Asn Gly Thr Val Glu Lys Arg Glu Asn Asp Thr Ala Asn Val Arg Pro
    290                 295                 300

Thr Arg Pro Ala Gly Pro Pro Pro Ala Thr Tyr Tyr Asp Ser Leu Ala
305                 310                 315                 320

Val Ser Gly Gln Gly Lys Glu Arg Leu Phe Thr Thr Asp Glu Val Arg
                325                 330                 335

Arg His Ile Leu Pro Thr Asp Gly Trp Leu Thr Cys His Glu Gly Val
            340                 345                 350

Tyr Asp Val Thr Asp Phe Leu Ala Lys His Pro Gly Gly Gly Val Ile
        355                 360                 365

Thr Leu Gly Leu Gly Arg Asp Cys Thr Ile Leu Ile Glu Ser Tyr His
    370                 375                 380

Pro Ala Gly Arg Pro Asp Lys Val Met Glu Lys Tyr Arg Ile Gly Thr
```

385 390 395 400

Leu Gln Asp Pro Lys Thr Phe Tyr Ala Trp Gly Glu Ser Asp Phe Tyr
405 410 415

Pro Glu Leu Lys Arg Arg Ala Leu Ala Arg Leu Lys Glu Ala Gly Gln
420 425 430

Ala Arg Arg Gly Gly Leu Gly Val Lys Ala Leu Leu Val Leu Thr Leu
435 440 445

Phe Phe Val Ser Trp Tyr Met Trp Val Ala His Lys Ser Phe Leu Trp
450 455 460

Ala Ala Val Trp Gly Phe Ala Gly Ser His Val Gly Leu Ser Ile Gln
465 470 475 480

His Asp Gly Asn His Gly Ala Phe Ser Arg Asn Thr Leu Val Asn Arg
485 490 495

Leu Ala Gly Trp Gly Met Asp Leu Ile Gly Ala Ser Ser Thr Val Trp
500 505 510

Glu Tyr Gln His Val Ile Gly His His Gln Tyr Thr Asn Leu Val Ser
515 520 525

Asp Thr Leu Phe Ser Leu Pro Glu Asn Asp Pro Asp Val Phe Ser Ser
530 535 540

Tyr Pro Leu Met Arg Met His Pro Asp Thr Ala Trp Gln Pro His His
545 550 555 560

Arg Phe Gln His Leu Phe Ala Phe Pro Leu Phe Ala Leu Met Thr Ile
565 570 575

Ser Lys Val Leu Thr Ser Asp Phe Ala Val Cys Leu Ser Met Lys Lys
580 585 590

Gly Ser Ile Asp Cys Ser Ser Arg Leu Val Pro Leu Glu Gly Gln Leu
595 600 605

Leu Phe Trp Gly Ala Lys Leu Ala Asn Phe Leu Leu Gln Ile Val Leu
610 615 620

Pro Cys Tyr Leu His Gly Thr Ala Met Gly Leu Ala Leu Phe Ser Val
625 630 635 640

```
Ala His Leu Val Ser Gly Glu Tyr Leu Ala Ile Cys Phe Ile Ile Asn
                645                 650                 655

His Ile Ser Glu Ser Cys Glu Phe Met Asn Thr Ser Phe Gln Thr Ala
            660                 665                 670

Ala Arg Arg Thr Glu Met Leu Gln Ala Ala His Gln Ala Ala Glu Ala
        675                 680                 685

Lys Lys Val Lys Pro Thr Pro Pro Pro Asn Asp Trp Ala Val Thr Gln
    690                 695                 700

Val Gln Cys Cys Val Asn Trp Arg Ser Gly Gly Val Leu Ala Asn His
705                 710                 715                 720

Leu Ser Gly Gly Leu Asn His Gln Ile Glu His His Leu Phe Pro Ser
                725                 730                 735

Ile Ser His Ala Asn Tyr Pro Thr Ile Ala Pro Val Val Lys Glu Val
            740                 745                 750

Cys Glu Glu Tyr Gly Leu Pro Tyr Lys Asn Tyr Val Thr Phe Trp Asp
        755                 760                 765

Ala Val Cys Gly Met Val Gln His Leu Arg Leu Met Gly Ala Pro Pro
    770                 775                 780

Val Pro Thr Asn Gly Asp Lys Lys Ser
785                 790
```

<210> 9
<211> 2569
<212> DNA
<213> Euglena gracilis

<400> 9

```
gcggccgcta gacatgccac cagttgtggt atggttaacc gaatatgaac aaacacccga      60

ttccagtcca agattgctca ataaggaaca gggtggggga gcaacttgtg agagtttcga     120

cgccatgggt gcctacagat taagtagatc cttcttgtcc aacttgttga ccttgcgctc     180

ggcacattct gagagctggt ttcgcagccg gtagatttcg tcgttggcct gttgcaactg     240

ggtccgcagc tcggagtcgc aaagattgtt cgctgtgagg atctcagcac ggatttcttc     300

taattcagaa gccgcagcac ggagttgatt cccaagggta tcaaggcggc catatgcttg     360

ctccaataat ttctcaacac ggtcgggggt gatgctcagc ttaacagatt cttctggtgg     420
```

```
ttttggaagc tctccagcag gctccacggg gggcgcaggg ggcgcggcgg gaaccagtgg    480
aagagttggc aacgtcgaca gggggtactg tgcttgaggg ccaggaatca cggacgggac    540
ttgcggaatg taagatggac cagtaggaaa tgacggaaat gttcgagcaa atccgccaaa    600
acccgtgtac gcaggtgccg gggcgtaatt aacaatcgac agcggccgcg aattcgcggc    660
cgctcacatt ccatttatgt gtggatttgc gggtcagaga tctacccacg gcctctgact    720
gctttgcagt cgttcgtgat ggtcaatatg ttggtgctgt ttggcaattt ctatgtcaag    780
caatactccc aaaagaacgg caagccggag aacggagcca cccctgagaa cggagcgaag    840
ccgcaacctt gcgagaacgg cacggtggaa aagcgagaga atgacaccgc caacgttcgg    900
cccacccgtc cagctggacc cccgccggcc acgtactacg actccctggc agtgtcgggg    960
cagggcaagg agcggctgtt caccaccgat gaggtgaggc ggcacatcct ccccaccgat   1020
ggctggctga cgtgccacga aggagtctac gatgtcactg atttccttgc caagcaccct   1080
ggtggcggtg tcatcacgct gggccttgga agggactgca caatcctcat cgagtcatac   1140
caccctgctg ggcgcccgga caaggtgatg gagaagtacc gcattggtac gctgcaggac   1200
cccaagacgt tctatgcttg gggagagtcc gatttctacc ctgagttgaa gcgccgggcc   1260
cttgcaaggc tgaaggaggc tggtcaggcg cggcgcggcg gccttggggt gaaggccctc   1320
ctggtgctca ccctcttctt cgtgtcgtgg tacatgtggg tggcccacaa gtccttcctc   1380
tgggccgccg tctggggctt cgccggctcc cacgtcgggc tgagcatcca gcacgatggc   1440
aaccacggcg cgttcagccg caacacactg gtgaaccgcc tggcggggtg gggcatggac   1500
ttgatcggcg cgtcgtccac ggtgtgggag taccagcacg tcatcggcca ccaccagtac   1560
accaacctcg tgtcggacac gctattcagt ctgcctgaga acgatccgga cgtcttctcc   1620
agctacccgc tgatgcgcat gcacccggat acggcgtggc agccgcacca ccgcttccag   1680
cacctgttcg cgttcccact gttcgccctg atgacaatca gcaaggtgct gaccagcgat   1740
ttcgctgtct gcctcagcat gaagaagggg tccatcgact gctcctccag gctcgtccca   1800
ctggaggggc agctgctgtt ctggggggcc aagctggcga acttcctgtt gcagattgtg   1860
ttgccatgct acctccacgg gacagctatg ggcctggccc tcttctctgt tgctcacctt   1920
gtgtcggggg agtacctcgc gatctgcttc atcatcaacc acatcagcga gtcttgtgag   1980
tttatgaata caagctttca aaccgccgcc cggaggacag agatgcttca ggcagcacat   2040
caggcagcgg aggccaagaa ggtgaagccc accccctccac cgaacgattg ggctgtgaca   2100
caggtccaat gctgcgtgaa ttggagatca ggtggcgtgt tggccaatca cctctctgga   2160
ggcttgaacc accagatcga gcatcatctg ttccccagca tctcgcatgc caactacccc   2220
```

accatcgccc ctgttgtgaa ggaggtgtgc gaggagtacg ggttgccgta caagaattac 2280

gtcacgttct gggatgcagt ctgtggcatg gttcagcacc tccggttgat gggtgctcca 2340

ccggtgccaa cgaacgggga caaaaagtca taagccacga catcatttgg ggctcactcc 2400

gtgcagcctt ttcttgggct gcccacgaag atgcgcgatg aggcacctgg tggttgccct 2460

ccgccggcct cggaaaacgg ttcgacgcct gctccttcag cccagagcac tccggcgaag 2520

agtgaaagag cactgacctg aattttatga tgacccattt agcggccgc 2569

<210> 10
<211> 1626
<212> DNA
<213> Euglena gracilis

<400> 10

```
atgttggtgc tgtttggcaa tttctatgtc aagcaatact cccaaaagaa cggcaagccg      60
gagaacggag ccacccctga gaacggagcg aagccgcaac cttgcgagaa cggcacggtg     120
gaaaagcgag agaatgacac cgccaacgtt cggcccaccc gtccagctgg acccccgccg     180
gccacgtact acgactccct ggcagtgtcg gggcagggca aggagcggct gttcaccacc     240
gatgaggtga ggcggcacat cctccccacc gatggctggc tgacgtgcca cgaaggagtc     300
tacgatgtca ctgatttcct tgccaagcac cctggtggcg gtgtcatcac gctgggcctt     360
ggaagggact gcacaatcct catcgagtca taccaccctg ctgggcgccc ggacaaggtg     420
atggagaagt accgcattgg tacgctgcag gaccccaaga cgttctatgc ttggggagag     480
tccgatttct accctgagtt gaagcgccgg gcccttgcaa ggctgaagga ggctggtcag     540
gcgcggcgcg gcggccttgg ggtgaaggcc ctcctggtgc tcaccctctt cttcgtgtcg     600
tggtacatgt gggtggccca caagtccttc ctctgggccg ccgtctgggg cttcgccggc     660
tcccacgtcg ggctgagcat ccagcacgat ggcaaccacg gcgcgttcag ccgcaacaca     720
ctggtgaacc gcctggcggg gtggggcatg gacttgatcg gcgcgtcgtc cacggtgtgg     780
gagtaccagc acgtcatcgg ccaccaccag tacaccaacc tcgtgtcgga cacgctattc     840
agtctgcctg agaacgatcc ggacgtcttc tccagctacc cgctgatgcg catgcacccg     900
gatacggcgt ggcagccgca ccaccgcttc cagcacctgt tcgcgttccc actgttcgcc     960
ctgatgacaa tcagcaaggt gctgaccagc gatttcgctg tctgcctcag catgaagaag    1020
gggtccatcg actgctcctc caggctcgtc ccactggagg ggcagctgct gttctggggg    1080
gccaagctgg cgaacttcct gttgcagatt gtgttgccat gctacctcca cgggacagct    1140
atgggcctgg ccctcttctc tgttgctcac cttgtgtcgg gggagtacct cgcgatctgc    1200
ttcatcatca accacatcag cgagtcttgt gagtttatga atacaagctt tcaaaccgcc    1260
gcccggagga cagagatgct tcaggcagca catcaggcag cggaggccaa gaaggtgaag    1320
cccacccctc caccgaacga ttgggctgtg acacaggtcc aatgctgcgt gaattggaga    1380
tcaggtggcg tgttggccaa tcacctctct ggaggcttga accaccagat cgagcatcat    1440
ctgttcccca gcatctcgca tgccaactac cccaccatcg cccctgttgt gaaggaggtg    1500
tgcgaggagt acgggttgcc gtacaagaat tacgtcacgt tctgggatgc agtctgtggc    1560
atggttcagc acctccggtt gatgggtgct ccaccggtgc caacgaacgg ggacaaaaag    1620
tcataa                                                               1626
```

<210> 11
<211> 300
<212> PRT

<213> Ostreococcus tauri

<400> 11

```
Met Ser Ala Ser Gly Ala Leu Leu Pro Ala Ile Ala Ser Ala Ala Tyr
1               5                   10                  15

Ala Tyr Ala Thr Tyr Ala Tyr Ala Phe Glu Trp Ser His Ala Asn Gly
            20                  25                  30

Ile Asp Asn Val Asp Ala Arg Glu Trp Ile Gly Ala Leu Ser Leu Arg
        35                  40                  45

Leu Pro Ala Ile Ala Thr Thr Met Tyr Leu Leu Phe Cys Leu Val Gly
    50                  55                  60

Pro Arg Leu Met Ala Lys Arg Glu Ala Phe Asp Pro Lys Gly Phe Met
65                  70                  75                  80

Leu Ala Tyr Asn Ala Tyr Gln Thr Ala Phe Asn Val Val Val Leu Gly
                85                  90                  95

Met Phe Ala Arg Glu Ile Ser Gly Leu Gly Gln Pro Val Trp Gly Ser
            100                 105                 110

Thr Met Pro Trp Ser Asp Arg Lys Ser Phe Lys Ile Leu Leu Gly Val
        115                 120                 125

Trp Leu His Tyr Asn Asn Lys Tyr Leu Glu Leu Leu Asp Thr Val Phe
    130                 135                 140

Met Val Ala Arg Lys Lys Thr Lys Gln Leu Ser Phe Leu His Val Tyr
145                 150                 155                 160
```

```
His His Ala Leu Leu Ile Trp Ala Trp Trp Leu Val Cys His Leu Met
                165                 170                 175

Ala Thr Asn Asp Cys Ile Asp Ala Tyr Phe Gly Ala Ala Cys Asn Ser
            180                 185                 190

Phe Ile His Ile Val Met Tyr Ser Tyr Tyr Leu Met Ser Ala Leu Gly
        195                 200                 205

Ile Arg Cys Pro Trp Lys Arg Tyr Ile Thr Gln Ala Gln Met Leu Gln
    210                 215                 220

Phe Val Ile Val Phe Ala His Ala Val Phe Val Leu Arg Gln Lys His
225                 230                 235                 240

Cys Pro Val Thr Leu Pro Trp Ala Gln Met Phe Val Met Thr Asn Met
                245                 250                 255

Leu Val Leu Phe Gly Asn Phe Tyr Leu Lys Ala Tyr Ser Asn Lys Ser
            260                 265                 270

Arg Gly Asp Gly Ala Ser Ser Val Lys Pro Ala Glu Thr Thr Arg Ala
        275                 280                 285

Pro Ser Val Arg Arg Thr Arg Ser Arg Lys Ile Asp
    290                 295                 300
```

<210> 12
<211> 358
<212> PRT
<213> Thalassiosira pseudonana

<400> 12

```
Met Cys Ser Ser Pro Pro Ser Gln Ser Lys Thr Thr Ser Leu Leu Ala
1               5                   10                  15

Arg Tyr Thr Thr Ala Ala Leu Leu Leu Leu Thr Leu Thr Thr Trp Cys
            20                  25                  30

His Phe Ala Phe Pro Ala Ala Thr Ala Thr Pro Gly Leu Thr Ala Glu
        35                  40                  45

Met His Ser Tyr Lys Val Pro Leu Gly Leu Thr Val Phe Tyr Leu Leu
    50                  55                  60

Ser Leu Pro Ser Leu Lys Tyr Val Thr Asp Asn Tyr Leu Ala Lys Lys
```

```
65                  70                  75                  80

Tyr Asp Met Lys Ser Leu Leu Thr Glu Ser Met Val Leu Tyr Asn Val
                85                  90                  95

Ala Gln Val Leu Leu Asn Gly Trp Thr Val Tyr Ala Ile Val Asp Ala
            100                 105                 110

Val Met Asn Arg Asp His Pro Phe Ile Gly Ser Arg Ser Leu Val Gly
        115                 120                 125

Ala Ala Leu His Ser Gly Ser Ser Tyr Ala Val Trp Val His Tyr Cys
    130                 135                 140

Asp Lys Tyr Leu Glu Phe Phe Asp Thr Tyr Phe Met Val Leu Arg Gly
145                 150                 155                 160

Lys Met Asp Gln Val Ser Phe Leu His Ile Tyr His His Thr Thr Ile
                165                 170                 175

Ala Trp Ala Trp Trp Ile Ala Leu Arg Phe Ser Pro Gly Gly Asp Ile
            180                 185                 190

Tyr Phe Gly Ala Leu Leu Asn Ser Ile Ile His Val Leu Met Tyr Ser
        195                 200                 205

Tyr Tyr Ala Leu Ala Leu Leu Lys Val Ser Cys Pro Trp Lys Arg Tyr
    210                 215                 220

Leu Thr Gln Ala Gln Leu Leu Gln Phe Thr Ser Val Val Val Tyr Thr
225                 230                 235                 240

Gly Cys Thr Gly Tyr Thr His Tyr Tyr His Thr Lys His Gly Ala Asp
                245                 250                 255

Glu Thr Gln Pro Ser Leu Gly Thr Tyr Tyr Phe Cys Cys Gly Val Gln
            260                 265                 270

Val Phe Glu Met Val Ser Leu Phe Val Leu Phe Ser Ile Phe Tyr Lys
        275                 280                 285

Arg Ser Tyr Ser Lys Lys Asn Lys Ser Gly Gly Lys Asp Ser Lys Lys
    290                 295                 300

Asn Asp Asp Gly Asn Asn Glu Asp Gln Cys His Lys Ala Met Lys Asp
305                 310                 315                 320
```

Ile Ser Glu Gly Ala Lys Glu Val Val Gly His Ala Ala Lys Asp Ala
325 330 335

Gly Lys Leu Val Ala Thr Ala Ser Lys Ala Val Lys Arg Lys Gly Thr
340 345 350

Arg Val Thr Gly Ala Met
355

<210> 13
<211> 515
<212> PRT
<213> Thraustochytrium aureum

<220>
<221> MISC_FEATURE
<222> (1)..(515)
<223> NCBI Accession No. AAN75707(GI 25956288), locus AAN75707, CDS AF391543

<400> 13

Met Thr Val Gly Phe Asp Glu Thr Val Thr Met Asp Thr Val Arg Asn
1 5 10 15

His Asn Met Pro Asp Asp Ala Trp Cys Ala Ile His Gly Thr Val Tyr
20 25 30

Asp Ile Thr Lys Phe Ser Lys Val His Pro Gly Gly Asp Ile Ile Met
35 40 45

Leu Ala Ala Gly Lys Glu Ala Thr Ile Leu Phe Glu Thr Tyr His Ile
50 55 60

Lys Gly Val Pro Asp Ala Val Leu Arg Lys Tyr Lys Val Gly Lys Leu
65 70 75 80

Pro Gln Gly Lys Lys Gly Glu Thr Ser His Met Pro Thr Gly Leu Asp
85 90 95

Ser Ala Ser Tyr Tyr Ser Trp Asp Ser Glu Phe Tyr Arg Val Leu Arg
100 105 110

Glu Arg Val Ala Lys Lys Leu Ala Glu Pro Gly Leu Met Gln Arg Ala
115 120 125

Arg Met Glu Leu Trp Ala Lys Ala Ile Phe Leu Leu Ala Gly Phe Trp

```
    130                 135                 140

Gly Ser Leu Tyr Ala Met Cys Val Leu Asp Pro His Gly Gly Ala Met
145                 150                 155                 160

Val Ala Ala Val Thr Leu Gly Val Phe Ala Ala Phe Val Gly Thr Cys
                165                 170                 175

Ile Gln His Asp Gly Ser His Gly Ala Phe Ser Lys Ser Arg Phe Met
            180                 185                 190

Asn Lys Ala Ala Gly Trp Thr Leu Asp Met Ile Gly Ala Ser Ala Met
        195                 200                 205

Thr Trp Glu Met Gln His Val Leu Gly His His Pro Tyr Thr Asn Leu
    210                 215                 220

Ile Glu Met Glu Asn Gly Leu Ala Lys Val Lys Gly Ala Asp Val Asp
225                 230                 235                 240

Pro Lys Lys Val Asp Gln Glu Ser Asp Pro Asp Val Phe Ser Thr Tyr
                245                 250                 255

Pro Met Leu Arg Leu His Pro Trp His Arg Gln Arg Phe Tyr His Lys
            260                 265                 270

Phe Gln His Leu Tyr Ala Pro Leu Ile Phe Gly Phe Met Thr Ile Asn
        275                 280                 285

Lys Val Ile Ser Gln Asp Val Gly Val Val Leu Arg Lys Arg Leu Phe
    290                 295                 300

Gln Ile Asp Ala Asn Cys Arg Tyr Gly Ser Pro Trp Asn Val Ala Arg
305                 310                 315                 320

Phe Trp Ile Met Lys Leu Leu Thr Thr Leu Tyr Met Val Ala Leu Pro
                325                 330                 335

Met Tyr Met Gln Gly Pro Ala Gln Gly Leu Lys Leu Phe Phe Met Ala
            340                 345                 350

His Phe Thr Cys Gly Glu Val Leu Ala Thr Met Phe Ile Val Asn His
        355                 360                 365

Ile Ile Glu Gly Val Ser Tyr Ala Ser Lys Asp Ala Val Lys Gly Val
    370                 375                 380
```

```
Met Ala Pro Pro Arg Thr Val His Gly Val Thr Pro Met Gln Val Thr
385                 390                 395                 400

Gln Lys Ala Leu Ser Ala Ala Glu Ser Thr Lys Ser Asp Ala Asp Lys
                405                 410                 415

Thr Thr Met Ile Pro Leu Asn Asp Trp Ala Ala Val Gln Cys Gln Thr
            420                 425                 430

Ser Val Asn Trp Ala Val Gly Ser Trp Phe Trp Asn His Phe Ser Gly
        435                 440                 445

Gly Leu Asn His Gln Ile Glu His His Cys Phe Pro Gln Asn Pro His
    450                 455                 460

Thr Val Asn Val Tyr Ile Ser Gly Ile Val Lys Glu Thr Cys Glu Glu
465                 470                 475                 480

Tyr Gly Val Pro Tyr Gln Ala Glu Ile Ser Leu Phe Ser Ala Tyr Phe
                485                 490                 495

Lys Met Leu Ser His Leu Arg Thr Leu Gly Asn Glu Asp Leu Thr Ala
            500                 505                 510

Trp Ser Thr
        515
```

<210> 14
<211> 509
<212> PRT
<213> Schizochytrium aggregatum

<400> 14

```
Met Thr Val Gly Gly Asp Glu Val Tyr Ser Met Ala Gln Val Arg Asp
1               5                   10                  15

His Asn Thr Pro Asp Asp Ala Trp Cys Ala Ile His Gly Glu Val Tyr
            20                  25                  30

Glu Leu Thr Lys Phe Ala Arg Thr His Pro Gly Gly Asp Ile Ile Leu
        35                  40                  45

Leu Ala Ala Gly Lys Glu Ala Thr Ile Leu Phe Glu Thr Tyr His Val
    50                  55                  60
```

```
Arg Pro Ile Ser Asp Ala Val Leu Arg Lys Tyr Arg Ile Gly Lys Leu
65                  70                  75                  80

Ala Ala Ala Gly Lys Asp Glu Pro Ala Asn Asp Ser Thr Tyr Tyr Ser
                85                  90                  95

Trp Asp Ser Asp Phe Tyr Lys Val Leu Arg Gln Arg Val Val Ala Arg
            100                 105                 110

Leu Glu Glu Arg Lys Ile Ala Arg Arg Gly Gly Pro Glu Ile Trp Ile
        115                 120                 125

Lys Ala Ala Ile Leu Val Ser Gly Phe Trp Ser Met Leu Tyr Leu Met
    130                 135                 140

Cys Thr Leu Asp Pro Asn Arg Gly Ala Ile Leu Ala Ala Ile Ala Leu
145                 150                 155                 160

Gly Ile Val Ala Ala Phe Val Gly Thr Cys Ile Gln His Asp Gly Asn
                165                 170                 175

His Gly Ala Phe Ala Phe Ser Pro Phe Met Asn Lys Leu Ser Gly Trp
            180                 185                 190

Thr Leu Asp Met Ile Gly Ala Ser Ala Met Thr Trp Glu Met Gln His
        195                 200                 205

Val Leu Gly His His Pro Tyr Thr Asn Leu Ile Glu Met Glu Asn Gly
    210                 215                 220

Thr Gln Lys Val Thr His Ala Asp Val Asp Pro Lys Lys Ala Asp Gln
225                 230                 235                 240

Glu Ser Asp Pro Asp Val Phe Ser Thr Tyr Pro Met Leu Arg Leu His
                245                 250                 255

Pro Trp His Arg Lys Arg Phe Tyr His Arg Phe Gln His Leu Tyr Ala
            260                 265                 270

Pro Leu Leu Phe Gly Phe Met Thr Ile Asn Lys Val Ile Thr Gln Asp
        275                 280                 285

Val Gly Val Val Leu Ser Lys Arg Leu Phe Gln Ile Asp Ala Asn Cys
    290                 295                 300

Arg Tyr Ala Ser Lys Ser Tyr Val Ala Arg Phe Trp Ile Met Lys Leu
```

```
305                 310                 315                 320

Leu Thr Val Leu Tyr Met Val Ala Leu Pro Val Tyr Thr Gln Gly Leu
                325                 330                 335

Val Asp Gly Leu Lys Leu Phe Phe Ile Ala His Phe Ser Cys Gly Glu
            340                 345                 350

Leu Leu Ala Thr Met Phe Ile Val Asn His Ile Ile Glu Gly Val Ser
        355                 360                 365

Tyr Ala Ser Lys Asp Ser Val Lys Gly Thr Met Ala Pro Pro Arg Thr
    370                 375                 380

Val His Gly Val Thr Pro Met His Asp Thr Arg Asp Ala Leu Gly Lys
385                 390                 395                 400

Glu Lys Ala Ala Thr Lys His Val Pro Leu Asn Asp Trp Ala Ala Val
                405                 410                 415

Gln Cys Gln Thr Ser Val Asn Trp Ser Ile Gly Ser Trp Phe Trp Asn
            420                 425                 430

His Phe Ser Gly Gly Leu Asn His Gln Ile Glu His His Leu Phe Pro
        435                 440                 445

Gly Leu Thr His Thr Thr Tyr Val Tyr Ile Gln Asp Val Val Gln Ala
    450                 455                 460

Thr Cys Ala Glu Tyr Gly Val Pro Tyr Gln Ser Glu Gln Ser Leu Phe
465                 470                 475                 480

Ser Ala Tyr Phe Lys Met Leu Ser His Leu Arg Ala Leu Gly Asn Glu
                485                 490                 495

Pro Met Pro Ser Trp Glu Lys Asp His Pro Lys Ser Lys
            500                 505
```

<210> 15
<211> 550
<212> PRT
<213> Thalassiosira pseudonana

<220>
<221> MISC_FEATURE
<222> (1)..(550)
<223> NCBI Accession No. AAX14506 (GI 60173017), locus AAX14506, CDS AY817156

<400> 15

```
Met Gly Asn Gly Asn Leu Pro Ala Ser Thr Ala Gln Leu Lys Ser Thr
1               5                   10                  15

Ser Lys Pro Gln Gln Gln His Glu His Arg Thr Ile Ser Lys Ser Glu
            20                  25                  30

Leu Ala Gln His Asn Thr Pro Lys Ser Ala Trp Cys Ala Val His Ser
        35                  40                  45

Thr Pro Ala Thr Asp Pro Ser His Ser Asn Asn Lys Gln His Ala His
    50                  55                  60

Leu Val Leu Asp Ile Thr Asp Phe Ala Ser Arg His Pro Gly Gly Asp
65                  70                  75                  80

Leu Ile Leu Leu Ala Ser Gly Lys Asp Ala Ser Val Leu Phe Glu Thr
                85                  90                  95

Tyr His Pro Arg Gly Val Pro Thr Ser Leu Ile Gln Lys Leu Gln Ile
            100                 105                 110

Gly Val Met Glu Glu Glu Ala Phe Arg Asp Ser Phe Tyr Ser Trp Thr
        115                 120                 125

Asp Ser Asp Phe Tyr Thr Val Leu Lys Arg Arg Val Val Glu Arg Leu
    130                 135                 140

Glu Glu Arg Gly Leu Asp Arg Arg Gly Ser Lys Glu Ile Trp Ile Lys
145                 150                 155                 160

Ala Leu Phe Leu Leu Val Gly Phe Trp Tyr Cys Leu Tyr Lys Met Tyr
                165                 170                 175

Thr Thr Ser Asp Ile Asp Gln Tyr Gly Ile Ala Ile Ala Tyr Ser Ile
            180                 185                 190

Gly Met Gly Thr Phe Ala Ala Phe Ile Gly Thr Cys Ile Gln His Asp
        195                 200                 205

Gly Asn His Gly Ala Phe Ala Gln Asn Lys Leu Leu Asn Lys Leu Ala
    210                 215                 220

Gly Trp Thr Leu Asp Met Ile Gly Ala Ser Ala Phe Thr Trp Glu Leu
```

```
225                 230                 235                 240

Gln His Met Leu Gly His His Pro Tyr Thr Asn Val Leu Asp Gly Val
                245                 250                 255

Glu Glu Glu Arg Lys Glu Arg Gly Glu Asp Val Ala Leu Glu Glu Lys
            260                 265                 270

Asp Gln Glu Ser Asp Pro Asp Val Phe Ser Ser Phe Pro Leu Met Arg
        275                 280                 285

Met His Pro His His Thr Thr Ser Trp Tyr His Lys Tyr Gln His Leu
    290                 295                 300

Tyr Ala Pro Pro Leu Phe Ala Leu Met Thr Leu Ala Lys Val Phe Gln
305                 310                 315                 320

Gln Asp Phe Glu Val Ala Thr Ser Gly Arg Leu Tyr His Ile Asp Ala
                325                 330                 335

Asn Val Arg Tyr Gly Ser Val Trp Asn Val Met Arg Phe Trp Ala Met
            340                 345                 350

Lys Val Ile Thr Met Gly Tyr Met Met Gly Leu Pro Ile Tyr Phe His
        355                 360                 365

Gly Val Leu Arg Gly Val Gly Leu Phe Val Ile Gly His Leu Ala Cys
    370                 375                 380

Gly Glu Leu Leu Ala Thr Met Phe Ile Val Asn His Val Ile Glu Gly
385                 390                 395                 400

Val Ser Tyr Gly Thr Lys Asp Leu Val Gly Gly Ala Ser His Gly Asp
                405                 410                 415

Glu Lys Lys Ile Val Lys Pro Thr Thr Val Leu Gly Asp Thr Pro Met
            420                 425                 430

Glu Lys Thr Arg Glu Glu Ala Leu Lys Ser Asn Ser Asn Asn Asn Lys
        435                 440                 445

Lys Lys Gly Glu Lys Asn Ser Val Pro Ser Val Pro Phe Asn Asp Trp
    450                 455                 460

Ala Ala Val Gln Cys Gln Thr Ser Val Asn Trp Ser Pro Gly Ser Trp
465                 470                 475                 480
```

```
Phe Trp Asn His Phe Ser Gly Gly Leu Ser His Gln Ile Glu His His
                485                 490                 495

Leu Phe Pro Ser Ile Cys His Thr Asn Tyr Cys His Ile Gln Asp Val
            500                 505                 510

Val Glu Ser Thr Cys Ala Glu Tyr Gly Val Pro Tyr Gln Ser Glu Ser
        515                 520                 525

Asn Leu Phe Val Ala Tyr Gly Lys Met Ile Ser His Leu Lys Phe Leu
    530                 535                 540

Gly Lys Ala Lys Cys Glu
545                 550
```

<210> 16
<211> 433
<212> PRT
<213> Isochrysis galbana

<220>
<221> MISC_FEATURE
<222> (1)..(433)
<223> NCBI Accession No. AAV33631 (GI 54307110), locus AAV33631, CDS AY630574

<400> 16

```
Met Cys Asn Ala Ala Gln Val Glu Thr Gln Ala Leu Arg Ala Lys Glu
1               5                   10                  15

Ala Ala Lys Pro Thr Trp Thr Lys Ile His Gly Arg Thr Val Asp Val
            20                  25                  30

Glu Thr Phe Arg His Pro Gly Gly Asn Ile Leu Asp Leu Phe Leu Gly
        35                  40                  45

Met Asp Ala Thr Thr Ala Phe Glu Thr Phe His Gly His His Lys Gly
    50                  55                  60

Ala Trp Lys Met Leu Lys Thr Leu Pro Glu Lys Glu Val Ala Ala Ala
65                  70                  75                  80

Asp Ile Pro Ala Gln Lys Glu Glu His Val Ala Glu Met Thr Arg Leu
                85                  90                  95

Met Ala Ser Trp Arg Glu Arg Gly Leu Phe Lys Pro Arg Pro Val Ala
```

100 105 110

Ser Ser Ile Tyr Gly Leu Cys Val Ile Phe Ala Ile Ala Ala Ser Val
115 120 125

Ala Cys Ala Pro Tyr Ala Pro Val Leu Ala Gly Ile Ala Val Gly Thr
130 135 140

Cys Trp Ala Gln Cys Gly Phe Leu Gln His Met Gly Gly His Arg Glu
145 150 155 160

Trp Gly Arg Thr Trp Ser Phe Ala Phe Gln His Leu Phe Glu Gly Leu
165 170 175

Leu Lys Gly Gly Ser Ala Ser Trp Trp Arg Asn Arg His Asn Lys His
180 185 190

His Ala Lys Thr Asn Val Leu Gly Glu Asp Gly Asp Leu Arg Thr Thr
195 200 205

Pro Phe Phe Ala Trp Asp Pro Thr Leu Ala Lys Lys Val Pro Asp Trp
210 215 220

Ser Leu Arg Thr Gln Ala Phe Thr Phe Leu Pro Ala Leu Gly Ala Tyr
225 230 235 240

Val Phe Val Phe Ala Phe Thr Val Arg Lys Tyr Ser Val Val Lys Arg
245 250 255

Leu Trp His Glu Val Ala Leu Met Val Ala His Tyr Ala Leu Phe Ser
260 265 270

Trp Ala Leu Ser Ala Ala Gly Ala Ser Leu Ser Ser Gly Leu Thr Phe
275 280 285

Tyr Cys Thr Gly Tyr Ala Trp Gln Gly Ile Tyr Leu Gly Phe Phe Phe
290 295 300

Gly Leu Ser His Phe Ala Val Glu Arg Val Pro Ser Thr Ala Thr Trp
305 310 315 320

Leu Glu Ser Thr Met Met Gly Thr Val Asp Trp Gly Gly Ser Ser Ala
325 330 335

Phe Cys Gly Tyr Leu Ser Gly Phe Leu Asn Ile Gln Ile Glu His His
340 345 350

```
Met Ala Pro Gln Met Pro Met Glu Asn Leu Arg Gln Ile Arg Ala Asp
        355                 360                 365

Cys Lys Ala Ala Ala His Lys Phe Gly Leu Pro Tyr Arg Glu Leu Thr
    370                 375                 380

Phe Val Ala Ala Thr Lys Leu Met Met Ser Gly Leu Tyr Arg Thr Gly
385                 390                 395                 400

Lys Asp Glu Leu Lys Leu Arg Ala Asp Arg Arg Lys Phe Thr Arg Ala
                405                 410                 415

Gln Ala Tyr Met Gly Ala Ala Ser Ala Leu Val Asp Thr Leu Lys Ala
            420                 425                 430

Asp
```

<210> 17
<211> 2523
<212> DNA
<213> Euglena anabaena

<400> 17

```
atggccgaag gcaagagcga tgggcctgtg gtgacccttc aaagcatgtg gaaaccgctt      60
gctttgatgg cagtagatgt cggcatattg gtcaatgtcc gccgcaaggc tttcactgag     120
tttgatgggc acagcaacgt ttttgcagat ccagtttaca ttccatttgt gatgaatctc     180
ttctacttga ccatgatctt tgctgggtgc cgttggatga agactcgcga gccctttgag     240
atcaagtcat atatgtttgc atacaatgca tatcagacaa tgatgaactt cctcattgtc     300
gtcgggttca tgtatgaggt gcacagcaca gggatgcgat attgggggtc caggatcgac     360
acctccacca agggcttggg cctcggtttc ctgatctatg cccactacca caacaaatat     420
gtggagtatg tcgacacgtt gttcatgatc ctgcgcaaga aaaacaacca gatctcgttc     480
ctccacgtct accaccattc gcttttgact tgggcctggt gggccgtggt ctactgggcc     540
ccgggaggag atgcctggtt cggagcatgc tacaattcct tcatccacgt gctgatgtac     600
tcctactacc tgtttgcaac gtttggcatc aggtgcccct ggaagaagat gctgacccag     660
ttgcagatgg tgcaattctg cttctgcttt gcccacgcga tgtatgttgg atggctgggc     720
catgaagtct acccgcgctg gttgacggcg ctgcaggcat ttgtgatgct aaacatgctg     780
gtgctgttcg gcaacttcta catgaagtcg tactccaagg ccagcaagct ggagccggcc     840
tcccccgtgt cccccgcctc cctcgcccag aaaccgttcg agaacgccaa ggtgaagcct     900
```

```
gggggccccg gcaagccaag cgagattgcg tcgctgccac cgccaattcg accagtcggg     960
aacccacctg cagcctacta cgatgccctg gcgacctcgg gcaccgggca ggaccgcaag    1020
ttcaccatgc gggaggtggc ccgccatatt gtgccgaccg acgggtggtt ggcatgccat    1080
gacggtgtct acgacatcac cgagttcata gggaaacatc ctggcggcga tgttatttct    1140
ctcggattgg gcagggactc cacaatcctg gttgagtcgt accaccctgc cggaaggcca    1200
gacaaggtca tggaaaagta ccgcatcggg acgctccagg accaccgcac gttctacgac    1260
tggcaggcct ccgcgttcta cgccgagctg aagcagcggg tggtgcagac gctaaaggag    1320
gccggccaac cgcggcgtgg gggcctgtcg gtcaaagcgg cgctggtcat ggcagcgttc    1380
gcggcgtcgt tctacctcat ggtgacccag ggatccttct tctgggccgc cgtctggggc    1440
ctcgccggct cccacattgg cctcagcatc cagcacgacg ggaaccacgg ggctttcagt    1500
aagagtggtc ggctgaaccg cctcgcaggc tggggcatgg acgttatcgg ggcctcctcg    1560
acggcctggg agtaccagca cgtcatcggg caccaccagt acaccaacct ggtatcggat    1620
cccgagttcg cgctgcctga gaacgacccg gacgtcttcg gcacctatcc gctgatgcgg    1680
atgcatccgg acaccccttg gaagccgcac caccagctgc agcatatgta cgcgttcccg    1740
ctgttcgccc tgatgaccat cagcaaggtc atcatcagtg acttcacgtt ctgcctcgcc    1800
aagcggcgcg ggccgatcga cttctccgcc aggctcgtgc cacttgaggg gcagatgctc    1860
ttctggggcg cgaagatcat ggggttcctg atgcagatag tcctgccgtg ctatctgcat    1920
ggcatcgccc atgggctggc gctgttcatc acagcccacc tggtgtcggg agagtacctc    1980
gcggtctgct tcatcatcaa ccacatctct gagtcatgcg actatttgaa tccaagttcc    2040
gtcatcgctg cgcggcggac ggaaatgctg aagcaggcgg agcaggaggc caaggcaaag    2100
cagaagcacc ccaccccacc gcccaacgac tgggccgcgt ctcaggtact gtgctgcgta    2160
aactggcgct ctggtggata tttctcaaac cacctctcag gcgggctgaa ccaccagatc    2220
gagcaccacc tcttccccag catctcacat gcgaactatc cgaccattgc cccagttgtg    2280
aaaggggtgt gcgaggagta cggcctcccc tacaagaact actcccagtt ctctgacgct    2340
atctatggaa tggtggagca cctcagggcg atgggcacga agcccgaaga caacggcaag    2400
ctggcgccac tgccgggctc cctggaggac gtgtgcccgg tcctgagtgc cgccgttgct    2460
gcccaacctg acggaagcac cgacggcagc gctgcgggtt gtccagcagt agccacactg    2520
gca                                                                  2523
```

<210> 18
<211> 841
<212> PRT

<213> Euglena anabaena

<400> 18

```
Met Ala Glu Gly Lys Ser Asp Gly Pro Val Val Thr Leu Gln Ser Met
1               5                   10                  15

Trp Lys Pro Leu Ala Leu Met Ala Val Asp Val Gly Ile Leu Val Asn
            20                  25                  30

Val Arg Arg Lys Ala Phe Thr Glu Phe Asp Gly His Ser Asn Val Phe
        35                  40                  45

Ala Asp Pro Val Tyr Ile Pro Phe Val Met Asn Leu Phe Tyr Leu Thr
    50                  55                  60

Met Ile Phe Ala Gly Cys Arg Trp Met Lys Thr Arg Glu Pro Phe Glu
65                  70                  75                  80

Ile Lys Ser Tyr Met Phe Ala Tyr Asn Ala Tyr Gln Thr Met Met Asn
                85                  90                  95

Phe Leu Ile Val Val Gly Phe Met Tyr Glu Val His Ser Thr Gly Met
            100                 105                 110

Arg Tyr Trp Gly Ser Arg Ile Asp Thr Ser Thr Lys Gly Leu Gly Leu
        115                 120                 125

Gly Phe Leu Ile Tyr Ala His Tyr His Asn Lys Tyr Val Glu Tyr Val
    130                 135                 140

Asp Thr Leu Phe Met Ile Leu Arg Lys Lys Asn Asn Gln Ile Ser Phe
145                 150                 155                 160

Leu His Val Tyr His His Ser Leu Leu Thr Trp Ala Trp Trp Ala Val
                165                 170                 175

Val Tyr Trp Ala Pro Gly Gly Asp Ala Trp Phe Gly Ala Cys Tyr Asn
            180                 185                 190

Ser Phe Ile His Val Leu Met Tyr Ser Tyr Tyr Leu Phe Ala Thr Phe
        195                 200                 205

Gly Ile Arg Cys Pro Trp Lys Lys Met Leu Thr Gln Leu Gln Met Val
    210                 215                 220

Gln Phe Cys Phe Cys Phe Ala His Ala Met Tyr Val Gly Trp Leu Gly
```

```
225                 230                 235                 240

His Glu Val Tyr Pro Arg Trp Leu Thr Ala Leu Gln Ala Phe Val Met
                245                 250                 255

Leu Asn Met Leu Val Leu Phe Gly Asn Phe Tyr Met Lys Ser Tyr Ser
            260                 265                 270

Lys Ala Ser Lys Leu Glu Pro Ala Ser Pro Val Ser Pro Ala Ser Leu
        275                 280                 285

Ala Gln Lys Pro Phe Glu Asn Ala Lys Val Lys Pro Gly Gly Pro Gly
    290                 295                 300

Lys Pro Ser Glu Ile Ala Ser Leu Pro Pro Pro Ile Arg Pro Val Gly
305                 310                 315                 320

Asn Pro Pro Ala Ala Tyr Tyr Asp Ala Leu Ala Thr Ser Gly Thr Gly
                325                 330                 335

Gln Asp Arg Lys Phe Thr Met Arg Glu Val Ala Arg His Ile Val Pro
            340                 345                 350

Thr Asp Gly Trp Leu Ala Cys His Asp Gly Val Tyr Asp Ile Thr Glu
        355                 360                 365

Phe Ile Gly Lys His Pro Gly Gly Asp Val Ile Ser Leu Gly Leu Gly
    370                 375                 380

Arg Asp Ser Thr Ile Leu Val Glu Ser Tyr His Pro Ala Gly Arg Pro
385                 390                 395                 400

Asp Lys Val Met Glu Lys Tyr Arg Ile Gly Thr Leu Gln Asp His Arg
                405                 410                 415

Thr Phe Tyr Asp Trp Gln Ala Ser Ala Phe Tyr Ala Glu Leu Lys Gln
            420                 425                 430

Arg Val Val Gln Thr Leu Lys Glu Ala Gly Gln Pro Arg Arg Gly Gly
        435                 440                 445

Leu Ser Val Lys Ala Ala Leu Val Met Ala Ala Phe Ala Ala Ser Phe
    450                 455                 460

Tyr Leu Met Val Thr Gln Gly Ser Phe Phe Trp Ala Ala Val Trp Gly
465                 470                 475                 480
```

```
Leu Ala Gly Ser His Ile Gly Leu Ser Ile Gln His Asp Gly Asn His
                485                 490                 495

Gly Ala Phe Ser Lys Ser Gly Arg Leu Asn Arg Leu Ala Gly Trp Gly
            500                 505                 510

Met Asp Val Ile Gly Ala Ser Ser Thr Ala Trp Glu Tyr Gln His Val
        515                 520                 525

Ile Gly His His Gln Tyr Thr Asn Leu Val Ser Asp Pro Glu Phe Ala
    530                 535                 540

Leu Pro Glu Asn Asp Pro Asp Val Phe Gly Thr Tyr Pro Leu Met Arg
545                 550                 555                 560

Met His Pro Asp Thr Pro Trp Lys Pro His His Gln Leu Gln His Met
                565                 570                 575

Tyr Ala Phe Pro Leu Phe Ala Leu Met Thr Ile Ser Lys Val Ile Ile
            580                 585                 590

Ser Asp Phe Thr Phe Cys Leu Ala Lys Arg Arg Gly Pro Ile Asp Phe
        595                 600                 605

Ser Ala Arg Leu Val Pro Leu Glu Gly Gln Met Leu Phe Trp Gly Ala
    610                 615                 620

Lys Ile Met Gly Phe Leu Met Gln Ile Val Leu Pro Cys Tyr Leu His
625                 630                 635                 640

Gly Ile Ala His Gly Leu Ala Leu Phe Ile Thr Ala His Leu Val Ser
                645                 650                 655

Gly Glu Tyr Leu Ala Val Cys Phe Ile Ile Asn His Ile Ser Glu Ser
            660                 665                 670

Cys Asp Tyr Leu Asn Pro Ser Ser Val Ile Ala Ala Arg Arg Thr Glu
        675                 680                 685

Met Leu Lys Gln Ala Glu Gln Glu Ala Lys Ala Lys Gln Lys His Pro
    690                 695                 700

Thr Pro Pro Pro Asn Asp Trp Ala Ala Ser Gln Val Leu Cys Cys Val
705                 710                 715                 720
```

```
Asn Trp Arg Ser Gly Gly Tyr Phe Ser Asn His Leu Ser Gly Gly Leu
                725                 730                 735

Asn His Gln Ile Glu His His Leu Phe Pro Ser Ile Ser His Ala Asn
            740                 745                 750

Tyr Pro Thr Ile Ala Pro Val Val Lys Gly Val Cys Glu Glu Tyr Gly
        755                 760                 765

Leu Pro Tyr Lys Asn Tyr Ser Gln Phe Ser Asp Ala Ile Tyr Gly Met
    770                 775                 780

Val Glu His Leu Arg Ala Met Gly Thr Lys Pro Glu Asp Asn Gly Lys
785                 790                 795                 800

Leu Ala Pro Leu Pro Gly Ser Leu Glu Asp Val Cys Pro Val Leu Ser
                805                 810                 815

Ala Ala Val Ala Ala Gln Pro Asp Gly Ser Thr Asp Gly Ser Ala Ala
            820                 825                 830

Gly Cys Pro Ala Val Ala Thr Leu Ala
        835                 840
```

<210> 19
<211> 2523
<212> DNA
<213> Euglena anabaena

<400> 19

```
atggccgaag gcaagagcga tgggcctgtg gtgacccttc aaagcatgtg gaaaccgctt     60
gctctgatgg caatagatgt cggcatattg gtcaatgtcc gccgcaaggc tttcactgag    120
tttgatgggc acagcaacgt tttcgcagat ccagtttaca ttccatttgt gatgaatctc    180
ttctacttga ccatgatctt tgctgggtgc cgttggatga agactcgcga accctttgag    240
atcaagtcat atatgtttgc atacaatgca tatcagacaa tgatgaactt cctcattgtc    300
gtcgggttca tgtatgaggt gcacagcaca gggatgcgat attgggggtc caggatcgac    360
acctccacca agggcttggg cctcggtttc ctgatctatg cccactacca caacaaatac    420
gtggagtatg tcgacacgtt gttcatgatc ctgcgcaaga aaaacaacca gatctcgttc    480
ctccacgtct accaccattc gcttttgact tgggcctggt gggccgtggt ctactgggcc    540
ccaggaggag atgcctggtt cggagcatgc tacaattcct tcatccacgt gctgatgtac    600
tcctactacc tgtttgcaac gtttggcatc aggtgcccct ggaagaagat gctgacccag    660
```

ttgcagatgg tgcaattctg cttctgcttt gcccacgcga tgtatgttgg atggctgggc 720
catgaagtct acccgcgctg gttgacggcg ctacaggcat ttgtgatgct aaacatgctg 780
gtgctgttcg gcaacttcta catgaagtcg tactccaagg ccagcaagct ggagccggcc 840
tcccccgtgt cccccgcctc cctcgcccag aagccgttcg agaacgccaa ggtgaagcct 900
gggggccccg gcaagccaag cgagattgcg tcgctgccac cgccaattcg accagtcggg 960
aacccacctg cagcctacta cgatgccctg gcgacctcgg gcaccgggca ggaccgcaag 1020
ttcaccatgc gggaggtggc ccgccatatt gtgccgaccg atgggtggtt ggcgtgccat 1080
gacggtgtct acgacatcac cgagttcata gggaaacatc ctggcggcga tgttatttct 1140
ctcggattgg gcagggactc cacaatcctg gttgagtcgt accaccctgc cggaaggcca 1200
gacaaggtca tggaaaagta ccgcatcggg acgctccagg accaccgcac gttctacgac 1260
tggcaggcct ccgcgttcta cgccgagctg aagcagcggg tggtgcagac gctaaaggag 1320
gccggccaac cgcggcgtgg gggcctgtcg gtcaaagcgg cgctggtcat ggcggcgttc 1380
gcagcgtcgt tctacctcat ggtgacccag ggatccttct tctgggccgc cgtctggggc 1440
ctcgccggct cccacattgg cctcagcatc cagcacgacg ggaaccacgg ggctttcagt 1500
aagagtggtc ggctgaaccg cctcgcgggc tggggcatgg acgtcatcgg ggcctcctcg 1560
acggcctggg agtaccagca cgtcatcggg caccaccagt acaccaacct ggtatcggat 1620
cccgagttcg cgctgcctga gaacgacccg gacgtcttcg gcacctatcc gctgatgcgg 1680
atgcatccgg acaccccttg gaagccgcac caccagctgc agcatgtgta cgcgttcccg 1740
ctgttcgccc tgatgaccat cagcaaggtc atcatcagcg acttcacgtt ctgcctcgcc 1800
aagcggcgcg ggccgatcga cttctccgcc aggctcgtgc cacttgaggg gcagatgctc 1860
ttctgggggg cgaagatcat ggggttcctg atgcagatag tcctgccgtg ctatctgcat 1920
ggcatcgccc atgggctggc gctgttcatc acagcccacc tggtgtcggg agagtacctc 1980
gcggtctgct tcatcatcaa ccacatctct gagtcatgcg actatttgaa tccaagttcc 2040
gtcatcgctg cgcggaggac ggaaatgctg aagcaggcgg agcaggaggc caaggcaaag 2100
cagaagcacc ccaccccacc gcccaacgac tgggccgcgt ctcaggtact gtgctgcgta 2160
aactggcgct ctggtggcta tttctcaaac cacctctcag gcgggctgaa ccaccagatc 2220
gagcaccacc tcttccccag catctcacat gcgaactatc cgaccattgc cccagttgtg 2280
aaaggggtgt gcgaggagta cggcctcccc tacaagaact actcccagtt ctccgacgct 2340
ctctatggaa tggtggagca cctcagggcg atgggcacga agccggcaga caacgacaag 2400
ctggcgccca ccgcgggctc cctggaggac gtgtgcccgg tcttgagcgc cgccgttgct 2460
gcccaacctg acggaagcac cgacggcagc gctgcgggtt gtccagcagt agccacactg 2520

gca 2523

<210> 20
<211> 841
<212> PRT
<213> Euglena anabaena

<400> 20

```
Met Ala Glu Gly Lys Ser Asp Gly Pro Val Val Thr Leu Gln Ser Met
1               5                   10                  15

Trp Lys Pro Leu Ala Leu Met Ala Ile Asp Val Gly Ile Leu Val Asn
            20                  25                  30

Val Arg Arg Lys Ala Phe Thr Glu Phe Asp Gly His Ser Asn Val Phe
        35                  40                  45

Ala Asp Pro Val Tyr Ile Pro Phe Val Met Asn Leu Phe Tyr Leu Thr
    50                  55                  60

Met Ile Phe Ala Gly Cys Arg Trp Met Lys Thr Arg Glu Pro Phe Glu
65                  70                  75                  80

Ile Lys Ser Tyr Met Phe Ala Tyr Asn Ala Tyr Gln Thr Met Met Asn
                85                  90                  95

Phe Leu Ile Val Val Gly Phe Met Tyr Glu Val His Ser Thr Gly Met
            100                 105                 110

Arg Tyr Trp Gly Ser Arg Ile Asp Thr Ser Thr Lys Gly Leu Gly Leu
        115                 120                 125

Gly Phe Leu Ile Tyr Ala His Tyr His Asn Lys Tyr Val Glu Tyr Val
    130                 135                 140

Asp Thr Leu Phe Met Ile Leu Arg Lys Lys Asn Asn Gln Ile Ser Phe
145                 150                 155                 160

Leu His Val Tyr His His Ser Leu Leu Thr Trp Ala Trp Trp Ala Val
                165                 170                 175

Val Tyr Trp Ala Pro Gly Gly Asp Ala Trp Phe Gly Ala Cys Tyr Asn
            180                 185                 190

Ser Phe Ile His Val Leu Met Tyr Ser Tyr Tyr Leu Phe Ala Thr Phe
        195                 200                 205
```

```
Gly Ile Arg Cys Pro Trp Lys Lys Met Leu Thr Gln Leu Gln Met Val
    210                 215                 220

Gln Phe Cys Phe Cys Phe Ala His Ala Met Tyr Val Gly Trp Leu Gly
225                 230                 235                 240

His Glu Val Tyr Pro Arg Trp Leu Thr Ala Leu Gln Ala Phe Val Met
                245                 250                 255

Leu Asn Met Leu Val Leu Phe Gly Asn Phe Tyr Met Lys Ser Tyr Ser
            260                 265                 270

Lys Ala Ser Lys Leu Glu Pro Ala Ser Pro Val Ser Pro Ala Ser Leu
        275                 280                 285

Ala Gln Lys Pro Phe Glu Asn Ala Lys Val Lys Pro Gly Gly Pro Gly
    290                 295                 300

Lys Pro Ser Glu Ile Ala Ser Leu Pro Pro Pro Ile Arg Pro Val Gly
305                 310                 315                 320

Asn Pro Pro Ala Ala Tyr Tyr Asp Ala Leu Ala Thr Ser Gly Thr Gly
                325                 330                 335

Gln Asp Arg Lys Phe Thr Met Arg Glu Val Ala Arg His Ile Val Pro
            340                 345                 350

Thr Asp Gly Trp Leu Ala Cys His Asp Gly Val Tyr Asp Ile Thr Glu
        355                 360                 365

Phe Ile Gly Lys His Pro Gly Gly Asp Val Ile Ser Leu Gly Leu Gly
    370                 375                 380

Arg Asp Ser Thr Ile Leu Val Glu Ser Tyr His Pro Ala Gly Arg Pro
385                 390                 395                 400

Asp Lys Val Met Glu Lys Tyr Arg Ile Gly Thr Leu Gln Asp His Arg
                405                 410                 415

Thr Phe Tyr Asp Trp Gln Ala Ser Ala Phe Tyr Ala Glu Leu Lys Gln
            420                 425                 430

Arg Val Val Gln Thr Leu Lys Glu Ala Gly Gln Pro Arg Arg Gly Gly
        435                 440                 445
```

```
Leu Ser Val Lys Ala Ala Leu Val Met Ala Ala Phe Ala Ala Ser Phe
    450                 455                 460

Tyr Leu Met Val Thr Gln Gly Ser Phe Phe Trp Ala Ala Val Trp Gly
465                 470                 475                 480

Leu Ala Gly Ser His Ile Gly Leu Ser Ile Gln His Asp Gly Asn His
                485                 490                 495

Gly Ala Phe Ser Lys Ser Gly Arg Leu Asn Arg Leu Ala Gly Trp Gly
            500                 505                 510

Met Asp Val Ile Gly Ala Ser Ser Thr Ala Trp Glu Tyr Gln His Val
        515                 520                 525

Ile Gly His His Gln Tyr Thr Asn Leu Val Ser Asp Pro Glu Phe Ala
    530                 535                 540

Leu Pro Glu Asn Asp Pro Asp Val Phe Gly Thr Tyr Pro Leu Met Arg
545                 550                 555                 560

Met His Pro Asp Thr Pro Trp Lys Pro His His Gln Leu Gln His Val
                565                 570                 575

Tyr Ala Phe Pro Leu Phe Ala Leu Met Thr Ile Ser Lys Val Ile Ile
            580                 585                 590

Ser Asp Phe Thr Phe Cys Leu Ala Lys Arg Arg Gly Pro Ile Asp Phe
        595                 600                 605

Ser Ala Arg Leu Val Pro Leu Glu Gly Gln Met Leu Phe Trp Gly Ala
    610                 615                 620

Lys Ile Met Gly Phe Leu Met Gln Ile Val Leu Pro Cys Tyr Leu His
625                 630                 635                 640

Gly Ile Ala His Gly Leu Ala Leu Phe Ile Thr Ala His Leu Val Ser
                645                 650                 655

Gly Glu Tyr Leu Ala Val Cys Phe Ile Ile Asn His Ile Ser Glu Ser
            660                 665                 670

Cys Asp Tyr Leu Asn Pro Ser Ser Val Ile Ala Ala Arg Arg Thr Glu
        675                 680                 685
```

```
Met Leu Lys Gln Ala Glu Gln Glu Ala Lys Ala Lys Gln Lys His Pro
    690                 695                 700

Thr Pro Pro Pro Asn Asp Trp Ala Ala Ser Gln Val Leu Cys Cys Val
705                 710                 715                 720

Asn Trp Arg Ser Gly Gly Tyr Phe Ser Asn His Leu Ser Gly Gly Leu
                725                 730                 735

Asn His Gln Ile Glu His His Leu Phe Pro Ser Ile Ser His Ala Asn
            740                 745                 750

Tyr Pro Thr Ile Ala Pro Val Val Lys Gly Val Cys Glu Glu Tyr Gly
        755                 760                 765

Leu Pro Tyr Lys Asn Tyr Ser Gln Phe Ser Asp Ala Leu Tyr Gly Met
    770                 775                 780

Val Glu His Leu Arg Ala Met Gly Thr Lys Pro Ala Asp Asn Asp Lys
785                 790                 795                 800

Leu Ala Pro Thr Ala Gly Ser Leu Glu Asp Val Cys Pro Val Leu Ser
                805                 810                 815

Ala Ala Val Ala Ala Gln Pro Asp Gly Ser Thr Asp Gly Ser Ala Ala
            820                 825                 830

Gly Cys Pro Ala Val Ala Thr Leu Ala
        835                 840
```

<210> 21
<211> 2523
<212> DNA
<213> Euglena anabaena

<400> 21

```
atggccgaag gcaagagcga tgggcctgtg gtgacccttc aaagcatgtg gaaaccgctt      60
gctttgatgg cagtagatgt cggcatattg gtcaatgtcc gccgcaaggc tttcactgag     120
tttgatgggc acagcaacgt ttttgcagat ccagtttaca ttccatttgt gatgaatctc     180
ttctacttga ccatgatctt tgctgggtgc cgttggatga agactcgcga gccctttgag     240
atcaagtcat atatgtttgc atacaatgca tatcagacaa tgatgaactt cctcattgtc     300
gtcgggttca tgtatgaggt gcacagcaca gggatgcgat attgggggtc caggatcgac     360
acctccacca agggcttggg cctcggtttc ctgatctatg cccactacca caacaaatat     420
gtggagtatg tcgacacgtt gttcatgatc ctgcgcaaga aaaacaacca gatctcgttc     480
```

```
ctccacgtct accaccattc gcttttgact tgggcctggt gggccgtggt ctactgggcc    540
ccgggaggag atgcctggtt cggagcatgc tacaattcct tcatccacgt gctgatgtac    600
tcctactacc tgtttgcaac gtttggcatc aggtgcccct ggaagaagat gctgacccag    660
ttgcagatgg tgcaattctg cttctgcttt gcccacgcga tgtatgttgg atggctgggc    720
catgaagtct acccgcgctg gttgacggcg ctgcaggcat ttgtgatgct aaacatgctg    780
gtgctgttcg gcaacttcta catgaagtcg tactccaagg ccagcaagct ggagccggcc    840
tcccccgcgt cccccgcctc cctcgcccag aaaccgttcg agaacgccaa ggtgaagcct    900
gggggccccg gcaagccaag cgagattgcg tcgctgccac cgccaattcg accagtcggg    960
aacccacctg cagcctacta cgatgccctg gcgacctcgg gcaccgggca ggaccgcaag   1020
ttcaccatgc gggaggtggc ccgccatatt gtgccgaccg acgggtggtt ggcatgccat   1080
gacggtgtct acgacatcac cgagttcata gggaaacatc ctggcggcga tgttatttct   1140
ctcggattgg gcagggactc cacaatcctg gttgagtcgt accaccctgc cggaaggcca   1200
gacaaggtca tggaaaagta ccgcatcggg acgctccagg accaccgcac gttctacgac   1260
tggcaggcct ccgcgttcta cgccgagctg aagcagcggg tggtgcagac gctaaaggag   1320
gccggccaac cgcggcgtgg gggcctgtcg gtcaaagcgg cgctggtcat ggcagcgttc   1380
gcggcgtcgt tctacctcat ggtgacccag ggatccttct tctgggccgc cgtctggggc   1440
ctcgccggct cccacattgg cctcagcatc cagcacgacg ggaaccacgg ggctttcagt   1500
aagagtggtc ggctgaaccg cctcgcaggc tggggcatgg acgttatcgg ggcctcctcg   1560
acggcctggg agtaccagca cgtcatcggg caccaccagt acaccaacct ggtatcggat   1620
cccgagttcg cgctgcctga gaacgacccg gacgtcttcg gcacctatcc gctgatgcgg   1680
atgcatccgg acaccccttg gaagccgcac caccagctgc agcatatgta cgcgttcccg   1740
ctgttcgccc tgatgaccat cagcaaggtc atcatcagtg acttcacgtt ctgcctcgcc   1800
aagcggcgcg ggccgatcga cttctccgcc aggctcgtgc cacttgaggg gcagatgctc   1860
ttctggggcg cgaagatcat ggggttcctg atgcagatag tcctgccgtg ctatctgcat   1920
ggcatcgccc atgggctggc gctgttcatc acagcccacc tggtgtcggg agagtacctc   1980
gcggtctgct tcatcatcaa ccacatctct gagtcatgcg actatttgaa tccaagttcc   2040
gtcatcgctg cgcggcggac ggaaatgctg aagcaggcgg agcaggaggc caaggcaaag   2100
cagaagcacc ccaccccacc gcccaacgac tgggccgcgt ctcaggtact gtgctgcgta   2160
aactggcgct ctggtggata tttctcaaac cacctctcag gcgggctgaa ccaccagatc   2220
gagcaccacc tcttccccag catctcacat gcgaactatc cgaccattgc cccagttgtg   2280
```

```
aaaggggtgt gcgaggagta cggcctcccc tacaagaact actcccagtt ctctgacgct   2340

atctatggaa tggtggagca cctcagggcg atgggcacga agcccgaaga caacggcaag   2400

ctggcgccac tgccgggctc cctggaggac gtgtgcccgg tcctgagtgc cgccgttgct   2460

gcccaacctg acggaagcac cgacggcagc gctgcgggtt gtccagcagt agccacactg   2520

gca                                                                 2523
```

<210> 22
<211> 841
<212> PRT
<213> Euglena anabaena

<400> 22

```
Met Ala Glu Gly Lys Ser Asp Gly Pro Val Val Thr Leu Gln Ser Met
1               5                   10                  15

Trp Lys Pro Leu Ala Leu Met Ala Val Asp Val Gly Ile Leu Val Asn
            20                  25                  30

Val Arg Arg Lys Ala Phe Thr Glu Phe Asp Gly His Ser Asn Val Phe
        35                  40                  45

Ala Asp Pro Val Tyr Ile Pro Phe Val Met Asn Leu Phe Tyr Leu Thr
    50                  55                  60

Met Ile Phe Ala Gly Cys Arg Trp Met Lys Thr Arg Glu Pro Phe Glu
65                  70                  75                  80

Ile Lys Ser Tyr Met Phe Ala Tyr Asn Ala Tyr Gln Thr Met Met Asn
                85                  90                  95

Phe Leu Ile Val Val Gly Phe Met Tyr Glu Val His Ser Thr Gly Met
            100                 105                 110

Arg Tyr Trp Gly Ser Arg Ile Asp Thr Ser Thr Lys Gly Leu Gly Leu
        115                 120                 125

Gly Phe Leu Ile Tyr Ala His Tyr His Asn Lys Tyr Val Glu Tyr Val
    130                 135                 140

Asp Thr Leu Phe Met Ile Leu Arg Lys Lys Asn Asn Gln Ile Ser Phe
145                 150                 155                 160

Leu His Val Tyr His His Ser Leu Leu Thr Trp Ala Trp Trp Ala Val
                165                 170                 175
```

```
Val Tyr Trp Ala Pro Gly Gly Asp Ala Trp Phe Gly Ala Cys Tyr Asn
            180                 185                 190

Ser Phe Ile His Val Leu Met Tyr Ser Tyr Tyr Leu Phe Ala Thr Phe
        195                 200                 205

Gly Ile Arg Cys Pro Trp Lys Lys Met Leu Thr Gln Leu Gln Met Val
    210                 215                 220

Gln Phe Cys Phe Cys Phe Ala His Ala Met Tyr Val Gly Trp Leu Gly
225                 230                 235                 240

His Glu Val Tyr Pro Arg Trp Leu Thr Ala Leu Gln Ala Phe Val Met
                245                 250                 255

Leu Asn Met Leu Val Leu Phe Gly Asn Phe Tyr Met Lys Ser Tyr Ser
            260                 265                 270

Lys Ala Ser Lys Leu Glu Pro Ala Ser Pro Ala Ser Pro Ala Ser Leu
        275                 280                 285

Ala Gln Lys Pro Phe Glu Asn Ala Lys Val Lys Pro Gly Gly Pro Gly
    290                 295                 300

Lys Pro Ser Glu Ile Ala Ser Leu Pro Pro Pro Ile Arg Pro Val Gly
305                 310                 315                 320

Asn Pro Pro Ala Ala Tyr Tyr Asp Ala Leu Ala Thr Ser Gly Thr Gly
                325                 330                 335

Gln Asp Arg Lys Phe Thr Met Arg Glu Val Ala Arg His Ile Val Pro
            340                 345                 350

Thr Asp Gly Trp Leu Ala Cys His Asp Gly Val Tyr Asp Ile Thr Glu
        355                 360                 365

Phe Ile Gly Lys His Pro Gly Gly Asp Val Ile Ser Leu Gly Leu Gly
    370                 375                 380

Arg Asp Ser Thr Ile Leu Val Glu Ser Tyr His Pro Ala Gly Arg Pro
385                 390                 395                 400

Asp Lys Val Met Glu Lys Tyr Arg Ile Gly Thr Leu Gln Asp His Arg
                405                 410                 415
```

```
Thr Phe Tyr Asp Trp Gln Ala Ser Ala Phe Tyr Ala Glu Leu Lys Gln
            420                 425                 430

Arg Val Val Gln Thr Leu Lys Glu Ala Gly Gln Pro Arg Arg Gly Gly
        435                 440                 445

Leu Ser Val Lys Ala Ala Leu Val Met Ala Ala Phe Ala Ala Ser Phe
    450                 455                 460

Tyr Leu Met Val Thr Gln Gly Ser Phe Phe Trp Ala Ala Val Trp Gly
465                 470                 475                 480

Leu Ala Gly Ser His Ile Gly Leu Ser Ile Gln His Asp Gly Asn His
                485                 490                 495

Gly Ala Phe Ser Lys Ser Gly Arg Leu Asn Arg Leu Ala Gly Trp Gly
            500                 505                 510

Met Asp Val Ile Gly Ala Ser Ser Thr Ala Trp Glu Tyr Gln His Val
        515                 520                 525

Ile Gly His His Gln Tyr Thr Asn Leu Val Ser Asp Pro Glu Phe Ala
    530                 535                 540

Leu Pro Glu Asn Asp Pro Asp Val Phe Gly Thr Tyr Pro Leu Met Arg
545                 550                 555                 560

Met His Pro Asp Thr Pro Trp Lys Pro His His Gln Leu Gln His Met
                565                 570                 575

Tyr Ala Phe Pro Leu Phe Ala Leu Met Thr Ile Ser Lys Val Ile Ile
            580                 585                 590

Ser Asp Phe Thr Phe Cys Leu Ala Lys Arg Arg Gly Pro Ile Asp Phe
        595                 600                 605

Ser Ala Arg Leu Val Pro Leu Glu Gly Gln Met Leu Phe Trp Gly Ala
    610                 615                 620

Lys Ile Met Gly Phe Leu Met Gln Ile Val Leu Pro Cys Tyr Leu His
625                 630                 635                 640

Gly Ile Ala His Gly Leu Ala Leu Phe Ile Thr Ala His Leu Val Ser
                645                 650                 655

Gly Glu Tyr Leu Ala Val Cys Phe Ile Ile Asn His Ile Ser Glu Ser
```

```
                660                 665                 670

Cys Asp Tyr Leu Asn Pro Ser Ser Val Ile Ala Ala Arg Arg Thr Glu
        675                 680                 685

Met Leu Lys Gln Ala Glu Gln Glu Ala Lys Ala Lys Gln Lys His Pro
    690                 695                 700

Thr Pro Pro Pro Asn Asp Trp Ala Ala Ser Gln Val Leu Cys Cys Val
705                 710                 715                 720

Asn Trp Arg Ser Gly Gly Tyr Phe Ser Asn His Leu Ser Gly Gly Leu
            725                 730                 735

Asn His Gln Ile Glu His His Leu Phe Pro Ser Ile Ser His Ala Asn
                740                 745                 750

Tyr Pro Thr Ile Ala Pro Val Val Lys Gly Val Cys Glu Glu Tyr Gly
        755                 760                 765

Leu Pro Tyr Lys Asn Tyr Ser Gln Phe Ser Asp Ala Ile Tyr Gly Met
    770                 775                 780

Val Glu His Leu Arg Ala Met Gly Thr Lys Pro Glu Asp Asn Gly Lys
785                 790                 795                 800

Leu Ala Pro Leu Pro Gly Ser Leu Glu Asp Val Cys Pro Val Leu Ser
            805                 810                 815

Ala Ala Val Ala Ala Gln Pro Asp Gly Ser Thr Asp Gly Ser Ala Ala
                820                 825                 830

Gly Cys Pro Ala Val Ala Thr Leu Ala
        835                 840
```

<210> 23
<211> 2442
<212> DNA
<213> Euglena anabaena

<400> 23

```
atggccgaag gcaagagcga tgggcctgtg gtgacccttc aaagcatgtg gaaaccgctt     60
gctttgatgg cagtagatgt cggcatattg gtcaacgtcc gccgcaaggc tttcactgag    120
tttgatgggc acagcaacgt ttttgcagat cccgtttaca ttccatttgt gatgaatctc    180
ttctacttga ccatgatctt tgctgggtgc cgttggatga agactcgcga acccttttgag    240
```

```
atcaagtcat atatgtttgc atacaatgca tatcagacga tgatgaactt cctcattgtc    300
gtcgggttca tgtatgaggt gcacagcaca gggatgcggt attgggggtc caggatcgac    360
acctccacca agggcttggg cctcggtttc ctgatctatg cccactacca caacaaatac    420
gtggagtatg tcgacacgtt gttcatgatc ctgcgcaaga aaaacaacca gatctcgttc    480
ctccacgtct accaccattc gcttttgact tgggcctggt gggccgtggt ctactgggcc    540
ccaggaggag atgcctggtt cggagcatgc tacaattcct ttatccacgt gctgatgtac    600
tcctactacc tgtttgcaac gtttggcatc aggtgcccct ggaagaagat gctgacccag    660
ttgcagatgg tgcaattctg cttctgcttt gcccacgcga tgtatgttgg atggctgggc    720
catgaagtct acccgcgctg gttgacggcg ctacaggcat ttgtgatgct aaacatgctg    780
gtgctgttcg gcaacttcta catgaagtcg tactccaagg ccagcaagct ggagccggcc    840
tcccccgtgt cccccgcctc cctcgcccag aagccgttcg agaacgccaa ggtgaagcct    900
gggggccccg gcaagccaag cgagattgcg tcgctgccac cgccaattcg accagtcggg    960
aacccacctg cagcctacta cgatgccctg gcgacctcgg gcaccgggca ggaccgcaag   1020
ttcaccatgc gggaggtggc ccgccatatt gtgccgaccg atgggtggtt ggcgtgccat   1080
gacggtgtct acgacatcac cgagttcata gggaaacatc ctggcggcga tgttatttct   1140
ctcggattgg gcagggactc cacaatcctg gttgagtcgt accaccctgc cggaaggcca   1200
gacaaggtca tggaaaagta ccgcatcggg acgctccagg accaccgcac gttctacgac   1260
tggcaggcct ccgcgttcta cgccgagctg aagcagcggg tggtgcagac gctaaaggag   1320
gccggccaac cgcggcgtgg gggcctgtcg gtcaaagcgg cgctggtcat ggcggcgttc   1380
gcagcgtcgt tctacctcat ggtgacccag ggatccttct tctgggccgc cgtctggggc   1440
ctcgccggct cccacattgg cctcagcatc cagcacgacg ggaaccacgg ggctttcagt   1500
aagagtggtc ggctgaaccg cctcgcgggc tggggcatgg acgtcatcgg ggcctcctcg   1560
acggcctggg agtaccagca cgtcatcggg caccaccagt acaccaacct ggtatcggat   1620
cccgagttcg cgctgcctga gaacgacccg gacgtcttcg gcacctatcc gctgatgcgg   1680
atgcatccgg acacccccttg gaagccgcac caccagctgc agcatatgta cgcgttcccg   1740
ctgttcgccc tgatgaccat cagcaaggtc atcatcagcg acttcacgtt ctgcctcgcc   1800
aagcggcgcg ggccgatcga cttctccgcc aggctcgtgc cacttgaggg gcagatgctc   1860
ttctgggggg cgaagatcat ggggttcctg atgcagatag tcctgccgtg ctatctgcat   1920
ggcatcgccc atgggctggc gctgttcatc acagcccacc tggtgtcggg agagtacctc   1980
gcggtctgct tcatcatcaa ccacatctct gagtcatgcg actatttgaa tccaagttcc   2040
gtcatcgctg cgcggcggac ggaaatgctg aagcaggcgg agcaggaggc caaggcaaag   2100
```

```
cagaagcacc ccaccccacc gcccaacgac tgggccgcgt ctcaggtact gtgctgcgta   2160

aactggcgct ctggtggata tttctcaaac cacctctcag gcgggctgaa ccaccagatc   2220

gagcaccacc tcttccccag catctcacat gcgaactatc cgaccattgc cccagttgtg   2280

aaaagggtgt gcgaggagta cggcctcccc tacaagaact actcccagtt ctctgacctc   2340

tctatggaat ggtggagcac ctcagggcga tgggcacgaa gccggcagac aacgacaagc   2400

tggcgccacc actgggctcc ctggaggacg tgtgcccggt cc                      2442
```

<210> 24
<211> 814
<212> PRT
<213> Euglena anabaena

<400> 24

```
Met Ala Glu Gly Lys Ser Asp Gly Pro Val Val Thr Leu Gln Ser Met
1               5                   10                  15

Trp Lys Pro Leu Ala Leu Met Ala Val Asp Val Gly Ile Leu Val Asn
            20                  25                  30

Val Arg Arg Lys Ala Phe Thr Glu Phe Asp Gly His Ser Asn Val Phe
        35                  40                  45

Ala Asp Pro Val Tyr Ile Pro Phe Val Met Asn Leu Phe Tyr Leu Thr
    50                  55                  60

Met Ile Phe Ala Gly Cys Arg Trp Met Lys Thr Arg Glu Pro Phe Glu
65                  70                  75                  80

Ile Lys Ser Tyr Met Phe Ala Tyr Asn Ala Tyr Gln Thr Met Met Asn
                85                  90                  95

Phe Leu Ile Val Val Gly Phe Met Tyr Glu Val His Ser Thr Gly Met
            100                 105                 110

Arg Tyr Trp Gly Ser Arg Ile Asp Thr Ser Thr Lys Gly Leu Gly Leu
        115                 120                 125

Gly Phe Leu Ile Tyr Ala His Tyr His Asn Lys Tyr Val Glu Tyr Val
    130                 135                 140

Asp Thr Leu Phe Met Ile Leu Arg Lys Lys Asn Asn Gln Ile Ser Phe
145                 150                 155                 160
```

```
Leu His Val Tyr His His Ser Leu Leu Thr Trp Ala Trp Trp Ala Val
                165                 170                 175

Val Tyr Trp Ala Pro Gly Gly Asp Ala Trp Phe Gly Ala Cys Tyr Asn
            180                 185                 190

Ser Phe Ile His Val Leu Met Tyr Ser Tyr Tyr Leu Phe Ala Thr Phe
        195                 200                 205

Gly Ile Arg Cys Pro Trp Lys Lys Met Leu Thr Gln Leu Gln Met Val
    210                 215                 220

Gln Phe Cys Phe Cys Phe Ala His Ala Met Tyr Val Gly Trp Leu Gly
225                 230                 235                 240

His Glu Val Tyr Pro Arg Trp Leu Thr Ala Leu Gln Ala Phe Val Met
                245                 250                 255

Leu Asn Met Leu Val Leu Phe Gly Asn Phe Tyr Met Lys Ser Tyr Ser
            260                 265                 270

Lys Ala Ser Lys Leu Glu Pro Ala Ser Pro Val Ser Pro Ala Ser Leu
        275                 280                 285

Ala Gln Lys Pro Phe Glu Asn Ala Lys Val Lys Pro Gly Gly Pro Gly
    290                 295                 300

Lys Pro Ser Glu Ile Ala Ser Leu Pro Pro Pro Ile Arg Pro Val Gly
305                 310                 315                 320

Asn Pro Pro Ala Ala Tyr Tyr Asp Ala Leu Ala Thr Ser Gly Thr Gly
                325                 330                 335

Gln Asp Arg Lys Phe Thr Met Arg Glu Val Ala Arg His Ile Val Pro
            340                 345                 350

Thr Asp Gly Trp Leu Ala Cys His Asp Gly Val Tyr Asp Ile Thr Glu
        355                 360                 365

Phe Ile Gly Lys His Pro Gly Gly Asp Val Ile Ser Leu Gly Leu Gly
    370                 375                 380

Arg Asp Ser Thr Ile Leu Val Glu Ser Tyr His Pro Ala Gly Arg Pro
385                 390                 395                 400

Asp Lys Val Met Glu Lys Tyr Arg Ile Gly Thr Leu Gln Asp His Arg
```

```
                405                 410                 415

Thr Phe Tyr Asp Trp Gln Ala Ser Ala Phe Tyr Ala Glu Leu Lys Gln
            420                 425                 430

Arg Val Val Gln Thr Leu Lys Glu Ala Gly Gln Pro Arg Arg Gly Gly
        435                 440                 445

Leu Ser Val Lys Ala Ala Leu Val Met Ala Ala Phe Ala Ala Ser Phe
    450                 455                 460

Tyr Leu Met Val Thr Gln Gly Ser Phe Phe Trp Ala Ala Val Trp Gly
465                 470                 475                 480

Leu Ala Gly Ser His Ile Gly Leu Ser Ile Gln His Asp Gly Asn His
                485                 490                 495

Gly Ala Phe Ser Lys Ser Gly Arg Leu Asn Arg Leu Ala Gly Trp Gly
            500                 505                 510

Met Asp Val Ile Gly Ala Ser Ser Thr Ala Trp Glu Tyr Gln His Val
        515                 520                 525

Ile Gly His His Gln Tyr Thr Asn Leu Val Ser Asp Pro Glu Phe Ala
    530                 535                 540

Leu Pro Glu Asn Asp Pro Asp Val Phe Gly Thr Tyr Pro Leu Met Arg
545                 550                 555                 560

Met His Pro Asp Thr Pro Trp Lys Pro His His Gln Leu Gln His Met
                565                 570                 575

Tyr Ala Phe Pro Leu Phe Ala Leu Met Thr Ile Ser Lys Val Ile Ile
            580                 585                 590

Ser Asp Phe Thr Phe Cys Leu Ala Lys Arg Arg Gly Pro Ile Asp Phe
        595                 600                 605

Ser Ala Arg Leu Val Pro Leu Glu Gly Gln Met Leu Phe Trp Gly Ala
    610                 615                 620

Lys Ile Met Gly Phe Leu Met Gln Ile Val Leu Pro Cys Tyr Leu His
625                 630                 635                 640

Gly Ile Ala His Gly Leu Ala Leu Phe Ile Thr Ala His Leu Val Ser
                645                 650                 655
```

```
Gly Glu Tyr Leu Ala Val Cys Phe Ile Ile Asn His Ile Ser Glu Ser
                660                 665                 670

Cys Asp Tyr Leu Asn Pro Ser Ser Val Ile Ala Ala Arg Arg Thr Glu
            675                 680                 685

Met Leu Lys Gln Ala Glu Gln Glu Ala Lys Ala Lys Gln Lys His Pro
    690                 695                 700

Thr Pro Pro Pro Asn Asp Trp Ala Ala Ser Gln Val Leu Cys Cys Val
705                 710                 715                 720

Asn Trp Arg Ser Gly Gly Tyr Phe Ser Asn His Leu Ser Gly Gly Leu
                    725                 730                 735

Asn His Gln Ile Glu His His Leu Phe Pro Ser Ile Ser His Ala Asn
                740                 745                 750

Tyr Pro Thr Ile Ala Pro Val Val Lys Arg Val Cys Glu Glu Tyr Gly
            755                 760                 765

Leu Pro Tyr Lys Asn Tyr Ser Gln Phe Ser Asp Leu Ser Met Glu Trp
    770                 775                 780

Trp Ser Thr Ser Gly Arg Trp Ala Arg Ser Arg Gln Thr Thr Thr Ser
785                 790                 795                 800

Trp Arg His His Trp Ala Pro Trp Arg Thr Cys Ala Arg Ser
                    805                 810
```

<210> 25
<211> 777
<212> DNA
<213> Euglena gracilis

<220>
<221> misc feature
<222> (1)..(777)
<223> delta-9 elongase

<300>
<302> DELTA-9 ELONGASES AND THEIR USE IN MAKING POLYUNSATURATED FATTY ACIDS

<310> US-2007-0118929-A1
<311> 2006-11-16
<312> 2007-05-24
<313> (1)..(777)

<300>
<302> DELTA-9 ELONGASES AND THEIR USE IN MAKING POLYUNSATURATED FATTY ACIDS

<310> WO 2007/061845
<311> 2006-11-16
<312> 2007-05-31
<313> (1)..(777)

<400> 25

```
atggaggtgg tgaatgaaat agtctcaatt gggcaggaag ttttacccaa agttgattat      60
gcccaactct ggagtgatgc cagtcactgt gaggtgcttt acttgtccat cgcatttgtc     120
atcttgaagt tcactcttgg ccccccttggt ccaaaaggtc agtctcgtat gaagtttgtt    180
ttcaccaatt acaaccttct catgtccatt tattcgttgg gatcattcct ctcaatggca     240
tatgccatgt acaccatcgg tgttatgtct gacaactgcg agaaggcttt tgacaacaac     300
gtcttcagga tcaccacgca gttgttctat ttgagcaagt tcctggagta tattgactcc     360
ttctatttgc cactgatggg caagcctctg acctggttgc aattcttcca tcatttgggg     420
gcaccgatgg atatgtggct gttctataat taccgaaatg aagctgtttg gatttttgtg     480
ctgttgaatg gtttcatcca ctggatcatg tacggttatt attggaccag attgatcaag     540
ctgaagttcc ccatgccaaa atccctgatt acatcaatgc agatcattca attcaatgtt     600
ggtttctaca ttgtctggaa gtacaggaac attccctgtt atcgccaaga tgggatgagg     660
atgtttggct ggttcttcaa ttacttttat gttggcacag tcttgtgttt gttcttgaat     720
ttctatgtgc aaacgtatat cgtcaggaag cacaagggag ccaaaaagat tcagtga        777
```

<210> 26
<211> 1260
<212> DNA
<213> Tetruetreptia pomquetensis CCMP1491

<220>
<221> misc_feature
<222> (1)..(1260)
<223> U.S. Patent Application No. 11/876,115 (filed October 22, 2007)

<400> 26

```
atgtctccta agcggcaagc tctgccaatc acaattgatg gcgcaactta tgatgtgtct      60
gcttgggtca atcaccaccc tggaggagct gacattatcg agaactatcg caaccgcgat     120
gcgaccgatg tcttcatggt gatgcactct caagaagccg tcgccaagtt gaagagaatg     180
cctgttatgg agccttcctc tcctgacaca cctgttgcac ccaagcctaa gcgtgatgag     240
ccccaggagg atttccgcaa gttgcgggag gaattcatct ccaagggtat gttcgagacg     300
agtttccttt ggtattttta caagacttca actaccgtcg gtttgatggt cctttccatc     360
ttgatgaccg tgtacacgaa ttggtatttc accgctgctt tggttcttgg cgtgtgctac     420
```

caacagctag gctggttgtc ccacgactat tgccatcacc aggttttcac aaaccgcaag 480
attaacgacg ctttcggtct ctttttcggt aacgtgatgc agggatactc acagacttgg 540
tggaaggata ggcacaatgg tcaccatgcc gccaccaatg tggtcggcca tgacccagat 600
attgataacc tccccatcct ggcttggtct cccgaagatg tcaagagggc tactccttcg 660
actcggaatc tcatcaagta ccagcagtac tacttcattc ccaccattgc atcccttagg 720
ttcatctggt gcctccaatc catcggcggc gtcatgtcct acaagagcga ggagaggaac 780
ctgtactaca agcgccagta cactaaggag gcgattggtc tggccctcca ctgggtgctc 840
aaggccactt tctattgcag tgccatgcct agctttgcca ccggtttggg atgcttcttg 900
atctccgagc tgctcggagg atttggcatt gccatcgttg tgtttctgaa tcactatcct 960
ttggacaagg ttgaggagac tgtctgggat gagcacgggt tcagcgccag ccagatccac 1020
gagacgttga acattaagcc cggccttctc accgattggg tctttggtgg tctcaactac 1080
cagattgagc accacttgtg gcccaacatg cccaggcaca acctcacggc agcttccctg 1140
gaggtgcaga agttgtgcgc caagcacaac ctgccctaca gggccccagc catcatcccc 1200
ggggttcaga aattggtcag cttcttaggc gagattgccc agctggctgc tgtccctgaa 1260

<210> 27
<211> 4300
<212> DNA
<213> Artificial Sequence

<220>
<223> plasmid pLF114-10

<400> 27

```
taatacgact cactataggg cgaattgggc ccgacgtcgc atgctcccgg ccgccatggc     60
ggccgcggga attcgattgg cggccgcacc atgtctccta agcggcaagc tctgccaatc    120
acaattgatg gcgcaactta tgatgtgtct gcttgggtca atcaccaccc tggaggagct    180
gacattatcg agaactatcg caaccgcgat gcgaccgatg tcttcatggt gatgcactct    240
caagaagccg tcgccaagtt gaagagaatg cctgttatgg agccttcctc tcctgacaca    300
cctgttgcac ccaagcctaa gcgtgatgag ccccaggagg atttccgcaa gttgcgggag    360
gaattcatct ccaagggtat gttcgagacg agtttccttt ggtattttta caagacttca    420
actaccgtcg gtttgatggt cctttccatc ttgatgaccg tgtacacgaa ttggtatttc    480
accgctgctt tggttcttgg cgtgtgctac caacagctag gctggttgtc ccacgactat    540
tgccatcacc aggttttcac aaaccgcaag attaacgacg ctttcggtct ctttttcggt    600
aacgtgatgc agggatactc acagacttgg tggaaggata ggcacaatgg tcaccatgcc    660
```

```
gccaccaatg tggtcggcca tgacccagat attgataacc tccccatcct ggcttggtct    720
cccgaagatg tcaagagggc tactccttcg actcggaatc tcatcaagta ccagcagtac    780
tacttcattc ccaccattgc atcccttagg ttcatctggt gcctccaatc catcggcggc    840
gtcatgtcct acaagagcga ggagaggaac ctgtactaca agcgccagta cactaaggag    900
gcgattggtc tggccctcca ctgggtgctc aaggccactt tctattgcag tgccatgcct    960
agctttgcca ccggtttggg atgcttcttg atctccgagc tgctcggagg atttggcatt   1020
gccatcgttg tgtttctgaa tcactatcct ttggacaagg ttgaggagac tgtctgggat   1080
gagcacgggt tcagcgccag ccagatccac gagacgttga acattaagcc cggccttctc   1140
accgattggg tctttggtgg tctcaactac cagattgagc accacttgtg gcccaacatg   1200
cccaggcaca acctcacggc agcttccctg gaggtgcaga agttgtgcgc caagcacaac   1260
ctgccctaca gggccccagc catcatcccc ggggttcaga aattggtcag cttcttaggc   1320
gagattgccc agctggctgc tgtccctgaa tgagcggccg caatcactag tgaattcgcg   1380
gccgcctgca ggtcgaccat atgggagagc tcccaacgcg ttggatgcat agcttgagta   1440
ttctatagtg tcacctaaat agcttggcgt aatcatggtc atagctgttt cctgtgtgaa   1500
attgttatcc gctcacaatt ccacacaaca tacgagccgg aagcataaag tgtaaagcct   1560
ggggtgccta atgagtgagc taactcacat taattgcgtt gcgctcactg cccgctttcc   1620
agtcgggaaa cctgtcgtgc cagctgcatt aatgaatcgg ccaacgcgcg gggagaggcg   1680
gtttgcgtat tgggcgctct tccgcttcct cgctcactga ctcgctgcgc tcggtcgttc   1740
ggctgcggcg agcggtatca gctcactcaa aggcggtaat acggttatcc acagaatcag   1800
gggataacgc aggaaagaac atgtgagcaa aaggccagca aaaggccagg aaccgtaaaa   1860
aggccgcgtt gctggcgttt ttccataggc tccgcccccc tgacgagcat cacaaaaatc   1920
gacgctcaag tcagaggtgg cgaaacccga caggactata aagataccag gcgtttcccc   1980
ctggaagctc cctcgtgcgc tctcctgttc cgaccctgcc gcttaccgga tacctgtccg   2040
cctttctccc ttcgggaagc gtggcgcttt ctcatagctc acgctgtagg tatctcagtt   2100
cggtgtaggt cgttcgctcc aagctgggct gtgtgcacga accccccgtt cagcccgacc   2160
gctgcgcctt atccggtaac tatcgtcttg agtccaaccc ggtaagacac gacttatcgc   2220
cactggcagc agccactggt aacaggatta gcagagcgag gtatgtaggc ggtgctacag   2280
agttcttgaa gtggtggcct aactacggct acactagaag aacagtattt ggtatctgcg   2340
ctctgctgaa gccagttacc ttcggaaaaa gagttggtag ctcttgatcc ggcaaacaaa   2400
ccaccgctgg tagcggtggt ttttttgttt gcaagcagca gattacgcgc agaaaaaaag   2460
gatctcaaga agatcctttg atcttttcta cggggtctga cgctcagtgg aacgaaaact   2520
```

```
cacgttaagg gattttggtc atgagattat caaaaaggat cttcacctag atccttttaa   2580
attaaaaatg aagttttaaa tcaatctaaa gtatatatga gtaaacttgg tctgacagtt   2640
accaatgctt aatcagtgag gcacctatct cagcgatctg tctatttcgt tcatccatag   2700
ttgcctgact ccccgtcgtg tagataacta cgatacggga gggcttacca tctggcccca   2760
gtgctgcaat gataccgcga gacccacgct caccggctcc agatttatca gcaataaacc   2820
agccagccgg aagggccgag cgcagaagtg gtcctgcaac tttatccgcc tccatccagt   2880
ctattaattg ttgccgggaa gctagagtaa gtagttcgcc agttaatagt ttgcgcaacg   2940
ttgttgccat tgctacaggc atcgtggtgt cacgctcgtc gtttggtatg gcttcattca   3000
gctccggttc ccaacgatca aggcgagtta catgatcccc catgttgtgc aaaaaagcgg   3060
ttagctcctt cggtcctccg atcgttgtca gaagtaagtt ggccgcagtg ttatcactca   3120
tggttatggc agcactgcat aattctctta ctgtcatgcc atccgtaaga tgcttttctg   3180
tgactggtga gtactcaacc aagtcattct gagaatagtg tatgcggcga ccgagttgct   3240
cttgcccggc gtcaatacgg gataataccg cgccacatag cagaacttta aaagtgctca   3300
tcattggaaa acgttcttcg gggcgaaaac tctcaaggat cttaccgctg ttgagatcca   3360
gttcgatgta acccactcgt gcacccaact gatcttcagc atcttttact ttcaccagcg   3420
tttctgggtg agcaaaaaca ggaaggcaaa atgccgcaaa aaagggaata agggcgacac   3480
ggaaatgttg aatactcata ctcttccttt ttcaatatta ttgaagcatt tatcagggtt   3540
attgtctcat gagcggatac atatttgaat gtatttagaa aaataaacaa ataggggttc   3600
cgcgcacatt tccccgaaaa gtgccacctg atgcggtgtg aaataccgca cagatgcgta   3660
aggagaaaat accgcatcag gaaattgtaa gcgttaatat tttgttaaaa ttcgcgttaa   3720
atttttgtta aatcagctca ttttttaacc aataggccga aatcggcaaa atcccttata   3780
aatcaaaaga atagaccgag atagggttga gtgttgttcc agtttggaac aagagtccac   3840
tattaaagaa cgtggactcc aacgtcaaag ggcgaaaaac cgtctatcag ggcgatggcc   3900
cactacgtga accatcaccc taatcaagtt ttttggggtc gaggtgccgt aaagcactaa   3960
atcggaaccc taaagggagc ccccgattta gagcttgacg gggaaagccg gcgaacgtgg   4020
cgagaaagga agggaagaaa gcgaaaggag cgggcgctag ggcgctggca agtgtagcgg   4080
tcacgctgcg cgtaaccacc acacccgccg cgcttaatgc gccgctacag ggcgcgtcca   4140
ttcgccattc aggctgcgca actgttggga agggcgatcg gtgcgggcct cttcgctatt   4200
acgccagctg gcgaaagggg gatgtgctgc aaggcgatta agttgggtaa cgccagggtt   4260
ttcccagtca cgacgttgta aaacgacggc cagtgaattg                         4300
```

<210> 28
<211> 31
<212> DNA
<213> Artificial Sequence

<220>
<223> oligonucleotide MGW511

<400> 28
gaattcgcgg ccgctcactg ccagggagtc g 31

<210> 29
<211> 11
<212> PRT
<213> Artificial Sequence

<220>
<223> conserved motif at C-terminal for C20elo domains

<220>
<221> misc_feature
<222> (4)..(5)
<223> Xaa can be any naturally occurring amino acid

<220>
<221> misc_feature
<222> (8)..(10)
<223> Xaa can be any naturally occurring amino acid

<400> 29

```
Val Leu Phe Xaa Xaa Phe Tyr Xaa Xaa Xaa Tyr
1               5                   10
```

<210> 30
<211> 4
<212> PRT
<213> Euglena gracilis

<400> 30

```
Lys Asn Gly Lys
1
```

<210> 31
<211> 5
<212> PRT
<213> Euglena gracilis

<400> 31

```
Pro Glu Asn Gly Ala
1               5
```

<210> 32
<211> 7
<212> PRT

<213> Euglena gracilis

<400> 32

Pro Cys Glu Asn Gly Thr Val
1 5

<210> 33
<211> 54
<212> DNA
<213> Euglena gracilis

<400> 33
cccgcccgtc cagctggact cccgccggcc acgtactacg actccctggc agtg 54

<210> 34
<211> 18
<212> PRT
<213> Euglena gracilis

<400> 34

Pro Ala Arg Pro Ala Gly Leu Pro Pro Ala Thr Tyr Tyr Asp Ser Leu
1 5 10 15

Ala Val

<210> 35
<211> 54
<212> DNA
<213> Euglena gracilis

<400> 35
cccacccgtc cagctggacc cccgccggcc acgtactacg actccctggc agtg 54

<210> 36
<211> 18
<212> PRT
<213> Euglena gracilis

<400> 36

Pro Thr Arg Pro Ala Gly Pro Pro Pro Ala Thr Tyr Tyr Asp Ser Leu
1 5 10 15

Ala Val

<210> 37
<211> 2379
<212> DNA
<213> Euglena gracilis

<400> 37

```
atggcggata gcccagtcat caacctcagc accatgtgga aaccccttc actgatggct      60
ttggaccttg ccgttttggg acatgtctgg aagcaggcac aacaggaggg cagcatttcg    120
gcctatgctg attctgtttg gactcctctc attatgtccg gtttatactt atcaatgatc    180
ttcgtggggt gccgctggat gaagaaccgt gaacccttg agatcaaaac atacatgttt     240
gcgtataacc tgtatcagac cttgatgaac ctttgcatcg tgttgggatt cttgtaccag    300
gtgcatgcca ctgggatgcg cttttgggga agtggtgtcg accgaagccc aaaaggtttg    360
ggcattggct tcttcattta tgcccactac cacaacaagt atgtggaata ttttgataca    420
ctttttatgg tgctgcgaaa gaagaacaac cagatttctt tccttcacgt gtatcatcat    480
gccctgttga catgggcttg gtttgctgtt gtgtatttcg cacctggagg tgatggctgg    540
tttggagctt gctacaattc ttccatccat gtcctgatgt actcttacta cttgcttgca    600
acttttggca tcagttgccc ttggaagaag atcttgacac agctccagat ggttcaattc    660
tgtttctgtt ttacacattc catttatgtg tggatttgcg ggtcagagat ctacccacgg    720
cctctgactg ctttgcagtc gttcgtgatg gtcaatatgt tggtgctgtt tggcaatttc    780
tatgtcaagc aatactccca aaagaacggc aagccggaga acggagccac ccctgagaac    840
ggagcgaagc cgcaaccttg cgagaacggc acggtggaaa agcgagagaa tgacaccgcc    900
aacgttcggc ccgcccgtcc agctggactc ccgccggcca cgtactacga ctccctggca    960
gtgtcggggc agggcaagga gcggctgttc accaccgatg aggtgaggcg gcacatcctc   1020
cccaccgatg gctggctgac gtgccacgaa ggagtctacg atgtcactga tttccttgcc   1080
aagcaccctg gtggcggtgt catcacgctg ggccttggaa gggactgcac aatcctcgtc   1140
gagtcatacc accctgctgg gcgcccggac aaggtgatgg agaagtaccg cattggtacg   1200
ctgcaggacc ccaagacgtt ctatgcttgg ggagagtccg atttctaccc tgagttgaag   1260
cgccgggccc ttgcaaggct gaaggaggct ggtcaggcgc ggcgcggcgg ccttggggtg   1320
aaggccctcc tggtgctcac cctcttcttc gtgtcgtggt acatgtgggt ggcccacaag   1380
tccttcctct gggccgccgt ctggggcttc gccggctccc acgtcgggct gagcatccag   1440
cacgacggca accacggcgc gttcagccgc agcacactgg tgaaccgcct ggcggggtgg   1500
ggcatggact tgatcggcgc gtcgtcaacg gtgtgggagt accagcacgt catcggccac   1560
caccagtaca ccaacctcgt gtcggacacg ctattcagtc tgcctgagaa cgatccggac   1620
gtcttctcca gctacccgct gatgcgcatg cacccggata cggcgtggca gccgcaccac   1680
cgcttccagc acctgttcgc gttcccactg ttcgccctga tgacaatcag caaggtgctg   1740
```

```
accagcgatt tcgctgtctg cctcagcatg aagaaggggt ccatcgactg ctcctccagg   1800
ctcgtcccac tggaggggca gctgctgttc tgggggccca agctggcgaa cttcctgttg   1860
cagattgtgt tgccatgcta cctccacggg acagctatgg gcctggccct cttctctgtt   1920
gcccaccttg tgtcggggga gtacctcgcg atctgcttca tcatcaacca catcagcgag   1980
tcttgtgagt ttatgaatac aagctttcaa accgccgccc ggaggacaga gatgcttcag   2040
gcagcccatc aggcagcgga ggccaagaag gtgaagccca cccctccacc gaacgattgg   2100
gctgtgacac aggtccaatg ctgcgtgaat tggagatcag gtggcgtgtt ggccaatcac   2160
ctctctggag gcttgaacca ccagatcgag catcatctgt tccccagcat ctcgcatgcc   2220
aactacccca tcatcgcccg tgttgtgaag gaggtgtgcg aggagtatgg gttgccgtac   2280
aagaactacg tcacgttctg ggatgcagtc tgtggcatgg ttcagcacct ccggttgatg   2340
ggtgctccac cggtgccaac gaacggggac aaaaagtca                          2379
```

<210> 38
<211> 3668
<212> DNA
<213> Artificial Sequence

<220>
<223> Plasmid pLF121-1

<220>
<221> misc_feature
<222> (3616)..(3655)
<223> n is a, c, g, or t

<400> 38

```
gtacaaagtt ggcattataa gaaagcattg cttatcaatt tgttgcaacg aacaggtcac     60
tatcagtcaa aataaaatca ttatttgcca tccagctgat atcccctata gtgagtcgta    120
ttacatggtc atagctgttt cctggcagct ctggcccgtg tctcaaaatc tctgatgtta    180
cattgcacaa gataaaaata tatcatcatg ttagaaaaac tcatcgagca tcaaatgaaa    240
ctgcaattta ttcatatcag gattatcaat accatatttt tgaaaaagcc gtttctgtaa    300
tgaaggagaa aactcaccga ggcagttcca taggatggca agatcctggt atcggtctgc    360
gattccgact cgtccaacat caatacaacc tattaatttc ccctcgtcaa aaataaggtt    420
atcaagtgag aaatcaccat gagtgacgac tgaatccggt gagaatggca aaagcttatg    480
catttctttc cagacttgtt caacaggcca gccattacgc tcgtcatcaa aatcactcgc    540
atcaaccaaa ccgttattca ttcgtgattg cgcctgagcg agacgaaata cgcgatcgct    600
gttaaaagga caattacaaa caggaatcga atgcaaccgg cgcaggaaca ctgccagcgc    660
atcaacaata ttttcacctg aatcaggata ttcttctaat acctggaatg ctgttttccc    720
```

```
ggggatcgca gtggtgagta accatgcatc atcaggagta cggataaaat gcttgatggt    780
cggaagaggc ataaattccg tcagccagtt tagtctgacc atctcatctg taacatcatt    840
ggcaacgcta cctttgccat gtttcagaaa caactctggc gcatcgggct tcccatacaa    900
tcgatagatt gtcgcacctg attgcccgac attatcgcga gcccatttat acccatataa    960
atcagcatcc atgttggaat ttaatcgcgg cctcgagcaa gacgtttccc gttgaatatg   1020
gctcatagat cttttctcca tcactgatag ggagtggtaa aataactcca tcaatgatag   1080
agtgtcaaca acatgaccaa aatcccttaa cgtgagttac gcgtattaat tgcgttgcgc   1140
tcactgcccg ctttccagtc gggaaacctg tcgtgccagc tgcattaatg aatcggccaa   1200
cgcgcgggga gaggcggttt gcgtattggg cgctcttccg cttcctcgct cactgactcg   1260
ctgcgctcgg tcgttcggct gcggcgagcg gtatcagctc actcaaaggc ggtaatacgg   1320
ttatccacag aatcagggga taacgcagga aagaacatgt gagcaaaagg ccagcaaaag   1380
gccaggaacc gtaaaaaggc cgcgttgctg gcgtttttcc ataggctccg cccccctgac   1440
gagcatcaca aaaatcgacg ctcaagtcag aggtggcgaa acccgacagg actataaaga   1500
taccaggcgt ttccccctgg aagctccctc gtgcgctctc ctgttccgac cctgccgctt   1560
accggatacc tgtccgcctt tctcccttcg ggaagcgtgg cgctttctca atgctcacgc   1620
tgtaggtatc tcagttcggt gtaggtcgtt cgctccaagc tgggctgtgt gcacgaaccc   1680
cccgttcagc ccgaccgctg cgccttatcc ggtaactatc gtcttgagtc caacccggta   1740
agacacgact tatcgccact ggcagcagcc actggtaaca ggattagcag agcgaggtat   1800
gtaggcggtg ctacagagtt cttgaagtgg tggcctaact acggttacac tagaagaaca   1860
gtatttggta tctgcgctct gctgaagcca gttaccttcg gaaaaagagt tggtagctct   1920
tgatccggca aacaaaccac cgctggtagc ggtggttttt ttgtttgcaa gcagcagatt   1980
acgcgcagaa aaaaaggatc tcaagaagat cctttgatct tttctacggg gtctgacgct   2040
cagggaacga cgcgtaccgc tagccaggaa gagtttgtag aaacgcaaaa aggccatccg   2100
tcaggatggc cttctgctta gtttgatgcc tggcagttta tggcgggcgt cctgcccgcc   2160
accctccggg ccgttgcttc acaacgttca aatccgctcc cggcggattt gtcctactca   2220
ggagagcgtt caccgacaaa caacagataa aacgaaaggc ccagtcttcc gactgagcct   2280
ttcgttttat ttgatgcctg gcagttccct actctcgcgt taacgctagc atggatgttt   2340
tcccagtcac gacgttgtaa aacgacggcc agtcttaagc tcgggcccca aataatgatt   2400
ttattttgac tgatagtgac ctgttcgttg caacaaattg atgagcaatg cttttttata   2460
atgccaactt tgtacaaaaa agttggtttt tttcggtcta aaatggaagc agccaaagaa   2520
```

```
ttggtttcca tcgtccaaga ggagctcccc aaggtggact atgcccagct ttggcaggat   2580
gccagcagct gtgaggtcct ttacctctcg gtggcattcg tggcgatcaa gttcatgctg   2640
cgcccactgg acctgaagcg ccaggccacc ttgaagaagc tgttcacagc atacaacttc   2700
ctcatgtcga tctattcctt tggctccttc ctggccatgg cctatgccct atcagtaact   2760
ggcatcctct ccggcgactg tgagacggcg ttcaacaacg atgtgttcag gatcacaact   2820
cagctgttct acctcagcaa gttcgtagag tacatcgact ccttctacct tccccttatg   2880
gacaagccac tgtcgttcct tcagttcttc catcatttgg gggcccccat tgacatgtgg   2940
ctattctaca aataccgcaa cgaaggagtc tggatctttg tcctgttgaa tgggttcatt   3000
cactggatca tgtacggtta ctattggacg cggctcatca agctgaactt ccccatgccc   3060
aagaacctga tcacctccat gcagatcatc cagttcaatg tcgggttcta catcgtctgg   3120
aagtaccgca atgtgccatg ctaccgccag gatgggatgc gcatgtttgc ctggatcttc   3180
aactactggt atgtcgggac ggtcttgctg ctgttcctca acttttacgt gcagacgtac   3240
atccggaagc cgaggaagaa ccgagggaag aaggagtagg ccacatggcg cctgcgctgg   3300
aggaaacggt acgctcggat ggtgcactgc acttgcactc cgccgtttct agcctcccct   3360
cgctctaacc actgcggcat gcctgcttga ggcgtgacgt tgcctcgtat gatacagttt   3420
acacccttcc cacagcccac ggagctggtg actgtttcca gcgtctgcag atcattgatc   3480
tggtgcaatg tgcacagacc aagcccctct aacgtcttgc ggtgtaccgc tcgacactca   3540
ctgcaagaga cagatggctg agcatgttat agccccttac attctaccct tcgtcccaac   3600
ctgaccgtca cattcnnnnn nnnnnnnnnn nnnnnnnnnn nnnnnnnnnn nnnnnaccca   3660
actttctt                                                            3668
```

<210> 39
<211> 774
<212> DNA
<213> Euglena anabaena

<400> 39

```
atggaagcag ccaaagaatt ggtttccatc gtccaagagg agctccccaa ggtggactat      60
gcccagcttt ggcaggatgc cagcagctgt gaggtccttt acctctcggt ggcattcgtg     120
gcgatcaagt tcatgctgcg cccactggac ctgaagcgcc aggccacctt gaagaagctg     180
ttcacagcat acaacttcct catgtcgatc tattcctttg gctccttcct ggccatggcc     240
tatgccctat cagtaactgg catcctctcc ggcgactgtg agacggcgtt caacaacgat     300
gtgttcagga tcacaactca gctgttctac ctcagcaagt tcgtagagta catcgactcc     360
ttctaccttc cccttatgga caagccactg tcgttccttc agttcttcca tcatttgggg     420
gcccccattg acatgtggct attctacaaa taccgcaacg aaggagtctg gatctttgtc     480
ctgttgaatg ggttcattca ctggatcatg tacggttact attggacgcg gctcatcaag     540
ctgaacttcc ccatgcccaa gaacctgatc acctccatgc agatcatcca gttcaatgtc     600
gggttctaca tcgtctggaa gtaccgcaat gtgccatgct accgccagga tgggatgcgc     660
atgtttgcct ggatcttcaa ctactggtat gtcgggacgg tcttgctgct gttcctcaac     720
ttttacgtgc agacgtacat ccggaagccg aggaagaacc gagggaagaa ggag           774
```

<210> 40
<211> 258
<212> PRT
<213> Euglena anabaena

<400> 40

```
Met Glu Ala Ala Lys Glu Leu Val Ser Ile Val Gln Glu Glu Leu Pro
1               5                   10                  15

Lys Val Asp Tyr Ala Gln Leu Trp Gln Asp Ala Ser Ser Cys Glu Val
            20                  25                  30

Leu Tyr Leu Ser Val Ala Phe Val Ala Ile Lys Phe Met Leu Arg Pro
        35                  40                  45

Leu Asp Leu Lys Arg Gln Ala Thr Leu Lys Lys Leu Phe Thr Ala Tyr
    50                  55                  60

Asn Phe Leu Met Ser Ile Tyr Ser Phe Gly Ser Phe Leu Ala Met Ala
65                  70                  75                  80

Tyr Ala Leu Ser Val Thr Gly Ile Leu Ser Gly Asp Cys Glu Thr Ala
                85                  90                  95

Phe Asn Asn Asp Val Phe Arg Ile Thr Thr Gln Leu Phe Tyr Leu Ser
            100                 105                 110

Lys Phe Val Glu Tyr Ile Asp Ser Phe Tyr Leu Pro Leu Met Asp Lys
        115                 120                 125

Pro Leu Ser Phe Leu Gln Phe Phe His His Leu Gly Ala Pro Ile Asp
    130                 135                 140

Met Trp Leu Phe Tyr Lys Tyr Arg Asn Glu Gly Val Trp Ile Phe Val
145                 150                 155                 160

Leu Leu Asn Gly Phe Ile His Trp Ile Met Tyr Gly Tyr Tyr Trp Thr
```

```
                165                 170                 175

Arg Leu Ile Lys Leu Asn Phe Pro Met Pro Lys Asn Leu Ile Thr Ser
            180                 185                 190

Met Gln Ile Ile Gln Phe Asn Val Gly Phe Tyr Ile Val Trp Lys Tyr
        195                 200                 205

Arg Asn Val Pro Cys Tyr Arg Gln Asp Gly Met Arg Met Phe Ala Trp
    210                 215                 220

Ile Phe Asn Tyr Trp Tyr Val Gly Thr Val Leu Leu Leu Phe Leu Asn
225                 230                 235                 240

Phe Tyr Val Gln Thr Tyr Ile Arg Lys Pro Arg Lys Asn Arg Gly Lys
                245                 250                 255

Lys Glu
```

<210> 41
<211> 31
<212> DNA
<213> Artificial Sequence

<220>
<223> EaD9-5Bbs primer

<400> 41
gaagacacca tggaagcagc caaagaattg g 31

<210> 42
<211> 30
<212> DNA
<213> Artificial Sequence

<220>
<223> EaD9-3fusion primer

<400> 42
agctggacgg gcgggctcct tcttccctcg 30

<210> 43
<211> 30
<212> DNA
<213> Artificial Sequence

<220>
<223> EgDHAsyn1Link-5fusion primer

<400> 43
cgagggaaga aggagcccgc ccgtccagct 30

<210> 44
<211> 852
<212> DNA
<213> Artificial Sequence

<220>
<223> EaD9elo1-EgDHAsyn1Link

<400> 44

```
gaagacacca tggaagcagc caaagaattg gtttccatcg tccaagagga gctccccaag     60
gtggactatg cccagctttg gcaggatgcc agcagctgtg aggtccttta cctctcggtg    120
gcattcgtgg cgatcaagtt catgctgcgc ccactggacc tgaagcgcca ggccaccttg    180
aagaagctgt tcacagcata caacttcctc atgtcgatct attcctttgg ctccttcctg    240
gccatggcct atgccctatc agtaactggc atcctctccg gcgactgtga gacggcgttc    300
aacaacgatg tgttcaggat cacaactcag ctgttctacc tcagcaagtt cgtagagtac    360
atcgactcct tctaccttcc ccttatggac aagccactgt cgttccttca gttcttccat    420
catttggggg cccccattga catgtggcta ttctacaaat accgcaacga aggagtctgg    480
atctttgtcc tgttgaatgg gttcattcac tggatcatgt acggttacta ttggacgcgg    540
ctcatcaagc tgaacttccc catgcccaag aacctgatca cctccatgca gatcatccag    600
ttcaatgtcg ggttctacat cgtctggaag taccgcaatg tgccatgcta ccgccaggat    660
gggatgcgca tgtttgcctg gatcttcaac tactggtatg tcgggacggt cttgctgctg    720
ttcctcaact tttacgtgca gacgtacatc cggaagccga ggaagaaccg agggaagaag    780
gagcccgccc gtccagctgg actcccgccg gccacgtact acgactccct ggcagtgagc    840
ggccgcgaat tc                                                        852
```

<210> 45
<211> 5559
<212> DNA
<213> Artificial Sequence

<220>
<223> Plasmid pLF124

<400> 45

```
ggccgcaagt atgaactaaa atgcacgtag gtgtaagagc tcatggagag catggaatat     60
tgtatccgac catgtaacag tataataact gagctccatc tcacttcttc tatgaataaa    120
caaaggatgt tatgatatat taacactcta tctatgcacc ttattgttct atgataaatt    180
tcctcttatt attataaatc atctgaatcg tgacggctta tggaatgctt caaatagtac    240
aaaaacaaat gtgtactata agactttcta aacaattcta actttagcat tgtgaacgag    300
```

```
acataagtgt taagaagaca taacaattat aatggaagaa gtttgtctcc atttatatat    360
tatatattac ccacttatgt attatattag gatgttaagg agacataaca attataaaga    420
gagaagtttg tatccattta tatattatat actacccatt tatatattat acttatccac    480
ttatttaatg tctttataag gtttgatcca tgatatttct aatattttag ttgatatgta    540
tatgaaaggg tactatttga actctcttac tctgtataaa ggttggatca tccttaaagt    600
gggtctattt aattttattg cttcttacag ataaaaaaaa aattatgagt tggtttgata    660
aaatattgaa ggatttaaaa taataataaa taacatataa tatatgtata taaatttatt    720
ataatataac atttatctat aaaaaagtaa atattgtcat aaatctatac aatcgtttag    780
ccttgctgga cgaatctcaa ttatttaaac gagagtaaac atatttgact ttttggttat    840
ttaacaaatt attatttaac actatatgaa attttttttt ttatcagcaa agaataaaat    900
taaattaagg aggacaatgg tgtcccaatc cttatacaac caacttccac aagaaagtca    960
agtcagagac aacaaaaaaa caagcaaagg aaattttta atttgagttg tcttgtttgc   1020
tgcataattt atgcagtaaa acactacaca taaccctttt agcagtaaag caatggttga   1080
ccgtgtgctt agcttctttt attttatttt tttatcagca aagaataaat aaaataaaat   1140
gagacacttc agggatgttt caacggatcc cccgggctgc aggaattcga tatcaagctt   1200
atcgataccg tcgacctcga gggggggccc ggtacccaat tcgccctata gtgagtcgta   1260
ttacgcgcgc tcactggccg tcgttttaca acgtcgtgac tgggaaaacc ctggcgttac   1320
ccaacttaat cgccttgcag cacatccccc tttcgccagc tggcgtaata gcgaagaggc   1380
ccgcaccgat cgcccttccc aacagttgcg cagcctgaat ggcgaatggg acgcgccctg   1440
tagcggcgca ttaagcgcgg cgggtgtggt ggttacgcgc agcgtgaccg ctacacttgc   1500
cagcgcccta gcgcccgctc ctttcgcttt cttcccttcc tttctcgcca cgttcgccgg   1560
ctttccccgt caagctctaa atcgggggct ccctttaggg ttccgattta gtgctttacg   1620
gcacctcgac cccaaaaaac ttgattaggg tgatggttca cgtagtgggc catcgccctg   1680
atagacggtt tttcgccctt tgacgttgga gtccacgttc tttaatagtg gactcttgtt   1740
ccaaactgga acaacactca accctatctc ggtctattct tttgatttat aagggatttt   1800
gccgatttcg gcctattggt taaaaaatga gctgatttaa caaaaattta acgcgaattt   1860
taacaaaata ttaacgctta caatttaggt ggcacttttc ggggaaatgt gcgcggaacc   1920
cctatttgtt tatttttcta aatacattca aatatgtatc cgctcatgag acaataaccc   1980
tgataaatgc ttcaataata ttgaaaaagg aagagtatga gtattcaaca tttccgtgtc   2040
gcccttattc cctttttgc ggcattttgc cttcctgttt ttgctcaccc agaaacgctg   2100
gtgaaagtaa aagatgctga agatcagttg ggtgcacgag tgggttacat cgaactggat   2160
```

ctcaacagcg gtaagatcct tgagagtttt cgccccgaag aacgttttcc aatgatgagc 2220
acttttaaag ttctgctatg tggcgcggta ttatcccgta ttgacgccgg gcaagagcaa 2280
ctcggtcgcc gcatacacta ttctcagaat gacttggttg agtactcacc agtcacagaa 2340
aagcatctta cggatggcat gacagtaaga gaattatgca gtgctgccat aaccatgagt 2400
gataacactg cggccaactt acttctgaca acgatcggag gaccgaagga gctaaccgct 2460
tttttgcaca acatggggga tcatgtaact cgccttgatc gttgggaacc ggagctgaat 2520
gaagccatac caaacgacga gcgtgacacc acgatgcctg tagcaatggc aacaacgttg 2580
cgcaaactat taactggcga actacttact ctagcttccc ggcaacaatt aatagactgg 2640
atggaggcgg ataaagttgc aggaccactt ctgcgctcgg cccttccggc tggctggttt 2700
attgctgata aatctggagc cggtgagcgt gggtctcgcg gtatcattgc agcactgggg 2760
ccagatggta agccctcccg tatcgtagtt atctacacga cggggagtca ggcaactatg 2820
gatgaacgaa atagacagat cgctgagata ggtgcctcac tgattaagca ttggtaactg 2880
tcagaccaag tttactcata tatactttag attgatttaa aacttcattt ttaatttaaa 2940
aggatctagg tgaagatcct ttttgataat ctcatgacca aaatccctta acgtgagttt 3000
tcgttccact gagcgtcaga ccccgtagaa aagatcaaag gatcttcttg agatcctttt 3060
tttctgcgcg taatctgctg cttgcaaaca aaaaaaccac cgctaccagc ggtggtttgt 3120
ttgccggatc aagagctacc aactcttttt ccgaaggtaa ctggcttcag cagagcgcag 3180
ataccaaata ctgtccttct agtgtagccg tagttaggcc accacttcaa gaactctgta 3240
gcaccgccta catacctcgc tctgctaatc ctgttaccag tggctgctgc cagtggcgat 3300
aagtcgtgtc ttaccgggtt ggactcaaga cgatagttac cggataaggc gcagcggtcg 3360
ggctgaacgg ggggttcgtg cacacagccc agcttggagc gaacgaccta caccgaactg 3420
agatacctac agcgtgagct atgagaaagc gccacgcttc ccgaagggag aaaggcggac 3480
aggtatccgg taagcggcag ggtcggaaca ggagagcgca cgagggagct tccaggggga 3540
aacgcctggt atctttatag tcctgtcggg tttcgccacc tctgacttga gcgtcgattt 3600
ttgtgatgct cgtcaggggg gcggagccta tggaaaaacg ccagcaacgc ggccttttta 3660
cggttcctgg ccttttgctg gccttttgct cacatgttct ttcctgcgtt atcccctgat 3720
tctgtggata accgtattac cgcctttgag tgagctgata ccgctcgccg cagccgaacg 3780
accgagcgca gcgagtcagt gagcgaggaa gcggaagagc gcccaatacg caaaccgcct 3840
ctccccgcgc gttggccgat tcattaatgc agctggcacg acaggtttcc cgactggaaa 3900
gcgggcagtg agcgcaacgc aattaatgtg agttagctca ctcattaggc accccaggct 3960

```
ttacacttta tgcttccggc tcgtatgttg tgtggaattg tgagcggata acaatttcac   4020
acaggaaaca gctatgacca tgattacgcc aagcgcgcaa ttaaccctca ctaaagggaa   4080
caaaagctgg agctccaccc tagaactagt ggatccttca tccatgccct tcatttgccg   4140
cttattaatt aatttggtaa cagtccgtac taatcagtta cttatccttc ccccatcata   4200
attaatcttg gtagtctcga atgccacaac actgactagt ctcttggatc ataagaaaaa   4260
gccaaggaac aaaagaagac aaaacacaat gagagtatcc tttgcatagc aatgtctaag   4320
ttcataaaat tcaaacaaaa acgcaatcac acacagtgga catcacttat ccactagctg   4380
atcaggatcg ccgcgtcaag aaaaaaaaac tggaccccaa aagccatgca caacaacacg   4440
tactcacaaa ggtgtcaatc gagcagccca aaacattcac caactcaacc catcatgagc   4500
cctcacattt gttgtttcta acccaacctc aaactcgtat tctcttccgc cacctcattt   4560
ttgtttattt caacacccgt caaactgcat gccaccccgt ggccaaatgt ccatgcatgt   4620
taacaagacc tatgactata aatagctgca atctcggccc aggttttcat catcaagaac   4680
cagttcaata tcctagtaca ccgtattaaa gaatttaaga tatactccat ggaagcagcc   4740
aaagaattgg tttccatcgt ccaagaggag ctccccaagg tggactatgc ccagctttgg   4800
caggatgcca gcagctgtga ggtcctttac ctctcggtgg cattcgtggc gatcaagttc   4860
atgctgcgcc cactggacct gaagcgccag gccaccttga agaagctgtt cacagcatac   4920
aacttcctca tgtcgatcta ttcctttggc tccttcctgg ccatggccta tgccctatca   4980
gtaactggca tcctctccgg cgactgtgag acggcgttca acaacgatgt gttcaggatc   5040
acaactcagc tgttctacct cagcaagttc gtagagtaca tcgactcctt ctaccttccc   5100
cttatggaca agccactgtc gttccttcag ttcttccatc atttgggggc ccccattgac   5160
atgtggctat tctacaaata ccgcaacgaa ggagtctgga tctttgtcct gttgaatggg   5220
ttcattcact ggatcatgta cggttactat tggacgcggc tcatcaagct gaacttcccc   5280
atgcccaaga acctgatcac ctccatgcag atcatccagt tcaatgtcgg gttctacatc   5340
gtctggaagt accgcaatgt gccatgctac cgccaggatg ggatgcgcat gtttgcctgg   5400
atcttcaact actggtatgt cgggacggtc ttgctgctgt tcctcaactt ttacgtgcag   5460
acgtacatcc ggaagccgag gaagaaccga gggaagaagg agcccgcccg tccagctgga   5520
ctcccgccgg ccacgtacta cgactccctg gcagtgagc                          5559
```

<210> 46
<211> 5559
<212> DNA
<213> Artificial Sequence

<220>
<223> Plasmid pKR1177

<400> 46

ggccgcaagt atgaactaaa atgcacgtag gtgtaagagc tcatggagag catggaatat 60
tgtatccgac catgtaacag tataataact gagctccatc tcacttcttc tatgaataaa 120
caaaggatgt tatgatatat taacactcta tctatgcacc ttattgttct atgataaatt 180
tcctcttatt attataaatc atctgaatcg tgacggctta tggaatgctt caaatagtac 240
aaaaacaaat gtgtactata agactttcta aacaattcta actttagcat tgtgaacgag 300
acataagtgt taagaagaca taacaattat aatggaagaa gtttgtctcc atttatatat 360
tatatattac ccacttatgt attatattag gatgttaagg agacataaca attataaaga 420
gagaagtttg tatccattta tatattatat actacccatt tatatattat acttatccac 480
ttatttaatg tctttataag gtttgatcca tgatatttct aatattttag ttgatatgta 540
tatgaaaggg tactatttga actctcttac tctgtataaa ggttggatca tccttaaagt 600
gggtctattt aattttattg cttcttacag ataaaaaaaa aattatgagt tggtttgata 660
aaatattgaa ggatttaaaa taataataaa taacatataa tatatgtata taaatttatt 720
ataatataac atttatctat aaaaaagtaa atattgtcat aaatctatac aatcgtttag 780
ccttgctgga cgaatctcaa ttatttaaac gagagtaaac atatttgact ttttggttat 840
ttaacaaatt attatttaac actatatgaa attttttttt ttatcagcaa agaataaaat 900
taaattaagg aggacaatgg tgtcccaatc cttatacaac caacttccac aagaaagtca 960
agtcagagac aacaaaaaaa caagcaaagg aaatttttta atttgagttg tcttgtttgc 1020
tgcataattt atgcagtaaa acactacaca taaccctttt agcagtaaag caatggttga 1080
ccgtgtgctt agcttctttt attttatttt tttatcagca aagaataaat aaaataaaat 1140
gagacacttc agggatgttt caacggatcc cccgggctgc aggaattcga tatcaagctt 1200
atcgataccg tcgacctcga gggggggccc ggtacccaat tcgccctata gtgagtcgta 1260
ttacgcgcgc tcactggccg tcgttttaca acgtcgtgac tgggaaaacc ctggcgttac 1320
ccaacttaat cgccttgcag cacatccccc tttcgccagc tggcgtaata gcgaagaggc 1380
ccgcaccgat cgcccttccc aacagttgcg cagcctgaat ggcgaatggg acgcgccctg 1440
tagcggcgca ttaagcgcgg cgggtgtggt ggttacgcgc agcgtgaccg ctacacttgc 1500
cagcgcccta gcgcccgctc ctttcgcttt cttcccttcc tttctcgcca cgttcgccgg 1560
ctttccccgt caagctctaa atcgggggct ccctttaggg ttccgattta gtgctttacg 1620
gcacctcgac cccaaaaaac ttgattaggg tgatggttca cgtagtgggc catcgccctg 1680
atagacggtt tttcgccctt tgacgttgga gtccacgttc tttaatagtg gactcttgtt 1740

ccaaactgga acaacactca accctatctc ggtctattct tttgatttat aagggatttt 1800

gccgatttcg gcctattggt taaaaaatga gctgatttaa caaaaattta acgcgaattt 1860

taacaaaata ttaacgctta caatttaggt ggcacttttc ggggaaatgt gcgcggaacc 1920

cctatttgtt tatttttcta aatacattca aatatgtatc cgctcatgag acaataaccc 1980

tgataaatgc ttcaataata ttgaaaaagg aagagtatga gtattcaaca tttccgtgtc 2040

gcccttattc ccttttttgc ggcattttgc cttcctgttt ttgctcaccc agaaacgctg 2100

gtgaaagtaa aagatgctga agatcagttg ggtgcacgag tgggttacat cgaactggat 2160

ctcaacagcg gtaagatcct tgagagtttt cgccccgaag aacgttttcc aatgatgagc 2220

acttttaaag ttctgctatg tggcgcggta ttatcccgta ttgacgccgg gcaagagcaa 2280

ctcggtcgcc gcatacacta ttctcagaat gacttggttg agtactcacc agtcacagaa 2340

aagcatctta cggatggcat gacagtaaga gaattatgca gtgctgccat aaccatgagt 2400

gataacactg cggccaactt acttctgaca acgatcggag gaccgaagga gctaaccgct 2460

tttttgcaca acatggggga tcatgtaact cgccttgatc gttgggaacc ggagctgaat 2520

gaagccatac caaacgacga gcgtgacacc acgatgcctg tagcaatggc aacaacgttg 2580

cgcaaactat taactggcga actacttact ctagcttccc ggcaacaatt aatagactgg 2640

atggaggcgg ataaagttgc aggaccactt ctgcgctcgg cccttccggc tggctggttt 2700

attgctgata aatctggagc cggtgagcgt gggtctcgcg gtatcattgc agcactgggg 2760

ccagatggta agccctcccg tatcgtagtt atctacacga cggggagtca ggcaactatg 2820

gatgaacgaa atagacagat cgctgagata ggtgcctcac tgattaagca ttggtaactg 2880

tcagaccaag tttactcata tatactttag attgatttaa aacttcattt ttaatttaaa 2940

aggatctagg tgaagatcct ttttgataat ctcatgacca aaatccctta acgtgagttt 3000

tcgttccact gagcgtcaga ccccgtagaa aagatcaaag gatcttcttg agatcctttt 3060

tttctgcgcg taatctgctg cttgcaaaca aaaaaaccac cgctaccagc ggtggtttgt 3120

ttgccggatc aagagctacc aactcttttt ccgaaggtaa ctggcttcag cagagcgcag 3180

ataccaaata ctgtccttct agtgtagccg tagttaggcc accacttcaa gaactctgta 3240

gcaccgccta catacctcgc tctgctaatc ctgttaccag tggctgctgc cagtggcgat 3300

aagtcgtgtc ttaccgggtt ggactcaaga cgatagttac cggataaggc gcagcggtcg 3360

ggctgaacgg ggggttcgtg cacacagccc agcttggagc gaacgaccta caccgaactg 3420

agatacctac agcgtgagct atgagaaagc gccacgcttc ccgaagggag aaaggcggac 3480

aggtatccgg taagcggcag ggtcggaaca ggagagcgca cgagggagct tccaggggga 3540

aacgcctggt atctttatag tcctgtcggg tttcgccacc tctgacttga gcgtcgattt 3600

ttgtgatgct cgtcaggggg gcggagccta tggaaaaacg ccagcaacgc ggccttttta 3660
cggttcctgg ccttttgctg gccttttgct cacatgttct ttcctgcgtt atcccctgat 3720
tctgtggata accgtattac cgcctttgag tgagctgata ccgctcgccg cagccgaacg 3780
accgagcgca gcgagtcagt gagcgaggaa gcggaagagc gcccaatacg caaaccgcct 3840
ctccccgcgc gttggccgat tcattaatgc agctggcacg acaggtttcc cgactggaaa 3900
gcgggcagtg agcgcaacgc aattaatgtg agttagctca ctcattaggc accccaggct 3960
ttacacttta tgcttccggc tcgtatgttg tgtggaattg tgagcggata acaatttcac 4020
acaggaaaca gctatgacca tgattacgcc aagcgcgcaa ttaaccctca ctaaagggaa 4080
caaaagctgg agctccaccc tagaactagt ggatccttca tccatgccct tcatttgccg 4140
cttattaatt aatttggtaa cagtccgtac taatcagtta cttatccttc ccccatcata 4200
attaatcttg gtagtctcga atgccacaac actgactagt ctcttggatc ataagaaaaa 4260
gccaaggaac aaaagaagac aaaacacaat gagagtatcc tttgcatagc aatgtctaag 4320
ttcataaaat tcaaacaaaa acgcaatcac acacagtgga catcacttat ccactagctg 4380
atcaggatcg ccgcgtcaag aaaaaaaaac tggaccccaa aagccatgca caacaacacg 4440
tactcacaaa ggtgtcaatc gagcagccca aaacattcac caactcaacc catcatgagc 4500
cctcacattt gttgtttcta acccaacctc aaactcgtat tctcttccgc cacctcattt 4560
ttgtttattt caacacccgt caaactgcat gccaccccgt ggccaaatgt ccatgcatgt 4620
taacaagacc tatgactata aatagctgca atctcggccc aggttttcat catcaagaac 4680
cagttcaata tcctagtaca ccgtattaaa gaatttaaga tatactccat ggaagcagcc 4740
aaagaattgg tttccatcgt ccaagaggag ctccccaagg tggactatgc ccagctttgg 4800
caggatgcca gcagctgtga ggtcctttac ctctcggtgg cattcgtggc gatcaagttc 4860
atgctgcgcc cactggacct gaagcgccag gccaccttga agaagctgtt cacagcatac 4920
aacttcctca tgtcgatcta ttcctttggc tccttcctgg ccatggccta tgccctatca 4980
gtaactggca tcctctccgg cgactgtgag acggcgttca acaacgatgt gttcaggatc 5040
acaactcagc tgttctacct cagcaagttc gtagagtaca tcgactcctt ctaccttccc 5100
cttatggaca agccactgtc gttccttcag ttcttccatc atttgggggc ccccattgac 5160
atgtggctat tctacaaata ccgcaacgaa ggagtctgga tctttgtcct gttgaatggg 5220
ttcattcact ggatcatgta cggttactat tggacgcggc tcatcaagct gaacttcccc 5280
atgcccaaga acctgatcac ctccatgcag atcatccagt tcaatgtcgg gttctacatc 5340
gtctggaagt accgcaatgt gccatgctac cgccaggatg ggatgcgcat gtttgcctgg 5400

atcttcaact actggtatgt cgggacggtc ttgctgctgt tcctcaactt ttacgtgcag 5460

acgtacatcc ggaagccgag gaagaaccga gggaagaagg agcccgcccg tccagctgga 5520

ctcccgccgg ccacgtacta cgactccctg gcagtgagc 5559

<210> 47
<211> 7916
<212> DNA
<213> Artificial Sequence

<220>
<223> Plasmid pKR1179

<400> 47

gatccgtcga cggcgcgccc gatcatccgg atatagttcc tcctttcagc aaaaaacccc 60

tcaagacccg tttagaggcc ccaaggggtt atgctagtta ttgctcagcg gtggcagcag 120

ccaactcagc ttcctttcgg gctttgttag cagccggatc gatccaagct gtacctcact 180

attcctttgc cctcggacga gtgctggggc gtcggtttcc actatcggcg agtacttcta 240

cacagccatc ggtccagacg gccgcgcttc tgcgggcgat ttgtgtacgc ccgacagtcc 300

cggctccgga tcggacgatt gcgtcgcatc gaccctgcgc ccaagctgca tcatcgaaat 360

tgccgtcaac caagctctga tagagttggt caagaccaat gcggagcata tacgcccgga 420

gccgcggcga tcctgcaagc tccggatgcc tccgctcgaa gtagcgcgtc tgctgctcca 480

tacaagccaa ccacggcctc cagaagaaga tgttggcgac ctcgtattgg gaatccccga 540

acatcgcctc gctccagtca atgaccgctg ttatgcggcc attgtccgtc aggacattgt 600

tggagccgaa atccgcgtgc acgaggtgcc ggacttcggg gcagtcctcg gcccaaagca 660

tcagctcatc gagagcctgc gcgacggacg cactgacggt gtcgtccatc acagtttgcc 720

agtgatacac atggggatca gcaatcgcgc atatgaaatc acgccatgta gtgtattgac 780

cgattccttg cggtccgaat gggccgaacc cgctcgtctg gctaagatcg gccgcagcga 840

tcgcatccat agcctccgcg accggctgca gaacagcggg cagttcggtt tcaggcaggt 900

cttgcaacgt gacaccctgt gcacggcggg agatgcaata ggtcaggctc tcgctgaatt 960

ccccaatgtc aagcacttcc ggaatcggga gcgcggccga tgcaaagtgc cgataaacat 1020

aacgatcttt gtagaaacca tcggcgcagc tatttacccg caggacatat ccacgccctc 1080

ctacatcgaa gctgaaagca cgagattctt cgccctccga gagctgcatc aggtcggaga 1140

cgctgtcgaa cttttcgatc agaaacttct cgacagacgt cgcggtgagt tcaggctttt 1200

ccatgggtat atctccttct taaagttaaa caaaattatt tctagaggga aaccgttgtg 1260

gtctccctat agtgagtcgt attaatttcg cgggatcgag atcgatccaa ttccaatccc 1320

acaaaaatct gagcttaaca gcacagttgc tcctctcaga gcagaatcgg gtattcaaca 1380

ccctcatatc aactactacg ttgtgtataa cggtccacat gccggtatat acgatgactg 1440
gggttgtaca aaggcggcaa caaacggcgt tcccggagtt gcacacaaga aatttgccac 1500
tattacagag gcaagagcag cagctgacgc gtacacaaca agtcagcaaa cagacaggtt 1560
gaacttcatc cccaaaggag aagctcaact caagcccaag agctttgcta aggccctaac 1620
aagcccacca aagcaaaaag cccactggct cacgctagga accaaaaggc ccagcagtga 1680
tccagcccca aaagagatct cctttgcccc ggagattaca atggacgatt tcctctatct 1740
ttacgatcta ggaaggaagt tcgaaggtga aggtgacgac actatgttca ccactgataa 1800
tgagaaggtt agcctcttca atttcagaaa gaatgctgac ccacagatgg ttagagaggc 1860
ctacgcagca ggtctcatca agacgatcta cccgagtaac aatctccagg agatcaaata 1920
ccttcccaag aaggttaaag atgcagtcaa aagattcagg actaattgca tcaagaacac 1980
agagaaagac atatttctca agatcagaag tactattcca gtatggacga ttcaaggctt 2040
gcttcataaa ccaaggcaag taatagagat tggagtctct aaaaaggtag ttcctactga 2100
atctaaggcc atgcatggag tctaagattc aaatcgagga tctaacagaa ctcgccgtga 2160
agactggcga acagttcata cagagtcttt tacgactcaa tgacaagaag aaaatcttcg 2220
tcaacatggt ggagcacgac actctggtct actccaaaaa tgtcaaagat acagtctcag 2280
aagaccaaag ggctattgag acttttcaac aaaggataat ttcgggaaac ctcctcggat 2340
tccattgccc agctatctgt cacttcatcg aaaggacagt agaaaaggaa ggtggctcct 2400
acaaatgcca tcattgcgat aaaggaaagg ctatcattca agatgcctct gccgacagtg 2460
gtcccaaaga tggaccccca cccacgagga gcatcgtgga aaaagaagac gttccaacca 2520
cgtcttcaaa gcaagtggat tgatgtgaca tctccactga cgtaagggat gacgcacaat 2580
cccactatcc ttcgcaagac ccttcctcta tataaggaag ttcatttcat ttggagagga 2640
cacgctcgag ctcatttctc tattacttca gccataacaa aagaactctt ttctcttctt 2700
attaaaccat gaaaaagcct gaactcaccg cgacgtctgt cgagaagttt ctgatcgaaa 2760
agttcgacag cgtctccgac ctgatgcagc tctcggaggg cgaagaatct cgtgctttca 2820
gcttcgatgt aggagggcgt ggatatgtcc tgcgggtaaa tagctgcgcc gatggtttct 2880
acaaagatcg ttatgtttat cggcactttg catcggccgc gctcccgatt ccggaagtgc 2940
ttgacattgg ggaattcagc gagagcctga cctattgcat ctcccgccgt gcacagggtg 3000
tcacgttgca agacctgcct gaaaccgaac tgcccgctgt tctgcagccg gtcgcggagg 3060
ccatggatgc gatcgctgcg gccgatctta gccagacgag cgggttcggc ccattcggac 3120
cgcaaggaat cggtcaatac actacatggc gtgatttcat atgcgcgatt gctgatcccc 3180

atgtgtatca ctggcaaact gtgatggacg acaccgtcag tgcgtccgtc gcgcaggctc 3240
tcgatgagct gatgctttgg gccgaggact gccccgaagt ccggcacctc gtgcacgcgg 3300
atttcggctc caacaatgtc ctgacggaca atggccgcat aacagcggtc attgactgga 3360
gcgaggcgat gttcggggat tcccaatacg aggtcgccaa catcttcttc tggaggccgt 3420
ggttggcttg tatggagcag cagacgcgct acttcgagcg gaggcatccg gagcttgcag 3480
gatcgccgcg gctccgggcg tatatgctcc gcattggtct tgaccaactc tatcagagct 3540
tggttgacgg caatttcgat gatgcagctt gggcgcaggg tcgatgcgac gcaatcgtcc 3600
gatccggagc cgggactgtc gggcgtacac aaatcgcccg cagaagcgcg gccgtctgga 3660
ccgatggctg tgtagaagta ctcgccgata gtggaaaccg acgccccagc actcgtccga 3720
gggcaaagga atagtgaggt acctaaagaa ggagtgcgtc gaagcagatc gttcaaacat 3780
ttggcaataa agtttcttaa gattgaatcc tgttgccggt cttgcgatga ttatcatata 3840
atttctgttg aattacgtta agcatgtaat aattaacatg taatgcatga cgttatttat 3900
gagatgggtt tttatgatta gagtcccgca attatacatt taatacgcga tagaaaacaa 3960
aatatagcgc gcaaactagg ataaattatc gcgcgcggtg tcatctatgt tactagatcg 4020
atgtcgaatc gatcaacctg cattaatgaa tcggccaacg cgcggggaga ggcggtttgc 4080
gtattgggcg ctcttccgct tcctcgctca ctgactcgct gcgctcggtc gttcggctgc 4140
ggcgagcggt atcagctcac tcaaaggcgg taatacggtt atccacagaa tcaggggata 4200
acgcaggaaa gaacatgtga gcaaaaggcc agcaaaaggc caggaaccgt aaaaaggccg 4260
cgttgctggc gtttttccat aggctccgcc cccctgacga gcatcacaaa aatcgacgct 4320
caagtcagag gtggcgaaac ccgacaggac tataaagata ccaggcgttt ccccctggaa 4380
gctccctcgt gcgctctcct gttccgaccc tgccgcttac cggatacctg tccgcctttc 4440
tcccttcggg aagcgtggcg ctttctcaat gctcacgctg taggtatctc agttcggtgt 4500
aggtcgttcg ctccaagctg ggctgtgtgc acgaaccccc cgttcagccc gaccgctgcg 4560
ccttatccgg taactatcgt cttgagtcca acccggtaag acacgactta tcgccactgg 4620
cagcagccac tggtaacagg attagcagag cgaggtatgt aggcggtgct acagagttct 4680
tgaagtggtg gcctaactac ggctacacta gaaggacagt atttggtatc tgcgctctgc 4740
tgaagccagt taccttcgga aaaagagttg gtagctcttg atccggcaaa caaaccaccg 4800
ctggtagcgg tggttttttt gtttgcaagc agcagattac gcgcagaaaa aaaggatctc 4860
aagaagatcc tttgatcttt tctacggggt ctgacgctca gtggaacgaa aactcacgtt 4920
aagggatttt ggtcatgaca ttaacctata aaaataggcg tatcacgagg ccctttcgtc 4980
tcgcgcgttt cggtgatgac ggtgaaaacc tctgacacat gcagctcccg gagacggtca 5040

cagcttgtct gtaagcggat gccgggagca gacaagcccg tcagggcgcg tcagcgggtg 5100

ttggcgggtg tcggggctgg cttaactatg cggcatcaga gcagattgta ctgagagtgc 5160

accatatgga catattgtcg ttagaacgcg gctacaatta atacataacc ttatgtatca 5220

tacacatacg atttaggtga cactatagaa cggcgcgcca agcttggatc tcctgcagga 5280

tctggccggc cggatctcgt acggatcctt catccatgcc cttcatttgc cgcttattaa 5340

ttaatttggt aacagtccgt actaatcagt tacttatcct tcccccatca taattaatct 5400

tggtagtctc gaatgccaca acactgacta gtctcttgga tcataagaaa aagccaagga 5460

acaaaagaag acaaaacaca atgagagtat cctttgcata gcaatgtcta agttcataaa 5520

attcaaacaa aaacgcaatc acacacagtg gacatcactt atccactagc tgatcaggat 5580

cgccgcgtca agaaaaaaaa actggacccc aaaagccatg cacaacaaca cgtactcaca 5640

aaggtgtcaa tcgagcagcc caaaacattc accaactcaa cccatcatga gccctcacat 5700

ttgttgtttc taacccaacc tcaaactcgt attctcttcc gccacctcat ttttgtttat 5760

ttcaacaccc gtcaaactgc atgccacccc gtggccaaat gtccatgcat gttaacaaga 5820

cctatgacta taaatagctg caatctcggc ccaggttttc atcatcaaga accagttcaa 5880

tatcctagta caccgtatta aagaatttaa gatatactcc atggaagcag ccaaagaatt 5940

ggtttccatc gtccaagagg agctccccaa ggtggactat gcccagcttt ggcaggatgc 6000

cagcagctgt gaggtccttt acctctcggt ggcattcgtg gcgatcaagt tcatgctgcg 6060

cccactggac ctgaagcgcc aggccacctt gaagaagctg ttcacagcat acaacttcct 6120

catgtcgatc tattcctttg gctccttcct ggccatggcc tatgccctat cagtaactgg 6180

catcctctcc ggcgactgtg agacggcgtt caacaacgat gtgttcagga tcacaactca 6240

gctgttctac ctcagcaagt tcgtagagta catcgactcc ttctaccttc cccttatgga 6300

caagccactg tcgttccttc agttcttcca tcatttgggg gcccccattg acatgtggct 6360

attctacaaa taccgcaacg aaggagtctg gatctttgtc ctgttgaatg ggttcattca 6420

ctggatcatg tacggttact attggacgcg gctcatcaag ctgaacttcc ccatgcccaa 6480

gaacctgatc acctccatgc agatcatcca gttcaatgtc gggttctaca tcgtctggaa 6540

gtaccgcaat gtgccatgct accgccagga tgggatgcgc atgtttgcct ggatcttcaa 6600

ctactggtat gtcgggacgg tcttgctgct gttcctcaac ttttacgtgc agacgtacat 6660

ccggaagccg aggaagaacc gagggaagaa ggagcccgcc cgtccagctg gactcccgcc 6720

ggccacgtac tacgactccc tggcagtgag cggccgcaag tatgaactaa aatgcacgta 6780

ggtgtaagag ctcatggaga gcatggaata ttgtatccga ccatgtaaca gtataataac 6840

```
tgagctccat ctcacttctt ctatgaataa acaaaggatg ttatgatata ttaacactct   6900
atctatgcac cttattgttc tatgataaat ttcctcttat tattataaat catctgaatc   6960
gtgacggctt atggaatgct tcaaatagta caaaaacaaa tgtgtactat aagactttct   7020
aaacaattct aactttagca ttgtgaacga gacataagtg ttaagaagac ataacaatta   7080
taatggaaga agtttgtctc catttatata ttatatatta cccacttatg tattatatta   7140
ggatgttaag gagacataac aattataaag agagaagttt gtatccattt atatattata   7200
tactacccat ttatatatta tacttatcca cttatttaat gtctttataa ggtttgatcc   7260
atgatatttc taatatttta gttgatatgt atatgaaagg gtactatttg aactctctta   7320
ctctgtataa aggttggatc atccttaaag tgggtctatt taattttatt gcttcttaca   7380
gataaaaaaa aaattatgag ttggtttgat aaaatattga aggatttaaa ataataataa   7440
ataacatata atatatgtat ataaatttat tataatataa catttatcta taaaaaagta   7500
aatattgtca taaatctata caatcgttta gccttgctgg acgaatctca attatttaaa   7560
cgagagtaaa catatttgac tttttggtta tttaacaaat tattatttaa cactatatga   7620
aatttttttt tttatcagca aagaataaaa ttaaattaag gaggacaatg gtgtcccaat   7680
ccttatacaa ccaacttcca caagaaagtc aagtcagaga caacaaaaaa acaagcaaag   7740
gaaatttttt aatttgagtt gtcttgtttg ctgcataatt tatgcagtaa aacactacac   7800
ataacccttt tagcagtaaa gcaatggttg accgtgtgct tagcttcttt tattttattt   7860
ttttatcagc aaagaataaa taaaataaaa tgagacactt cagggatgtt tcaacg       7916
```

<210> 48
<211> 9190
<212> DNA
<213> Artificial Sequence

<220>
<223> Plasmid pKR1183

<400> 48

ggccgcaagt atgaactaaa atgcacgtag gtgtaagagc tcatggagag catggaatat 60

tgtatccgac catgtaacag tataataact gagctccatc tcacttcttc tatgaataaa 120

caaaggatgt tatgatatat taacactcta tctatgcacc ttattgttct atgataaatt 180

tcctcttatt attataaatc atctgaatcg tgacggctta tggaatgctt caaatagtac 240

aaaaacaaat gtgtactata agactttcta aacaattcta actttagcat tgtgaacgag 300

acataagtgt taagaagaca taacaattat aatggaagaa gtttgtctcc atttatatat 360

tatatattac ccacttatgt attatattag gatgttaagg agacataaca attataaaga 420

gagaagtttg tatccattta tatattatat actacccatt tatatattat acttatccac 480

ttatttaatg tctttataag gtttgatcca tgatatttct aatattttag ttgatatgta 540
tatgaaaggg tactatttga actctcttac tctgtataaa ggttggatca tccttaaagt 600
gggtctattt aattttattg cttcttacag ataaaaaaaa aattatgagt tggtttgata 660
aaatattgaa ggatttaaaa taataataaa taacatataa tatatgtata taaatttatt 720
ataatataac atttatctat aaaaaagtaa atattgtcat aaatctatac aatcgtttag 780
ccttgctgga cgaatctcaa ttatttaaac gagagtaaac atatttgact ttttggttat 840
ttaacaaatt attatttaac actatatgaa attttttttt ttatcagcaa agaataaaat 900
taaattaagg aggacaatgg tgtcccaatc cttatacaac caacttccac aagaaagtca 960
agtcagagac aacaaaaaaa caagcaaagg aaattttta atttgagttg tcttgtttgc 1020
tgcataattt atgcagtaaa acactacaca taacccttttt agcagtaaag caatggttga 1080
ccgtgtgctt agcttctttt attttatttt tttatcagca aagaataaat aaaataaaat 1140
gagacacttc agggatgttt caacggatcc gtcgacggcg cgcccgatca tccggatata 1200
gttcctcctt tcagcaaaaa acccctcaag acccgtttag aggccccaag gggttatgct 1260
agttattgct cagcggtggc agcagccaac tcagcttcct ttcgggcttt gttagcagcc 1320
ggatcgatcc aagctgtacc tcactattcc tttgccctcg gacgagtgct ggggcgtcgg 1380
tttccactat cggcgagtac ttctacacag ccatcggtcc agacggccgc gcttctgcgg 1440
gcgatttgtg tacgcccgac agtcccggct ccggatcgga cgattgcgtc gcatcgaccc 1500
tgcgcccaag ctgcatcatc gaaattgccg tcaaccaagc tctgatagag ttggtcaaga 1560
ccaatgcgga gcatatacgc ccggagccgc ggcgatcctg caagctccgg atgcctccgc 1620
tcgaagtagc gcgtctgctg ctccatacaa gccaaccacg gcctccagaa gaagatgttg 1680
gcgacctcgt attgggaatc cccgaacatc gcctcgctcc agtcaatgac cgctgttatg 1740
cggccattgt ccgtcaggac attgttggag ccgaaatccg cgtgcacgag gtgccggact 1800
tcggggcagt cctcggccca aagcatcagc tcatcgagag cctgcgcgac ggacgcactg 1860
acggtgtcgt ccatcacagt ttgccagtga tacacatggg gatcagcaat cgcgcatatg 1920
aaatcacgcc atgtagtgta ttgaccgatt ccttgcggtc cgaatgggcc gaacccgctc 1980
gtctggctaa gatcggccgc agcgatcgca tccatagcct ccgcgaccgg ctgcagaaca 2040
gcgggcagtt cggtttcagg caggtcttgc aacgtgacac cctgtgcacg gcgggagatg 2100
caataggtca ggctctcgct gaattcccca atgtcaagca cttccggaat cgggagcgcg 2160
gccgatgcaa agtgccgata aacataacga tctttgtaga aaccatcggc gcagctattt 2220
acccgcagga catatccacg ccctcctaca tcgaagctga aagcacgaga ttcttcgccc 2280

tccgagagct gcatcaggtc ggagacgctg tcgaactttt cgatcagaaa cttctcgaca 2340
gacgtcgcgg tgagttcagg cttttccatg ggtatatctc cttcttaaag ttaaacaaaa 2400
ttatttctag agggaaaccg ttgtggtctc cctatagtga gtcgtattaa tttcgcggga 2460
tcgagatcga tccaattcca atcccacaaa aatctgagct taacagcaca gttgctcctc 2520
tcagagcaga atcgggtatt caacaccctc atatcaacta ctacgttgtg tataacggtc 2580
cacatgccgg tatatacgat gactggggtt gtacaaaggc ggcaacaaac ggcgttcccg 2640
gagttgcaca caagaaattt gccactatta cagaggcaag agcagcagct gacgcgtaca 2700
caacaagtca gcaaacagac aggttgaact tcatccccaa aggagaagct caactcaagc 2760
ccaagagctt tgctaaggcc ctaacaagcc caccaaagca aaaagcccac tggctcacgc 2820
taggaaccaa aaggcccagc agtgatccag ccccaaaaga gatctccttt gccccggaga 2880
ttacaatgga cgatttcctc tatctttacg atctaggaag gaagttcgaa ggtgaaggtg 2940
acgacactat gttcaccact gataatgaga aggttagcct cttcaatttc agaaagaatg 3000
ctgacccaca gatggttaga gaggcctacg cagcaggtct catcaagacg atctacccga 3060
gtaacaatct ccaggagatc aaataccttc ccaagaaggt taaagatgca gtcaaaagat 3120
tcaggactaa ttgcatcaag aacacagaga aagacatatt tctcaagatc agaagtacta 3180
ttccagtatg gacgattcaa ggcttgcttc ataaaccaag gcaagtaata gagattggag 3240
tctctaaaaa ggtagttcct actgaatcta aggccatgca tggagtctaa gattcaaatc 3300
gaggatctaa cagaactcgc cgtgaagact ggcgaacagt tcatacagag tcttttacga 3360
ctcaatgaca agaagaaaat cttcgtcaac atggtggagc acgacactct ggtctactcc 3420
aaaaatgtca aagatacagt ctcagaagac caaagggcta ttgagacttt tcaacaaagg 3480
ataatttcgg gaaacctcct cggattccat tgcccagcta tctgtcactt catcgaaagg 3540
acagtagaaa aggaaggtgg ctcctacaaa tgccatcatt gcgataaagg aaaggctatc 3600
attcaagatg cctctgccga cagtggtccc aaagatggac ccccacccac gaggagcatc 3660
gtggaaaaag aagacgttcc aaccacgtct tcaaagcaag tggattgatg tgacatctcc 3720
actgacgtaa gggatgacgc acaatcccac tatccttcgc aagacccttc ctctatataa 3780
ggaagttcat ttcatttgga gaggacacgc tcgagctcat ttctctatta cttcagccat 3840
aacaaaagaa ctcttttctc ttcttattaa accatgaaaa agcctgaact caccgcgacg 3900
tctgtcgaga agtttctgat cgaaaagttc gacagcgtct ccgacctgat gcagctctcg 3960
gagggcgaag aatctcgtgc tttcagcttc gatgtaggag ggcgtggata tgtcctgcgg 4020
gtaaatagct gcgccgatgg tttctacaaa gatcgttatg tttatcggca ctttgcatcg 4080
gccgcgctcc cgattccgga agtgcttgac attggggaat tcagcgagag cctgacctat 4140

tgcatctccc gccgtgcaca gggtgtcacg ttgcaagacc tgcctgaaac cgaactgccc 4200
gctgttctgc agccggtcgc ggaggccatg gatgcgatcg ctgcggccga tcttagccag 4260
acgagcgggt tcggcccatt cggaccgcaa ggaatcggtc aatacactac atggcgtgat 4320
ttcatatgcg cgattgctga tccccatgtg tatcactggc aaactgtgat ggacgacacc 4380
gtcagtgcgt ccgtcgcgca ggctctcgat gagctgatgc tttgggccga ggactgcccc 4440
gaagtccggc acctcgtgca cgcggatttc ggctccaaca atgtcctgac ggacaatggc 4500
cgcataacag cggtcattga ctggagcgag gcgatgttcg gggattccca atacgaggtc 4560
gccaacatct tcttctggag gccgtggttg gcttgtatgg agcagcagac gcgctacttc 4620
gagcggaggc atccggagct tgcaggatcg ccgcggctcc gggcgtatat gctccgcatt 4680
ggtcttgacc aactctatca gagcttggtt gacggcaatt tcgatgatgc agcttgggcg 4740
cagggtcgat gcgacgcaat cgtccgatcc ggagccggga ctgtcgggcg tacacaaatc 4800
gcccgcagaa gcgcggccgt ctggaccgat ggctgtgtag aagtactcgc cgatagtgga 4860
aaccgacgcc ccagcactcg tccgagggca aaggaatagt gaggtaccta aagaaggagt 4920
gcgtcgaagc agatcgttca aacatttggc aataaagttt cttaagattg aatcctgttg 4980
ccggtcttgc gatgattatc atataatttc tgttgaatta cgttaagcat gtaataatta 5040
acatgtaatg catgacgtta tttatgagat gggtttttat gattagagtc ccgcaattat 5100
acatttaata cgcgatagaa aacaaaatat agcgcgcaaa ctaggataaa ttatcgcgcg 5160
cggtgtcatc tatgttacta gatcgatgtc gaatcgatca acctgcatta atgaatcggc 5220
caacgcgcgg ggagaggcgg tttgcgtatt gggcgctctt ccgcttcctc gctcactgac 5280
tcgctgcgct cggtcgttcg gctgcggcga gcggtatcag ctcactcaaa ggcggtaata 5340
cggttatcca cagaatcagg ggataacgca ggaaagaaca tgtgagcaaa aggccagcaa 5400
aaggccagga accgtaaaaa ggccgcgttg ctggcgtttt tccataggct ccgcccccct 5460
gacgagcatc acaaaaatcg acgctcaagt cagaggtggc gaaacccgac aggactataa 5520
agataccagg cgtttccccc tggaagctcc ctcgtgcgct ctcctgttcc gaccctgccg 5580
cttaccggat acctgtccgc ctttctccct tcgggaagcg tggcgctttc tcaatgctca 5640
cgctgtaggt atctcagttc ggtgtaggtc gttcgctcca agctgggctg tgtgcacgaa 5700
ccccccgttc agcccgaccg ctgcgcctta tccggtaact atcgtcttga gtccaacccg 5760
gtaagacacg acttatcgcc actggcagca gccactggta acaggattag cagagcgagg 5820
tatgtaggcg gtgctacaga gttcttgaag tggtggccta actacggcta cactagaagg 5880
acagtatttg gtatctgcgc tctgctgaag ccagttacct tcggaaaaag agttggtagc 5940

tcttgatccg gcaaacaaac caccgctggt agcggtggtt tttttgtttg caagcagcag 6000
attacgcgca gaaaaaaagg atctcaagaa gatcctttga tcttttctac ggggtctgac 6060
gctcagtgga acgaaaactc acgttaaggg attttggtca tgacattaac ctataaaaat 6120
aggcgtatca cgaggccctt tcgtctcgcg cgtttcggtg atgacggtga aaacctctga 6180
cacatgcagc tcccggagac ggtcacagct tgtctgtaag cggatgccgg gagcagacaa 6240
gcccgtcagg gcgcgtcagc gggtgttggc gggtgtcggg gctggcttaa ctatgcggca 6300
tcagagcaga ttgtactgag agtgcaccat atggacatat tgtcgttaga acgcggctac 6360
aattaataca taaccttatg tatcatacac atacgattta ggtgacacta tagaacggcg 6420
cgccaagctt ggatctcctg caggatctgg ccggccggat ctcgtacgga tccttcatcc 6480
atgcccttca tttgccgctt attaattaat ttggtaacag tccgtactaa tcagttactt 6540
atccttcccc catcataatt aatcttggta gtctcgaatg ccacaacact gactagtctc 6600
ttggatcata agaaaaagcc aaggaacaaa agaagacaaa acacaatgag agtatccttt 6660
gcatagcaat gtctaagttc ataaaattca aacaaaaacg caatcacaca cagtggacat 6720
cacttatcca ctagctgatc aggatcgccg cgtcaagaaa aaaaaactgg accccaaaag 6780
ccatgcacaa caacacgtac tcacaaaggt gtcaatcgag cagcccaaaa cattcaccaa 6840
ctcaacccat catgagccct cacatttgtt gtttctaacc caacctcaaa ctcgtattct 6900
cttccgccac ctcatttttg tttatttcaa cacccgtcaa actgcatgcc accccgtggc 6960
caaatgtcca tgcatgttaa caagacctat gactataaat agctgcaatc tcggcccagg 7020
ttttcatcat caagaaccag ttcaatatcc tagtacaccg tattaaagaa tttaagatat 7080
actccatgga agcagccaaa gaattggttt ccatcgtcca agaggagctc cccaaggtgg 7140
actatgccca gctttggcag gatgccagca gctgtgaggt cctttacctc tcggtggcat 7200
tcgtggcgat caagttcatg ctgcgcccac tggacctgaa gcgccaggcc accttgaaga 7260
agctgttcac agcatacaac ttcctcatgt cgatctattc ctttggctcc ttcctggcca 7320
tggcctatgc cctatcagta actggcatcc tctccggcga ctgtgagacg gcgttcaaca 7380
acgatgtgtt caggatcaca actcagctgt tctacctcag caagttcgta gagtacatcg 7440
actccttcta ccttcccctt atggacaagc cactgtcgtt ccttcagttc ttccatcatt 7500
tgggggcccc cattgacatg tggctattct acaaataccg caacgaagga gtctggatct 7560
ttgtcctgtt gaatgggttc attcactgga tcatgtacgg ttactattgg acgcggctca 7620
tcaagctgaa cttccccatg cccaagaacc tgatcacctc catgcagatc atccagttca 7680
atgtcgggtt ctacatcgtc tggaagtacc gcaatgtgcc atgctaccgc caggatggga 7740
tgcgcatgtt tgcctggatc ttcaactact ggtatgtcgg gacggtcttg ctgctgttcc 7800

```
tcaactttta cgtgcagacg tacatccgga agccgaggaa gaaccgaggg aagaaggagc   7860
ccgcccgtcc agctggactc ccgccggcca cgtactacga ctccctggca gtgagcggcc   7920
gcaccatgtc tcctaagcgg caagctctgc caatcacaat tgatggcgca acttatgatg   7980
tgtctgcttg ggtcaatcac caccctggag gagctgacat tatcgagaac tatcgcaacc   8040
gcgatgcgac cgatgtcttc atggtgatgc actctcaaga agccgtcgcc aagttgaaga   8100
gaatgcctgt tatggagcct tcctctcctg acacacctgt tgcacccaag cctaagcgtg   8160
atgagcccca ggaggatttc cgcaagttgc gggaggaatt catctccaag ggtatgttcg   8220
agacgagttt cctttggtat ttttacaaga cttcaactac cgtcggtttg atggtccttt   8280
ccatcttgat gaccgtgtac acgaattggt atttcaccgc tgctttggtt cttggcgtgt   8340
gctaccaaca gctaggctgg ttgtcccacg actattgcca tcaccaggtt ttcacaaacc   8400
gcaagattaa cgacgctttc ggtctctttt tcggtaacgt gatgcaggga tactcacaga   8460
cttggtggaa ggataggcac aatggtcacc atgccgccac caatgtggtc ggccatgacc   8520
cagatattga taacctcccc atcctggctt ggtctcccga agatgtcaag agggctactc   8580
cttcgactcg gaatctcatc aagtaccagc agtactactt cattcccacc attgcatccc   8640
ttaggttcat ctggtgcctc caatccatcg gcggcgtcat gtcctacaag agcgaggaga   8700
ggaacctgta ctacaagcgc cagtacacta aggaggcgat tggtctggcc ctccactggg   8760
tgctcaaggc cactttctat tgcagtgcca tgcctagctt tgccaccggt ttgggatgct   8820
tcttgatctc cgagctgctc ggaggatttg gcattgccat cgttgtgttt ctgaatcact   8880
atcctttgga caaggttgag gagactgtct gggatgagca cgggttcagc gccagccaga   8940
tccacgagac gttgaacatt aagcccggcc ttctcaccga ttgggtcttt ggtggtctca   9000
actaccagat tgagcaccac ttgtggccca acatgcccag gcacaacctc acggcagctt   9060
ccctggaggt gcagaagttg tgcgccaagc acaacctgcc ctacagggcc ccagccatca   9120
tccccggggt tcagaaattg gtcagcttct taggcgagat tgcccagctg gctgctgtcc   9180
ctgaatgagc                                                          9190
```

<210> 49
<211> 99
<212> DNA
<213> Euglena anabaena

<400> 49

```
cctgggggcc ccggcaagcc aagcgagatt gcgtcgctgc caccgccaat tcgaccagtc     60
gggaacccac ctgcagccta ctacgatgcc ctggcgacc                            99
```

<210> 50
<211> 33
<212> PRT
<213> Euglena anabaena

<400> 50

```
Pro Gly Gly Pro Gly Lys Pro Ser Glu Ile Ala Ser Leu Pro Pro Pro
1               5                   10                  15

Ile Arg Pro Val Gly Asn Pro Pro Ala Ala Tyr Tyr Asp Ala Leu Ala
            20                  25                  30

Thr
```

## Description

This application claims the benefit of U.S. Provisional Application No. 61/041956, filed April 3, 2008.

### FIELD OF THE INVENTION

This invention is in the field of biotechnology. More specifically, this invention pertains to polynucleotide sequences encoding novel multizymes uses outside the area of fatty acid biosynthesis.

### BACKGROUND OF THE INVENTION

Multizymes and their use in the production of long chain polyunsaturated fatty acids constitute the subject matter of Applicants' Assignee's application having U.S. Application No. 12/061,738 (filed April 3, 2008, which published October 16, 2008 under number US 2008/0254191).

Multizymes are defined in the related non-provisional filing as comprising a single polypeptide having at least two independent and separable enzymatic activities. The concept of a multizyme originated with a unique DHA synthase gene that was isolated from Euglena and Euglenoid homologs. This DHA synthase was found to be a fusion of a delta-4 desaturase and a C20 condensing enzyme (an Elo-type elongase). This was the first time that two enzymes with very different activities/functions were found to be fused in this way.

Another unique aspect of this DHA synthase gene was the linker region that joined the two genes encoding these enzymes. Initially, multizymes were studied with respect to synthesized long chain polyunsaturated fatty acids. However, it has since been found that this unique linker can be used to make multizymes whose applications extend far beyond the synthesis of long chain polyunsaturated fatty acids.

The present invention focuses on making multizymes with uses extending beyond the realm of fatty acid biosynthesis.

### SUMMARY OF THE INVENTION

The present invention concerns a multizyme comprising a single polypeptide having at least two independent and separable activities wherein the independent and separable activities are joined together using a linker, wherein said linker is a sequence of a DHA synthase locus of a Euglenoid comprising the amino acid sequence set forth in any one of SEQ ID NOs: 34, 36 or 50, or an amino acid sequence having at least 95% sequence identity therewith, and wherein said linker can be natural or synthetic and retains the ability to form a multizyme as if it were a linker from a Euglenoid DHA synthase locus, provided that the multizyme does not comprise an enzyme involved in fatty acid biosynthesis.

In a second embodiment, the multizyme of the invention can be used to increase total flux of all combined reactions of the multizyme when compared with the total flux of the combined reactions when each reaction is produced separately when not linked together as the multizyme.

In a third embodiment, the multizyme of the invention can be used in a reaction sequence to lower an amount of at least one intermediate produced by the multizyme when compared to the amount of substantially the same intermediate produced by each individual enzymatic activity in a reaction sequence when not linked together as a multizyme.

In a fourth embodiment, the multizyme of the invention can be used in a reaction to reduce the extent of any feedback inhibition of at least one enzymatic activity of the multizyme when compared to the amount of feedback inhibition of a corresponding enzymatic activity that occurs when said individual enzymatic activity is not part of a multizyme.

In a fifth embodiment, the multizyme of the invention can be used in a reaction to protect an activity of the multizyme from competitive inhibition by a substrate other than that produced by a preceding enzymatic activity of the multizyme.

In a sixth embodiment, the multizyme of the invention can be used such that a product of a reaction of the multizyme is substantially used in a subsequent reaction of the multizyme.

In a seventh embodiment, the multizyme of the invention can be used such that at least one independent and separable activity of the multizyme is prevented from associating with polypeptide complexes other than the multizyme of which it is a part.

In an eighth embodiment, the multizyme of the invention can be used such that a product of an enzymatic reaction of the multizyme is channeled into a transporter activity which is comprised by the multizyme.

In a ninth embodiment, the multizyme of the invention can be used such that at least one activity of the multizyme that is capable of multiple functions when expressed as an individual polypeptide is restricted to a single function when it is part of the multizyme.

In a tenth embodiment, the multizyme of the invention can be used such that at least two enzymatic activities which when linked together have resistance to an herbicide inhibitor of at least one of the individual activities. For example, the multizyme can comprise a 4-hydroxyphenyl pyruvate dioxygenase linked to an activity that produces hydroxyphenylpyruvate or it can comprise a 4-hydroxyphenyl pyruvate dioxygenase linked to a prephenate dehydrogenase or a bifunctional chorismate mutase.

In an eleventh embodiment, expression of the multizyme of the invention in a transgenic plant confers an increase in seed yield or drought resistance of said plant.

Other embodiments include the following:

a multizyme comprising a 1-hydroxy-2-methyl-2-(*E*)-butenyl 4-diphosphate reductase linked to adenosine phosphate-isopentenyltransferase;
a multizyme comprising an herbicide resistant acetolactate synthase peptide linked to a second herbicide resistant acetolactate synthase peptide;
a multizyme comprising a soluble nitrate reductase linked to a nitrate transporter peptide; or
a multizyme comprising an acyl-ACP-thioesterase protein linked to a beta-ketoacyl-ACP synthetase II protein.

BRIEF DESCRIPTION OF THE DRAWINGS AND SEQUENCE LISTINGS

The invention can be more fully understood from the following detailed description and the accompanying drawings and Sequence Listing, which form a part of this application.

FIG. 1 is a representative omega-3 and omega-6 fatty acid pathway providing for the conversion of myristic acid through various intermediates to DHA.
FIG. 2 shows a Clustal W alignment between a portion of the coding sequence of EgDHAsyn2 (SEQ ID NO:7), the cDNA sequence of the *Euglena gracilis* delta-4 desaturase (SEQ ID NO:9) (NCBI Accession No. AY278558 (GI 33466345), locus AY278558, Meyer et al., Biochemistry 42(32):9779-9788 (2003)) and the coding sequence of the *Euglena gracilis* delta-4 desaturase (SEQ ID NO:10) (Meyer et al., *supra).* EgDHAsyn2_CDS is nt 621-940 of SEQ ID NO:7, EgD4_cDNA is nt 621-931 of SEQ ID NO:9, and EgD4_CDS is nt 1-184 of SEQ ID NO:7..
FIGs. 3A and 3B show a Clustal W alignment between the amino acid sequence of EgDHAsyn1 (SEQ ID NO:5), EgDHAsyn2 (SEQ ID NO:8) and EgC20elo1 (SEQ ID NO:3).

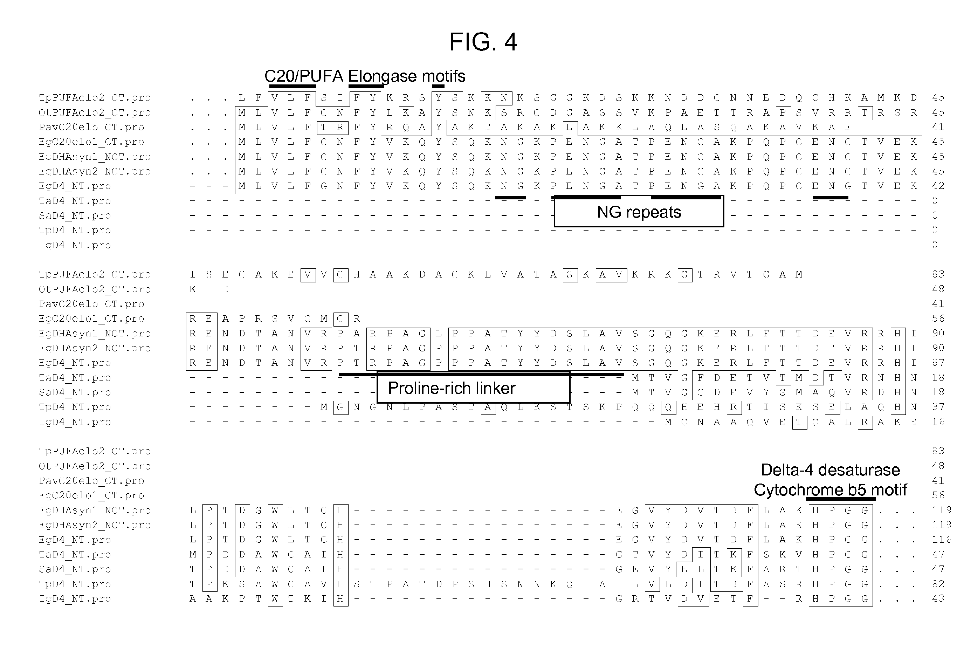

FIG. 4 shows an alignment of an interior fragment of EgDHAsyn1 (EgDHAsyn1_NCT; amino acids 253-365 of SEQ ID NO:5) and EgDHAsyn2 (EgDHAsyn2_NCT; amino acids 253-365 of SEQ ID NO:8) spanning both the C20 elongase region and the delta-4 desaturase domain (based on homology) with the C-termini of C20 elongases (EgC20elo1_CT, amino acids 246-298 of SEQ ID NO:3; PavC20elo_CT, amino acids 240-277 of SEQ ID NO:1; OtPUFAelo2_CT, amino acids 256-300 of SEQ ID NO:11; TpPUFAelo2_CT, amino acids 279-358 of SEQ ID NO:26) and the N-termini of delta-4 desaturases (EgD4_NT, amino acids 1-116 of SEQ ID NO:6; TaD4_NT, amino acids 1-47 of SEQ ID NO:13; SaD4_NT, amino acids 1-47 of SEQ ID NO:14; TpD4_NT, amino acids 1-82 of SEQ ID NO:15; IgD4_NT, amino acids 1-43 of SEQ ID NO:16).
FIGs. 5A, 5B and 5C show a Clustal W alignment of the amino acid sequences for EaDHAsyn1 (SEQ ID NO:18), EaDHAsyn2 (SEQ ID NO:20), EaDHAsyn3 (SEQ ID NO:22) and EaDHAsyn4 (SEQ ID NO:24).
FIG. 6. shows a schematic representation of the cytokinin biosynthetic pathway with native enzymes (top) and the cytokinin biosynthetic pathway with a HMBDPR-IPT multizyme (bottom).
FIG. 7 shows the Modified Hoagland's solution-16X concentration for semi-hydroponics maize growth.
FIG. 8 shows the effect of different nitrate concentrations on the growth and development of Gaspe Flint derived maize lines.
FIG. 9 shows a schematic representation of the ARA biosynthetic pathway. LA= linoleic acid, EDA = eicosadienoic acid, JUP = juniperonic acid, HGLA = dihomo-gamma- linolenic acid , ARA = arachidonic acid.

The sequence descriptions summarize the Sequences Listing attached hereto. The Sequence Listing contains one letter codes for nucleotide sequence characters and the single and three letter codes for amino acids as defined in the IUPAC-IUB standards described in Nucleic Acids Research 13:3021-3030 (1985) and in the Biochemical Journal 219(2):345-373 (1984).

SEQ ID NOs:1-50 are primers, ORFs encoding genes or proteins (or portions thereof), or plasmids, as identified in Table 1.

TABLE 1

| Summary Of Nucleic Acid And Protein SEQ ID Numbers | | |
|---|---|---|
| **Description and Abbreviation** | **Nucleic acid SEQ ID NO.** | **Protein SEQ ID NO.** |
| *Pavlova sp.* CCMP459 C20-polyunsaturated fatty acid elongase (GenBank Accession No. AAV33630) | -- | 1 (277 AA) |
| *Euglena gracilis* clone eeg1c.pk005.p14.f coding sequence ("EgC20elo1") | 2 (897 bp) | 3 (298 AA) |
| *Euglena gracilis* clone eeg1c.pk016.e6.f coding sequence (DHA synthase 1 or "EgDHAsyn1") | 4 (2382 bp) | 5 (793 AA) |
| *Euglena gracilis* delta-4 fatty acid desaturase (GenBank Accession No. AAQ19605) | -- | 6 (541 AA) |
| *Euglena gracilis* clone eeg1c-1 coding sequence (DHA synthase 2 or "EgDHAsyn2") | 7 (2382 bp) | 8 (793 AA) |
| *Euglena gracilis* delta-4 desaturase cDNA sequence (GenBank Accession No. AY278558) | 9 (2569 bp) | -- |
| *Euglena gracilis* delta-4 desaturase-coding sequence (GenBank Accession No. AY278558) | 10 (1626 bp) | -- |
| *Ostreococcus tauri* PUFA elongase 2 (GenBank Accession No. AAV67798) | -- | 11 (300 AA) |
| *Thalassiosira pseudonana* PUFA elongase 2 (GenBank Accession No. AAV67800) | -- | 12 (358 AA) |
| *Thraustochytrium aureum* delta-4 desaturase (GenBank Accession No. AAN75707) | -- | 13 (515 AA) |
| *Schizochytrium aggregatum* delta-4 desaturase (PCT Publication No. WO 2002/090493) | -- | 14 (509 AA) |
| *Thalassiosira pseuduonana* delta-4 fatty acid desaturase (GenBank Accession No. AAX14506) | -- | 15 (550 AA) |
| *Isochrysis galbana* delta-4 desaturase (GenBank Accession No. AAV33631) | -- | 16 (433 AA) |
| *Euglena anabaena* coding sequence (DHA synthase 1 or "EaDHAsyn1") | 17 (2523 bp) | 18 (841 AA) |
| *Euglena anabaena* coding sequence (DHA synthase 2 or "EaDHAsyn2") | 19 (2523 bp) | 20 (841 AA) |
| *Euglena anabaena* coding sequence (DHA synthase 3 or "EaDHAsyn3") | 21 (2523 bp) | 22 (841 AA) |
| *Euglena anabaena* coding sequence (DHA synthase 4 or "EaDHAsyn4") | 23 (2442 bp) | 24 (814 AA) |
| *Euglena gracilis* delta-9 elongase ("EgD9elo" or "EgD9e" or EgD9E") | 25 (777 bp) | -- |
| *Tetruetreptia pomquetensis* CCMP1491 delta-8 desaturase ("TpomD8") | 26 (1260 bp) | -- |
| Plasmid pLF114-10 | 27 (4300 bp) | -- |
| MWG511 primer | 28 | -- |
| Conserved motif at C-terminal for C20 elongase domains | -- | 29 (11 AA) |
| NG motif located at the C-terminus of each of the C20 elongase domains of EgDHAsyn1, EgDHAsyn2 and EgC20elo1 | -- | 30 |
| PENGA motif located at the C-terminus of each of the C20 elongase domains of EgDHAsyn1, EgDHAsyn2 and EgC20elo1 | -- | 31 |
| PCENGTV motif located at the C-terminus of each of the C20 elongase domains of EgDHAsyn1, EgDHAsyn2 and EgC20elo1 | | 32 |
| *Euglena gracilis* EgDHAsyn1 proline-rich linker | 33 (54 bp) | 34 (18 AA) |
| *Euglena gracilis* EgDHAsyn2 proline-rich linker | 35 (54 bp) | 36 (18 AA) |

(continued)

| Summary Of Nucleic Acid And Protein SEQ ID Numbers | | |
|---|---|---|
| **Description and Abbreviation** | **Nucleic acid SEQ ID NO.** | **Protein SEQ ID NO.** |
| *Euglena gracilis* EgDHAsyn1* (internal *Nco*I site removed) | 37 (2379 bp) | -- |
| Plasmid pLF121-1 | 38 (3668 bp) | -- |
| *Euglena anabaena* delta-9 elongase 1 ("EaD9Elo1"); also referred to herein as "EaD9E" and "EaD9e" | 39 (774 bp) | 40 (258 AA) |
| EaD9-5Bbs primer | 41 | -- |
| EaD9-3fusion primer | 42 | -- |
| EgDHAsyn1Link-5fusion primer | 43 | -- |
| EaD9Elo1-EgDHAsyn1Link linker | 44 (852 bp) | -- |
| Plasmid pLF124 | 45 (5559 bp) | -- |
| Plasmid pKR1177 | 46 (5559 bp) | -- |
| Plasmid pKR1179 | 47 (7916 bp) | -- |
| Plasmid pKR1183 | 48 (9190 bp) | -- |
| *Euglena anabaena* EaDHAsyn1 proline-rich linker | 49 (99 bp) | 50 (33 AA) |

## DETAILED DESCRIPTION OF THE INVENTION

As was discussed above, the concept of a multizyme originated with a unique DHA synthase gene that was isolated from Euglena and Euglenoid homologs. This DHA synthase was found to be a fusion of a delta-4 desaturase and a C20 condensing enzyme (an Elo-type elongase). This was the first time that two enzymes with very different activities/functions were found to be fused in this way.

Accordingly, the present invention is concerned with making a variety of multizymes using the linker(s) described herein for a plethora of applications extending beyond fatty acid biosynthesis and, more particularly, beyond their use in the production of long chain polyunsaturated fatty acids that constitutes the subject matter of Applicants' Assignee's Application No. 12/061,738 (filed April 3, 2008 which published October 16, 2008 under No. US 2008/0254191).

### Definitions

As used herein and in the appended claims, the singular forms "a", "an", and "the" include plural reference unless the context clearly dictates otherwise. Thus, for example, reference to "a plant" includes a plurality of such plants, reference to "a cell" includes one or more cells and equivalents thereof known to those skilled in the art, and so forth.

The term "invention" or "present invention" as used herein is not meant to be limiting to any one specific embodiment of the invention but applies generally to any and all embodiments of the invention as described in the claims and specification.

In the context of this disclosure, a number of terms and abbreviations are used. The following definitions are provided.

"Open reading frame" is abbreviated ORF.

"Polymerase chain reaction" is abbreviated PCR.

"American Type Culture Collection" is abbreviated ATCC.

"Polyunsaturated fatty acid(s)" is abbreviated PUFA(s).

"Triacylglycerols" are abbreviated TAGs.

The terms "down-regulate or down-regulation", as used herein, refer to a reduction or decrease in the level of expression of a gene or polynucleotide.

The term "multizyme" refers to a single polypeptide having at least two independent and separable enzymatic activities. Preferably, the multizyme comprises a first enzymatic activity linked to a second enzymatic activity.

The term "fusion protein" is used interchangeably with the term "multizyme". Thus, a "fusion protein" refers to a single polypeptide having at least two independent and separable enzymatic activities.

The term "fusion gene" refers to a polynucleotide or gene that encodes a multizyme. A fusion gene can be constructed by linking at least two DNA fragments, wherein each DNA fragment encodes for an independent and separate enzyme activity. An example of a fusion gene is described herein below in Example 4, in which the Hybrid1-HGLA Synthase fusion gene was constructed by linking the *Euglena anabaena* delta-9 elongase (EaD9Elo1; SEQ ID NO:39) and the *Tetruetreptia pomquetensis* CCMP1491 delta-8 desaturase (TpomD8; SEQ ID NO:26) using the *Euglena gracilis* DHA synthase 1 proline-rich linker (EgDHAsyn1Link; SEQ ID NO:33). This and other examples of fusion genes are described in Applicants' Assignee's Application No. 12/061,738 (filed April 3, 2008, which published October 16, 2008 under No. US 2008/0254191).

A "domain" or "functional domain" is a discrete, continuous part or subsequence of a polypeptide that can be associated with a function (e.g. enzymatic activity). As used herein, the term "domain" includes but is not limited to fatty acid biosynthetic enzymes and portions of fatty acid biosynthetic enzymes that retain enzymatic activity.

"DHA synthase" is an example of a multizyme. Specifically, a DHA synthase comprises a C20 elongase linked to a delta-4 desaturase using any of the linkers described herein.

The term "linker" refers to the bond or link between two or more polypeptides each having independent and separable enzymatic activities.

The preferred linker is a linker derived from a sequence of DHA synthase locus of a Euglenoid wherein the linker can be natural or synthetic. An example of a linker is shown in SEQ ID NO:34 (the EgDHAsyn1 proline-rich linker). Those skilled in the art will appreciate that it may be possible to make suitable amino acid substitutions in the linker such that it can function like a linker derived from a sequence of DHA synthase Euglenoid locus. Furthermore, it is possible to increase the distance between the polypeptides by using 2X, 3X, etc. the linker whether it is the sequence set forth in SEQ ID NO:34 or a comparable linker with one or more amino acid substitutions as described herein.

The term "Euglenoid" refers to Euglenophyceae which is a group of unicellular colorless or photosynthetic flagellates ("euglenoids") found living in freshwater, marine, soil, and parasitic environments. The class is characterized by solitary unicells, wherein most are free-swimming and have two flagella (one of which may be nonemergent) arising from an anterior invagination known as a reservoir. Photosynthetic euglenoids contain one to many grass-green chloroplasts, which vary from minute disks to expanded plates or ribbons. Colorless euglenoids depend on osmotrophy or phagotrophy for nutrient assimilation. About 1000 species have been described and classified into about 40 genera and 6 orders. Examples of Euglenophyceae include, but are by no means limited to, the following genera: *Euglena, Eutreptiella* and *Tetruetreptia.*

The term "fatty acids" refers to long-chain aliphatic acids (alkanoic acids) of varying chain lengths, from about $C_{12}$ to $C_{22}$ (although both longer and shorter chain-length acids are known). The predominant chain lengths are between $C_{16}$ and $C_{22}$. Additional details concerning the differentiation between "saturated fatty acids" versus "unsaturated fatty acids", "monounsaturated fatty acids" versus "polyunsaturated fatty acids" (or "PUFAs"), and "omega-6 fatty acids" (ω-6 or n-6) versus "omega-3 fatty acids" (omega-3 or n-3) are provided in U.S. Patent 7,238,482.

Fatty acids are described herein by a simple notation system of "X:Y", wherein X is the total number of carbon (C) atoms in the particular fatty acid and Y is the number of double bonds. The number following the fatty acid designation indicates the position of the double bond from the carboxyl end of the fatty acid with the "c" affix for the *cis*-configuration of the double bond (e.g., palmitic acid (16:0), stearic acid (18:0), oleic acid (18:1, 9c), petroselinic acid (18:1, 6c), LA (18:2, 9c,12c), GLA (18:3, 6c,9c,12c) and ALA (18:3, 9c,12c,15c)). Unless otherwise specified, 18:1, 18:2 and 18:3 refer to oleic, LA and ALA fatty acids, respectively. If not specifically written as otherwise, double bonds are assumed to be of the *cis* configuration. For instance, the double bonds in 18:2 (9,12) would be assumed to be in the *cis* configuration.

Nomenclature used to describe PUFAs in the present disclosure is shown below in Table 2. In the column titled "Shorthand Notation", the omega-reference system is used to indicate the number of carbons, the number of double bonds and the position of the double bond closest to the omega carbon, counting from the omega carbon (which is numbered 1 for this purpose). The remainder of the table summarizes the common names of omega-3 and omega-6 fatty acids and their precursors, the abbreviations that will be used throughout the remainder of the specification, and each compounds' chemical name.

TABLE 2

| Nomenclature of Polyunsaturated Fatty Acids and Precursors | | | |
|---|---|---|---|
| Common Name | Abbreviation | Chemical Name | Shorthand Notation |
| myristic | -- | tetradecanoic | 14:0 |
| palmitic | PA or Palmitate | hexadecanoic | 16:0 |
| palmitoleic | -- | 9-hexadecenoic | 16:1 |
| stearic | -- | octadecanoic | 18:0 |

(continued)

| Nomenclature of Polyunsaturated Fatty Acids and Precursors | | | |
|---|---|---|---|
| Common Name | Abbreviation | Chemical Name | Shorthand Notation |
| oleic | -- | *cis*-9-octadecenoic | 18:1 |
| linoleic | LA | *cis*-9,12-octadecadienoic | 18:2 ω-6 |
| gamma-linolenic | GLA | *cis*-6,9,12-octadecatrienoic | 18:3 ω-6 |
| eicosadienoic | EDA | *cis*-11,14-eicosadienoic | 20:2 ω-6 |
| dihomo-gamma-linolenic | DGLA or HGLA (used interchangeably herein) | *cis*-8,11,14-eicosatrienoic | 20:3 ω-6 |
| sciadonic | SCI | *cis*-5,11,14-eicosatrienoic | 20:3b ω-6 |
| arachidonic | ARA | cis-5,8,11,14-eicosatetraenoic | 20:4 ω-6 |
| alpha-linolenic | ALA | cis-9,12,15-octadecatrienoic | 18:3 ω-3 |
| stearidonic | STA | cis-6,9,12,15-octadecatetraenoic | 18:4 ω-3 |
| eicosatrienoic | ETrA or ERA | *cis*-11,14,17-eicosatrienoic | 20:3 ω-3 |
| eicosa-tetraenoic | ETA | *cis-8,11,14,17*-eicosatetraenoic | 20:4 ω-3 |
| juniperonic | JUP | *cis*-5,11,14,17-eicosatrienoic | 20:4b ω-3 |
| eicosa-pentaenoic | EPA | *cis*-5,8,11,14,17-eicosapentaenoic | 20:5 ω-3 |
| docosa-trienoic | DRA | *cis*-10,13,16-docosatrienoic | 22:3 ω-3 |
| docosa-tetraenoic | DTA | *cis*-7,10,13,16-docosatetraenoic | 22:4 ω-3 |
| docosa-pentaenoic | DPAn-6 | *cis*-4,7,10,13,16-docosapentaenoic | 22:5 ω-6 |
| docosa-pentaenoic | DPA | *cis*-7,10,13,16,19-docosapentaenoic | 22:5 ω-3 |
| docosa-hexaenoic | DHA | *cis*-4,7,10,13,16,19-docosahexaenoic | 22:6 ω-3 |

A metabolic, or biosynthetic, pathway, in a biochemical sense, can be regarded as a series of chemical reactions occurring within a cell, catalyzed by enzymes, to achieve either the formation of a metabolic product to be used or stored by the cell, or the initiation of another metabolic pathway (then called a flux generating step). Many of these pathways are elaborate, and involve a step by step modification of the initial substance to shape it into a product having the exact chemical structure desired.

The term "PUFA biosynthetic pathway" refers to a metabolic process that converts oleic acid to LA, EDA, GLA, DGLA, ARA, DTA, DPAn-6, ALA, STA, ETrA, ETA, EPA, DPA and DHA. This process is well described in the literature (e.g., see PCT Publication No. WO 2006/052870). Simplistically, this process involves elongation of the carbon chain through the addition of carbon atoms and desaturation of the molecule through the addition of double bonds, via a series of special desaturation and elongation enzymes (i.e., "PUFA biosynthetic pathway enzymes") present in the endoplasmic reticulum membrane. More specifically, "PUFA biosynthetic pathway enzyme" refers to any of the following enzymes (and genes which encode said enzymes) associated with the biosynthesis of a PUFA, including: a delta-4 desaturase, a delta-5 desaturase, a delta-6 desaturase, a delta-12 desaturase, a delta-15 desaturase, a delta-17 desaturase, a delta-9 desaturase, a delta-8 desaturase, a delta-9 elongase, a $C_{14/16}$ elongase, a $C_{16/18}$ elongase, a $C_{18/20}$ elongase, a $C_{20/22}$ elongase, a DHA synthase and/or a multizyme of the instant invention.

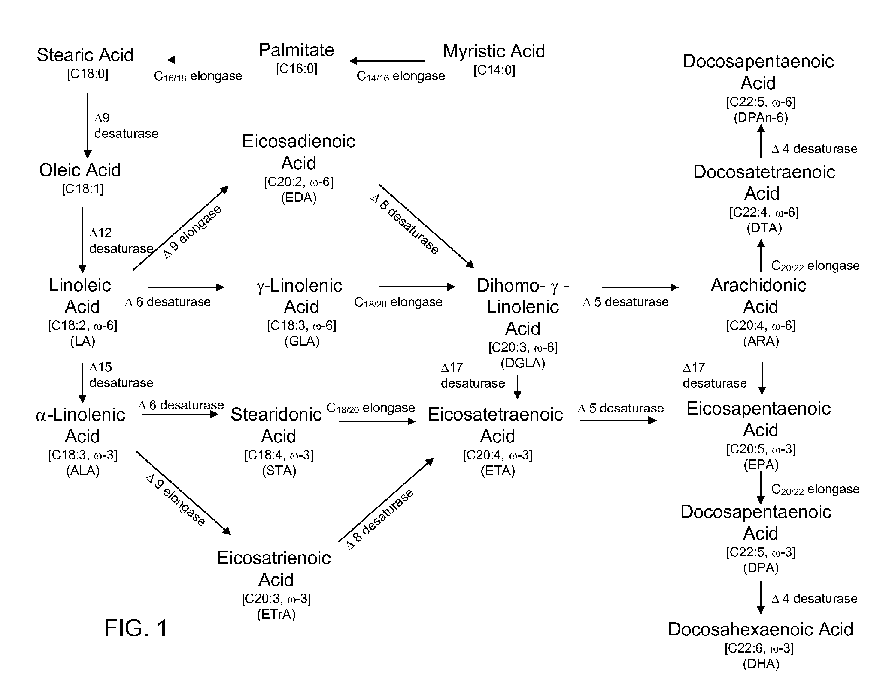

The term "omega-3/omega-6 fatty acid biosynthetic pathway" refers to a set of genes which, when expressed under the appropriate conditions encode enzymes that catalyze the production of either or both omega-3 and omega-6 fatty acids. Typically the genes involved in the omega-3/omega-6 fatty acid biosynthetic pathway encode PUFA biosynthetic pathway enzymes. A representative pathway is illustrated in FIG. 1, providing for the conversion of myristic acid through various intermediates to DHA, which demonstrates how both omega-3 and omega-6 fatty acids may be produced from a common source. The pathway is naturally divided into two portions where one portion will generate omega-3 fatty acids and the other portion, omega-6 fatty acids.

The term "functional" as used herein in context with the omega-3/omega-6 fatty acid biosynthetic pathway means that some (or all) of the genes in the pathway express active enzymes, resulting in *in vivo* catalysis or substrate conversion. It should be understood that "omega-3/omega-6 fatty acid biosynthetic pathway" or "functional omega-3/omega-6 fatty acid biosynthetic pathway" does not imply that all the PUFA biosynthetic pathway enzyme genes are required, as a number of fatty acid products will only require the expression of a subset of the genes of this pathway.

The term "delta-6 desaturase/ delta-6 elongase pathway" refers to a PUFA biosynthetic pathway that minimally includes at least one delta-6 desaturase and at least one $C_{18/20}$ elongase, thereby enabling biosynthesis of DGLA and/or ETA from LA and ALA, respectively, with GLA and/or STA as intermediate fatty acids. With expression of other desaturases and elongases, ARA, DTA, DPAn-6, EPA, DPA, and DHA may also be synthesized.

The term "delta-9 elongase/delta-8 desaturase pathway" refers to a PUFA biosynthetic pathway that minimally comprises at least one delta-9 elongase and at least one delta-8 desaturase, thereby enabling biosynthesis of DGLA and/or ETA from LA and ALA, respectively, with EDA and/or ETrA as intermediate fatty acids With expression of other desaturases and elongases, ARA, DTA, DPAn-6, EPA, DPA and DHA may also be synthesized. This pathway may be advantageous as the biosynthesis of GLA and/or STA is excluded.

The term "intermediate fatty acid" refers to any fatty acid produced in a fatty acid metabolic pathway that can be further converted to an intended product fatty acid in this pathway by the action of other metabolic pathway enzymes. For instance, when EPA is produced using the delta-9 elongase/delta-8 desaturase pathway, EDA, ETrA, DGLA, ETA and ARA can be produced and are considered "intermediate fatty acids" since these fatty acids can be further converted to EPA via action of other metabolic pathway enzymes.

The term "by-product fatty acid" refers to any fatty acid produced in a fatty acid metabolic pathway that is not the intended fatty acid product of the pathway nor an "intermediate fatty acid" of the pathway. For instance, when EPA is produced using the delta-9 elongase/delta-8 desaturase pathway, sciadonic acid (SCI) and juniperonic acid (JUP) also can be produced by the action of a delta-5 desaturase on either EDA or ETrA, respectively. They are considered to be "by-product fatty acids" since neither can be further converted to EPA by the action of other metabolic pathway enzymes.

The terms "triacylglycerol", "oil" and "TAGs" refer to neutral lipids composed of three fatty acyl residues esterified to a glycerol molecule (and such terms will be used interchangeably throughout the present disclosure herein). Such oils can contain long-chain PUFAs, as well as shorter saturated and unsaturated fatty acids and longer chain saturated fatty acids. Thus, "oil biosynthesis" generically refers to the synthesis of TAGs in the cell.

"Percent (%) PUFAs in the total lipid and oil fractions" refers to the percent of PUFAs relative to the total fatty acids in those fractions. The term "total lipid fraction" or "lipid fraction" both refer to the sum of all lipids (i.e., neutral and polar) within an oleaginous organism, thus including those lipids that are located in the phosphatidylcholine (PC) fraction, phosphatidylethanolamine (PE) fraction and triacylglycerol (TAG or oil) fraction. However, the terms "lipid" and "oil" will be used interchangeably throughout the specification.

The terms "conversion efficiency" and "percent substrate conversion" refer to the efficiency by which a particular enzyme (e.g., a desaturase) can convert substrate to product. The conversion efficiency is measured according to the following formula: ([product]/[substrate + product])*100, where 'product' includes the immediate product and all products in the pathway derived from it.

"Desaturase" is a polypeptide that can desaturate, i.e., introduce a double bond, in one or more fatty acids to produce a fatty acid or precursor of interest. Despite use of the omega-reference system throughout the specification to refer to specific fatty acids, it is more convenient to indicate the activity of a desaturase by counting from the carboxyl end of the substrate using the delta-system. For example, delta-8 desaturases will desaturate a fatty acid between the eighth and ninth carbon atom numbered from the carboxyl-terminal end of the molecule and can, for example, catalyze the conversion of EDA to DGLA and/or ETrA to ETA. Other useful fatty acid desaturases include, for example: (1) delta-5 desaturases that catalyze the conversion of DGLA to ARA and/or ETA to EPA; (2) delta-6 desaturases that catalyze the conversion of LA to GLA and/or ALA to STA; (3) delta-4 desaturases that catalyze the conversion of DPA to DHA and/or DTA to DPAn-6; (4) delta-12 desaturases that catalyze the conversion of oleic acid to LA; (5) delta-15 desaturases that catalyze the conversion of LA to ALA and/or GLA to STA; (6) delta-17 desaturases that catalyze the conversion of ARA to EPA and/or DGLA to ETA; and (7) delta-9 desaturases that catalyze the conversion of palmitic acid to palmitoleic acid (16:1) and/or stearic acid to oleic acid (18:1). In the art, delta-15 and delta-17 desaturases are also occasionally referred to as "omega-3 desaturases", "w-3 desaturases", and/or "n-3 desaturases", based on their ability to convert omega-6 fatty acids into their omega-3 counterparts (e.g., conversion of LA into ALA and ARA into EPA, respectively). It can be desirable to empirically determine the specificity of a particular fatty acid desaturase by transforming a suitable host with the gene for the fatty acid desaturase and determining its effect on the fatty acid profile of the host.

The term "delta-4 desaturase" refers to an enzyme that will desaturate a fatty acid between the fourth and fifth carbon atom numbered from the carboxyl-terminal end of the molecule and that can, for example, catalyze the conversion of DPA to DHA and/or DTA to DPAn-6. For the purposes herein, the term "EgDHAsyn1" refers to a DHA synthase enzyme (SEQ ID NO:5) isolated from *Euglena gracilis,* encoded by SEQ ID NO:4 herein.

The term "EgDHAsyn2" refers to a DHA synthase enzyme (SEQ ID NO:8) isolated from *Euglena gracilis,* encoded by SEQ ID NO:7 herein. The term "EaDHAsyn1" refers to a DHA synthase enzyme (SEQ ID NO:18) isolated from *Euglena anabaena,* encoded by SEQ ID NO:17 herein. The term "EaDHAsyn2" refers to a DHA synthase enzyme (SEQ ID NO:20) isolated from *Euglena anabaena,* encoded by SEQ ID NO:19 herein. The term "EaDHAsyn3" refers to a DHA synthase enzyme (SEQ ID NO:22) isolated from *Euglena anabaena,* encoded by SEQ ID NO:21 herein. The term "EaDHAsyn4" refers to an enzyme (SEQ ID NO:24) isolated from *Euglena anabaena,* encoded by SEQ ID NO:23 herein.

The term "elongase system" refers to a suite of four enzymes that are responsible for elongation of a fatty acid carbon chain to produce a fatty acid that is two carbons longer than the fatty acid substrate that the elongase system acts upon. More specifically, the process of elongation occurs in association with fatty acid synthase, whereby CoA is the acyl carrier (Lassner et al., Plant Cell 8:281-292 (1996)). In the first step, which has been found to be both substrate-specific and also rate-limiting, malonyl-CoA is condensed with a long-chain acyl-CoA to yield carbon dioxide ($CO_2$) and a β-ketoacyl-CoA (where the acyl moiety has been elongated by two carbon atoms). Subsequent reactions include reduction to β-hydroxyacyl-CoA, dehydration to an enoyl-CoA and a second reduction to yield the elongated acyl-CoA. Examples of reactions catalyzed by elongase systems are the conversion of GLA to DGLA, STA to ETA, LA to EDA, ALA to ETrA and EPA to DPA.

For the purposes herein, an enzyme catalyzing the first condensation reaction (i.e., conversion of malonyl-CoA and long-chain acyl-CoA to β-ketoacyl-CoA) will be referred to generically as an "elongase". In general, the substrate selectivity of elongases is somewhat broad but segregated by both chain length and the degree of unsaturation. Accordingly, elongases can have different specificities. For example, a $C_{14/16}$ elongase will utilize a $C_{14}$ substrate (e.g., myristic acid); a $C_{16/18}$ elongase will utilize a $C_{16}$ substrate (e.g., palmitate); a $C_{18/20}$ elongase will utilize a $C_{18}$ substrate (e.g., GLA, STA); and a $C_{20/22}$ elongase will utilize a $C_{20}$ substrate (e.g., ARA, EPA). Similarly, a "delta-9 elongase" is able to catalyze the conversion of LA to EDA and/or ALA to ETrA.

It is should be noted that some elongases have broad specificity and thus a single enzyme may be capable of catalyzing several elongase reactions. Thus, for example, a delta-9 elongase may also act as a $C_{16/18}$ elongase, $C_{18/20}$ elongase and/or $C_{20/22}$ elongase and may have alternate, but not preferred, specificities for delta-5 and delta-6 fatty acids such as EPA and/or GLA, respectively.

The term "C20 elongase" as used herein refers to an enzyme which utilizes a C20 substrate such as EPA or ARA, for example. The term "C20/delta-5 elongase" refers to an enzyme that utilizes a C20 substrate with a delta-5 double bond.

Similarly for the purposes herein, the term "EgD9elo" or "EgD9e" refers to a delta-9 elongase isolated from *Euglena gracilis* (see SEQ ID NO:25; also see U.S. Application No. 11/601,563 (filed November 16, 2006, which published as US-2007-0118929-A1 on May 24, 2007)).

As used herein, "nucleic acid" means a polynucleotide and includes a single or double-stranded polymer of deoxyribonucleotide or ribonucleotide bases. Nucleic acids may also include fragments and modified nucleotides. Thus, the terms "polynucleotide", "nucleic acid sequence", "nucleotide sequence" or "nucleic acid fragment" are used interchangeably and refer to a polymer of RNA or DNA that is single- or double-stranded, optionally containing synthetic, non-natural or altered nucleotide bases. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of chimeric genes to produce the desired phenotype in a transformed plant. Chimeric genes can be designed for use in suppression by linking a nucleic acid fragment or subfragment thereof, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential in the structure, the stability, or the activity of a protein. The terms "homology", "homologous", "substantially similar" and "corresponding substantially" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. It is therefore understood, as those skilled in the art will appreciate, that the invention encompasses more than the specific exemplary sequences.

Moreover, the skilled artisan recognizes that substantially similar nucleic acid sequences encompassed by this invention are also defined by their ability to hybridize (under moderately stringent conditions, e.g., 0.5X SSC, 0.1% SDS, 60 °C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30 °C for short probes (e.g., 10 to 50 nucleotides) and at least about 60 °C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37 °C, and a wash in 1X to 2X SSC (20X SSC = 3.0 M NaCl/0.3 M trisodium citrate) at 50 to 55 °C. Exemplary moderate stringency conditions include hybridization in 40 to 45% formamide, 1 M NaCl, 1% SDS at 37 °C, and a wash in 0.5X to 1X SSC at 55 to 60 °C. Exemplary high stringency conditions include hybridization in 50% formamide, 1 M NaCl, 1% SDS at 37 °C, and a wash in 0.1 X SSC at 60 to 65 °C.

Specificity is typically the function of post-hybridization washes, the critical factors being the ionic strength and temperature of the final wash solution. For DNA-DNA hybrids, the $T_m$ can be approximated from the equation of Meinkoth et al., Anal. Biochem. 138:267-284 (1984): $T_m$ = 81.5 °C + 16.6 (log M) + 0.41 (%GC)-0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The $T_m$ is the temperature (under defined ionic strength and pH) at which 50% of a complementary target sequence hybridizes to a perfectly matched probe. $T_m$ is reduced by about 1°C for each 1% of mismatching; thus, $T_m$, hybridization and/or wash conditions can be adjusted to hybridize to sequences of the desired identity. For example, if sequences with ≥90% identity are sought, the $T_m$ can be decreased 10 °C. Generally, stringent conditions are selected to be about 5 °C lower than the thermal melting point ($T_m$) for the specific sequence and its complement at a defined ionic strength and pH. However, severely stringent conditions can utilize a hybridization and/or wash at 1, 2, 3, or 4 °C lower than the thermal melting point ($T_m$); moderately stringent conditions can utilize a hybridization and/or wash at 6, 7, 8, 9, or 10 °C lower than the thermal melting point ($T_m$); low stringency conditions can utilize a hybridization and/or wash at 11, 12, 13, 14, 15, or 20 °C lower than the thermal melting point ($T_m$). Using the equation, hybridization and wash compositions, and desired $T_m$, those of ordinary skill will understand that variations in the stringency of hybridization and/or wash solutions are inherently described. If the desired degree of mismatching results in a $T_m$ of less than 45 °C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistry and Molecular Biology--Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel et al., Eds., Greene Publishing and Wiley-Interscience, New York (1995). Hybridization and/or wash conditions can be applied for at least 10, 30, 60, 90, 120, or 240 minutes.

"Sequence identity" or "identity" in the context of nucleic acid or polypeptide sequences refers to the nucleic acid bases or amino acid residues in two sequences that are the same when aligned for maximum correspondence over a specified comparison window.

Thus, "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90%, or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign™ program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, CABIOS. 5:151-153 (1989); Higgins, D.G. et al., Comput. Appl. Biosci. 8:189-191 (1992)) and found in the MegAlign™ program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal V method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, *supra;* Higgins, D.G. et al., *supra)* and found in the MegAlign™ v6.1 program of the LASERGENE bioinformatics computing suite (DNASTAR Inc., Madison, WI). Default parameters for multiple alignment correspond to GAP PENALTY=10, GAP LENGTH PENALTY=0.2, Delay Divergen Seqs(%)=30, DNA Transition Weight=0.5, Protein Weight Matrix=Gonnet Series, DNA Weight Matrix=IUB. After alignment of the sequences using the Clustal W program, it is possible to obtain a "percent identity" by viewing the "sequence distances" table in the same program.

"BLASTN method of alignment" is an algorithm provided by the National Center for Biotechnology Information (NCBI) to compare nucleotide sequences using default parameters.

It is well understood by one skilled in the art that many levels of sequence identity are useful in identifying polypeptides and/or nucleotides, from other species, wherein such polypeptides and/or nucleotides have the same or similar function or activity. Useful examples of percent identities include, but are not limited to, 50%, 55%, 60%, 65%, 70%, 75%, 80%, 85%, 90%, or 95%, or any integer percentage from 50% to 100%. Indeed, any integer amino acid identity and/or nucleotide identity from 50% to 100% may be useful in describing the present invention, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

Also, of interest is any full-length or partial complement of a nucleotide sequence comprised within an isolated nucleotide fragment.

"Gene" refers to a nucleic acid fragment that expresses a specific protein and can include either the coding region alone or the coding region in addition to the regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

The term "genome" as it applies to plant cells encompasses not only chromosomal DNA found within the nucleus, but organelle DNA found within subcellular components (e.g., mitochondrial, plastid) of the cell.

A "codon-optimized gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same that plant is homozygous at that locus. If the alleles present at a given locus on a chromosome differ that plant is heterozygous at that locus.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, introns, polyadenylation recognition sequences, RNA processing sites, effector binding sites and stem-loop structures.

"Promoter" refers to a DNA sequence capable of controlling the expression of a coding sequence or functional RNA. The promoter sequence consists of proximal and more distal upstream elements, the latter elements often referred to as enhancers. Accordingly, an "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". New promoters of various types useful in plant cells are constantly being discovered; numerous examples may be found in the compilation by Okamuro, J. K., and Goldberg, R. B. Biochemistry of Plants 15:1-82 (1989).

"Translation leader sequence" refers to a polynucleotide sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G. D., Mol. Biotechnol. 3:225-236 (1995)).

"3' non-coding sequences", "transcription terminator" or "termination sequences" refer to DNA sequences located downstream of a coding sequence, including polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht, I. L., et al. Plant Cell 1:671-680 (1989).

"RNA transcript" refers to the product resulting from RNA polymerase-catalyzed transcription of a DNA sequence. When the RNA transcript is a perfect complementary copy of the DNA sequence, it is referred to as the primary transcript. An RNA transcript is referred to as the mature RNA when it is an RNA sequence derived from post-transcriptional processing of the primary transcript. "Messenger RNA" or "mRNA" refers to the RNA that is without introns and that can be translated into protein by the cell. "cDNA" refers to a DNA that is complementary to, and synthesized from, an mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks or reduces the expression of a target gene (U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is affected by the other. For example, a promoter is operably linked with a coding sequence when it is capable of affecting the expression of that coding sequence (i.e., the coding sequence is under the transcriptional control of the promoter). Coding sequences can be operably linked to regulatory sequences in a sense or antisense orientation. In another example, the complementary RNA regions of the invention can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook, J., Fritsch, E.F. and Maniatis, T. Molecular Cloning: A Laboratory Manual; Cold Spring Harbor Laboratory: Cold Spring Harbor, NY (1989). Transformation methods are well known to those skilled in the art and are described *infra.*

"PCR" or "polymerase chain reaction" is a technique for the synthesis of large quantities of specific DNA segments and consists of a series of repetitive cycles (Perkin Elmer Cetus Instruments, Norwalk, CT). Typically, the double-stranded DNA is heat denatured, the two primers complementary to the 3' boundaries of the target segment are annealed at low temperature and then extended at an intermediate temperature. One set of these three consecutive steps is referred to as a "cycle".

The term "recombinant" refers to an artificial combination of two otherwise separated segments of sequence, e.g., by chemical synthesis or by the manipulation of isolated segments of nucleic acids by genetic engineering techniques.

A "plasmid" or "vector" is an extra chromosomal element often carrying genes that are not part of the central metabolism of the cell, and usually in the form of circular double-stranded DNA fragments. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing an expression cassette(s) into a cell. "Expression cassette" refers to a fragment of DNA containing a foreign gene and having elements in addition to the foreign gene that allow for enhanced expression of that gene in a foreign host. "Transformation cassette" refers to a fragment of DNA containing a foreign gene and having elements in addition to the foreign gene that facilitate transformation of a particular host cell.

The terms "recombinant construct", "expression construct", "chimeric construct", "construct", and "recombinant DNA construct" are used interchangeably herein. A recombinant construct comprises an artificial combination of nucleic acid fragments, e.g., regulatory and coding sequences that are not found together in nature. For example, a recombinant construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells comprising any of the isolated nucleic acid fragments of the invention. The skilled artisan will also recognize that different independent transformation events will result in different levels and patterns of expression (Jones et al., EMBO J. 4:2411-2418 (1985); De Almeida et al., Mol. Gen. Genetics 218:78-86 (1989)), and thus that multiple events must be screened in order to obtain lines displaying the desired expression level and pattern. Such screening may be accomplished by Southern analysis of DNA, Northern analysis of mRNA expression, immunoblotting analysis of protein expression, or phenotypic analysis, among others.

The term "expression", as used herein, refers to the production of a functional end-product (e.g., an mRNA or a protein [either precursor or mature]).

The term "introduced" means providing a nucleic acid (e.g., expression construct) or protein into a cell. Introduced includes reference to the incorporation of a nucleic acid into a eukaryotic or prokaryotic cell where the nucleic acid may be incorporated into the genome of the cell, and includes reference to the transient provision of a nucleic acid or protein to the cell. Introduced includes reference to stable or transient transformation methods, as well as sexually crossing. Thus, "introduced" in the context of inserting a nucleic acid fragment (e.g., a recombinant construct/expression construct) into a cell, means "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid or mitochondrial DNA), converted into an autonomous replicon, or transiently expressed (e.g., transfected mRNA).

"Mature" protein refers to a post-translationally processed polypeptide (i.e., one from which any pre- or propeptides present in the primary translation product have been removed). "Precursor" protein refers to the primary product of translation of mRNA (i.e., with pre- and propeptides still present). Pre- and propeptides may be but are not limited to intracellular localization signals.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms.

As used herein, "transgenic" refers to a plant or a cell which comprises within its genome a heterologous polynucleotide. Preferably, the heterologous polynucleotide is stably integrated within the genome such that the polynucleotide is passed on to successive generations. The heterologous polynucleotide may be integrated into the genome alone or as part of an expression construct. Transgenic is used herein to include any cell, cell line, callus, tissue, plant part or plant, the genotype of which has been altered by the presence of heterologous nucleic acid including those transgenics initially so altered as well as those created by sexual crosses or asexual propagation from the initial transgenic. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

"Antisense inhibition" refers to the production of antisense RNA transcripts capable of suppressing the expression of the target protein. "Co-suppression" refers to the production of sense RNA transcripts capable of suppressing the expression of identical or substantially similar foreign or endogenous genes (U.S. Patent No. 5,231,020). Co-suppression constructs in plants previously have been designed by focusing on overexpression of a nucleic acid sequence having homology to an endogenous mRNA, in the sense orientation, which results in the reduction of all RNA having homology to the overexpressed sequence (Vaucheret et al., Plant J. 16:651-659 (1998); Gura, Nature 404:804-808 (2000)). The overall efficiency of this phenomenon is low, and the extent of the RNA reduction is widely variable. More recent work has described the use of "hairpin" structures that incorporate all, or part, of an mRNA encoding sequence in a complementary orientation that results in a potential "stem-loop" structure for the expressed RNA (PCT Publication No. WO 99/53050; PCT Publication No. WO 02/00904). This increases the frequency of co-suppression in the recovered transgenic plants. Another variation describes the use of plant viral sequences to direct the suppression, or "silencing", of proximal mRNA encoding sequences (PCT Publication No. WO 98/36083). Both of these co-suppressing phenomena have not been elucidated mechanistically, although genetic evidence has begun to unravel this complex situation (Elmayan et al., Plant Cell 10:1747-1757 (1998)).

The term "oleaginous" refers to those organisms that tend to store their energy source in the form of lipid (Weete, In: Fungal Lipid Biochemistry, 2nd Ed., Plenum, 1980). A class of plants identified as oleaginous are commonly referred to as "oilseed" plants. Examples of oilseed plants include, but are not limited to: soybean (*Glycine* and *Soja sp.),* flax *(Linum sp.),* rapeseed *(Brassica sp.),* maize, cotton, safflower *(Carthamus sp.)* and sunflower *(Helianthus sp.).*

Within oleaginous microorganisms the cellular oil or TAG content generally follows a sigmoid curve, wherein the concentration of lipid increases until it reaches a maximum at the late logarithmic or early stationary growth phase and then gradually decreases during the late stationary and death phases (Yongmanitchai and Ward, Appl. Environ. Microbiol. 57:419-25 (1991)). The term "oleaginous yeast" refers to those microorganisms classified as yeasts that make oil. It is not uncommon for oleaginous microorganisms to accumulate in excess of about 25% of their dry cell weight as oil. Examples of oleaginous yeast include, but are no means limited to, the following genera: *Yarrowia, Candida, Rhodotorula, Rhodosporidium, Cryptococcus, Trichosporon* and *Lipomyces.*

The term "plant" refers to whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores.

"Progeny" comprises any subsequent generation of a plant.

In one embodiment, the present invention concerns a multizyme comprising a single polypeptide having at least two independent and separable activities wherein the independent and separable activities are joined together using a linker derived from a sequence of a DHA synthase locus of a Euglenoid wherein said linker can be natural or synthetic provided that the multizyme does not comprise (i) a fatty acid desaturase linked to a fatty acid elongase or (ii) a fatty acid desaturase linked to another fatty acid desaturase.

Examples of suitable activities that can be linked together include, but are not limited to nitrite reductase and glutamine synthetase; sucrose:sucrose fructosyltransferase (SST) and fructan:fructan fructosyltransrerase (FFT); a prokaryotic prephenate dehydrogenase or bifunctional chorismate mutase/prephenate dehydrogenase or a plant prephenate dehydrogenase (PDH) linked to a HPPD (4-hydroxyphenylpyruvate dioxygenase) activity; 1-Hydroxy-2-methyl-2-(E)-butenyl 4-diphosphate reductase (HMBDP reductase, also referred to as IDS; EC 1.17.1.2) linked to an adenosine phosphate isopentenyltransferase (IPT; EC 2.5.1.27); a herbicide resistant plant acetolactate synthase subunit linked to another herbicide resistant plant acetolactate synthase subunit; a soluble nitrate reductase (NR) to a nitrate transporter (NT); or an acyl-ACP thioesterase linked to a soluble beta-ketopacylsynthase II.

Examples of suitable transferases include but are not limited to acyl transferases such as glycerol-3-phosphate O-acyltransferase (also called glycerol - phosphate acyl transferase or glycerol -3-phosphate acyl transferase; GPAT), 2-acylglycerol O-acyltransferase, 1-acylglycerol-3-phosphate O-acyltransferase (also called 1-acylglycerol-phosphate acyltransferase or lyso-phosphatidic acid acyltransferase; AGPAT or LPAAT or LPAT), 2-acylglycerol-3-phosphate O-acyltransferase, 1-acylglycerophosphocholine O-acyltransferase (also called lyso-lecithin acyltransferase or lyso-phosphatidylcholine acyltransferase; AGPCAT or LLAT or LPCAT), 2-acylglycerophosphocholine O-acyltransferase, diacylglycerol O-acyltransferase (also called diglyceride acyltransferase; DAGAT or DGAT) and phospholipid:diacylglycerol acyltransferase (PDAT).

An example of a suitable acyl CoA synthetase includes but is not limited to long-chain-fatty-acid-CoA ligase (also called acyl-activating enzyme or acyl-CoA synthetase).

An example of a suitable thioesterase includes but is not limited to oleoyl-[acyl-carrier-protein] hydrolase (also called acyl-[acyl-carrier-protein] hydrolase, acyl-ACP-hydrolase or acyl-ACP-thioesterase).

The link used to form the multizyme should be derived from a sequence of a DHA synthase locus of a Euglenoid. The linker can be natural or synthetic provided that the multizyme does not comprise (i) a fatty acid desaturase linked to a fatty acid elongase; or (ii) a fatty acid desaturase linked to another fatty acid desaturase.

It is possible that amino acid substitutions could be made while retaining the ability to form a multizyme as if it was a linker derived from a sequence of a DHA synthase locus of a Euglenoid. Furthermore, the Euglenoid DHA synthase linker or substituted linker can be used at least once or more than once to extend the linkage between at least two polypeptides, if so needed.

As is discussed above, multizymes were discovered when a unique DHA synthase gene that was isolated from Euglena and Euglenoid homologs. This DHA synthase was found to be a fusion of a delta-4 desaturase and a C20 condensing enzyme (an Elo-type elongase). This was the first time that two enzymes with very different activities/functions were found to be fused in this way. Data described herein confirm that linking of the two domains within each synthase results in increased efficiency or flux, as compared to efficiency or flux observed when the enzymatic domains exist as independent entities, i.e., not linked together in a multizyme.

Nucleotide sequences encoding DHA synthases have been isolated from *Euglena gracilis* and *Euglena anabaena,* as summarized below in Table 3.

TABLE 3

| Summary Of *Euglena* DHA Synthases | | | |
|---|---|---|---|
| DHA Synthase Designation | Organism | Nucleotide SEQ ID NO | Amino Acid SEQ ID NO |
| EgDHAsyn1 | *E. gracilis* | 4 | 5 |
| EgDHAsyn1* | *E. gracilis* | 37 | 5 |
| EgDHAsyn2 | *E. gracilis* | 7 | 8 |
| EaDHAsyn1 | *E. anabaena* | 17 | 18 |
| EaDHAsyn2 | *E. anabaena* | 19 | 20 |
| EaDHAsyn3 | *E. anabaena* | 21 | 22 |
| EaDHAsyn4 | *E. anabaena* | 23 | 24 |

Those skilled in the art will appreciate that EgDHAsyn1, EgDHAsyn2, EaDHAsyn1, EaDHAsyn2 , EaDHAsyn3 and EaDHAsyn34 DHA synthase sequences can be codon-optimized for expression in a particular host organism. As is well known in the art, this can be a useful means to further optimize the expression of the enzyme in the alternate host, since use of host-preferred codons can substantially enhance the expression of the foreign gene encoding the polypeptide. EgDHAsyn1, for example, can be codon-optimized for expression in *Yarrowia lipolytica,* thereby yielding EgDHAsyn1S (as taught in U.S. Patent 7,238,482 and U.S. Patent 7,125,672).

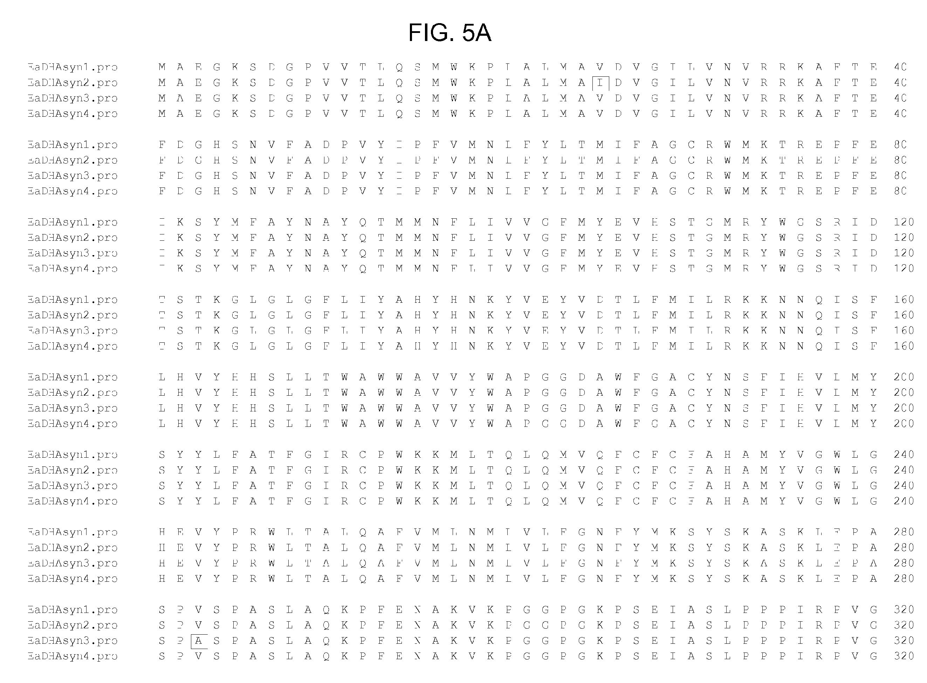

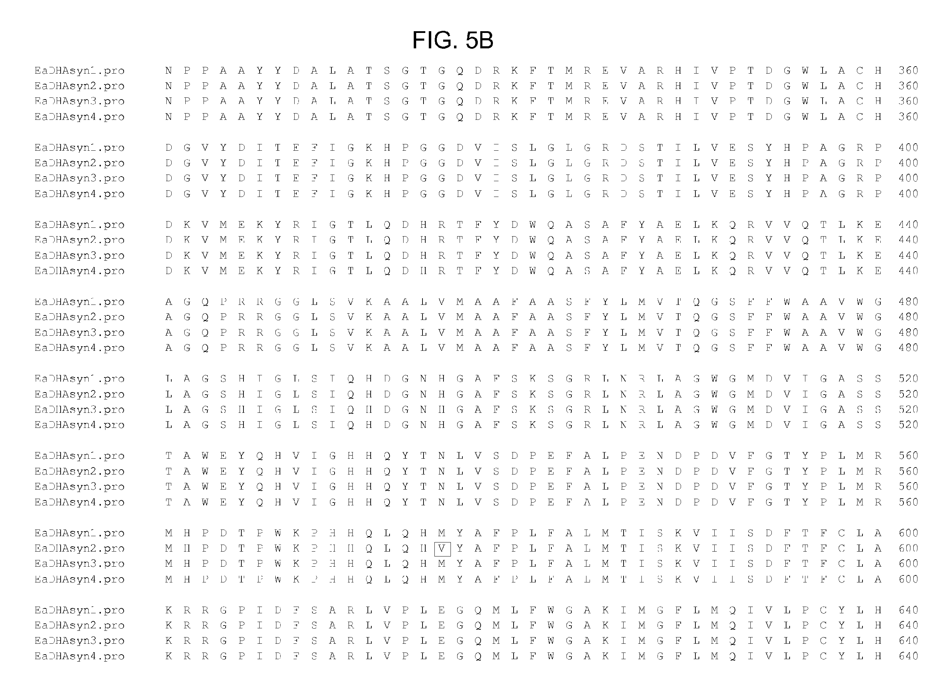

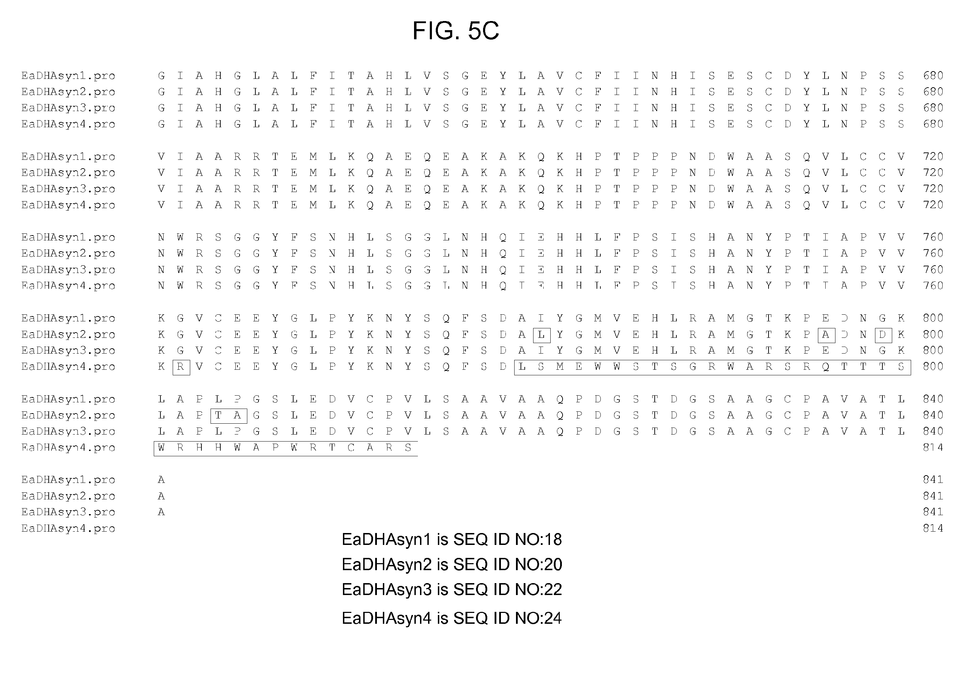

An alignment of the amino acid sequences for EaDHAsyn1 (SEQ ID NO:18), EaDHAsyn2 (SEQ ID NO:20), EaDHAsyn3 (SEQ ID NO:22) and EaDHAsyn4 (SEQ ID NO:24) using the Clustal W method is shown in FIGs. 5A, 5B and 5C.

When compared to the amino acid sequence of EgDHAsyn1 (SEQ ID NO:5) using BLASTP, the amino acid sequences of EaDHAsyn1 (SEQ ID NO:18), EaDHAsyn2 (SEQ ID NO:20), EaDHAsyn3 (SEQ ID NO:22) and EaDHAsyn4 (SEQ ID NO:24) were 70% (558/791), 70% (558/791), 70% (559/791) and 70% (548/775) identical, respectively.

As was the case for EgDHAsyn1 (SEQ ID NO:5) and EgDHAsyn2 (SEQ ID NO:8), all four EaDHAsyn sequences have a proline-rich linker region (from approximately P300 to T332 based on numbering for EaDHAsyn1). The linker appears to be slightly longer than that for EgDHAsyn1 (SEQ ID NO:5) or EgDHAsyn2 (SEQ ID NO:8). All four EaDHAsyn sequences also lack the NG repeat motif found upstream of the proline-rich motif of EgDHAsyn1 and EgDHAsyn2; but, this region, as was the case for EgDHAsyn1 and EgDHAsyn2, is also slightly proline-rich in all four EaDHAsyn sequences and may play a role in the linker function.

The nucleotide and amino acid sequences for the C20 elongase domains of EaDHAsyn1, EaDHAsyn2, EaDHAsyn3 and EaDHAsyn4 are described in Applicants' Assignee's application having U.S. Application No. 12/061,738 (filed April 3, 2008, which published October 16, 2008 under No. US 2008/0254191).

The nucleotide and amino acid sequence for the proline-rich linker of EaDHAsyn1 is set forth in SEQ ID NO:49 and SEQ ID NO:50, respectively. The nucleotide and amino acid sequences for the proline-rich linkers of EaDHAsyn2, EaDHAsyn3 and EaDHAsyn4 are identical to that for EaDHAsyn1.

One skilled in the art would be able to use the teachings herein to create various other codon-optimized DHA synthase proteins suitable for optimal expression in alternate hosts, based on the wildtype EgDHAsyn1, EgDHAsyn2, EaDHAsyn1, EaDHAsyn2 and/or EaDHAsyn3 sequences described above in Table 3.

It may be desirable to modify a portion of the codons encoding EgDHAsyn1, EgDHAsyn2, EaDHAsyn1, EaDHAsyn2 and/or EaDHAsyn3 to enhance expression of the gene in a host organism including, but not limited to, a plant or plant part.

Any of the DHA synthase sequences described herein (i.e., EgDHAsyn1, EgDHAsyn2, EaDHAsyn1, EaDHAsyn2 and EaDHAsyn3) or portions thereof may be used to search for DHA synthase homologs in the same or other bacterial, algal, fungal, euglenoid or plant species using sequence analysis software. In general, such computer software matches similar sequences by assigning degrees of homology to various substitutions, deletions, and other modifications.

Alternatively, any of the instant DHA synthase sequences or portions thereof may also be employed as hybridization reagents for the identification of DHA synthase homologs. The basic components of a nucleic acid hybridization test include a probe, a sample suspected of containing the gene or gene fragment of interest and a specific hybridization method. Probes of the present invention are typically single-stranded nucleic acid sequences that are complementary to the nucleic acid sequences to be detected. Probes are "hybridizable" to the nucleic acid sequence to be detected. Although the probe length can vary from 5 bases to tens of thousands of bases, typically a probe length of about 15 bases to about 30 bases is suitable. Only part of the probe molecule needs to be complementary to the nucleic acid sequence to be detected. In addition, the complementarity between the probe and the target sequence need not be perfect. Hybridization does occur between imperfectly complementary molecules with the result that a certain fraction of the bases in the hybridized region are not paired with the proper complementary base.

Hybridization methods are well defined. Typically the probe and sample must be mixed under conditions that will permit nucleic acid hybridization. This involves contacting the probe and sample in the presence of an inorganic or organic salt under the proper concentration and temperature conditions. The probe and sample nucleic acids must be in contact for a long enough time that any possible hybridization between the probe and sample nucleic acid may occur. The concentration of probe or target in the mixture will determine the time necessary for hybridization to occur. The higher the probe or target concentration, the shorter the hybridization incubation time needed. Optionally, a chaotropic agent may be added (e.g., guanidinium chloride, guanidinium thiocyanate, sodium thiocyanate, lithium tetrachloroacetate, sodium perchlorate, rubidium tetrachloroacetate, potassium iodide, cesium trifluoroacetate). If desired, one can add formamide to the hybridization mixture, typically 30-50% (v/v).

Various hybridization solutions can be employed. Typically, these comprise from about 20 to 60% volume, preferably 30%, of a polar organic solvent. A common hybridization solution employs about 30-50% v/v formamide, about 0.15 to 1 M sodium chloride, about 0.05 to 0.1 M buffers (e.g., sodium citrate, Tris-HCl, PIPES or HEPES (pH range about 6-9)), about 0.05 to 0.2% detergent (e.g., sodium dodecylsulfate), or between 0.5-20 mM EDTA, FICOLL (Pharmacia Inc.) (about 300-500 kdal), polyvinylpyrrolidone (about 250-500 kdal), and serum albumin. Also included in the typical hybridization solution will be unlabeled carrier nucleic acids from about 0.1 to 5 mg/mL, fragmented nucleic DNA (e.g., calf thymus or salmon sperm DNA, or yeast RNA), and optionally from about 0.5 to 2% wt/vol glycine. Other additives may also be included, such as volume exclusion agents that include a variety of polar water-soluble or swellable agents (e.g., polyethylene glycol), anionic polymers (e.g., polyacrylate or polymethylacrylate) and anionic saccharidic polymers (e.g., dextran sulfate).

Nucleic acid hybridization is adaptable to a variety of assay formats. One of the most suitable is the sandwich assay format. The sandwich assay is particularly adaptable to hybridization under non-denaturing conditions. A primary component of a sandwich-type assay is a solid support. The solid support has adsorbed to it or covalently coupled to it an immobilized nucleic acid probe that is unlabeled and complementary to one portion of the sequence.

Any of the DHA synthase nucleic acid fragments described herein (or any homologs identified thereof) may be used to isolate genes encoding homologous proteins from the same or other bacterial, algal, fungal, euglenoid or plant species. Isolation of homologous genes using sequence-dependent protocols is well known in the art. Examples of sequence-dependent protocols include, but are not limited to: (1) methods of nucleic acid hybridization; (2) methods of DNA and RNA amplification, as exemplified by various uses of nucleic acid amplification technologies [e.g., polymerase chain reaction (PCR), Mullis et al., U.S.

Patent 4,683,202; ligase chain reaction (LCR), Tabor et al., *Proc. Acad. Sci.* USA 82:1074 (1985); or strand displacement amplification (SDA), Walker et al., Proc. Natl. Acad. Sci. U.S.A., 89:392 (1992)]; and (3) methods of library construction and screening by complementation.

For example, genes encoding similar proteins or polypeptides to a multizyme or an individual domain thereof (such as the DHA synthases) described herein, could be isolated directly by using all or a portion of the instant nucleic acid fragments as DNA hybridization probes to screen libraries from e.g. any desired yeast or fungus using methodology well known to those skilled in the art.

Specific oligonucleotide probes based upon the instant nucleic acid sequences can be designed and synthesized by methods known in the art (Maniatis, *supra).* Moreover, the entire sequences can be used directly to synthesize DNA probes by methods known to the skilled artisan (e.g., random primers DNA labeling, nick translation or end-labeling techniques), or RNA probes using available *in vitro* transcription systems. In addition, specific primers can be designed and used to amplify a part of (or full-length of) the instant sequences. The resulting amplification products can be labeled directly during amplification reactions or labeled after amplification reactions, and used as probes to isolate full-length DNA fragments under conditions of appropriate stringency.

Typically, in PCR-type amplification techniques, the primers have different sequences and are not complementary to each other. Depending on the desired test conditions, the sequences of the primers should be designed to provide for both efficient and faithful replication of the target nucleic acid. Methods of PCR primer design are common and well known in the art (Thein and Wallace, "The use of oligonucleotide as specific hybridization probes in the Diagnosis of Genetic Disorders", in Human Genetic Diseases: A Practical Approach, K. E. Davis Ed., (1986) pp 33-50, IRL: Herndon, VA; and Rychlik, W., In Methods in Molecular Biology, White, B. A. Ed., (1993) Vol. 15, pp 31-39, PCR Protocols: Current Methods and Applications. Humania: Totowa, NJ).

Generally two short segments of the instant sequences may be used in PCR protocols to amplify longer nucleic acid fragments encoding homologous genes from DNA or RNA. PCR may also be performed on a library of cloned nucleic acid fragments wherein the sequence of one primer is derived from the instant nucleic acid fragments, and the sequence of the other primer takes advantage of the presence of the polyadenylic acid tracts to the 3' end of the mRNA precursor encoding eukaryotic genes.

Alternatively, the second primer sequence may be based upon sequences derived from the cloning vector. For example, the skilled artisan can follow the RACE protocol (Frohman et al., Proc. Natl Acad. Sci. U.S.A., 85:8998 (1988)) to generate cDNAs by using PCR to amplify copies of the region between a single point in the transcript and the 3' or 5' end. Primers oriented in the 3' and 5' directions can be designed from the instant sequences. Using commercially available 3' RACE or 5' RACE systems (Gibco/BRL, Gaithersburg, MD), specific 3' or 5' cDNA fragments can be isolated (Ohara et al., Proc. Natl Acad. Sci. U.S.A., 86:5673 (1989); Loh et al., Science 243:217 (1989)).

Any of the multizymes, DHA synthases, or individual domains described herein may be modified. As is well known to those skilled in the art, *in vitro* mutagenesis and selection, chemical mutagenesis, "gene shuffling" methods or other means can be employed to obtain mutations of naturally occurring genes. Alternatively, multizymes may be synthesized by domain swapping, wherein a functional domain from any enzyme may be exchanged with or added to a functional domain in an alternate enzyme to thereby result in a novel protein.

As was noted above, multizymes of the invention can be used in a variety of applications that extend beyond the biosynthesis of long chain polyunsaturated fatty acids.

A multizyme of the invention can be used to increase total flux of all combined reactions of the multizyme when compared with the total flux of the combined reactions when each reaction is produced separately when not linked together as the multizyme. A metabolic pathway can be constructed as a single multizyme and the total flux through the multizyme pathway will be greater than the individual reactions of the pathway expressed together but as separate activities. Such a multizyme pathway, with two or more of the activities of the pathway linked in a multizyme, can be engineered into a transgenic plant to increase the accumulation of a novel product when compared with a pathway engineered from individually expressed activities. A fusion of nitrite reductase and glutamine synthetase enzymes can result in an increased flux of nitrite to glutamate and ultimately lead to increased amino acid production. The importance of the nitrogen assimilating enzymes is well known in the art (see for example Heldt H. 1996. Plant Biochemistry and Molecular Biology. Oxford University press. ISBN 019850180, chapter 10, page 247-277).

Another example can be found in fusing a sucrose:sucrose fructosyltransferase (SST) with a fructan:fructan fructosyltransrerase (FFT) which can result in an increased flux of sucrose (the substrate of SST through the SST-FFT pathway leading to increased inulin production. SST and FFT are the enzymes responsible for inulin biosynthesis using the Euglenoid linker are more efficient at moving substrate through both reactions, and thus increasing the flux through the reactions, than enzymes expressed together as separate proteins.

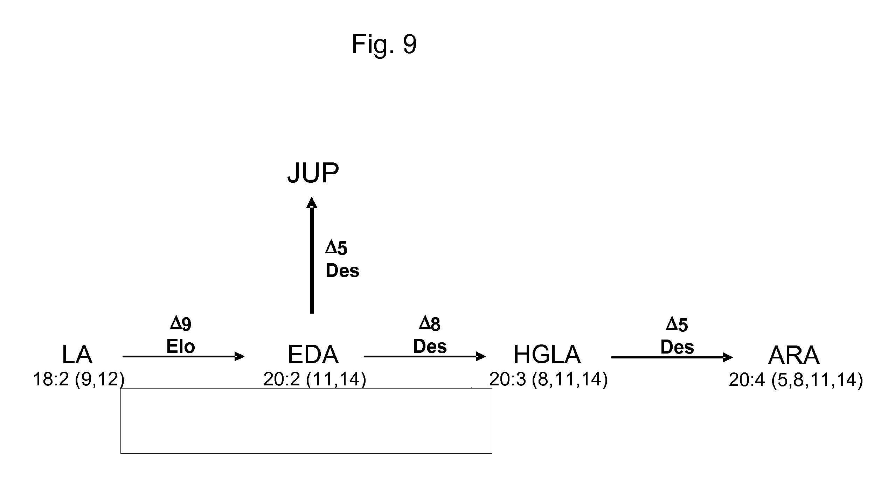

Linking a delta-9 elongase together with a delta-8 desaturase using a linker to form a multizyme (DGLA and/or ETA synthase), for example, could result in increased flux through these steps leading to reduced availability of the EDA/ERA intermediate fatty acids to delta-5 desaturase and thus reduced concentrations of SCI and JUP (FIG. 9). Evidence for an increased flux of all combined reactions using a multizyme involved in fatty acid metabolism is found in Example 61 of Applicants' Assignee's U.S.Application No. 12/061,738 (filed April 3, 2008, which published October 16, 2008 under No. US 2008/0254191), (triple fusion for ARA biosynthesis).

A multizyme of the invention can be used in a reaction sequence to lower an amount of at least one intermediate produced by the multizyme when compared to the amount of substantially the same intermediate produced by each individual enzymatic activity in a reaction sequence when not linked together as a multizyme.

A metabolic pathway can be constructed as a single multizyme or with two or more of the activities of the pathway linked in a multizyme and the intermediates and by-products (non target products) of the pathway will be eliminated or reduced when compared with a pathway engineered from individually expressed activities. For instance, linking a HMBDP reductase with an IPT in a multizyme using the Euglemoid linker, may lead to a decrease in the intermediates DMAPP (dimethylallyl diphosphate) and IPP (isopentenyl diphosphate) during production of iPRMP (isopentenyladenine riboside monophosphate).

Similarly, linking an SST and an FFT may reduce the buildup of short chain inulin polymers (product of SST).The specific genes included within a particular expression cassette will depend on the host cell, the availability of substrate and the desired end product(s).

A multizyme of the invention can be used in a reaction to reduce the extent of any feedback inhibition of at least one enzymatic activity of the multizyme when compared to the amount of feedback inhibition of a corresponding enzymatic activity that occurs when said individual enzymatic activity is not part of a multizyme. A multizyme can be used to engineer a pathway that is normally regulated by the feedback inhibition of a pathway product of one the activities in the pathway. If the regulated activity is part of a multizyme it will be resistant to inhibition by the pathway intermediate or product and thus the pathway will overproduce a final product.

A multizyme of the invention can be used in a reaction to protect an activity of the multizyme from competitive inhibition by a substrate other than that produced by a preceding enzymatic activity of the multizyme. Many enzyme inhibitors, such as herbicides and antibiotics, are enzyme substrate analogs that competitively inhibit the enzyme. An enzyme resistant to competitive inhibition, and thus herbicide or antibiotic resistance, can be engineered by linking it in a multizyme to another activity that supplies the appropriate substrate. Thus a multizyme of the invention can be used such that at least two enzymatic activities which when linked together have resistance to an herbicide inhibitor of at least one of the individual activities. For example, the multizyme can comprise a 4-hydroxyphenyl pyruvate dioxygenase linked to an activity that produces 4-hydroxyphenylpyruvate or it can comprise a 4-hydroxyphenyl pyruvate dioxygenase linked to a prephenate dehydrogenase or a bifunctional chorismate PDH. A HPPD/PDH mutase/ multizyme can be created by joining a HPPD with a prokaryotic or plant PDH or with a prokaryotic bifunctional chorismate mutase/PDH together using a linker derived from a sequence of a DHA synthase locus of a Euglenoid. Alternatively, a HPPD/ tyrosine aminotransferase multizyme can be created by joining a HPPD and a tyrosine aminotransferase. Fusing HPPD with a prokaryotic or plant PDH or with a prokaryotic bifunctional chorismate mutase/PDH can protect the HPPD from competitive-substrate inhibition and create a herbicide-resistant HPPD activity.

A multizyme of the invention can be used such that a product of a reaction of the multizyme is substantially used in a subsequent reaction of the multizyme.

Linking a HMBDP reductase with an IPT in a multizyme using a linker derived from a sequence of a DHA synthase locus of a Euglenoid can channel carbon into cytokinin by maximizing DMAPP production and reducing IPP formation. Such a multizyme can also serve to divert more DMAPP into the CK pathway rather than to isoprenenoids.

In a seventh embodiment, the multizyme of the invention can be used such that at least one independent and separable activity of the multizyme is prevented from associating with polypeptide complexes other than the multizyme of which it is a part. An activity may be regulated by its association with another polypeptide, or it may form homo- or hetero- dimers with similar activities and this may regulate enzyme activity. Association of a modified protein with a native homolog of the protein may reduce the efficacy of the modification and this may be prevented by engineering multizyme homodimers that prevent the formation of heterodimers. For example a herbicide resistant acetolactate synthase (HRA) activity may be linked to a second HRA activity to prevent association of the HRA subunit with a non-herbicide resistant ALS

In an eighth embodiment, the multizyme of the invention can be used such that a product of an enzymatic reaction of the multizyme is channeled into a transporter activity which is comprised by the multizyme. The efficiency of transport across a membrane and utilization of the transported molecule may be increased by linking the transporter to an activity that utilizes the transported molecule. For example, nitrogen utilization of a plant may be increased by linker a nitrate transporter to a nitrate reductase activity. A NR/NT multizyme can be created by joining a soluble nitrate reductase (NR) to a nitrate transporter (NT) using a linker derived from a sequence of a DHA synthase locus of a Euglenoid. The fusion of a nitrate transporter to a root nitrate reductase may increase the nitrate uptake from the root environment into the root cells and immediately convert the nitrate to nitrite which is then available for further assimilation of nitrogen compounds such as amides in the root. A similar NR/NT fusion can be produced for above ground processes resulting in an increased nitrate uptake from the xylem cells into the mesophyll cells of leaves coupled with a conversion of nitrate to nitrite which is then available for further assimilation of nitrogen compounds such as amino acids in vegetative tissues. The specification of expression of the multizyme will depend on the promoter used to drive the gene fusion as explained above.

A multizyme of the invention can be used such that at least one activity of the multizyme that is capable of multiple functions when expressed as an individual polypeptide is restricted to a single function when it is part of the multizyme. Many activities in secondary metabolism and fatty acid metabolism are capable of utilizing multiple substrates to produce multiple products. If the substrate presented to a target activity is limited to a single species, the product of a second activity linked in a multizyme, then the number of different products produced by the target activity will be limited to one, desired product. For example, a type II acyl-ACP thioesterase, which is normally active on palmitoyl-ACPI and stearoyl-ACP can be linked to a beta-ketoacyl synthase II such that the thioesterase is only presented with stearoyl-ACP.

A multizyme of the invention can be used such that at least two enzymatic activities which when linked together have resistance to an herbicide inhibitor of at least one of the individual activities. For example, the multizyme can comprise a 4-hydroxyphenyl pyruvate dioxygenase linked to an activity that produces 4-hydroxyphenylpyruvate or it can comprise a 4-hydroxyphenyl pyruvate dioxygenase linked to a prephenate dehydrogenase or a bifunctional chorismate mutase/PDH.

Expression of a multizyme of the invention in a transgenic plant can confer an increase in seed yield or drought resistance of said plant. For example, increased cytokinin production may be engineered by linking an adenosine phosphate isopentenyltransferase activity to a source of dimethylallyl diphosphate (DMAPP) such as a 1-Hydroxy-2-methyl-2-(E)-butenyl 4-diphosphate reductase thus increasing flux into cytokinin biosynthesis and protecting DMAPP from other activities such as cis prenyltransferase activity.

Other embodiments include the following:

a multizyme comprising a 1-hydroxy-2-methyl-2-(*E*)-butenyl 4-diphosphate reductase linked to adenosine phosphate-isopentenyltransferase;
a multizyme comprising an herbicide resistant acetolactate synthase peptide linked to a second herbicide resistant acetolactate synthase peptide;
a multizyme comprising a soluble nitrate reductase linked to a nitrate transporter peptide; or
a multizyme comprising an acyl-ACP-thioesterase protein linked to a beta-ketoacyl-ACP synthetase II protein.

Plant Expression Systems, Cassettes and Vectors, and Transformation

A recombinant DNA construct can be made that contain isolated polynucleotides encoding for the independent and separable activities of the multizyme wherein the independent and separable activities are joined together using a linker derived from a sequence of a DHA synthase locus of a Euglenoid, and wherein the isolated polynucleotides are operably linked to at least one regulatory sequence suitable for expression in a host cell such as a plant.

A promoter is a DNA sequence that directs cellular machinery of a plant to produce RNA from the contiguous coding sequence downstream (3') of the promoter. The promoter region influences the rate, developmental stage, and cell type in which the RNA transcript of the gene is made. The RNA transcript is processed to produce mRNA which serves as a template for translation of the RNA sequence into the amino acid sequence of the encoded polypeptide. The 5' non-translated leader sequence is a region of the mRNA upstream of the protein coding region that may play a role in initiation and translation of the mRNA. The 3' transcription termination/polyadenylation signal is a non-translated region downstream of the protein coding region that functions in the plant cell to cause termination of the RNA transcript and the addition of polyadenylate nucleotides to the 3' end of the RNA.

The origin of the promoter chosen to drive expression of the multizyme coding sequence is not important as long as it has sufficient transcriptional activity to accomplish the invention by expressing translatable mRNA for the desired nucleic acid fragments in the desired host tissue at the right time. Either heterologous or non-heterologous (i.e., endogenous) promoters can be used to practice the invention. For example, suitable promoters in plants include, but are not limited to: the alpha prime subunit of beta conglycinin promoter, the Kunitz trypsin inhibitor 3 promoter, the annexin promoter, the glycinin Gy1 promoter, the beta subunit of beta conglycinin promoter, the P34/Gly Bd m 30K promoter, the albumin promoter, the Leg A1 promoter and the Leg A2 promoter.

The annexin, or P34, promoter is described in PCT Publication No. WO 2004/071178 (published August 26, 2004). The level of activity of the annexin promoter is comparable to that of many known strong promoters, such as: (1) the CaMV 35S promoter (Atanassova et al., Plant Mol. Biol. 37:275-285 (1998); Battraw and Hall, Plant Mol. Biol. 15:527-538 (1990); Holtorf et al., Plant Mol. Biol. 29:637-646 (1995); Jefferson et al., EMBO J. 6:3901-3907 (1987); Wilmink et al., Plant Mol. Biol. 28:949-955 (1995)); (2) the *Arabidopsis* oleosin promoters (Plant et al., Plant Mol. Biol. 25:193-205 (1994); Li, Texas A&M University Ph.D. dissertation, pp. 107-128 (1997)); (3) the *Arabidopsis* ubiquitin extension protein promoters (Callis et al., J Biol. Chem. 265(21):12486-93 (1990)); (4) a tomato ubiquitin gene promoter (Rollfinke et al., Gene. 211(2):267-76 (1998)); (5) a soybean heat shock protein promoter (Schoffl et al., Mol Gen Genet. 217(2-3):246-53 (1989)); and, (6) a maize H3 histone gene promoter (Atanassova et al., Plant Mol Biol. 37(2):275-85 (1989)).

Another useful feature of the annexin promoter is its expression profile in developing seeds. The annexin promoter is most active in developing seeds at early stages (before 10 days after pollination) and is largely quiescent in later stages. The expression profile of the annexin promoter is different from that of many seed-specific promoters, e.g., seed storage protein promoters, which often provide highest activity in later stages of development (Chen et al., Dev. Genet. 10:112-122 (1989); Ellerstrom et al., Plant Mol. Biol. 32:1019-1027 (1996); Keddie et al., Plant Mol. Biol. 24:327-340 (1994); Plant et al., (*supra*); Li, *(supra)).* The annexin promoter has a more conventional expression profile but remains distinct from other known seed specific promoters. Thus, the annexin promoter will be a very attractive candidate when overexpression, or suppression, of a gene in embryos is desired at an early developing stage. For example, it may be desirable to overexpress a gene regulating early embryo development or a gene involved in the metabolism prior to seed maturation.

Following identification of an appropriate promoter suitable for expression of at least two independent and separable activities of the multizyme, the promoter is then operably linked in a sense orientation using conventional means well known to those skilled in the art.

Standard recombinant DNA and molecular cloning techniques used herein are well known in the art and are described more fully in Sambrook, J. et al., In Molecular Cloning: A Laboratory Manual; 2nd ed.; Cold Spring Harbor Laboratory Press: Cold Spring Harbor, New York, 1989 (hereinafter "Sambrook et al., 1989") or Ausubel, F. M., Brent, R., Kingston, R. E., Moore, D. D., Seidman, J. G., Smith, J. A. and Struhl, K., Eds.; In Current Protocols in Molecular Biology; John Wiley and Sons: New York, 1990 (hereinafter "Ausubel et al., 1990"). For example, a fusion gene can be constructed by linking at least two DNA fragments in frame so as not to introduce a stop codon (in-frame fusion). The resulting fusion gene will be such that each DNA fragment encodes for at least one independent and separable enzymatic activity.

Once the recombinant construct has been made, it may then be introduced into a plant cell of choice by methods well known to those of ordinary skill in the art (e.g., transfection, transformation and electroporation). Expression in a plant cell may be accomplished in a transient or stable fashion as is described above.

Methods for transforming dicots (primarily by use of *Agrobacterium tumefaciens*) and obtaining transgenic plants have been published, among others, for: cotton (U.S. Patent No. 5,004,863; U.S. Patent No. 5,159,135); soybean (U.S. Patent No. 5,569,834; U.S. Patent No. 5,416,011); *Brassica* (U.S. Patent No. 5,463,174); peanut (Cheng et al. Plant Cell Rep. 15:653-657 (1996); McKently et al. Plant Cell Rep. 14:699-703 (1995)); papaya (Ling, K. et al. Bio/technology 9:752-758 (1991)); and pea (Grant et al. Plant Cell Rep. 15:254-258 (1995)). For a review of other commonly used methods of plant transformation see Newell, C.A. (Mol. Biotechnol. 16:53-65 (2000)). One of these methods of transformation uses *Agrobacterium rhizogenes* (Tepfler, M. and Casse-Delbart, F. Microbiol. Sci. 4:24-28 (1987)). Transformation of soybeans using direct delivery of DNA has been published using PEG fusion (PCT Publication No. WO 92/17598), electroporation (Chowrira, G.M. et al., Mol. Biotechnol. 3:17-23 (1995); Christou, P. et al., Proc. Natl. Acad. Sci. U.S.A. 84:3962-3966 (1987)), microinjection and particle bombardement (McCabe, D.E. et. al., Bio/Technology 6:923 (1988); Christou et al., Plant Physiol. 87:671-674 (1988)). The choice of transformation protocols used for generating transgenic plants and plant cells can vary depending on the type of plant or plant cell, *i.e.,* monocot or dicot, targeted for transformation. Examples of transformation protocols particularly suited for a particular plant type include those for: potato (Tu et al., 1998, Plant Molecular Biology 37:829-838; Chong et al., 2000, Transgenic Research 9:71-78); soybean (Christou et al., 1988, Plant Physiol. 87:671-674; McCabe et al., 1988, BioTechnology 6:923-926; Finer and McMullen, 1991, In Vitro Cell Dev. Biol. 27P:175-182; Singh et al., 1998, Theor. Appl. Genet. 96:319-324); maize (Klein et al., 1988, Proc. Natl. Acad. Sci. 85:4305-4309; Klein et al., 1988, Biotechnology 6:559-563; Klein et al., 1988, Plant Physiol. 91:440-444; Fromm et al., 1990, Biotechnology 8:833-839; Tomes et al., 1995, "Direct DNA Transfer into Intact Plant Cells via Microprojectile Bombardment," in Plant Cell, Tissue, and Organ Culture: Fundamental Methods, ed. Gamborg (Springer-Verlag, Berlin)); cereals (Hooykaas-Van Slogteren et al., 1984, Nature 311:763-764; U.S. Patent No. 5,736,369).

The transformed plant cell is then cultured and regenerated under suitable conditions permitting expression multizyme which is then optionally recovered and purified.

There are a variety of methods for the regeneration of plants from plant tissue. The particular method of regeneration will depend on the starting plant tissue and the particular plant species to be regenerated. The regeneration, development and cultivation of plants from single plant protoplast transformants or from various transformed explants is well known in the art (Weissbach and Weissbach, In: Methods for Plant Molecular Biology, (Eds.), Academic: San Diego, CA (1988)). This regeneration and growth process typically includes the steps of selection of transformed cells and culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil. Preferably, the regenerated plants are self-pollinated to provide homozygous transgenic plants. Otherwise, pollen obtained from the regenerated plants is crossed to seed-grown plants of agronomically important lines. Conversely, pollen from plants of these important lines is used to pollinate regenerated plants. A transgenic plant of the present invention containing a desired polypeptide is cultivated using methods well known to one skilled in the art.

In addition to the above discussed procedures, practitioners are familiar with the standard resource materials which describe specific conditions and procedures for: the construction, manipulation and isolation of macromolecules (e.g., DNA molecules, plasmids, etc.); the generation of recombinant DNA fragments and recombinant expression constructs; and, the screening and isolating of clones. See, for example: Sambrook et al., Molecular Cloning: A Laboratory Manual, Cold Spring Harbor: NY (1989); Maliga et al., Methods in Plant Molecular Biology, Cold Spring Harbor: NY (1995); Birren et al., Genome Analysis: Detecting Genes, Vol.1, Cold Spring Harbor: NY (1998); Birren et al., Genome Analysis: Analyzing DNA, Vol.2, Cold Spring Harbor: NY (1998); Plant Molecular Biology: A Laboratory Manual, eds. Clark, Springer: NY (1997).

Plant parts include differentiated and undifferentiated tissues including, but not limited to the following: roots, stems, shoots, leaves, pollen, seeds, tumor tissue and various forms of cells and culture (e.g., single cells, protoplasts, embryos and callus tissue). The plant tissue may be in plant or in a plant organ, tissue or cell culture.

The term "plant organ" refers to plant tissue or a group of tissues that constitute a morphologically and functionally distinct part of a plant. The term "genome" refers to the following: (1) the entire complement of genetic material (genes and non-coding sequences) that is present in each cell of an organism, or virus or organelle; and/or (2) a complete set of chromosomes inherited as a (haploid) unit from one parent.

Examples of oilseed plants include, but are not limited to: soybean, *Brassica* species, sunflower, maize, cotton, flax and safflower.

Irrespective of the host selected for expression of a multizyme of the invention, multiple transformants must be screened in order to obtain a strain displaying the desired expression level and pattern. Such screening may be accomplished by Southern analysis of DNA blots (Southern, J. Mol. Biol., 98:503 (1975)), Northern analysis of mRNA expression (Kroczek, J. Chromatogr. Biomed. Appl., 618(1-2):133-145 (1993)), Western and/or Elisa analyses of protein expression, phenotypic analysis, or GC analysis of the PUFA products.

## EXAMPLES

The present invention is further defined in the following Examples, in which parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions. Thus, various modifications of the invention in addition to those shown and described herein will be apparent to those skilled in the art from the foregoing description. Such modifications are also intended to fall within the scope of the appended claims.

The meaning of abbreviations is as follows: "sec" means second(s), "min" means minute(s), "h" means hour(s), "d" means day(s), "μL" means microliter(s), "mL" means milliliter(s), "L" means liter(s), "μM" means micromolar, "mM" means millimolar, "M" means molar, "mmol" means millimole(s), "μmole" mean micromole(s), "g" means gram(s), "μg" means microgram(s), "ng" means nanogram(s), "U" means unit(s), "bp" means base pair(s) and "kB" means kilobase(s).

## GENERAL METHODS:

### Nomenclature for Expression Cassettes:

The structure of an expression cassette will be represented by a simple notation system of "X::Y::Z", wherein X describes the promoter fragment, Y describes the gene coding region fragment, and Z describes the terminator fragment, which are all operably linked to one another.

The identification of DHA synthase 1 (EgDHAsyn1) and DHA synthase 2 (EgDHAsyn2) from *Euglena gracilis* cDNA Library eeg1c is described in Applicants' Assignee's Application No. 12/061,738 (filed April 3, 2008, which published October 16, 2008 under No. US 2008/0254191).

## EXAMPLE 1

### Primary Structure Analysis of EgC20elo1, EgDHAsyn1 and EgDHAsyn2

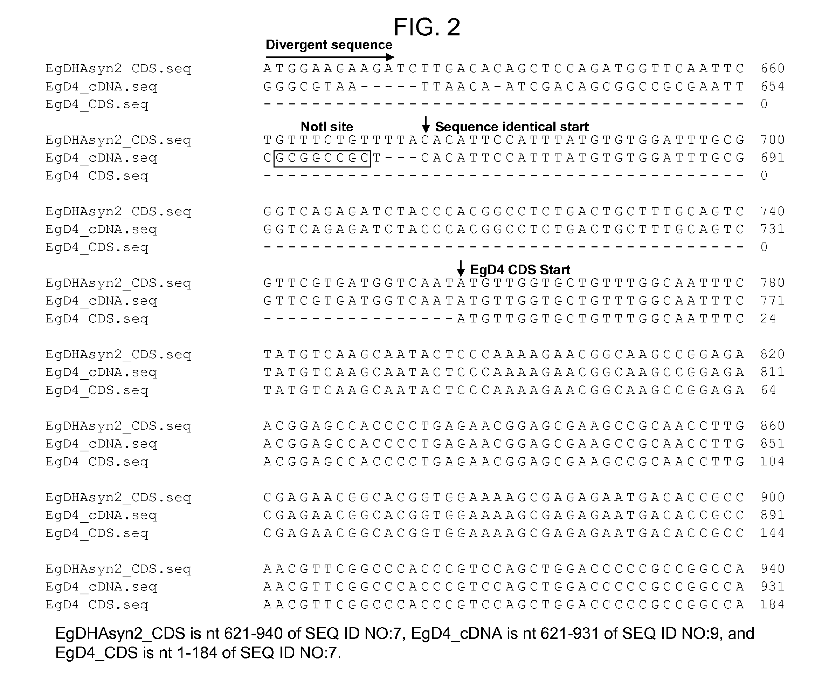

Given the 100% amino acid identity between the C-terminus of EgDHAsyn2 (SEQ ID NO:8) and the *Euglena gracilis* delta-4 desaturase (SEQ ID NO:6), a nucleotide sequence alignment was carried out between the coding sequence of EgDHAsyn2 (SEQ ID NO:7), the cDNA sequence of the *Euglena gracilis* delta-4 desaturase (SEQ ID NO:9) (NCBI Accession No. AY278558 (GI 33466345), locus AY278558, Meyer et al., Biochemistry 42(32):9779-9788 (2003)) and the coding sequence of the *Euglena gracilis* delta-4 desaturase (SEQ ID NO:10) (Meyer et al., *supra).* Sequence alignment was performed by the Clustal W method (using the MegAlign™ v6.1 program of the LASERGENE bioinformatics computing suite (DNASTAR Inc.) with the default parameters for multiple alignment (GAP PENALTY=10, GAP LENGTH PENALTY=0.2, Delay Divergen Seqs(%)=30, DNA Transition Weight=0.5, Protein Weight Matrix=Gonnet Series, DNA Weight Matrix=IUB). The alignment is shown in FIG. 2. The *Euglena gracilis* delta-4 desaturase coding sequence is named EgD4_CDS (SEQ ID NO:10), the *Euglena gracilis* delta-4 desaturase cDNA sequence is named EgD4_cDNA (SEQ ID NO:9) and the *Euglena gracilis* DHA synthase 2 coding sequence is named EgDHAsyn2_CDS (SEQ ID NO:7).

The 5' end (where the sequences are divergent) and the 3' end (where the sequences are identical) of the alignment are truncated in order to fit the alignment on one page. FIG. 2 illustrates that the sequences are highly divergent from the start of the *Euglena gracilis* delta-4 desaturase cDNA to 83 bp upstream of the coding sequence (CDS) start site. It is clear from the alignment that the nucleotide sequences for EgD4_cDNA and EgDHAsyn2_CDS are identical from 83 bp upstream of the CDS start site of the *Euglena gracilis* delta-4 desaturase cDNA sequence (SEQ ID NO:9), which is equivalent to nucleotide 674 of the EgDHAsyn2_CDS (SEQ ID NO:7), through to the end of the sequences. At the exact point of divergence, a *Not*I site can be found in the *Euglena gracilis* cDNA sequence (nucleotides 656-663 of SEQ ID NO:9) and since *Not*I linkers were used in the original cloning of the *Euglena gracilis* delta-4 desaturase cDNA (see Meyer et al., *supra)* it is likely that what was cloned was an incomplete, not full-length, transcript for EgDHAsyn2.

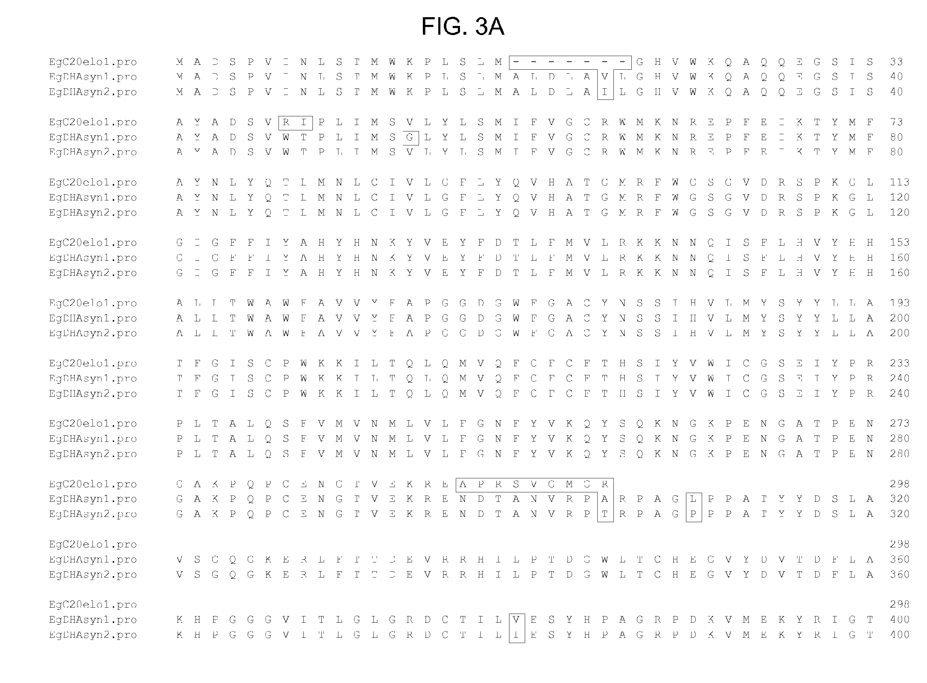

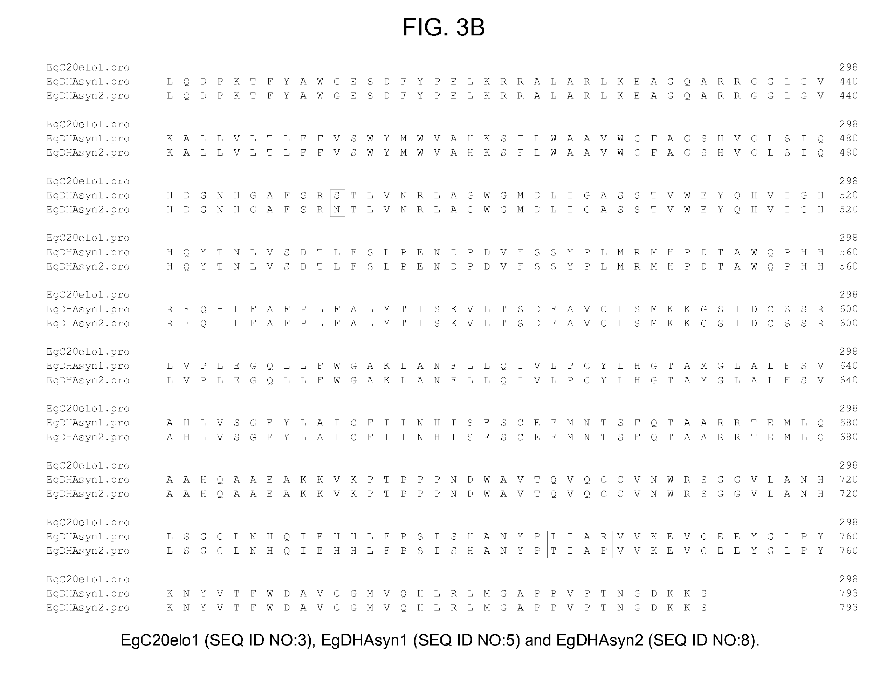

The amino acid sequence EgDHAsyn1 (SEQ ID NO:5) was compared to EgDHAsyn2 (SEQ ID NO:8) and EgC20elo1 (SEQ ID NO:3) using the Clustal W method as described above and the alignment is shown in FIGs. 3A and 3B. Compared to EgDHAsyn1 and EgDHAsyn2, EgC20elo1 has a deletion of 7 amino acids (i.e., A L D L A [V/I] L) and 2 other amino acid substitutions (i.e., W47R, T48I; based on numbering for EgDHAsyn1) at the N-terminus. After amino acid 289 of EgC20elo1, the sequences are very different when compared to the DHA synthases with EgDHAsyn1 and EgDHAsyn2 having an additional 498 amino acids at the C-terminus with homology to delta-4 fatty acid desaturases while EgC20elo1 ends after only 9 additional amino acids. The amino acid sequences of EgDHAsyn1 (SEQ ID NO:5) and EgDHAsyn2 (SEQ ID NO:8) have 8 amino acid differences between the 2 sequences (i.e., V25I, G54V, A305T, L310P, V380I, S491 N, I744T, R747P; based on numbering for EgDHAsyn1). The last four differences occur in the delta-4 desaturase domain.

FIG. 4 shows an alignment of an interior fragment of EgDHAsyn1 (labeled as "EgDHAsyn1_NCT.pro"; amino acids 253-365 of SEQ ID NO:5) and EgDHAsyn2 (labeled as "EgDHAsyn2_NCT.pro"; amino acids 253-365 of SEQ ID NO:8) spanning both the C20 elongase region and the delta-4 desaturase domain (based on homology) with the C-termini of C20 elongases (EgC20elo1_CT.pro, amino acids 246-298 of SEQ ID NO:3; PavC20elo_CT.pro, amino acids 240-277 of SEQ ID NO:2; OtPUFAelo2_CT.pro, amino acids 256-300 of SEQ ID NO:11; TpPUFAelo2_CT.pro, amino acids 279-358 of SEQ ID NO:12) and the N-termini of delta-4 desaturases (EgD4_NT.pro, amino acids 1-116 of SEQ ID NO:6; TaD4_NT.pro, amino acids 1-47 of SEQ ID NO:13; SaD4_NT.pro, amino acids 1-47 of SEQ ID NO:14; TpD4_NT.pro, amino acids 1-82 of SEQ ID NO:15; IgD4_NT.pro, amino acids 1-43 of SEQ ID NO:16) is shown. A conserved motif at the C-terminus of all the C20 elongase domains (i.e., VLFXXFYXXXY (SEQ ID NO:29)) is also present at the N-terminus of EgD4 and further supports EgD4 being an incomplete DHA synthase.

At the C-terminus of the C20 elongase domain of EgDHAsyn1, EgDHAsyn2 and EgC20elo1, there is a repeated sequence containing an NG motif (i.e., KNGK (SEQ ID NO:30), PENGA (SEQ ID NO:31),, PCENGTV (SEQ ID NO:32); called NG repeats and indicated in FIG. 4 with lines under the sequence). Although the pattern occurs with a high probability of occurrence, a scan of the NG repeated region using Prosite shows the last NG motif (i.e., NGTV) in this region as a potential N-glycosylation site. After the NG repeat region, both EgDHAsyn1 and EgDHAsyn2 contain a proline-rich region (labeled "Proline-rich linker" in FIG. 4), which may act as a linker between the C20 elongase and delta-4 desaturase domains. The linker may play a role in keeping the C20 elongase and delta-4 desaturase domains in the proper structural orientation to allow efficient conversion of EPA to DHA. Although the proline-rich linker is shown in FIG. 4 as extending from P304 to V321 (based on numbering for EgDHAsyn1), the NG repeat region is also somewhat proline-rich and may also play a role in this linker function.

The nucleotide and corresponding amino acid sequences for the proline-rich linker of EgDHAsyn1, as defined in FIG. 4, are set forth in SEQ ID NO:33 and SEQ ID NO:34, respectively. The nucleotide and corresponding amino acid sequences for the proline-rich linker of EgDHAsyn2, as defined in FIG. 4, are set forth in SEQ ID NO:35 and SEQ ID NO:36, respectively.

The nucleotide and corresponding amino acid sequences for the EgDHAsyn1 C20 elongase domain from EgDHAsyn1 are set forth in SEQ ID NO:201 and SEQ ID NO:202, respectively. The nucleotide and corresponding amino acid sequences for the EgDHAsyn2 C20 elongase domain are set forth in SEQ ID NO:203 and SEQ ID NO:204, respectively.

EXAMPLE 2

Production and Model System Transformation of Somatic Soybean Embryo Cultures with Soybean Expression Vectors

Culture Conditions:

Soybean embryogenic suspension cultures (cv. Jack) are maintained in 35 mL liquid medium SB196 *(infra)* on a rotary shaker, 150 rpm, 26 °C with cool white fluorescent lights on 16:8 hr day/night photoperiod at light intensity of 60-85 μE/m2/s. Cultures are subcultured every 7 days to two weeks by inoculating approximately 35 mg of tissue into 35 mL of fresh liquid SB196 (the preferred subculture interval is every 7 days).

Soybean embryogenic suspension cultures are transformed with the soybean expression plasmids by the method of particle gun bombardment (Klein et al., Nature 327:70 (1987)) using a DuPont Biolistic PDS1000/HE instrument (helium retrofit) for all transformations.

Soybean Embryogenic Suspension Culture Initiation:

Soybean cultures are initiated twice each month with 5-7 days between each initiation. Pods with immature seeds from available soybean plants are picked 45-55 days after planting. Seeds are removed from the pods and placed into a sterilized magenta box. The soybean seeds are sterilized by shaking them for 15 min in a 5% Clorox solution with 1 drop of Ivory soap (i.e., 95 mL of autoclaved distilled water plus 5 mL Clorox and 1 drop of soap, mixed well). Seeds are rinsed using 2 1-liter bottles of sterile distilled water and those less than 4 mm are placed on individual microscope slides. The small end of the seed is cut and the cotyledons are pressed out of the seed coat. When cultures are being prepared for production transformation, cotyledons are transferred to plates containing SB1 medium (25-30 cotyledons per plate). Plates are wrapped with fiber tape and are maintained at 26 °C with cool white fluorescent lights on 16:8 h day/night photoperiod at light intensity of 60-80 μE/m2/s for eight weeks, with a media change after 4 weeks. When cultures are being prepared for model system experiments, cotyledons are transferred to plates containing SB199 medium (25-30 cotyledons per plate) for 2 weeks, and then transferred to SB1 for 2-4 weeks. Light and temperature conditions are the same as described above. After incubation on SB1 medium, secondary embryos are cut and placed into SB196 liquid media for 7 days.

Preparation of DNA for Bombardment:

Either an intact plasmid or a DNA plasmid fragment containing the genes of interest and the selectable marker gene are used for bombardment. Fragments from soybean expression plasmids, the construction of which is described herein, are obtained by gel isolation of digested plasmids. In each case, 100 μg of plasmid DNA is used in 0.5 mL of the specific enzyme mix described below. Plasmids are digested with *Asc*I (100 units) in NEBuffer 4 (20 mM Tris-acetate, 10 mM magnesium acetate, 50 mM potassium acetate, 1 mM dithiothreitol, pH 7.9), 100 μg/mL BSA, and 5 mM beta-mercaptoethanol at 37 °C for 1.5 hr. The resulting DNA fragments are separated by gel electrophoresis on 1% SeaPlaque GTG agarose (BioWhitaker Molecular Applications) and the DNA fragments containing gene cassettes are cut from the agarose gel. DNA is purified from the agarose using the GELase digesting enzyme following the manufacturer's protocol.

A 50 μL aliquot of sterile distilled water containing 3 mg of gold particles (3 mg gold) is added to 30 μL of a 10 ng/μL DNA solution (either intact plasmid or DNA fragment prepared as described herein), 25 μL 5M $CaCl_2$ and 20 μL of 0.1 M spermidine. The mixture is shaken 3 min on level 3 of a vortex shaker and spun for 10 sec in a bench microfuge. The supernatant is removed, followed by a wash with 400 μL 100% ethanol and another brief centrifugation. The 400 μl ethanol is removed and the pellet is resuspended in 40 μL of 100% ethanol. Five μL of DNA suspension is dispensed to each flying disk of the Biolistic PDS1000/HE instrument disk. Each 5 μL aliquot contained approximately 0.375 mg gold per bombardment (e.g., per disk).

For model system transformations, the protocol is identical except for a few minor changes (i.e., 1 mg of gold particles is added to 5 μL of a 1 μg/μL DNA solution, 50 μL of a 2.5M $CaCl_2$ is used and the pellet is ultimately resuspended in 85 μL of 100% ethanol thus providing 0.058 mg of gold particles per bombardment).

Tissue Preparation and Bombardment with DNA:

Approximately 150-200 mg of seven day old embryogenic suspension cultures is placed in an empty, sterile 60 x 15 mm petri dish and the dish is covered with plastic mesh. The chamber is evacuated to a vacuum of 27-28 inches of mercury, and tissue is bombarded one or two shots per plate with membrane rupture pressure set at 1100 PSI. Tissue is placed approximately 3.5 inches from the retaining /stopping screen. Model system transformation conditions are identical except 100-150 mg of embryogenic tissue is used, rupture pressure is set at 650 PSI and tissue is place approximately 2.5 inches from the retaining screen.

Selection of Transformed Embryos:

Transformed embryos are selected either using hygromycin (when the hygromycin B phosphotransferase (HPT) gene is used as the selectable marker) or chlorsulfuron (when the acetolactate synthase (ALS) gene is used as the selectable marker).

Following bombardment, the tissue is placed into fresh SB196 media and cultured as described above. Six to eight days post-bombardment, the SB196 is exchanged with fresh SB196 containing either 30 mg/L hygromycin or 100 ng/mL chlorsulfuron, depending on the selectable marker used. The selection media is refreshed weekly. Four to six weeks post-selection, green, transformed tissue is observed growing from untransformed, necrotic embryogenic clusters.

Embryo Maturation:

For production transformations, isolated, green tissue is removed and inoculated into multiwell plates to generate new, clonally propagated, transformed embryogenic suspension cultures. Transformed embryogenic clusters are cultured for four-six weeks in multiwell plates at 26 °C in SB196 under cool white fluorescent (Phillips cool white Econowatt F40/CW/RS/EW) and Agro (Phillips F40 Agro) bulbs (40 watt) on a 16:8 hr photoperiod with light intensity of 90-120 $\mu E/m^2s$. After this time embryo clusters are removed to a solid agar media, SB166, for one-two weeks and then subcultured to SB103 medium for 3-4 weeks to mature embryos. After maturation on plates in SB103, individual embryos are removed from the clusters, dried and screened for alterations in their fatty acid compositions as described *supra.*

For model system transformations, embryos are matured in soybean histodifferentiation and maturation liquid medium (SHaM liquid media; Schmidt et al., Cell Biology and Morphogenesis 24:393 (2005)) using a modified procedure. Briefly, after 4 weeks of selection in SB196 as described above, embryo clusters are removed to 35 mL of SB228 (SHaM liquid media) in a 250 mL Erlenmeyer flask. Tissue is maintained in SHaM liquid media on a rotary shaker at 130 rpm and 26 °C with cool white fluorescent lights on a 16:8 hr day/night photoperiod at a light intensity of 60-85 μE/m2/s for 2 weeks as embryos matured. Embryos grown for 2 weeks in SHaM liquid media are equivalent in size and fatty acid content to embryos cultured on SB166/SB103 for 5-8 weeks.

After maturation in SHaM liquid media, individual embryos are removed from the clusters, dried and screened for alterations in their fatty acid compositions as described *supra.*

Media Recipes:

SB 196 - FN Lite Liquid Proliferation Medium (per liter)

| | |
|---|---|
| MS FeEDTA - 100x Stock 1 | 10 mL |
| MS Sulfate - 100x Stock 2 | 10 mL |
| FN Lite Halides - 100x Stock 3 | 10 mL |
| FN Lite P, B, Mo - 100x Stock 4 | 10 mL |
| B5 vitamins (1 mL/L) | 1.0 mL |
| 2,4-D (10mg/L final concentration) | 1.0 mL |
| $KNO_3$ | 2.83 gm |
| $(NH_4)_2SO_4$ | 0.463 gm |
| asparagine | 1.0 gm |
| sucrose (1%) | 10 gm |
| pH 5.8 | |

FN Lite Stock Solutions

| Stock Number | | 1000 mL | 500 mL |
|---|---|---|---|
| 1 | MS Fe EDTA 100x Stock | | |
| | $Na_2$ EDTA* | 3.724 g | 1.862 g |
| | $FeSO_4$ - $7H_2O$ | 2.784 g | 1.392 g |
| *Add first, dissolve in dark bottle while stirring | | | |
| 2 | MS Sulfate 100x stock | | |
| | $MgSO_4$ - $7H_2O$ | 37.0 g | 18.5 g |
| | $MnSO_4$ - $H_2O$ | 1.69 g | 0.845 g |
| | $ZnSO_4$ - $7H_2O$ | 0.86 g | 0.43 g |
| | $CuSO_4$ - $5H_2O$ | 0.0025 g | 0.00125 g |
| 3 | FN Lite Halides 100x Stock | | |
| | $CaCl_2$- $2H_2O$ | 30.0 g | 15.0 g |
| | KI | 0.083 g | 0.0715 g |
| | $CoCl_2$ - $6H_2O$ | 0.0025 g | 0.00125 g |
| 4 | FN Lite P, B, Mo 100x Stock | | |
| | $KH_2PO_4$ | 18.5 g | 9.25 g |
| | $H_3BO_3$ | 0.62 g | 0.31 g |

(continued)

| | | |
|---|---|---|
| $Na_2MoO_4 - 2H_2O$ | 0.025 g | 0.0125 g |

SB1 Solid Medium (per liter)

1 package MS salts (Gibco/ BRL - Cat. No. 11117-066)
1 mL B5 vitamins 1000X stock
31.5 g glucose
2 mL 2,4-D (20 mg/L final concentration) pH 5.7
8 g TC agar

SB199 Solid Medium (per liter)

1 package MS salts (Gibco/ BRL - Cat. No. 11117-066)
1 mL B5 vitamins 1000X stock
30g Sucrose
4 ml 2,4-D (40 mg/L final concentration)
pH 7.0
2 gm Gelrite

SB 166 Solid Medium (per liter)

1 package MS salts (Gibco/ BRL - Cat. No. 11117-066)
1 mL B5 vitamins 1000X stock
60 g maltose
750 mg $MgCl_2$ hexahydrate
5 g activated charcoal
pH 5.7
2 g gelrite

SB 103 Solid Medium (per liter)

1 package MS salts (Gibco/ BRL - Cat. No. 11117-066)
1 mL B5 vitamins 1000X stock
60 g maltose
750 mg MgCl2 hexahydrate
pH 5.7
2 g gelrite

SB 71-4 Solid Medium (per liter)

1 bottle Gamborg's B5 salts w/ sucrose (Gibco/ BRL - Cat. No. 21153-036)
pH 5.7
5 g TC agar

2,4-D Stock

Obtain premade from Phytotech Cat. No. D 295 - concentration 1 mg/mL

B5 Vitamins Stock (per 100 mL)

Store aliquots at -20 °C

10 g myo-inositol
100 mg nicotinic acid
100 mg pyridoxine HCl
1 g thiamine

If the solution does not dissolve quickly enough, apply a low level of heat via the hot stir plate.

SB 228- Soybean Histodifferentiation & Maturation (SHaM) (per liter)

| | |
|---|---|
| DDI $H_2O$ | 600 mL |
| FN-Lite Macro Salts for SHaM 10X | 100 mL |
| MS Micro Salts 1000x | 1 mL |
| MS FeEDTA 100x | 10 mL |
| CaCl 100x | 6.82 mL |
| B5 Vitamins 1000x | 1 mL |
| L-Methionine | 0.149 g |
| Sucrose | 30 g |
| Sorbitol | 30 g |
| Adjust volume to 900 mL | |
| pH 5.8 | |
| Autoclave | |
| Add to cooled media (≤30 °C): | |
| *Glutamine (final concentration 30 mM) 4% | 110 mL |

*Note: Final volume will be 1010 mL after glutamine addition.

Since glutamine degrades relatively rapidly, it may be preferable to add immediately prior to using media. Expiration 2 weeks after glutamine is added; base media can be kept longer without glutamine.

FN-lite Macro for SHAM 10X- Stock #1 (per liter)

| | |
|---|---|
| $(NH_4)_2SO_4$ (ammonium sulfate) | 4.63 g |
| $KNO_3$ (potassium nitrate) | 28.3 g |
| $MgSO_4$*$7H_20$ (magnesium sulfate heptahydrate) | 3.7 g |
| $KH_2PO_4$ (potassium phosphate, monobasic) | 1.85 g |
| Bring to volume | |
| Autoclave | |

MS Micro 1000X- Stock #2 (per 1 liter)

| | |
|---|---|
| $H_3BO_3$ (boric acid) | 6.2 g |
| $MnSO_4$*$H_2O$ (manganese sulfate monohydrate) | 16.9 g |
| ZnSO4*$7H_20$ (zinc sulfate heptahydrate) | 8.6 g |
| $Na_2MoO_4$*$2H_20$ (sodium molybdate dihydrate) | 0.25 g |
| $CuSO_4$*$5H_20$ (copper sulfate pentahydrate) | 0.025 g |
| $CoCl_2$*$6H_20$ (cobalt chloride hexahydrate) | 0.025 g |
| KI (potassium iodide) | 0.8300 g |
| Bring to volume | |
| Autoclave | |

FeEDTA 100X- Stock #3 (per liter)

| | |
|---|---|
| $Na_2EDTA$* (sodium EDTA) | 3.73 g |
| $FeSO_4$*$7H_20$ (iron sulfate heptahydrate) | 2.78 g |
| *EDTA must be completely dissolved before adding iron. | |

Bring to Volume

Solution is photosensitive. Bottle(s) should be wrapped in foil to omit light.

Autoclave

Ca 100X- Stock #4 (per liter)

| | |
|---|---|
| $CaCl_2$*$2H_20$ (calcium chloride dihydrate) | 44 g |
| Bring to Volume | |
| Autoclave | |

B5 Vitamin 1000X- Stock #5 (per liter)

| | |
|---|---|
| Thiamine*HCl | 10 g |
| Nicotinic Acid | 1 g |
| Pyridoxine*HCl | 1 g |
| Myo-Inositol | 100 g |
| Bring to Volume | |
| Store frozen | |

4% Glutamine- Stock #6 (per liter)

| | |
|---|---|
| DDI water heated to 30 °C | 900 mL |
| L-Glutamine | 40 g |
| Gradually add while stirring and applying low heat. | |
| Do not exceed 35 °C. | |

(continued)

| |
|---|
| Bring to Volume |
| Filter Sterilize |
| Store frozen* |
| *Note: Warm thawed stock in 31 °C bath to fully dissolve crystals. |

EXAMPLE 3

Chlorsulfuron Selection (ALS) and Plant Regeneration

Chlorsulfuron (ALS) Selection:

Following bombardment, the tissue is divided between 2 flasks with fresh SB196 media and cultured as described in Example 2. Six to seven days post-bombardment, the SB196 is exchanged with fresh SB196 containing selection agent of 100 ng/mL chlorsulfuron (chlorsulfuron stock is 1 mg/mL in 0.01 N ammonium hydroxide). The selection media is refreshed weekly. Four to six weeks post selection, green, transformed tissue may be observed growing from untransformed, necrotic embryogenic clusters. Isolated, green tissue is removed and inoculated into multiwell plates containing SB196 and embryos are matured as described in Example 2.

Regeneration of Soybean Somatic Embryos Into Plants:

In order to obtain whole plants from embryogenic suspension cultures, the tissue must be regenerated. Embryos are matured as described in Example 2. After subculturing on medium SB103 for 3 weeks, individual embryos can be removed from the clusters and screened for alterations in their fatty acid compositions as described herein. It should be noted that any detectable phenotype, resulting from the expression of the genes of interest, could be screened at this stage. This would include, but not be limited to, alterations in fatty acid profile, protein profile and content, carbohydrate content, growth rate, viability, or the ability to develop normally into a soybean plant.

Matured individual embryos are desiccated by placing them into an empty, small petri dish (35 x 10 mm) for approximately 4 to 7 days. The plates are sealed with fiber tape (creating a small humidity chamber). Desiccated embryos are planted into SB71-4 medium where they are left to germinate under the same culture conditions described above. Germinated plantlets are removed from germination medium and rinsed thoroughly with water and then are planted in Redi-Earth in 24-cell pack tray, covered with clear plastic dome. After 2 weeks the dome is removed and plants hardened off for a further week. If plantlets looked hardy they are transplanted to 10" pot of Redi-Earth with up to 3 plantlets per pot. After 10 to 16 weeks, mature seeds are harvested, chipped and analyzed for trait of interest.

EXAMPLE 4

Construction of Soybean Expression Vector pKR1183 for Expression of a *Euglena anabaena* delta-9 elongase-*Tetruetreptia pomquetensis* CCMP1491 Delta-8 Desaturase Fusion Gene (Hybrid1-HGLA Synthase)

An in-frame fusion between the *Euglena anabaena* delta-9 elongase (EaD9Elo1; SEQ ID NO:11), the *Euglena gracilis* DHA synthase 1 proline-rich linker (EgDHAsyn1Link; SEQ ID NO:33) and the *Tetruetreptia pomquetensis* CCMP1491 delta-8 desaturase (TpomD8; SEQ ID NO:26;) was constructed using the conditions described below (see also Applicants' Assignee's Application No. 12/061,738 (filed April 3, 2008, which published October 16, 2008 under No. US 2008/0254191)).

An initial in-frame fusion between the EaD9Elo1 and the EgDHAsyn1Link (EaD9elo-EgDHAsyn1Link) was made, flanked by an *Nco*I site at the 5'end and a *Not*I site at the 3' end, by PCR amplification. EaD9Elo1 (SEQ ID NO:39) was amplified from pLF121-1 (SEQ ID NO:38) with oligonucleotides EaD9-5Bbs (SEQ ID NO:41) and EaD9-3fusion (SEQ ID NO:42), using the Phusion™ High-Fidelity DNA Polymerase (Cat. No. F553S, Finnzymes Oy, Finland) following the manufacturer's protocol. EgDHAsyn1Link (SEQ ID NO:33) was amplified in a similar way from pKR1049 (described in Applicants' Assignee's application having U.S. Application No. 12/061,738 (filed April 3, 2008, which published October 16, 2008; Attorney Docket No. BB-1585 USNA)), with oligonucleotides EgDHAsyn1Link-5fusion (SEQ ID NO:43) and MWG511 (SEQ ID NO:28). The two resulting PCR products were combined and re-amplified using EaD9-5Bbs (SEQ ID NO:41) and MWG511 (SEQ ID NO:28) to form EaD9Elo1-EgDHAsyn1Link. The sequence of the EaD9Elo1-EgDHAsyn1Link is shown in SEQ ID NO:44. EaD9Elo1-EgDHAsyn1Link does not contain an in-frame stop codon upstream of the *Not*I site at the 3' end and therefore, a DNA fragment cloned into the *Not*I site can give rise to an in-frame fusion with the EgD9elo1-EgDHAsyn1Link if the correct frame is chosen. EaD9Elo1-EgDHAsyn1Link was cloned into the pCR-Blunt® cloning vector using the Zero Blunt® PCR Cloning Kit (Invitrogen Corporation), following the manufacturer's protocol, to produce pLF124 (SEQ ID NO:45).

The *Bbs*I/*Not*I DNA fragment of pLF124 (SEQ ID NO:45), containing EaD9Elo1-EgDHAsyn1Link, was cloned into the *Nco*I/*Not*I DNA fragment from KS366 containing the promoter for the α' subunit of β-conglycinin, to produce pKR1177 (SEQ ID NO:46).

The *Bam*HI DNA fragment of pKR1177 (SEQ ID NO:46), containing EaD9Elo1-EgDHAsyn1Link, was cloned into the *Bam*HI DNA fragment of pKR325, previously described in PCT Publication No. WO 2006/012325 to produce pKR1179 (SEQ ID NO:47).

The *Not*I fragment from pLF114-10 (SEQ ID NO:27), containing TpomD8 was cloned into the *Not*I fragment of pKR1179 (SEQ ID NO:47) to produce pKR1183 (SEQ ID NO:48).

## EXAMPLE 5

## Expression of Chimeric Genes in Monocot Cells

A chimeric gene comprising a fusion gene encoding the instant multizyme in sense orientation with respect to the maize 27 kD zein promoter that is located 5' to the fusion gene, and the 10 kD zein 3' end that is located 3' to the fusion gene, can be constructed. The fusion gene may be generated by polymerase chain reaction (PCR) of cDNA clones using appropriate oligonucleotide primers. Cloning sites (Ncol or Smal) can be incorporated into the oligonucleotides to provide proper orientation of the DNA fragment when inserted into the digested vector pML103 as described below. Amplification is then performed in a standard PCR. The amplified DNA is then digested with restriction enzymes Ncol and Smal and fractionated on an agarose gel. The appropriate band can be isolated from the gel and combined with a 4.9 kb Ncol-Smal fragment of the plasmid pML103. Plasmid pML103 has been deposited under the terms of the Budapest Treaty at ATCC (American Type Culture Collection, 10801 University Blvd., Manassas, VA 20110-2209), and bears accession number ATCC 97366. The DNA segment from pML103 contains a 1.05 kb Sall-Ncol promoter fragment of the maize 27 kD zein gene and a 0.96 kb Smal-Sall fragment from the 3' end of the maize 10 kD zein gene in the vector pGem9Zf(+) (Promega). Vector and insert DNA can be ligated at 15°C overnight, essentially as described (Maniatis). The ligated DNA may then be used to transform *E. coli* XL1-Blue (Epicurian Coli XL-1 Blue□; Stratagene). Bacterial transformants can be screened by restriction enzyme digestion of plasmid DNA and limited nucleotide sequence analysis using the dideoxy chain termination method (Sequenase□ DNA Sequencing Kit; U.S. Biochemical). The resulting plasmid construct would comprise a chimeric gene encoding, in the 5' to 3' direction, the maize 27 kD zein promoter, a fusion gene encoding the instant polypeptides, and the 10 kD zein 3' region.

The chimeric gene described above can then be introduced into corn cells by the following procedure. Immature corn embryos can be dissected from developing caryopses derived from crosses of the inbred corn lines H99 and LH132. The embryos are isolated 10 to 11 days after pollination when they are 1.0 to 1.5 mm long. The embryos are then placed with the axis-side facing down and in contact with agarose-solidified N6 medium (Chu et al. (1975) Sci. Sin. Peking 18:659-668). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and subcultured on this medium every 2 to 3 weeks.

The plasmid, p35S/Ac (obtained from Dr. Peter Eckes, Hoechst Ag, Frankfurt, Germany) may be used in transformation experiments in order to provide for a selectable marker. This plasmid contains the *Pat* gene (see European Patent Publication 0 242 236) which encodes phosphinothricin acetyl transferase (PAT). The enzyme PAT confers resistance to herbicidal glutamine synthetase inhibitors such as phosphinothricin. The *pat* gene in p35S/Ac is under the control of the 35S promoter from Cauliflower Mosaic Virus (Odell et al. (1985) Nature 313:810-812) and the 3' region of the nopaline synthase gene from the T-DNA of the Ti plasmid of *Agrobacterium tumefaciens.*

The particle bombardment method (Klein et al. (1987) Nature 327:70-73) may be used to transfer genes to the callus culture cells. According to this method, gold particles (1 μm in diameter) are coated with DNA using the following technique. Ten μg of plasmid DNAs are added to 50 μL of a suspension of gold particles (60 mg per mL). Calcium chloride (50 μL of a 2.5 M solution) and spermidine free base (20 μL of a 1.0 M solution) are added to the particles. The suspension is vortexed during the addition of these solutions. After 10 minutes, the tubes are briefly centrifuged (5 sec at 15,000 rpm) and the supernatant removed. The particles are resuspended in 200 μL of absolute ethanol, centrifuged again and the supernatant removed. The ethanol rinse is performed again and the particles resuspended in a final volume of 30 μL of ethanol. An aliquot (5 μL) of the DNA-coated gold particles can be placed in the center of a Kapton□ flying disc (Bio-Rad Labs). The particles are then accelerated into the corn tissue with a Biolistic□ PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains gluphosinate (2 mg per liter) and lacks casein or proline. The tissue continues to grow slowly on this medium. After an additional 2 weeks the tissue can be transferred to fresh N6 medium containing gluphosinate. After 6 weeks, areas of about 1 cm in diameter of actively growing callus can be identified on some of the plates containing the gluphosinate-supplemented medium. These calli may continue to grow when sub-cultured on the selective medium.

Plants can be regenerated from the transgenic callus by first transferring clusters of tissue to N6 medium supplemented with 0.2 mg per liter of 2,4-D. After two weeks the tissue can be transferred to regeneration medium (Fromm et al. (1990) Bio/Technology 8:833-839).

EXAMPLE 6

Synthesis Of Multizymes For Herbicide Resistance

This example describes the synthesis of multizymes wherein the use of the multizyme in a reaction protects an activity of the multizyme from competitive inhibition by a substrate other than that produced by a preceding enzymatic activity of the multizyme.

HPPD (4-hydroxyphenylpyruvate dioxygenase) is a known herbicide target and inhibition of HPPD activity leads to plant bleaching and death (Mayonado et al. (1989) Pestic. Biochem. Physiol. 35:138-145; Schultz et al. (1993) FEBS lett. 318:162-166; Secor (1994) Plant Phys. 106:1429-1433). HPPD inhibitors such as diketonitrile are competitive with p-hydroxyphenylpyruvate, the substrate of HPPD. The level of tolerance to different HPPD-inhibitors may vary depending on the HPPD enzyme. HPPD catalyzes the second step in the pathway for the catabolism of tyrosine, that is common to essentially all aerobic forms of life (Moran GR, Archives of biochemistry and biophysics 2005 433:117-28). In plants, HPPD is essential in the biosynthesis of tocopherols (vitamin E) and plastoquinones, which are involved in electron transport during photosynthesis.

In order to produce plants containing HPPD herbicide resistance, it is desirable to reduce the susceptibility of HPPD to herbicides. Fusing HPPD with a prokaryotic prephenate dehydrogenase (PDH) or bifunctional chorismate mutase/prephenate dehydrogenase or a plant prephenate dehydrogenase (PDH) (or another suitable source of hydroxyphenylpyruvate such as tyrosine aminotransferase) can protect the HPPD from competitive-substrate inhibition and create a herbicide-resistant HPPD activity.

An HPPD/PDH multizyme is created by joining HPPD with a prokaryotic or plant PDH or with a prokaryotic bifunctional chorismate mutase/PDH. An HPPD/ tyrosine aminotransferase multizyme is created by joining an HPPD and a plant or microbial tyrosine aminotransferase.

HPPD genes from many organism have been identified to date and are well known in the art. Some examples include, but are not limited to HPPDs from carrots (NCBI GI:2231615), barley (NCBI GI::2695710), and *Arabidopsis thaliana* (Garcia,I., Rodgers et al. 1999 Plant Physiol. 119:1507-1516) with at least two different variants existing in this last plant; HPPD cDNA's from rice, soybean, Vernonia, Catalpa described in U.S. Patent 7226745 (SEQ ID NOs: 11 & 13, 15, 17, 19 , 31 of U.S. Patent 7226745 respectively); *Mycosphaerella graminicola* (Keon J and Hargreaves J. FEMS Microbiol Lett. 1998 Apr 15;161(2):337-43.), Human (Awata H, Endo F, Matsuda I. Genomics. 1994 Oct;23(3):534-9 and *Coccidiodes immitis.* (Wyckoff EE, Pishko EJ, Kirkland TN, Cole GT. Gene. 1995 Aug 8;161(1):107-11.) carrots (NCBI GI:2231615), barley (NCBI GI::2695710), and *Arabidopsis thaliana* (Garcia,I., Rodgers et al. 1999 Plant Physiol. 119:1507-1516) maize (US 20030066102); cotton, tomato, canola (US 20050289664); wheat, oat, sorghum and other grasses (US 20040058427); *Pseudomonas* (US 6,245,986). Microbial Prephenate dehydrogenase genes include, but are not limited to, *Candida albicans* (NCBI GI:3635639), *Saccharomyces cerevisiae*(NCBI GI: 852464), *Frankia sp. EAN1pec*(NCBI GI: 5673396),*Bacillus licheniformis ATCC 14580* (NCBI GI: 3099695), *Bacillus cereus* (NCBI GI: 1205287) and Aquifex aeolicus (Bonvin J, et al. Protein Sci. 2006 15(6):1417-32). Plant genes that have been annotated as coding for prephenate dehydrogenase include those in Arabidopsis thaliana (NCBI GI:15218283) and white spruce (NCBI, GI:1350504), but the corresponding enzyme activities have not been demonstrated. Prephenate dehydrogenase activity has not been observed in non-legumes (Siehl, DL, Biosynthesis of Tryptophan, Tyrosine and Phenylalanine from Chorismate, in Plant Amino Acids: Biochemistry and Biotechnology, Marcel Dekker, 1999). Instead, non-legume genes annotated as coding for prephenate dehydrogenase likely are arogenate dehydrogenases. Extracts of soybean leaves have prephenate dehydrogenase activity that can be chromatographically resolved from arogenate dehydrogenase (Siehl, DL, ibid). The soybean genes coding for these activities have not been identified. A tyrosine aminotransferase gene in *Arabidopsis thaliana* is present in NCBI (GI: 817022).

The independent and separate activities of the HPPD/PDH multizyme or the HPPD/tyrosine aminotransferase multizyme (e.g, HPPD, PDH, bifunctional chorismate mutase/PDH or tyrosine aminotransferase) are joined together using a linker derived from a sequence of a DHA synthase locus of a Euglenoid. An example of such a linker is the EgDHAsyn1 proline-rich linker (SEQ ID NO:34). The gene fusion encoding the multizyme may be produced as described in Example 4, or as described by Examples 15-24 of Applicants' Assignee's application having U.S. Application No. 12/061,738 (filed April 3, 2008, which published October 16, 2008 under No. US 2008/0254191). Furthermore, any number of methods known to those skilled in the art may be used to produce the gene fusion. Additional nucleotides can be added to the 3' end of the EgDHAsyn1 proline-rich linker sequence to enable cloning when making the fusions for all constructs. Thus, an additional 4 amino acids can be included between the end of the EgDHAsyn1 proline-rich linker (SEQ ID NO:34; PARPAGLPPATYYDSLAV) and the start of the bifunctional chorismate mutase/PDH, PDH or tyrosine aminotransferase used.

For comparison, constructs that co-expressed the independent and separate activities of the multizyme (HPPD, bifunctional chorismate mutase/PDH, PDH and tyrosine aminotransferase) are also created. The multizyme may be engineered with a protease recognition site at the fusion point so that fusion partners can be separated by protease digestion to yield intact mature enzyme. Examples of such proteases include thrombin, enterokinase and factor Xa. However, any protease can be used which specifically cleaves the peptide connecting the multiple enzymes.

The multizyme fusion gene can be expressed in either microbial cells as described in General Methods, monocot cells as described in EXAMPLE 5 or dicot cells as described in Example 2 and 3, or expression in eukaryotic cell culture, *in planta,* and using viral expression systems in suitably infected organisms or cell lines. Purification of the instant multizymes, if desired, may utilize any number of separation technologies familiar to those skilled in the art of protein purification. Examples of such methods include, but are not limited to, homogenization, filtration, centrifugation, heat denaturation, ammonium sulfate precipitation, desalting, pH precipitation, ion exchange chromatography, hydrophobic interaction chromatography and affinity chromatography, wherein the affinity ligand represents a substrate, substrate analog or inhibitor. The purification protocol may include the use of an affinity resin containing ligands which are specific for the independent and separate activities of the multizyme.

Crude, partially purified or purified enzyme may be utilized in assays for the evaluation of compounds for their ability to inhibit enzymatic activation of the instant multizymes disclosed herein. Assays may be conducted under well known experimental conditions which permit optimal enzymatic activity. For example, assays for HPPD are presented by Norris et al. (1995) Plant Cell 7: 2139-2149; assays for chorismate mutase by Kim SK, et al. 2006 (J Bacteriol. 2006, 188(24):8638-48) and for prephenate dehydrogenase assays by Xu S, et al. 2006. Protein Expr Purif. 49(2):151-8.

EXAMPLE 7

Synthesis Of Multizymes For Increased Cytokinin Production

This example describes the synthesis of multizymes having at least two independent and separable activities wherein the independent and separable activities are joined together using a linker derived from a sequence of a DHA synthase locus of a Euglenoid and wherein the product of a first reaction of the multizyme is substantially used in a subsequent reaction of the multizyme.

Cytokinins (CKs) are plant hormones that play a role in cell division, cell differentiation, release of lateral buds from apical dominance, and delay of senescence. (Mok, D. W., and Mok, M. C. (2001) Annu. Rev. Plant Physiol. Plant Mol. Biol. 52, 89-1181). Most natural CKs, including isopentenyladenine (iP) and *trans-zeatin* (tZ) are derivatives of $N^6$-prenylated adenine. (Mok, D. W., and Mok, M. C. (2001) Annu. Rev. Plant Physiol. Plant Mol. Biol. 52, 89-1181). In CK biosynthesis, the prenylation of AMP, ADP or ATP is catalyzed by adenosine phosphate isopentenyltransferase (adenosine phosphate -IPT; EC 2.5.1.27), which utilizes dimethylallyl diphosphate (DMAPP) as a substrate. This enzyme activity leads to the formation of isopentenyladenine riboside monophosphate (iPRMP) (Taya, Y., Tanaka, Y., and Nishimura, S. (1978) Nature 271, 545-547). Subsequent conversion of iPRMP to iP is catalyzed by 5'-nucleotidase and adenosine nucleosidase (Chen, C.-M., and Kristopeit, S. M. (1981) Plant Physiol. 67, 494-498).

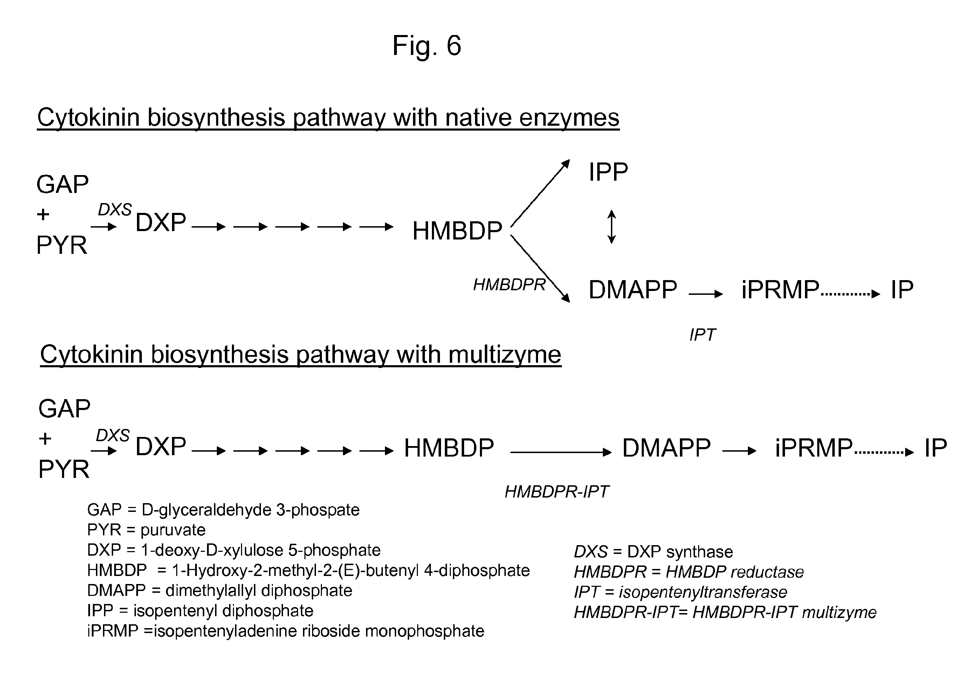

Isoprenoids are synthesized through the condensation of five-carbon intermediates, isopentenyl diphosphate (IPP) and dimethylallyl diphosphate (DMAPP), derived form the 2-C-methyl-D-erythritol 4-phosphate (MEP) pathway in plastids and the mevalonate (MVA) pathway in the cytosol. (FIG. 6) Kasahara et al 2004 (J. Biol. Chem. 2004;279:14049-14054) demonstrated a critical contribution of the plastid-located MEP pathway in providing the prenyl precursor to tZ- and iP-type CKs in *Arabidopsis* seedlings. In the MEP pathway, 1-Hydroxy-2-methyl-2-(E)-butenyl 4-diphosphate (HMBDP) is reduced by HMBDP reductase (IDS; EC 1.17.1.2) to produce both IPP and DMAPP (Altincicek B, Kollas A, Eberl M, Wiesner J, Sanderbrand S, Hintz M, Beck E, Jomaa H (2001) LytB, a novel gene of the 2-C-methyl-D-erythritol 4-phosphate pathway of isoprenoid biosynthesis in Escherichia coli. FEBS Lett 499:37-40; Hecht S, Eisenreich W, Adam P, Amslinger S, Kis K, Bacher A, Arigoni D, Rohdich F (2001) Studies on the nonmevalonate pathway to terpenes: the role of the GcpE (IspG) protein. Proc Natl Acad Sci USA 98:14837-14842).

A fusion with HMBDP reductase and IPT can channel carbon into cytokinin by maximizing DMAPP production and reducing IPP formation (FIG. 6). Such a multizyme can also serve to divert more DMAPP into the CK pathway rather than to isoprenenoids. Furthermore, the use of cell-cycle specific promoters (as described in US2006/0010515A1) to drive the expression of the HMBDP-IPT fusion gene may result a yield increase

A HMBDP/IPT multizyme is created by joining an HMBDP reductase and IPT. IPT genes from several organisms have been identified to date (Takei, et al T. (2001) J. Biol. Chem. 276,, 26405-26410, Kakimoto, (2001) Plant Cell Physiol. 42, 677-685; Sakamoto et al Plant Physiol 2006 Sep;142(1):54-62; Sugawara et al Proc Natl Acead Sci USA 2008 Feb 19;105(7):2734-9). Plant and microbial HMBDP reductase IPT genes have also been identified (Campos et al Biochem J 2001 Jan 1;353(Pt 1):59-67; Lu et al Mol Biol Rep 2007 2007 May 26 [Epub ahead of print]; Kim et al Planta 2008 Jan;227(2):287-98)

The HMBDP reductase and IPT are joined together using a linker derived from a sequence of a DHA synthase locus of a Euglenoid. An example of such a linker is the EgDHAsyn1 proline-rich linker (SEQ ID NO:34). The gene fusion encoding the multizyme may be produced as described in Example 4, or as described by Examples 15-24 of Applicants' Assignee's application having U.S. Application No. 12/061,738 (filed April 3, 2008, which published October 16, 2008 under No. US 2008/0254191). Furthermore, any number of methods known to those skilled in the art may be used to produce the gene fusion. Additional nucleotides can be added to the 3' end of the EgDHAsyn1 proline-rich linker sequence to enable cloning when making the fusions for all constructs. Thus, an additional 4 amino acids can be included between the end of the EgDHAsyn1 proline-rich linker (SEQ ID NO:34; PARPAGLPPATYYDSLAV) and the start of HMBDP reductase or the IPT used.

For comparison, constructs that co-expressed the independent and separate activities of the multizyme (HMBDP reductase and IPT) are also created. The multizymes may be engineered with a protease recognition site at the fusion point so that fusion partners can be separated by protease digestion to yield intact mature enzyme. Examples of such proteases include thrombin, enterokinase and factor Xa. However, any protease can be used which specifically cleaves the peptide connecting the multiple enzymes.

The multizyme fusion gene can be expressed in either microbial cells as described in General Methods, monocot cells as in Example 5 or dicot cells as described in Example 2 and 3, or expression in eukaryotic cell culture, *in planta,* and using viral expression systems in suitably infected organisms or cell lines. Purification of the instant multizymes, if desired, may utilize any number of separation technologies familiar to those skilled in the art of protein purification. Examples of such methods include, but are not limited to, homogenization, filtration, centrifugation, heat denaturation, ammonium sulfate precipitation, desalting, pH precipitation, ion exchange chromatography, hydrophobic interaction chromatography and affinity chromatography, wherein the affinity ligand represents a substrate, substrate analog or inhibitor. The purification protocol may include the use of an affinity resin containing ligands which are specific for the independent and separate activities of the multizyme.

Crude, partially purified or purified enzyme may be utilized in assays for the evaluation of compounds for their ability to inhibit enzymatic activation of the instant multizymes disclosed herein. Assays may be conducted under well known experimental conditions which permit optimal enzymatic activity. For example, assays for HMBDP reductase (also referred to as *lytB* or IDS or isopentenyl diphosphate/dimethylalyl diphosphate synthase) are presented by Rohdich F, Hecht S, Gärtner K, Adam P, Krieger C, Amslinger S, Arigoni D, Bacher A, Eisenreich W (2002) Studies on the nonmevalonate terpene biosynthetic pathway: metabolic role of IspH (LytB) protein. Proc Natl Acad Sci USA 99: 1158-1163) and assays for IPT by Kakimoto, T. (2001) Plant Cell Physiol. 42, 677-685 and Takei, et al . (2003) J. Plant Res. 116, 265-269.

Hence, this example describes the synthesis of multizymes having at least two independent and separable activities (HMBDP reductase and IPT) wherein the independent and separable activities are joined together using a linker derived from a sequence of a DHA synthase locus of a Euglenoid and wherein the product of a first reaction of the multizyme (DMAPP) is substantially used in a subsequent reaction (IPT) of the multizyme. In this manner, the production of IPP can be reduced to a minimum due to the unique properties of the multizyme.

## EXAMPLE 8

### Synthesis Of Multizymes wherein at least one independent and separable activity or the multizyme is prevented from associating with polypeptide complexes other than the multizyme of which it is a part.

This example describes the synthesis of a multizyme wherein at least one independent and separable activity of the multizyme is prevented from associating with polypeptide complexes other than the multizyme of which it is a part.

The availability of crops that have an efficient and effective resistance to herbicides continues to be of great importance to growers. Developing more effective resistance mechanism can allow for better weed management. A highly herbicide-resistant acetolactate synthase (HRA) has been described (Bedbrook J.R., Chaleff R.S., Falco S.C.,

Mazur B.J., Somerville C.R., and Yadev N.S. 1995). Nucleic acid fragment encoding herbicide resistant plant acetolactate synthase. U. S. Patent 5,378,824.; Lee K.Y., Townsend J., Tepperman J., Black M., Chui C.F., Mazur B., Dunsmuir P., and Bedbrook J. 1988. The resistance mechanism to ALS-inhibiting herbicides is a double-mutant, highly resistant ALS (HRA) that is insensitive to all five classes of ALS herbicides (J. Green. 2006 Weed technology 21:547-558). Plants such as soybean produce a native acetolactate synthase (ALS) that can form a heterodimer with HRA in plant expressing the HRA monomer. In order to increase the herbicide resistance of HRA, it is desirable to prevent the formation of the HRA and ALS heterodimer and instead stimulate the formation of a HRA-HRA homodimer.

A HRA/HRA multizyme is made by joining an herbicide resistant acetolactate (HRA) synthase peptide to a second acetolactate synthase peptide. The acetolactate synthases are joined together using a linker derived from a sequence of a DHA synthase locus of a Euglenoid. An example of such a linker is the EgDHAsyn1 proline-rich linker (SEQ ID NO:34). The gene fusion encoding the multizyme may be produced as described in Example 4, described herein, or as described by Examples 15-24 of Applicants' Assignee's application having U.S. Application No. 12/061,738 (filed April 3, 2008, which published October 16, 2008 under No. US 2008/0254191),. Furthermore, any number of methods known to those skilled in the art may be used to produce the gene fusion. Additional nucleotides can be added to the 3' end of the EgDHAsyn1 proline-rich linker sequence to enable cloning when making the fusions for all constructs. Thus, an additional 4 amino acids can be included between the end of the EgDHAsyn1 proline-rich linker (SEQ ID NO:34; PARPAGLPPATYYDSLAV) and the start of the second HRA used. The HRA- EgDHAsyn1 proline-rich linker -HRA gene fusion encodes a homodimer that can prevent HRA peptides to form a heterodimer with a native acetolactase synthase (ALS).

The multizyme fusion gene can be expressed in either microbial cells as described in General Methods, monocot cells as described in Example 5 or dicot cells as described in Example 2 and 3, or expression in eukaryotic cell culture, *in planta,* and using viral expression systems in suitably infected organisms or cell lines. Purification of the instant multizymes, if desired, may utilize any number of separation technologies familiar to those skilled in the art of protein purification.

For comparison, a construct that expresses the monomer of HRA is also created using techniques well known in the art. Such a construct encodes a monomer of HRA that will form a heterodimer with the native ALS when expressed in a plant tissue that enables expression of native ALS.

Plants expressing the HRA-HRA homodimer versus the HRA-ALS heterodimer can be evaluated for altered herbicide resistance or altered agronomic properties. The multizyme can be evaluated by making transgenic soy plants and spraying them with sulfonyl ureas like rimsulfuron (Resolve) to see if they have improved tolerance using herbicide greenhouse tests well known to one skilled in the art.

EXAMPLE 9

Synthesis of Multizymes for increased Nitrogen Utilization

This example describes the synthesis of a multizyme wherein a product of an enzymatic reaction of the multizyme is channeled into a transporter activity which is comprised by the activity of the multizyme.

A NR / NT multizyme is created by joining a soluble nitrate reductase (NR) to a nitrate transporter (NT). The NR and the NT are joined together using a linker derived from a sequence of a DHA synthase locus of a Euglenoid. An example of such a linker is the EgDHAsyn1 proline-rich linker (SEQ ID NO:34). The gene fusion encoding the multizyme may be produced as described in Example 4, or as described by Examples 15-24 of Applicants' Assignee's application having U.S. Application No. 12/061,738 (filed April 3, 2008, which published October 16, 2008 under No. US 2008/0254191). Furthermore, any number of methods known to those skilled in the art may be used to produce the gene fusion. Additional nucleotides can be added to the 3' end of the EgDHAsyn1 proline-rich linker sequence to enable cloning when making the fusions for all constructs. Thus, an additional 4 amino acids can be included between the end of the EgDHAsyn1 proline-rich linker (SEQ ID NO:34; PARPAGLPPATYYDSLAV) and the start of the NT used. The NR/NT multizyme can channel nitrogen through the transporter more effectively when compared to an individual and separate nitrate transported. Hence, the multizyme enables the product of the first protein (e. g. the nitrate reductase) to be channeled into a second protein which is a nitrate transporter resulting in an increased nitrogen uptake and utilization.

The NR/NT fusion gene can be expressed in either microbial cells as described in General Methods, monocot cells as described in Example 5 or dicot cells as described in Example 2 and 3, or expression in eukaryotic cell culture, *in planta,* and using viral expression systems in suitably infected organisms or cell lines. Purification of the instant multizymes, if desired, may utilize any number of separation technologies familiar to those skilled in the art of protein purification.

For comparison, a construct that expresses the independent and separate activities of the multizyme (the NR and the nitrate transporter) are also created using techniques well known in the art. Plants expressing the NR/NT multizyme can be evaluated for altered nitrogen utilization using as described in, but not limited to, Example 5, or evaluated for altered agronomic traits using methods well known in the art.

EXAMPLE 10

Transformation of Gaspe Flint Derived Maize Lines containing the NR/NT multizyme

Maize plants can be transformed as described in Example 5 to express the NR/NT gene fusion of Example 9. In addition to the promoters previously described other promoters such the S2B promoter, the maize ROOTMET2 promoter, the maize Cyclo, the CR1 BIO, the CRWAQ81 and the maize ZRP2.4447 may be useful in expressing genes in maize. Furthermore, a variety of terminators, such as, but not limited to the PINII terminator, can be used to achieve expression of the gene of interest in Gaspe Flint Derived Maize Lines.

Recipient Plants

Recipient plant cells can be from a uniform maize line having a short life cycle ("fast cycling"), a reduced size, and high transformation potential. Typical of these plant cells for maize are plant cells from any of the publicly available Gaspe Flint (GBF) line varieties. One possible candidate plant line variety is the F1 hybrid of GBF x QTM (Quick Turnaround Maize, a publicly available form of Gaspe Flint selected for growth under greenhouse conditions) disclosed in Tomes et al. U.S. Patent Application Publication No. 2003/0221212. Transgenic plants obtained from this line are of such a reduced size that they can be grown in four inch pots (1/4 the space needed for a normal sized maize plant) and mature in less than 2.5 months. (Traditionally 3.5 months is required to obtain transgenic T0 seed once the transgenic plants are acclimated to the greenhouse.) Another suitable line is a double haploid line of GS3 (a highly transformable line) X Gaspe Flint. Yet another suitable line is a transformable elite inbred line carrying a transgene which causes early flowering, reduced stature, or both.

Transformation Protocol

Any suitable method may be used to introduce the transgenes into the maize cells, including but not limited to inoculation type procedures using *Agrobacterium* based vectors. Transformation may be performed on immature embryos of the recipient (target) plant.

Precision Growth and Plant Tracking

The event population of transgenic (TO) plants resulting from the transformed maize embryos is grown in a controlled greenhouse environment using a modified randomized block design to reduce or eliminate environmental error. A randomized block design is a plant layout in which the experimental plants are divided into groups (e.g., thirty plants per group), referred to as blocks, and each plant is randomly assigned a location with the block.

For a group of thirty plants, twenty-four transformed, experimental plants and six control plants (plants with a set phenotype) (collectively, a "replicate group") are placed in pots which are arranged in an array (a.k.a. a replicate group or block) on a table located inside a greenhouse. Each plant, control or experimental, is randomly assigned to a location with the block which is mapped to a unique, physical greenhouse location as well as to the replicate group. Multiple replicate groups of thirty plants each may be grown in the same greenhouse in a single experiment. The layout (arrangement) of the replicate groups should be determined to minimize space requirements as well as environmental effects within the greenhouse. Such a layout may be referred to as a compressed greenhouse layout.

An alternative to the addition of a specific control group is to identify those transgenic plants that do not express the gene of interest. A variety of techniques such as RT-PCR can be applied to quantitatively assess the expression level of the introduced gene. T0 plants that do not express the transgene can be compared to those which do.

Each plant in the event population is identified and tracked throughout the evaluation process, and the data gathered from that plant is automatically associated with that plant so that the gathered data can be associated with the transgene carried by the plant. For example, each plant container can have a machine readable label (such as a Universal Product Code (UPC) bar code) which includes information about the plant identity, which in turn is correlated to a greenhouse location so that data obtained from the plant can be automatically associated with that plant.

Alternatively any efficient, machine readable, plant identification system can be used, such as two-dimensional matrix codes or even radio frequency identification tags (RFID) in which the data is received and interpreted by a radio frequency receiver/processor. See U.S. Published Patent Application No. 2004/0122592.

Phenotypic Analysis Using Three-Dimensional Imaging

Each greenhouse plant in the T0 event population, including any control plants, is analyzed for agronomic characteristics of interest, and the agronomic data for each plant is recorded or stored in a manner so that it is associated with the identifying data (see above) for that plant. Confirmation of a phenotype (gene effect) can be accomplished in the T1 generation with a similar experimental design to that described above.

The T0 plants are analyzed at the phenotypic level using quantitative, nondestructive imaging technology throughout the plant's entire greenhouse life cycle to assess the traits of interest. Preferably, a digital imaging analyzer is used for automatic multi-dimensional analyzing of total plants. The imaging may be done inside the greenhouse. Two camera systems, located at the top and side, and an apparatus to rotate the plant, are used to view and image plants from all sides. Images are acquired from the top, front and side of each plant. All three images together provide sufficient information to evaluate the biomass, size and morphology of each plant.

Due to the change in size of the plants from the time the first leaf appears from the soil to the time the plants are at the end of their development, the early stages of plant development are best documented with a higher magnification from the top. This may be accomplished by using a motorized zoom lens system that is fully controlled by the imaging software.

In a single imaging analysis operation, the following events occur: (1) the plant is conveyed inside the analyzer area, rotated 360 degrees so its machine readable label can be read, and left at rest until its leaves stop moving; (2) the side image is taken and entered into a database; (3) the plant is rotated 90 degrees and again left at rest until its leaves stop moving, and (4) the plant is transported out of the analyzer.

Plants are allowed at least six hours of darkness per twenty four hour period in order to have a normal day/night cycle.

Imaging Instrumentation

Any suitable imaging instrumentation may be used, including but not limited to light spectrum digital imaging instrumentation commercially available from LemnaTec GmbH of Wurselen, Germany. The images are taken and analyzed with a LemnaTec Scanalyzer HTS LT-0001-2 having a 1/2" IT Progressive Scan IEE CCD imaging device. The imaging cameras may be equipped with a motor zoom, motor aperture and motor focus. All camera settings may be made using LemnaTec software. Preferably, the instrumental variance of the imaging analyzer is less than about 5% for major components and less than about 10% for minor components.

Software

The imaging analysis system comprises a LemnaTec HTS Bonit software program for color and architecture analysis and a server database for storing data from about 500,000 analyses, including the analysis dates. The original images and the analyzed images are stored together to allow the user to do as much reanalyzing as desired. The database can be connected to the imaging hardware for automatic data collection and storage. A variety of commercially available software systems (e.g. Matlab, others) can be used for quantitative interpretation of the imaging data, and any of these software systems can be applied to the image data set.

Conveyor System

A conveyor system with a plant rotating device may be used to transport the plants to the imaging area and rotate them during imaging. For example, up to four plants, each with a maximum height of 1.5 m, are loaded onto cars that travel over the circulating conveyor system and through the imaging measurement area. In this case the total footprint of the unit (imaging analyzer and conveyor loop) is about 5 m x 5 m.

The conveyor system can be enlarged to accommodate more plants at a time. The plants are transported along the conveyor loop to the imaging area and are analyzed for up to 50 seconds per plant. Three views of the plant are taken. The conveyor system, as well as the imaging equipment, should be capable of being used in greenhouse environmental conditions.

Illumination

Any suitable mode of illumination may be used for the image acquisition. For example, a top light above a black background can be used. Alternatively, a combination of top- and backlight using a white background can be used. The illuminated area should be housed to ensure constant illumination conditions. The housing should be longer than the measurement area so that constant light conditions prevail without requiring the opening and closing or doors. Alternatively, the illumination can be varied to cause excitation of either transgene (e.g., green fluorescent protein (GFP), red fluorescent protein (RFP)) or endogenous (e.g. Chlorophyll) fluorophores.

Biomass Estimation Based on Three-Dimensional Imaging

For best estimation of biomass the plant images should be taken from at least three axes, preferably the top and two side (sides 1 and 2) views. These images are then analyzed to separate the plant from the background, pot and pollen control bag (if applicable). The volume of the plant can be estimated by the calculation:

$$Volume(voxels) = \sqrt{TopArea(pixels)} \times \sqrt{Side1Area(pixels)} \times \sqrt{Side2Area(pixels)}$$

In the equation above the units of volume and area are "arbitrary units". Arbitrary units are entirely sufficient to detect gene effects on plant size and growth in this system because what is desired is to detect differences (both positive-larger and negative-smaller) from the experimental mean, or control mean. The arbitrary units of size (e.g. area) may be trivially converted to physical measurements by the addition of a physical reference to the imaging process. For instance, a physical reference of known area can be included in both top and side imaging processes. Based on the area of these physical references a conversion factor can be determined to allow conversion from pixels to a unit of area such as square centimeters ($cm^2$). The physical reference may or may not be an independent sample. For instance, the pot, with a known diameter and height, could serve as an adequate physical reference.

Color Classification

The imaging technology may also be used to determine plant color and to assign plant colors to various color classes. The assignment of image colors to color classes is an inherent feature of the LemnaTec software. With other image analysis software systems color classification may be determined by a variety of computational approaches.

For the determination of plant size and growth parameters, a useful classification scheme is to define a simple color scheme including two or three shades of green and, in addition, a color class for chlorosis, necrosis and bleaching, should these conditions occur. A background color class which includes non plant colors in the image (for example pot and soil colors) is also used and these pixels are specifically excluded from the determination of size. The plants are analyzed under controlled constant illumination so that any change within one plant over time, or between plants or different batches of plants (e.g. seasonal differences) can be quantified.

In addition to its usefulness in determining plant size growth, color classification can be used to assess other yield component traits. For these other yield component traits additional color classification schemes may be used. For instance, the trait known as "staygreen", which has been associated with improvements in yield, may be assessed by a color classification that separates shades of green from shades of yellow and brown (which are indicative of senescing tissues). By applying this color classification to images taken toward the end of the T0 or T1 plants' life cycle, plants that have increased amounts of green colors relative to yellow and brown colors (expressed, for instance, as Green/Yellow Ratio) may be identified. Plants with a significant difference in this Green/Yellow ratio can be identified as carrying transgenes which impact this important agronomic trait.

The skilled plant biologist will recognize that other plant colors arise which can indicate plant health or stress response (for instance anthocyanins), and that other color classification schemes can provide further measures of gene action in traits related to these responses.

Plant Architecture Analysts

Transgenes which modify plant architecture parameters may also be identified using the present invention, including such parameters as maximum height and width, internodal distances, angle between leaves and stem, number of leaves starting at nodes and leaf length. The LemnaTec system software may be used to determine plant architecture as follows. The plant is reduced to its main geometric architecture in a first imaging step and then, based on this image, parameterized identification of the different architecture parameters can be performed. Transgenes that modify any of these architecture parameters either singly or in combination can be identified by applying the statistical approaches previously described.

Pollen Shed Date

Pollen shed date is an important parameter to be analyzed in a transformed plant, and may be determined by the first appearance on the plant of an active male flower. To find the male flower object, the upper end of the stem is classified by color to detect yellow or violet anthers. This color classification analysis is then used to define an active flower, which in turn can be used to calculate pollen shed date.

Alternatively, pollen shed date and other easily visually detected plant attributes (e.g. pollination date, first silk date) can be recorded by the personnel responsible for performing plant care. To maximize data integrity and process efficiency this data is tracked by utilizing the same barcodes utilized by the LemnaTec light spectrum digital analyzing device. A computer with a barcode reader, a palm device, or a notebook PC may be used for ease of data capture recording time of observation, plant identifier, and the operator who captured the data.

Orientation of the Plants

Mature maize plants grown at densities approximating commercial planting often have a planar architecture. That is, the plant has a clearly discernable broad side, and a narrow side. The image of the plant from the broadside is determined. To each plant a well defined basic orientation is assigned to obtain the maximum difference between the broadside and edgewise images. The top image is used to determine the main axis of the plant, and an additional rotating device is used to turn the plant to the appropriate orientation prior to starting the main image acquisition.

EXAMPLE 11

Screening of transgenic Maize Lines

Expressing the NR/NT multizyme under Nitrogen Limiting Conditions

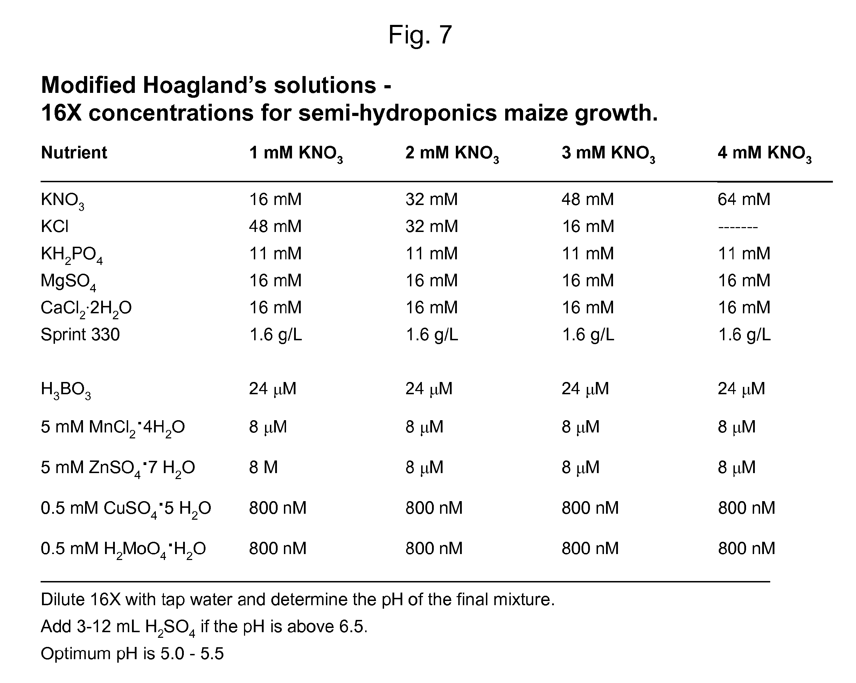

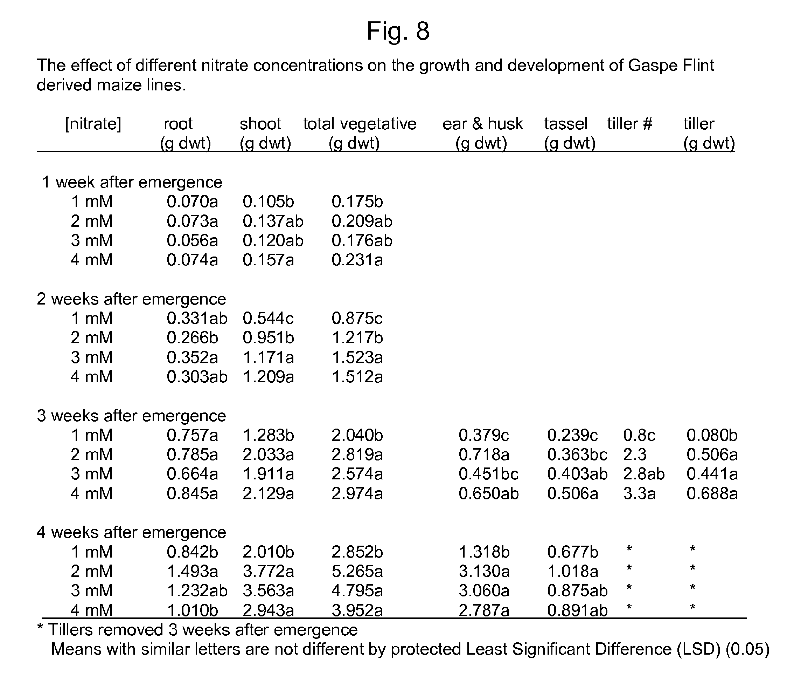

Transgenic plants containing the NR/NT multizyme are planted in Turface, a commercial potting medium, and watered four times each day with 1 mM $KNO_3$ growth medium and with 2 mM $KNO_3$, or higher, growth medium (see FIG. 7). Control plants grown in 1 mM $KNO_3$ medium will be less green, produce less biomass and have a smaller ear at anthesis (see FIG. 8 for an illustration of sample data).

Statistics are used to decide if differences seen between treatments are really different. FIG. 8 illustrates one method which places letters after the values. Those values in the same column that have the same letter (not group of letters) following them are not significantly different. Using this method, if there are no letters following the values in a column, then there are no significant differences between any of the values in that column or, in other words, all the values in that column are equal.

Expression of a transgene will result in plants with improved plant growth in 1 mM $KNO_3$ when compared to a transgenic null. Thus biomass and greenness (as described in Example 10) will be monitored during growth and compared to a transgenic null. Improvements in growth, greenness and ear size at anthesis will be indications of increased nitrogen tolerance.

EXAMPLE 12

Creation Of Multizymes For High Stearic-Low palmitic Oilseed plants

This example describes the creation of a multizyme wherein at least one activity of the multizyme that is capable of multiple functions when expressed as an individual polypeptide is restricted to a single function when it is part of the multizyme.

A multizyme is created by joining a type B acyl-ACP thioesterase (16:0/18:0 specific) with a beta-ketoacyl-ACP synthetase II protein (KAS II). The type B acyl-ACP thioesterase and KAS II are joined together using a linker derived from a sequence of a DHA synthase locus of a Euglenoid. An example of such a linker is the EgDHAsyn1 proline-rich linker (SEQ ID NO:34). The gene fusion encoding the multizyme may be produced as described in Example 4, or as described by Examples 15-24 of Applicants' Assignee's application having U.S. Application No. 12/061,738 (filed April 3, 2008, which published October 16, 2008 under No. US 2008/0254191). Furthermore, any number of methods known to those skilled in the art may be used to produce the gene fusion. Additional nucleotides can be added to the 3' end of the EgDHAsyn1 proline-rich linker sequence to enable cloning when making the fusions for all constructs. Thus, an additional 4 amino acids can be included between the end of the EgDHAsyn1 proline-rich linker (SEQ ID NO:34; PARPAGLPPATYYDSLAV) and the start of the KASII used.

For comparison, constructs that co-expressed the independent and separate activities of the multizyme (type B acyl-ACP thioesterase and KASII) are also created. The multizymes may be engineered with a protease recognition site at the fusion point so that fusion partners can be separated by protease digestion to yield intact mature enzyme. Examples of such proteases include thrombin, enterokinase and factor Xa. However, any protease can be used which specifically cleaves the peptide connecting the multiple enzymes.

The multizyme fusion gene can be expressed in either microbial cells as described in General Methods, monocot cells as described in Example 5 or dicot cells as described in Example 2 and 3, or expression in eukaryotic cell culture, *in planta,* and using viral expression systems in suitably infected organisms or cell lines. Purification of the instant multizymes, if desired, may utilize any number of separation technologies familiar to those skilled in the art of protein purification. Examples of such methods include, but are not limited to, homogenization, filtration, centrifugation, heat denaturation, ammonium sulfate precipitation, desalting, pH precipitation, ion exchange chromatography, hydrophobic interaction chromatography and affinity chromatography, wherein the affinity ligand represents a substrate, substrate analog or inhibitor.

Crude, partially purified or purified enzyme may be utilized in assays for the evaluation of compounds for their ability to inhibit enzymatic activation of the instant multizymes disclosed herein. Assays may be conducted under well known experimental conditions which permit optimal enzymatic activity. For example, an assay for KAS II (beta-ketoacyl-ACP synthetase) is described in US Patent 5,500,361 issued on March 19, 1996 and assays for thioesterase is described in US Reissued Patent RE37,317, reissued on August 7, 2001 Plants expressing acyl-ACP thioesterase / KASII multizyme can be evaluated for their oil quality composition. The expression of an acyl-ACP thioesterase / KASII multizyme in an oilseed plastid can result in the channeling of 18:0-ACP in to the thioesterase so that it releases only 18:0 and not 16:0 during plastidal acyl-ACP synthesis (and also prevent the 18:0 being desaturated). This may lead to the creation of an oilseed containing a high stearic, low palmitic oil.

## Claims

**1.** A multizyme comprising a single polypeptide having at least two independent and separable activities wherein the independent and separable activities are joined together using a linker, wherein said linker is a sequence of a DHA synthase locus of a Euglenoid comprising the amino acid sequence set forth in any one of SEQ ID NOs: 34, 36 or 50, or an amino acid sequence having at least 95% sequence identity therewith, and wherein said linker can be natural or synthetic and retains the ability to form a multizyme as if it were a linker from a Euglenoid DHA synthase locus, provided that the multizyme does not comprise an enzyme involved in fatty acid biosynthesis.

**2.** The multizyme of claim 1 wherein use of the multizyme increases total flux of all combined reactions of the multizyme when compared with the total flux of the combined reactions when each reaction is produced separately when not linked together as the multizyme.

**3.** The multizyme of claim 1 wherein use of the multizyme in a reaction sequence lowers an amount of at least one intermediate produced by the multizyme when compared to the amount of substantially the same intermediate produced by each individual enzymatic activity in a reaction sequence when not linked together as a multizyme.

**4.** The multizyme of claim 1 wherein use of the multizyme in a reaction reduces the extent of any feedback inhibition of at least one enzymatic activity of the multizyme when compared to the amount of feedback inhibition of a corresponding enzymatic activity that occurs when said individual enzymatic activity is not part of a multizyme.

**5.** The multizyme of claim 1 wherein use of the multizyme in a reaction protects an activity of the multizyme from competitive inhibition by a substrate other than that produced by a preceding enzymatic activity of the multizyme.

**6.** The multizyme of claim 1 wherein a product of a reaction of the multizyme is substantially used in a subsequent reaction of the multizyme.

**7.** The multizyme of claim 1 wherein at least one independent and separable activity of the multizyme is prevented from associating with polypeptide complexes other than the multizyme of which it is a part.

**8.** The multizyme of claim 1 wherein a product of an enzymatic reaction of the multizyme is channeled into a transporter activity which is comprised by the multizyme.

**9.** The multizyme of claim 1 wherein at least one activity of the multizyme that is capable of multiple functions when expressed as an individual polypeptide is restricted to a single function when it is part of the multizyme.

**10.** The multizyme of claim 5 wherein said multizyme comprises at least two enzymatic activities which when linked together have resistance to an herbicide inhibitor of at least one of the individual activities,
preferably wherein said multizyme comprises a 4-hydroxyphenyl pyruvate dioxygenase linked to an activity that produces hydroxyphenyl pyruvate, or

preferably wherein said multizyme comprises a 4-hydroxyphenyl pyruvate dioxygenase linked to a prephenate dehydrogenase or a bifunctional chorismate mutase.

11. The multizyme of claim 6 wherein expression of said multizyme in a transgenic plant confers an increase in seed yield or drought resistance of said plant, or
wherein said multizyme comprises a 1-hydroxy-2-methyl-2-(E)-butenyl 4-diphosphate reductase linked to adenosine phosphate-isopentenyltransferase.

12. The multizyme of claim 7 wherein said multizyme comprises an herbicide resistant acetolactate synthase peptide linked to a second herbicide resistant acetolactate synthase peptide.

13. The multizyme of claim 8 wherein said multizyme comprises a soluble nitrate reductase linked to a nitrate transporter peptide.

14. The multizyme of claim 9 wherein said multizyme comprises an acyl-ACP-thioesterase protein linked to a beta-ketoacyl-ACP synthetase II protein.

**Patentansprüche**

1. Multizym, umfassend ein einzelnes Polypeptid mit wenigstens zwei unabhängigen und trennbaren Aktivitäten, wobei die unabhängigen und trennbaren Aktivitäten unter Verwendung eines Verknüpfers miteinander verbunden sind, wobei der genannte Verknüpfer eine Sequenz eines DHA-Synthaseorts eines Euglenoids ist, umfassend die Aminosäuresequenz, die in einer der SEQ ID NOs: 34, 36 oder 50 festgelegt ist oder eine Aminosäuresequenz mit einer Identität mit derselben von wenigstens 95 % und wobei der genannte Verknüpfer natürlich oder synthetisch sein kann und die Fähigkeit, ein Multizym zu bilden, beibehalten kann, als wäre er ein Verknüpfer aus einem Euglenoid-DHA-Synthaseort, mit der Maßgabe, dass das Multizym kein Enzym enthält, das an der Fettsäure-Biosynthese beteiligt ist.

2. Multizym nach Anspruch 1, wobei die Verwendung des Multizyms den Gesamtflux aller kombinierten Reaktionen des Multizyms im Vergleich zum Gesamtflux der kombinierten Reaktionen erhöht, wenn jede Reaktion separat produziert wird, wenn diese nicht als das Multizym miteinander verknüpft sind.

3. Multizym nach Anspruch 1, wobei die Verwendung des Multizyms in einer Reaktionssequenz eine Menge wenigstens eines Zwischenprodukts senkt, das durch das Multizym produziert wird, im Vergleich zur Menge des im Wesentlichen selben Zwischenprodukts, das durch jede einzelne enzymatische Aktivität in einer Reaktionssequenz produziert wird, wenn diese nicht als ein Multizym miteinander verknüpft sind.

4. Multizym nach Anspruch 1, wobei die Verwendung des Multizyms in einer Reaktion das Ausmaß einer Feedback-Hemmung wenigstens einer enzymatischen Aktivität des Multizyms reduziert, im Vergleich zur Menge der Feedback-Hemmung einer entsprechenden enzymatischen Aktivität, die stattfindet, wenn die genannte einzelne enzymatische Aktivität nicht Teil eines Multizyms ist.

5. Multizym nach Anspruch 1, wobei die Verwendung des Multizyms in einer Reaktion eine Aktivität des Multizyms vor der kompetitiven Hemmung durch ein Substrat schützt, das ein anderes ist, als dasjenige, das durch eine vorhergehende enzymatische Aktivität des Multizyms produziert wurde.

6. Multizym nach Anspruch 1, wobei ein Produkt einer Reaktion des Multizyms im Wesentlichen in einer darauffolgenden Reaktion des Multizyms verwendet wird.

7. Multizym nach Anspruch 1, wobei wenigstens eine unabhängige und trennbare Aktivität des Multizyms an der Assoziierung mit Polypeptidkomplexen, außer dem Multizym, von dem diese ein Teil ist, gehindert wird.

8. Multizym nach Anspruch 1, wobei ein Produkt einer enzymatischen Reaktion des Multizyms in eine Transporteraktivität geleitet wird, die aus dem Multizym besteht.

9. Multizym nach Anspruch 1, wobei wenigstens eine Aktivität des Multizyms, das zu mehrfachen Funktionen fähig ist wenn als ein einzelnes Polypeptid exprimiert, auf eine einzelne Funktion beschränkt ist, wenn es Teil des Multizyms ist.

**10.** Multizym nach Anspruch 5, wobei das genannte Multizym wenigstens zwei enzymatische Aktivitäten umfasst, die beim Zusammenverknüpfen eine Resistenz gegenüber einem Herbizidhemmer wenigstens einer der einzelnen Aktivitäten aufweist,
bevorzugt wobei das genannte Multizym eine 4-Hydroxyphenylpyruvatdioxygenase umfasst, die mit einer Aktivität verknüpft ist, die Hydroxyphenylpyruvat produziert oder
bevorzugt wobei das genannte Multizym eine 4-Hydroxyphenylpyruvatdioxygenase umfasst, die mit einer Prephenatdehydrogenase oder einer bifunktionellen Chorismatmutase verknüpft ist.

**11.** Multizym nach Anspruch 6, wobei die Expression des genannten Multizyms in einer transgenen Pflanze eine Steigerung an Saatausbeute oder Trockenresistenz der genannten Pflanze verleiht oder
wobei das genannte Multizym eine 1-Hydroxy-2-methyl-2-(E)-butenyl-4-diphosphatreduktase umfasst, die mit einer Adenosinphosphatisopentenyltransferase verknüpft ist.

**12.** Multizym nach Anspruch 7, wobei das genannte Multizym ein Herbizid resistente Acetolaktatsynthasepeptid umfasst, das mit einem zweiten Herbizid resistenten Acetolaktatsynthasepeptid verknüpft ist.

**13.** Multizym nach Anspruch 8, wobei das genannte Multizym eine lösliche Nitratreduktase umfasst, die mit einem Nitrattransporterpeptid verknüpft ist.

**14.** Multizym nach Anspruch 9, wobei das genannte Multizym ein Acyl-ACP-Thioesteraseprotein umfasst, das mit einem Beta-Ketoacyl-ACP-Synthase II-Protein verknüpft ist.

## Revendications

**1.** Multizyme comprenant un polypeptide unique ayant au moins deux activités indépendantes et séparables dans laquelle les activités indépendantes et séparables sont reliées à l'aide d'un lieur, dans laquelle ledit lieur est une séquence d'un locus de DHA synthase d'un Euglénoïde comprenant la séquence d'acides aminés figurant dans l'une quelconque des SEQ ID Nos : 34, 36 ou 50, ou une séquence d'acides aminés ayant au moins 95 % d'identité de séquence avec celles-ci, et dans laquelle ledit lieur peut être naturel ou synthétique et garde la capacité de former une multizyme comme s'il s'agissait d'un lieur d'un locus de DHA synthase d'un Euglénoïde, à condition que la multizyme ne comprenne pas une enzyme jouant un rôle dans la biosynthèse des acides gras.

**2.** Multizyme selon la revendication 1, dans laquelle l'utilisation de la multizyme augmente le flux total de toutes les réactions combinées de la multizyme en comparaison avec le flux total des réactions combinées lorsque chaque réaction est produite séparément lorsqu'elles ne sont pas reliées au sein de la multizyme.

**3.** Multizyme selon la revendication 1, dans laquelle l'utilisation de la multizyme dans une séquence réactionnelle réduit une quantité d'au moins un intermédiaire produit par la multizyme en comparaison avec la quantité de sensiblement le même intermédiaire produit par chaque activité enzymatique individuelle dans une séquence réactionnelle lorsqu'elles ne sont pas reliées au sein d'une multizyme.

**4.** Multizyme selon la revendication 1, dans laquelle l'utilisation de la multizyme dans une réaction réduit l'étendue de toute rétro-inhibition d'au moins une activité enzymatique de la multizyme en comparaison avec la quantité de rétro-inhibition d'une activité enzymatique correspondante qui est présente lorsque ladite activité enzymatique individuelle ne fait pas partie d'une multizyme.

**5.** Multizyme selon la revendication 1, dans laquelle l'utilisation de la multizyme dans une réaction protège une activité de la multizyme de l'inhibition compétitive par un substrat autre que celui produit par une activité enzymatique précédente de la multizyme.

**6.** Multizyme selon la revendication 1, dans laquelle un produit d'une réaction de la multizyme est sensiblement utilisé dans une réaction subséquente de la multizyme.

**7.** Multizyme selon la revendication 1, dans laquelle au moins une activité indépendante et séparable de la multizyme est empêchée de s'associer avec des complexes de polypeptides autres que la multizyme dont elle fait partie.

**8.** Multizyme selon la revendication 1, dans laquelle un produit d'une réaction enzymatique de la multizyme est canalisé dans une activité de transporteur qui fait partie de la multizyme.

**9.** Multizyme selon la revendication 1, dans laquelle au moins une activité de la multizyme qui est capable de multiples fonctions lorsqu'elle est exprimée sous la forme d'un polypeptide individuel est restreinte à une seule fonction lorsqu'elle fait partie de la multizyme.

**10.** Multizyme selon la revendication 5, dans laquelle ladite multizyme comprend au moins deux activités enzymatiques qui lorsqu'elles sont reliées présentent une résistance à un inhibiteur herbicide d'au moins l'une des activités individuelles,
de préférence dans laquelle ladite multizyme comprend une 4-hydroxyphényl pyruvate dioxygénase liée à une activité qui produit de l'hydroxyphényl pyruvate, ou
de préférence dans laquelle ladite multizyme comprend une 4-hydroxyphényl pyruvate dioxygénase liée à une préphénate déshydrogénase ou une chorismate mutase bifonctionnelle.

**11.** Multizyme selon la revendication 6, dans laquelle l'expression de ladite multizyme dans une plante transgénique confère une augmentation du rendement des semences ou une résistance à la sécheresse à ladite plante, ou
dans laquelle ladite multizyme comprend une 1-hydroxy-2-méthyl-2-(E)-butény1 4-diphosphate réductase liée à une adénosine phosphate-isopentényltransférase.

**12.** Multizyme selon la revendication 7, dans laquelle ladite multizyme comprend un peptide d'acétolactate synthase résistante aux herbicides lié à un deuxième peptide d'acétolactate synthase résistante aux herbicides.

**13.** Multizyme selon la revendication 8, dans laquelle ladite multizyme comprend une nitrate réductase soluble liée à un peptide transporteur de nitrate.

**14.** Multizyme selon la revendication 9, dans laquelle ladite multizyme comprend une protéine acyle-ACP-thioestérase liée à une protéine bêta-cétoacyl-ACP synthétase II.

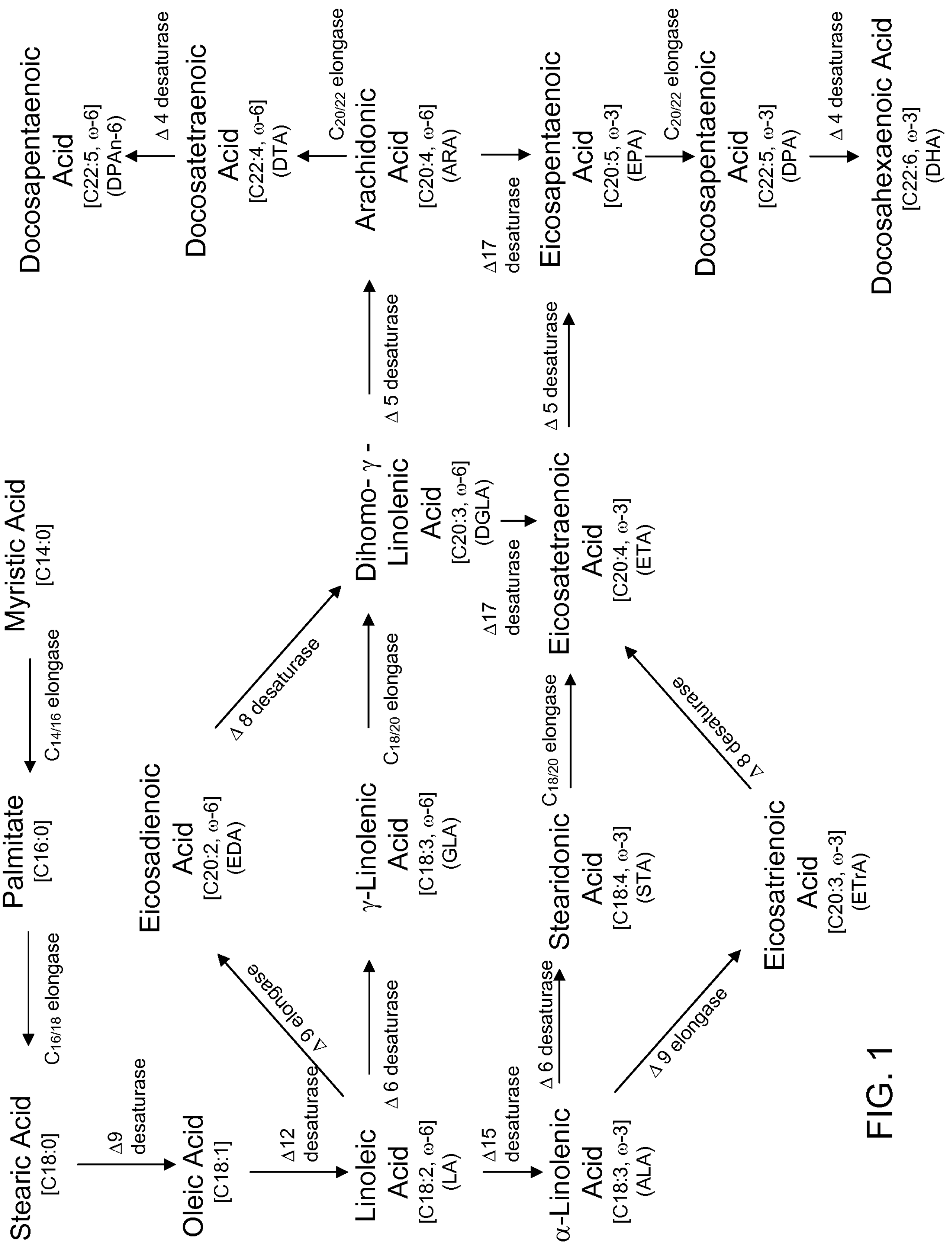
Stearic Acid [C18:0]
Δ9 desaturase
Oleic Acid [C18:1]
Δ12 desaturase
Linoleic Acid [C18:2, ω-6] (LA)
Δ15 desaturase
α-Linolenic Acid [C18:3, ω-3] (ALA)
$C_{16/18}$ elongase
Palmitate [C16:0]
$C_{14/16}$ elongase
Myristic Acid [C14:0]
Δ 9 elongase
Eicosadienoic Acid [C20:2, ω-6] (EDA)
Δ 8 desaturase
Δ 6 desaturase
γ-Linolenic Acid [C18:3, ω-6] (GLA)
$C_{18/20}$ elongase
Dihomo- γ - Linolenic Acid [C20:3, ω-6] (DGLA)
Δ 5 desaturase
Arachidonic Acid [C20:4, ω-6] (ARA)
$C_{20/22}$ elongase
Docosatetraenoic Acid [C22:4, ω-6] (DTA)
Δ 4 desaturase
Docosapentaenoic Acid [C22:5, ω-6] (DPAn-6)
Δ 6 desaturase
Stearidonic Acid [C18:4, ω-3] (STA)
$C_{18/20}$ elongase
Δ17 desaturase
Eicosatetraenoic Acid [C20:4, ω-3] (ETA)
Δ 5 desaturase
Δ17 desaturase
Eicosapentaenoic Acid [C20:5, ω-3] (EPA)
$C_{20/22}$ elongase
Docosapentaenoic Acid [C22:5, ω-3] (DPA)
Δ 4 desaturase
Docosahexaenoic Acid [C22:6, ω-3] (DHA)
Δ 9 elongase
Eicosatrienoic Acid [C20:3, ω-3] (ETrA)
Δ 8 desaturase

FIG. 1

# FIG. 2

```
                                Divergent sequence
                                ---------->
EgDHAsyn2_CDS.seq   ATGGAAGAAGATCTTGACACAGCTCCAGATGGTTCAATTC 660
EgD4_cDNA.seq       GGGCGTAA-----TTAACA-ATCGACAGCGGCCGCGAATT 654
EgD4_CDS.seq        ---------------------------------------- 0

                      NotI site    ↓ Sequence identical start
EgDHAsyn2_CDS.seq   TGTTTCTGTTTTACACATTCCATTTATGTGTGGATTTGCG 700
EgD4_cDNA.seq       C[GCGGCCGC]T---CACATTCCATTTATGTGTGGATTTGCG 691
EgD4_CDS.seq        ---------------------------------------- 0

EgDHAsyn2_CDS.seq   GGTCAGAGATCTACCCACGGCCTCTGACTGCTTTGCAGTC 740
EgD4_cDNA.seq       GGTCAGAGATCTACCCACGGCCTCTGACTGCTTTGCAGTC 731
EgD4_CDS.seq        ---------------------------------------- 0

                                    ↓ EgD4 CDS Start
EgDHAsyn2_CDS.seq   GTTCGTGATGGTCAATATGTTGGTGCTGTTTGGCAATTTC 780
EgD4_cDNA.seq       GTTCGTGATGGTCAATATGTTGGTGCTGTTTGGCAATTTC 771
EgD4_CDS.seq        ----------------ATGTTGGTGCTGTTTGGCAATTTC 24

EgDHAsyn2_CDS.seq   TATGTCAAGCAATACTCCCAAAAGAACGGCAAGCCGGAGA 820
EgD4_cDNA.seq       TATGTCAAGCAATACTCCCAAAAGAACGGCAAGCCGGAGA 811
EgD4_CDS.seq        TATGTCAAGCAATACTCCCAAAAGAACGGCAAGCCGGAGA 64

EgDHAsyn2_CDS.seq   ACGGAGCCACCCCTGAGAACGGAGCGAAGCCGCAACCTTG 860
EgD4_cDNA.seq       ACGGAGCCACCCCTGAGAACGGAGCGAAGCCGCAACCTTG 851
EgD4_CDS.seq        ACGGAGCCACCCCTGAGAACGGAGCGAAGCCGCAACCTTG 104

EgDHAsyn2_CDS.seq   CGAGAACGGCACGGTGGAAAAGCGAGAGAATGACACCGCC 900
EgD4_cDNA.seq       CGAGAACGGCACGGTGGAAAAGCGAGAGAATGACACCGCC 891
EgD4_CDS.seq        CGAGAACGGCACGGTGGAAAAGCGAGAGAATGACACCGCC 144

EgDHAsyn2_CDS.seq   AACGTTCGGCCCACCCGTCCAGCTGGACCCCCGCCGGCCA 940
EgD4_cDNA.seq       AACGTTCGGCCCACCCGTCCAGCTGGACCCCCGCCGGCCA 931
EgD4_CDS.seq        AACGTTCGGCCCACCCGTCCAGCTGGACCCCCGCCGGCCA 184
```

EgDHAsyn2_CDS is nt 621-940 of SEQ ID NO:7, EgD4_cDNA is nt 621-931 of SEQ ID NO:9, and EgD4_CDS is nt 1-184 of SEQ ID NO:7.

# FIG. 3A

```
EgC20elo1.pro    M A D S P V I N L S T M W K P L S L M - - - - - - - G H V W K Q A Q Q E G S I S  33
EgDHAsyn1.pro    M A D S P V I N L S T M W K P L S L M A L D L A V L G H V W K Q A Q Q E G S I S  40
EgDHAsyn2.pro    M A D S P V I N L S T M W K P L S L M A L D L A I L G H V W K Q A Q Q E G S I S  40

EgC20elo1.pro    A Y A D S V R I P L I M S V L Y L S M I F V G C R W M K N R E P F E I K T Y M F  73
EgDHAsyn1.pro    A Y A D S V W T P L I M S G L Y L S M I F V G C R W M K N R E P F E I K T Y M F  80
EgDHAsyn2.pro    A Y A D S V W T P L I M S V L Y L S M I F V G C R W M K N R E P F E I K T Y M F  80

EgC20elo1.pro    A Y N L Y Q T L M N L C I V L G F L Y Q V H A T G M R F W G S G V D R S P K G L  113
EgDHAsyn1.pro    A Y N L Y Q T L M N L C I V L G F L Y Q V H A T G M R F W G S G V D R S P K G L  120
EgDHAsyn2.pro    A Y N L Y Q T L M N L C I V L G F L Y Q V H A T G M R F W G S G V D R S P K G L  120

EgC20elo1.pro    G I G F F I Y A H Y H N K Y V E Y F D T L F M V L R K K N N Q I S F L H V Y H H  153
EgDHAsyn1.pro    G I G F F I Y A H Y H N K Y V E Y F D T L F M V L R K K N N Q I S F L H V Y H H  160
EgDHAsyn2.pro    G I G F F I Y A H Y H N K Y V E Y F D T L F M V L R K K N N Q I S F L H V Y H H  160

EgC20elo1.pro    A L L T W A W F A V V Y F A P G G D G W F G A C Y N S S I H V L M Y S Y Y L L A  193
EgDHAsyn1.pro    A L L T W A W F A V V Y F A P G G D G W F G A C Y N S S I H V L M Y S Y Y L L A  200
EgDHAsyn2.pro    A L L T W A W F A V V Y F A P G G D G W F G A C Y N S S I H V L M Y S Y Y L L A  200

EgC20elo1.pro    T F G I S C P W K K I L T Q L Q M V Q F C F C F T H S I Y V W I C G S E I Y P R  233
EgDHAsyn1.pro    T F G I S C P W K K I L T Q L Q M V Q F C F C F T H S I Y V W I C G S E I Y P R  240
EgDHAsyn2.pro    T F G I S C P W K K I L T Q L Q M V Q F C F C F T H S I Y V W I C G S E I Y P R  240

EgC20elo1.pro    P L T A L Q S F V M V N M L V L F G N F Y V K Q Y S Q K N G K P E N G A T P E N  273
EgDHAsyn1.pro    P L T A L Q S F V M V N M L V L F G N F Y V K Q Y S Q K N G K P E N G A T P E N  280
EgDHAsyn2.pro    P L T A L Q S F V M V N M L V L F G N F Y V K Q Y S Q K N G K P E N G A T P E N  280

EgC20elo1.pro    G A K P Q P C E N G T V E K R E A P R S V G M G R                                298
EgDHAsyn1.pro    G A K P Q P C E N G T V E K R E N D T A N V R P A R P A G L P P A T Y Y D S L A  320
EgDHAsyn2.pro    G A K P Q P C E N G T V E K R E N D T A N V R P T R P A G P P P A T Y Y D S L A  320

EgC20elo1.pro                                                                                     298
EgDHAsyn1.pro    V S G Q G K E R L F T T D E V R R H I L P T D G W L T C H E G V Y D V T D F L A  360
EgDHAsyn2.pro    V S G Q G K E R L F T T D E V R R H I L P T D G W L T C H E G V Y D V T D F L A  360

EgC20elo1.pro                                                                                     298
EgDHAsyn1.pro    K H P G G G V I T L G L G R D C T I L V E S Y H P A G R P D K V M E K Y R I G T  400
EgDHAsyn2.pro    K H P G G G V I T L G L G R D C T I L I E S Y H P A G R P D K V M E K Y R I G T  400
```

# FIG. 3B

```
EgC20elo1.pro                                                                                  298
EgDHAsyn1.pro  L Q D P K T F Y A W G E S D F Y P E L K R R A L A R L K E A G Q A R R G G L G V  440
EgDHAsyn2.pro  L Q D P K T F Y A W G E S D F Y P E L K R R A L A R L K E A G Q A R R G G L G V  440

EgC20elo1.pro                                                                                  298
EgDHAsyn1.pro  K A L L V L T L F F V S W Y M W V A H K S F L W A A V W G F A G S H V G L S I Q  480
EgDHAsyn2.pro  K A L L V L T L F F V S W Y M W V A H K S F L W A A V W G F A G S H V G L S I Q  480

EgC20elo1.pro                                                                                  298
EgDHAsyn1.pro  H D G N H G A F S R S T L V N R L A G W G M D L I G A S S T V W E Y Q H V I G H  520
EgDHAsyn2.pro  H D G N H G A F S R N T L V N R L A G W G M D L I G A S S T V W E Y Q H V I G H  520

EgC20elo1.pro                                                                                  298
EgDHAsyn1.pro  H Q Y T N L V S D T L F S L P E N D P D V F S S Y P L M R M H P D T A W Q P H H  560
EgDHAsyn2.pro  H Q Y T N L V S D T L F S L P E N D P D V F S S Y P L M R M H P D T A W Q P H H  560

EgC20elo1.pro                                                                                  298
EgDHAsyn1.pro  R F Q H L F A F P L F A L M T I S K V L T S D F A V C L S M K K G S I D C S S R  600
EgDHAsyn2.pro  R F Q H L F A F P L F A L M T I S K V L T S D F A V C L S M K K G S I D C S S R  600

EgC20elo1.pro                                                                                  298
EgDHAsyn1.pro  L V P L E G Q L L F W G A K L A N F L L Q I V L P C Y L H G T A M G L A L F S V  640
EgDHAsyn2.pro  L V P L E G Q L L F W G A K L A N F L L Q I V L P C Y L H G T A M G L A L F S V  640

EgC20elo1.pro                                                                                  298
EgDHAsyn1.pro  A H L V S G E Y L A I C F I I N H I S E S C E F M N T S F Q T A A R R T E M L Q  680
EgDHAsyn2.pro  A H L V S G E Y L A I C F I I N H I S E S C E F M N T S F Q T A A R R T E M L Q  680

EgC20elo1.pro                                                                                  298
EgDHAsyn1.pro  A A H Q A A E A K K V K P T P P P N D W A V T Q V Q C C V N W R S G G V L A N H  720
EgDHAsyn2.pro  A A H Q A A E A K K V K P T P P P N D W A V T Q V Q C C V N W R S G G V L A N H  720

EgC20elo1.pro                                                                                  298
EgDHAsyn1.pro  L S G G L N H Q I E H H L F P S I S H A N Y P I I A R V V K E V C E E Y G L P Y  760
EgDHAsyn2.pro  L S G G L N H Q I E H H L F P S I S H A N Y P T I A P V V K E V C E E Y G L P Y  760

EgC20elo1.pro                                                                                  298
EgDHAsyn1.pro  K N Y V T F W D A V C G M V Q H L R L M G A P P V P T N G D K K S              793
EgDHAsyn2.pro  K N Y V T F W D A V C G M V Q H L R L M G A P P V P T N G D K K S              793
```

EgC20elo1 (SEQ ID NO:3), EgDHAsyn1 (SEQ ID NO:5) and EgDHAsyn2 (SEQ ID NO:8).

FIG. 4

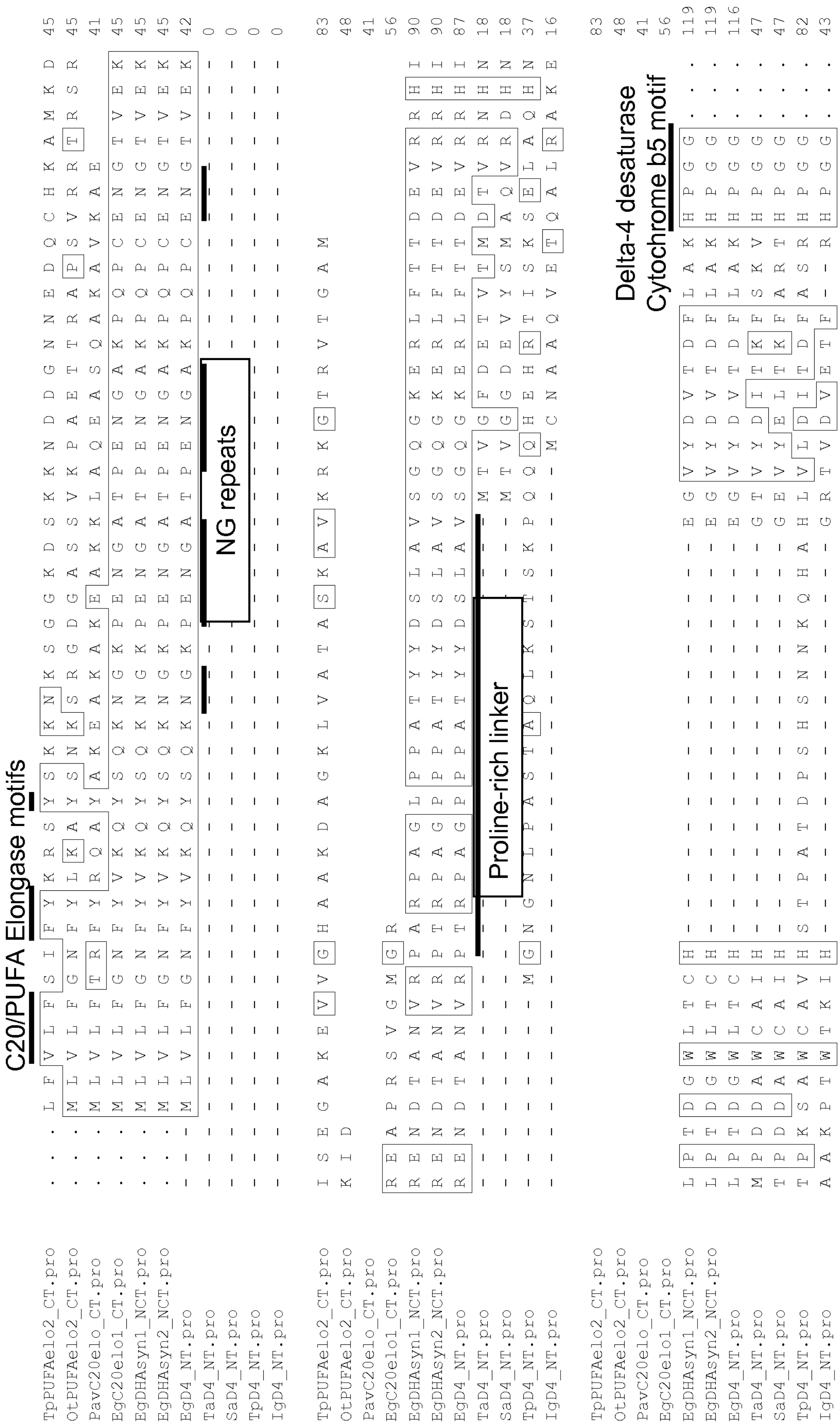
C20/PUFA Elongase motifs
NG repeats
Proline-rich linker
Delta-4 desaturase
Cytochrome b5 motif
TpPUFAelo2_CT.pro
OtPUFAelo2_CT.pro
PavC20elo_CT.pro
EgC20elo1_CT.pro
EgDHAsyn1_NCT.pro
EgDHAsyn2_NCT.pro
EgD4_NT.pro
TaD4_NT.pro
SaD4_NT.pro
TpD4_NT.pro
IgD4_NT.pro

# FIG. 5A

```
EaDHAsyn1.pro   M A E G K S D G P V V T L Q S M W K P L A L M A V D V G I L V N V R R K A F T E  40
EaDHAsyn2.pro   M A E G K S D G P V V T L Q S M W K P L A L M A I D V G I L V N V R R K A F T E  40
EaDHAsyn3.pro   M A E G K S D G P V V T L Q S M W K P L A L M A V D V G I L V N V R R K A F T E  40
EaDHAsyn4.pro   M A E G K S D G P V V T L Q S M W K P L A L M A V D V G I L V N V R R K A F T E  40

EaDHAsyn1.pro   F D G H S N V F A D P V Y I P F V M N L F Y L T M I F A G C R W M K T R E P F E  80
EaDHAsyn2.pro   F D G H S N V F A D P V Y I P F V M N L F Y L T M I F A G C R W M K T R E P F E  80
EaDHAsyn3.pro   F D G H S N V F A D P V Y I P F V M N L F Y L T M I F A G C R W M K T R E P F E  80
EaDHAsyn4.pro   F D G H S N V F A D P V Y I P F V M N L F Y L T M I F A G C R W M K T R E P F E  80

EaDHAsyn1.pro   I K S Y M F A Y N A Y Q T M M N F L I V V G F M Y E V H S T G M R Y W G S R I D  120
EaDHAsyn2.pro   I K S Y M F A Y N A Y Q T M M N F L I V V G F M Y E V H S T G M R Y W G S R I D  120
EaDHAsyn3.pro   I K S Y M F A Y N A Y Q T M M N F L I V V G F M Y E V H S T G M R Y W G S R I D  120
EaDHAsyn4.pro   I K S Y M F A Y N A Y Q T M M N F L I V V G F M Y E V H S T G M R Y W G S R I D  120

EaDHAsyn1.pro   T S T K G L G L G F L I Y A H Y H N K Y V E Y V D T L F M I L R K K N N Q I S F  160
EaDHAsyn2.pro   T S T K G L G L G F L I Y A H Y H N K Y V E Y V D T L F M I L R K K N N Q I S F  160
EaDHAsyn3.pro   T S T K G L G L G F L I Y A H Y H N K Y V E Y V D T L F M I L R K K N N Q I S F  160
EaDHAsyn4.pro   T S T K G L G L G F L I Y A H Y H N K Y V E Y V D T L F M I L R K K N N Q I S F  160

EaDHAsyn1.pro   L H V Y H H S L L T W A W W A V V Y W A P G G D A W F G A C Y N S F I H V L M Y  200
EaDHAsyn2.pro   L H V Y H H S L L T W A W W A V V Y W A P G G D A W F G A C Y N S F I H V L M Y  200
EaDHAsyn3.pro   L H V Y H H S L L T W A W W A V V Y W A P G G D A W F G A C Y N S F I H V L M Y  200
EaDHAsyn4.pro   L H V Y H H S L L T W A W W A V V Y W A P G G D A W F G A C Y N S F I H V L M Y  200

EaDHAsyn1.pro   S Y Y L F A T F G I R C P W K K M L T Q L Q M V Q F C F C F A H A M Y V G W L G  240
EaDHAsyn2.pro   S Y Y L F A T F G I R C P W K K M L T Q L Q M V Q F C F C F A H A M Y V G W L G  240
EaDHAsyn3.pro   S Y Y L F A T F G I R C P W K K M L T Q L Q M V Q F C F C F A H A M Y V G W L G  240
EaDHAsyn4.pro   S Y Y L F A T F G I R C P W K K M L T Q L Q M V Q F C F C F A H A M Y V G W L G  240

EaDHAsyn1.pro   H E V Y P R W L T A L Q A F V M L N M L V L F G N F Y M K S Y S K A S K L E P A  280
EaDHAsyn2.pro   H E V Y P R W L T A L Q A F V M L N M L V L F G N F Y M K S Y S K A S K L E P A  280
EaDHAsyn3.pro   H E V Y P R W L T A L Q A F V M L N M L V L F G N F Y M K S Y S K A S K L E P A  280
EaDHAsyn4.pro   H E V Y P R W L T A L Q A F V M L N M L V L F G N F Y M K S Y S K A S K L E P A  280

EaDHAsyn1.pro   S P V S P A S L A Q K P F E N A K V K P G G P G K P S E I A S L P P P I R P V G  320
EaDHAsyn2.pro   S P V S P A S L A Q K P F E N A K V K P G G P G K P S E I A S L P P P I R P V G  320
EaDHAsyn3.pro   S P A S P A S L A Q K P F E N A K V K P G G P G K P S E I A S L P P P I R P V G  320
EaDHAsyn4.pro   S P V S P A S L A Q K P F E N A K V K P G G P G K P S E I A S L P P P I R P V G  320
```

FIG. 5B

```
EaDHAsyn1.pro   N P P A A Y Y D A L A T S G T G Q D R K F T M R E V A R H I V P T D G W L A C H   360
EaDHAsyn2.pro   N P P A A Y Y D A L A T S G T G Q D R K F T M R E V A R H I V P T D G W L A C H   360
EaDHAsyn3.pro   N P P A A Y Y D A L A T S G T G Q D R K F T M R E V A R H I V P T D G W L A C H   360
EaDHAsyn4.pro   N P P A A Y Y D A L A T S G T G Q D R K F T M R E V A R H I V P T D G W L A C H   360

EaDHAsyn1.pro   D G V Y D I T E F I G K H P G G D V I S L G L G R D S T I L V E S Y H P A G R P   400
EaDHAsyn2.pro   D G V Y D I T E F I G K H P G G D V I S L G L G R D S T I L V E S Y H P A G R P   400
EaDHAsyn3.pro   D G V Y D I T E F I G K H P G G D V I S L G L G R D S T I L V E S Y H P A G R P   400
EaDHAsyn4.pro   D G V Y D I T E F I G K H P G G D V I S L G L G R D S T I L V E S Y H P A G R P   400

EaDHAsyn1.pro   D K V M E K Y R I G T L Q D H R T F Y D W Q A S A F Y A E L K Q R V V Q T L K E   440
EaDHAsyn2.pro   D K V M E K Y R I G T L Q D H R T F Y D W Q A S A F Y A E L K Q R V V Q T L K E   440
EaDHAsyn3.pro   D K V M E K Y R I G T L Q D H R T F Y D W Q A S A F Y A E L K Q R V V Q T L K E   440
EaDHAsyn4.pro   D K V M E K Y R I G T L Q D H R T F Y D W Q A S A F Y A E L K Q R V V Q T L K E   440

EaDHAsyn1.pro   A G Q P R R G G L S V K A A L V M A A F A A S F Y L M V T Q G S F F W A A V W G   480
EaDHAsyn2.pro   A G Q P R R G G L S V K A A L V M A A F A A S F Y L M V T Q G S F F W A A V W G   480
EaDHAsyn3.pro   A G Q P R R G G L S V K A A L V M A A F A A S F Y L M V T Q G S F F W A A V W G   480
EaDHAsyn4.pro   A G Q P R R G G L S V K A A L V M A A F A A S F Y L M V T Q G S F F W A A V W G   480

EaDHAsyn1.pro   L A G S H I G L S I Q H D G N H G A F S K S G R L N R L A G W G M D V I G A S S   520
EaDHAsyn2.pro   L A G S H I G L S I Q H D G N H G A F S K S G R L N R L A G W G M D V I G A S S   520
EaDHAsyn3.pro   L A G S H I G L S I Q H D G N H G A F S K S G R L N R L A G W G M D V I G A S S   520
EaDHAsyn4.pro   L A G S H I G L S I Q H D G N H G A F S K S G R L N R L A G W G M D V I G A S S   520

EaDHAsyn1.pro   T A W E Y Q H V I G H H Q Y T N L V S D P E F A L P E N D P D V F G T Y P L M R   560
EaDHAsyn2.pro   T A W E Y Q H V I G H H Q Y T N L V S D P E F A L P E N D P D V F G T Y P L M R   560
EaDHAsyn3.pro   T A W E Y Q H V I G H H Q Y T N L V S D P E F A L P E N D P D V F G T Y P L M R   560
EaDHAsyn4.pro   T A W E Y Q H V I G H H Q Y T N L V S D P E F A L P E N D P D V F G T Y P L M R   560

EaDHAsyn1.pro   M H P D T P W K P H H Q L Q H M Y A F P L F A L M T I S K V I I S D F T F C L A   600
EaDHAsyn2.pro   M H P D T P W K P H H Q L Q H V Y A F P L F A L M T I S K V I I S D F T F C L A   600
EaDHAsyn3.pro   M H P D T P W K P H H Q L Q H M Y A F P L F A L M T I S K V I I S D F T F C L A   600
EaDHAsyn4.pro   M H P D T P W K P H H Q L Q H M Y A F P L F A L M T I S K V I I S D F T F C L A   600

EaDHAsyn1.pro   K R R G P I D F S A R L V P L E G Q M L F W G A K I M G F L M Q I V L P C Y L H   640
EaDHAsyn2.pro   K R R G P I D F S A R L V P L E G Q M L F W G A K I M G F L M Q I V L P C Y L H   640
EaDHAsyn3.pro   K R R G P I D F S A R L V P L E G Q M L F W G A K I M G F L M Q I V L P C Y L H   640
EaDHAsyn4.pro   K R R G P I D F S A R L V P L E G Q M L F W G A K I M G F L M Q I V L P C Y L H   640
```

# FIG. 5C

```
EaDHAsyn1.pro   G I A H G L A L F I T A H L V S G E Y L A V C F I I N H I S E S C D Y L N P S S   680
EaDHAsyn2.pro   G I A H G L A L F I T A H L V S G E Y L A V C F I I N H I S E S C D Y L N P S S   680
EaDHAsyn3.pro   G I A H G L A L F I T A H L V S G E Y L A V C F I I N H I S E S C D Y L N P S S   680
EaDHAsyn4.pro   G I A H G L A L F I T A H L V S G E Y L A V C F I I N H I S E S C D Y L N P S S   680

EaDHAsyn1.pro   V I A A R R T E M L K Q A E Q E A K A K Q K H P T P P P N D W A A S Q V L C C V   720
EaDHAsyn2.pro   V I A A R R T E M L K Q A E Q E A K A K Q K H P T P P P N D W A A S Q V L C C V   720
EaDHAsyn3.pro   V I A A R R T E M L K Q A E Q E A K A K Q K H P T P P P N D W A A S Q V L C C V   720
EaDHAsyn4.pro   V I A A R R T E M L K Q A E Q E A K A K Q K H P T P P P N D W A A S Q V L C C V   720

EaDHAsyn1.pro   N W R S G G Y F S N H L S G G L N H Q I E H H L F P S I S H A N Y P T I A P V V   760
EaDHAsyn2.pro   N W R S G G Y F S N H L S G G L N H Q I E H H L F P S I S H A N Y P T I A P V V   760
EaDHAsyn3.pro   N W R S G G Y F S N H L S G G L N H Q I E H H L F P S I S H A N Y P T I A P V V   760
EaDHAsyn4.pro   N W R S G G Y F S N H L S G G L N H Q I E H H L F P S I S H A N Y P T I A P V V   760

EaDHAsyn1.pro   K G V C E E Y G L P Y K N Y S Q F S D A I Y G M V E H L R A M G T K P E D N G K   800
EaDHAsyn2.pro   K G V C E E Y G L P Y K N Y S Q F S D A L Y G M V E H L R A M G T K P A D N D K   800
EaDHAsyn3.pro   K G V C E E Y G L P Y K N Y S Q F S D A I Y G M V E H L R A M G T K P E D N G K   800
EaDHAsyn4.pro   K R V C E E Y G L P Y K N Y S Q F S D L S M E W W S T S G R W A R S R Q T T T S   800

EaDHAsyn1.pro   L A P L P G S L E D V C P V L S A A V A A Q P D G S T D G S A A G C P A V A T L   840
EaDHAsyn2.pro   L A P T A G S L E D V C P V L S A A V A A Q P D G S T D G S A A G C P A V A T L   840
EaDHAsyn3.pro   L A P L P G S L E D V C P V L S A A V A A Q P D G S T D G S A A G C P A V A T L   840
EaDHAsyn4.pro   W R H H W A P W R T C A R S                                                       814

EaDHAsyn1.pro   A                                                                                 841
EaDHAsyn2.pro   A                                                                                 841
EaDHAsyn3.pro   A                                                                                 841
EaDHAsyn4.pro                                                                                     814
```

EaDHAsyn1 is SEQ ID NO:18

EaDHAsyn2 is SEQ ID NO:20

EaDHAsyn3 is SEQ ID NO:22

EaDHAsyn4 is SEQ ID NO:24

Fig. 6

Cytokinin biosynthesis pathway with native enzymes

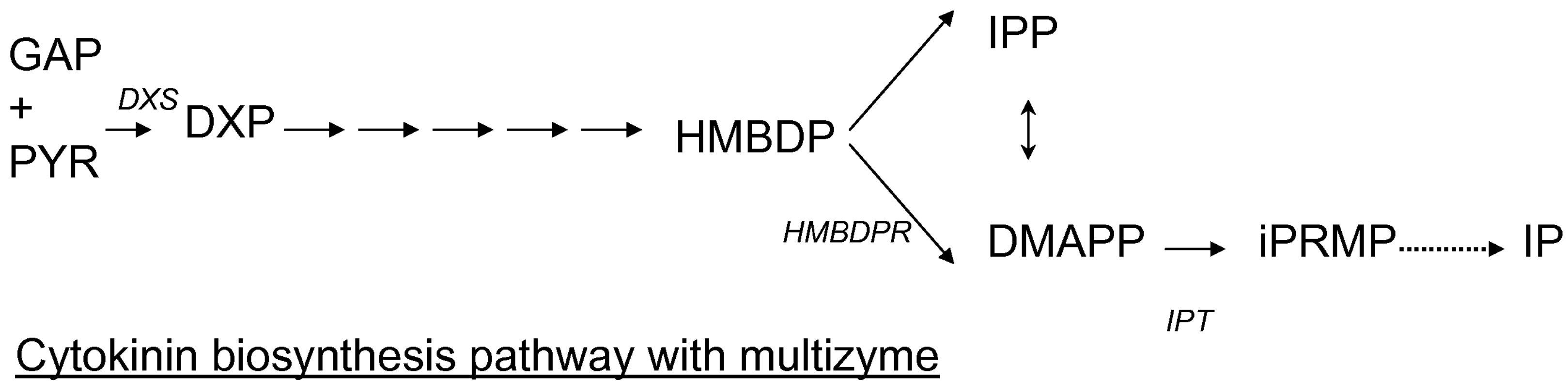


Cytokinin biosynthesis pathway with multizyme

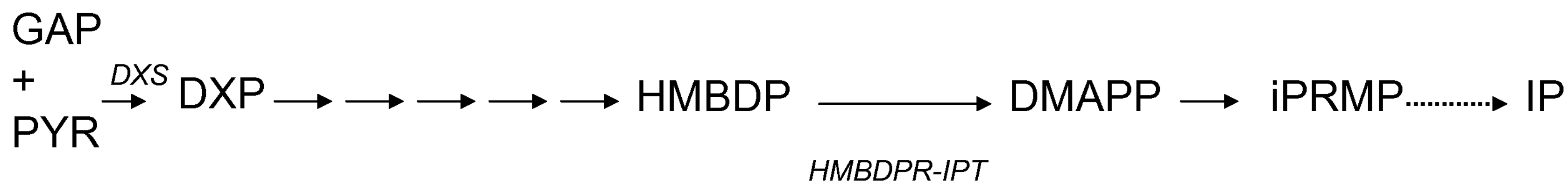


GAP = D-glyceraldehyde 3-phospate
PYR = puruvate
DXP = 1-deoxy-D-xylulose 5-phosphate
HMBDP = 1-Hydroxy-2-methyl-2-(E)-butenyl 4-diphosphate
DMAPP = dimethylallyl diphosphate
IPP = isopentenyl diphosphate
iPRMP =isopentenyladenine riboside monophosphate

*DXS* = DXP synthase
*HMBDPR = HMBDP reductase*
*IPT = isopentenyltransferase*
*HMBDPR-IPT= HMBDPR-IPT multizyme*

Fig. 7

**Modified Hoagland's solutions -**
**16X concentrations for semi-hydroponics maize growth.**

| Nutrient | 1 mM $KNO_3$ | 2 mM $KNO_3$ | 3 mM $KNO_3$ | 4 mM $KNO_3$ |
|---|---|---|---|---|
| $KNO_3$ | 16 mM | 32 mM | 48 mM | 64 mM |
| KCl | 48 mM | 32 mM | 16 mM | ------- |
| $KH_2PO_4$ | 11 mM | 11 mM | 11 mM | 11 mM |
| $MgSO_4$ | 16 mM | 16 mM | 16 mM | 16 mM |
| $CaCl_2 \cdot 2H_2O$ | 16 mM | 16 mM | 16 mM | 16 mM |
| Sprint 330 | 1.6 g/L | 1.6 g/L | 1.6 g/L | 1.6 g/L |
| $H_3BO_3$ | 24 μM | 24 μM | 24 μM | 24 μM |
| 5 mM $MnCl_2 \cdot 4H_2O$ | 8 μM | 8 μM | 8 μM | 8 μM |
| 5 mM $ZnSO_4 \cdot 7\ H_2O$ | 8 M | 8 μM | 8 μM | 8 μM |
| 0.5 mM $CuSO_4 \cdot 5\ H_2O$ | 800 nM | 800 nM | 800 nM | 800 nM |
| 0.5 mM $H_2MoO_4 \cdot H_2O$ | 800 nM | 800 nM | 800 nM | 800 nM |

Dilute 16X with tap water and determine the pH of the final mixture.

Add 3-12 mL $H_2SO_4$ if the pH is above 6.5.

Optimum pH is 5.0 - 5.5

# Fig. 8

The effect of different nitrate concentrations on the growth and development of Gaspe Flint derived maize lines.

| [nitrate] | root (g dwt) | shoot (g dwt) | total vegetative (g dwt) | ear & husk (g dwt) | tassel (g dwt) | tiller # | tiller (g dwt) |
|---|---|---|---|---|---|---|---|
| 1 week after emergence | | | | | | | |
| 1 mM | 0.070a | 0.105b | 0.175b | | | | |
| 2 mM | 0.073a | 0.137ab | 0.209ab | | | | |
| 3 mM | 0.056a | 0.120ab | 0.176ab | | | | |
| 4 mM | 0.074a | 0.157a | 0.231a | | | | |
| 2 weeks after emergence | | | | | | | |
| 1 mM | 0.331ab | 0.544c | 0.875c | | | | |
| 2 mM | 0.266b | 0.951b | 1.217b | | | | |
| 3 mM | 0.352a | 1.171a | 1.523a | | | | |
| 4 mM | 0.303ab | 1.209a | 1.512a | | | | |
| 3 weeks after emergence | | | | | | | |
| 1 mM | 0.757a | 1.283b | 2.040b | 0.379c | 0.239c | 0.8c | 0.080b |
| 2 mM | 0.785a | 2.033a | 2.819a | 0.718a | 0.363bc | 2.3 | 0.506a |
| 3 mM | 0.664a | 1.911a | 2.574a | 0.451bc | 0.403ab | 2.8ab | 0.441a |
| 4 mM | 0.845a | 2.129a | 2.974a | 0.650ab | 0.506a | 3.3a | 0.688a |
| 4 weeks after emergence | | | | | | | |
| 1 mM | 0.842b | 2.010b | 2.852b | 1.318b | 0.677b | * | * |
| 2 mM | 1.493a | 3.772a | 5.265a | 3.130a | 1.018a | * | * |
| 3 mM | 1.232ab | 3.563a | 4.795a | 3.060a | 0.875ab | * | * |
| 4 mM | 1.010b | 2.943a | 3.952a | 2.787a | 0.891ab | * | * |

* Tillers removed 3 weeks after emergence
Means with similar letters are not different by protected Least Significant Difference (LSD) (0.05)

Fig. 9

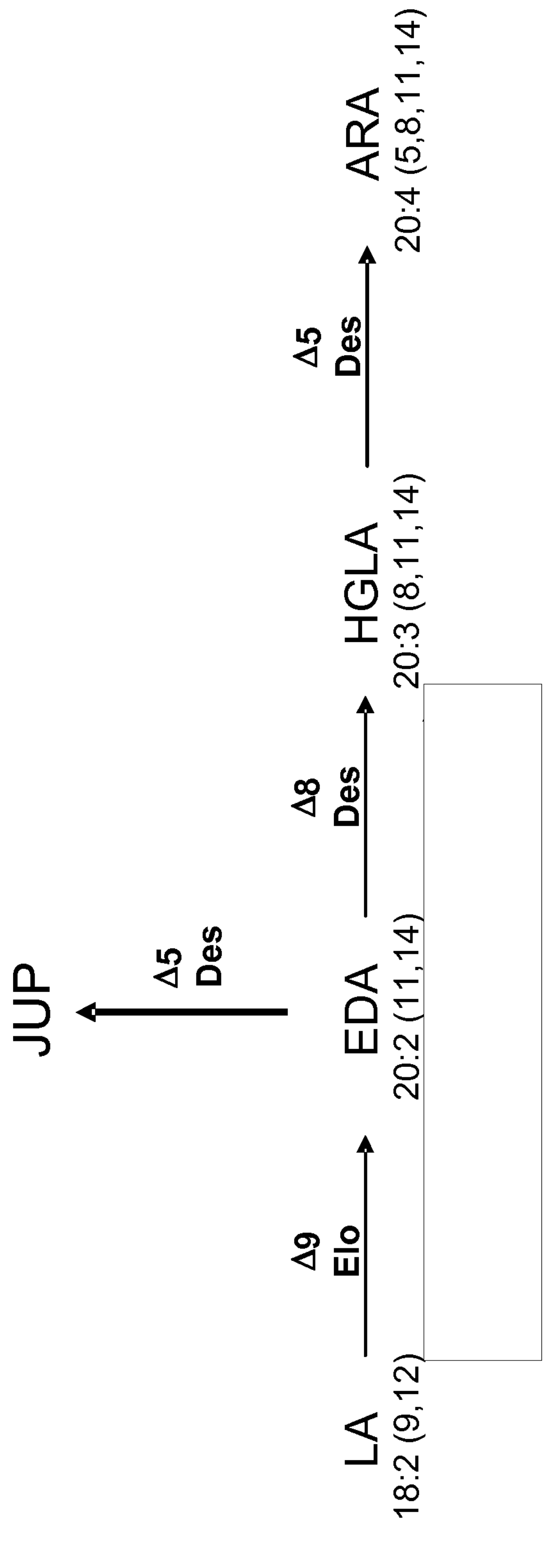
LA
18:2 (9,12)
Δ9
Elo
EDA
20:2 (11,14)
Δ5
Des
JUP
Δ8
Des
HGLA
20:3 (8,11,14)
Δ5
Des
ARA
20:4 (5,8,11,14)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61041956 A
- US 06173808 A
- US 20080254191 A
- WO 2002090493 A
- US 12061738 B
- US 7238482 B
- WO 2006052870 A
- US 60156306 A
- US 20070118929 A1
- US 5107065 A
- US 5231020 A
- WO 9953050 A
- WO 0200904 A
- WO 9836083 A
- US 7125672 B
- US 4683202 A
- US 8210741985 A
- WO 2004071178 A
- US 5004863 A
- US 5159135 A
- US 5569834 A
- US 5416011 A
- US 5463174 A
- WO 9217598 A
- US 5736369 A
- WO 2006012325 A
- EP 0242236 A
- US 7226745 B
- US 20030066102 A
- US 20050289664 A
- US 20040058427 A
- US 6245986 B
- US 20060010515 A1
- US 5378824 A
- US 20030221212 A
- US 20040122592 A
- US 5500361 A
- US RE37317 E
- WO 61041956 A
- WO 2007061845 A
- US 87611507 A

### Non-patent literature cited in the description


- **MEYER et al.** *Biochemistry,* 2003, vol. 42 (32), 9779-9788
- *Nucleic Acids Research,* 1985, vol. 13, 3021-3030
- *Biochemical Journal,* 1984, vol. 219 (2), 345-373
- **LASSNER et al.** *Plant Cell,* 1996, vol. 8, 281-292
- **MEINKOTH et al.** *Anal. Biochem.,* 1984, vol. 138, 267-284
- Overview of principles of hybridization and the strategy of nucleic acid probe assays. **TIJSSEN.** Laboratory Techniques in Biochemistry and Molecular Biology--Hybridization with Nucleic Acid Probes. Elsevier, 1993
- Current Protocols in Molecular Biology. Greene Publishing and Wiley-Interscience, 1995
- **HIGGINS ; SHARP.** *CABIOS,* 1989, vol. 5, 151-153
- **HIGGINS, D.G. et al.** *Comput. Appl. Biosci.,* 1992, vol. 8, 189-191
- **OKAMURO, J. K. ; GOLDBERG, R. B.** *Biochemistry of Plants,* 1989, vol. 15, 1-82
- **TURNER, R. ; FOSTER, G. D.** *Mol. Biotechnol.,* 1995, vol. 3, 225-236
- **INGELBRECHT, I. L. et al.** *Plant Cell,* 1989, vol. 1, 671-680
- **SAMBROOK, J. ; FRITSCH, E.F. ; MANIATIS, T.** Molecular Cloning: A Laboratory Manual. Cold Spring Harbor Laboratory, 1989
- **JONES et al.** *EMBO J.,* 1985, vol. 4, 2411-2418
- **DE ALMEIDA et al.** *Mol. Gen. Genetics,* 1989, vol. 218, 78-86
- **VAUCHERET et al.** *Plant J.,* 1998, vol. 16, 651-659
- **GURA.** *Nature,* 2000, vol. 404, 804-808
- **ELMAYAN et al.** *Plant Cell,* 1998, vol. 10, 1747-1757
- **YONGMANITCHAI ; WARD.** *Appl. Environ. Microbiol.,* 1991, vol. 57, 419-25
- **WALKER et al.** *Proc. Natl. Acad. Sci. U.S.A.,* 1992, vol. 89, 392

- The use of oligonucleotide as specific hybridization probes in the Diagnosis of Genetic Disorders. **THEIN ; WALLACE.** Human Genetic Diseases: A Practical Approach. IRL, 1986, 33-50
- **RYCHLIK, W.** Methods in Molecular Biology. 1993, vol. 15, 31-39
- PCR Protocols: Current Methods and Applications. Humania
- **FROHMAN et al.** *Proc. Natl Acad. Sci. U.S.A.,* 1988, vol. 85, 8998
- **OHARA et al.** *Proc. Natl Acad. Sci. U.S.A.,* 1989, vol. 86, 5673
- **LOH et al.** *Science,* 1989, vol. 243, 217
- **HELDT H.** Plant Biochemistry and Molecular Biology. Oxford University press, 1996, 247-277
- **ATANASSOVA et al.** *Plant Mol. Biol.,* 1998, vol. 37, 275-285
- **BATTRAW ; HALL.** *Plant Mol. Biol.,* 1990, vol. 15, 527-538
- **HOLTORF et al.** *Plant Mol. Biol.,* 1995, vol. 29, 637-646
- **JEFFERSON et al.** *EMBO J.,* 1987, vol. 6, 3901-3907
- **WILMINK et al.** *Plant Mol. Biol.,* 1995, vol. 28, 949-955
- **PLANT et al.** *Plant Mol. Biol,* 1994, vol. 25, 193-205
- **LI.** *Texas A&M University Ph.D. dissertation,* 1997, 107-128
- **CALLIS et al.** *J Biol. Chem.,* 1990, vol. 265 (21), 12486-93
- **ROLLFINKE et al.** *Gene,* 1998, vol. 211 (2), 267-76
- **SCHOFFL et al.** *Mol Gen Genet.,* 1989, vol. 217 (2-3), 246-53
- **ATANASSOVA et al.** *Plant Mol Biol.,* 1989, vol. 37 (2), 275-85
- **CHEN et al.** *Dev. Genet.,* 1989, vol. 10, 112-122
- **ELLERSTROM et al.** *Plant Mol. Biol.,* 1996, vol. 32, 1019-1027
- **KEDDIE et al.** *Plant Mol. Biol.,* 1994, vol. 24, 327-340
- **SAMBROOK, J. et al.** Molecular Cloning: A Laboratory Manual. Cold Spring Harbor Laboratory Press, 1989
- Current Protocols in Molecular Biology. John Wiley and Sons, 1990
- **CHENG et al.** *Plant Cell Rep.,* 1996, vol. 15, 653-657
- **MCKENTLY et al.** *Plant Cell Rep.,* 1995, vol. 14, 699-703
- **LING, K. et al.** *Bio/technology,* 1991, vol. 9, 752-758
- **GRANT et al.** *Plant Cell Rep.,* 1995, vol. 15, 254-258
- **NEWELL, C.A.** *Mol. Biotechnol.,* 2000, vol. 16, 53-65
- **TEPFLER, M. ; CASSE-DELBART, F.** *Microbiol. Sci.,* 1987, vol. 4, 24-28
- **CHOWRIRA, G.M. et al.** *Mol. Biotechnol.,* 1995, vol. 3, 17-23
- **CHRISTOU, P. et al.** *Proc. Natl. Acad. Sci. U.S.A.,* 1987, vol. 84, 3962-3966
- **MCCABE, D.E.** *Bio/Technology,* 1988, vol. 6, 923
- **CHRISTOU et al.** *Plant Physiol.,* 1988, vol. 87, 671-674
- **TU et al.** *Plant Molecular Biology,* 1998, vol. 37, 829-838
- **CHONG et al.** *Transgenic Research,* vol. 9, 71-78
- **MCCABE et al.** *BioTechnology,* 1988, vol. 6, 923-926
- **FINER ; MCMULLEN.** *In Vitro Cell Dev. Biol.,* 1991, vol. 27P, 175-182
- **SINGH et al.** *Theor. Appl. Genet.,* 1998, vol. 96, 319-324
- **KLEIN et al.** *Proc. Natl. Acad. Sci.,* 1988, vol. 85, 4305-4309
- **KLEIN et al.** *Biotechnology,* 1988, vol. 6, 559-563
- **KLEIN et al.** *Plant Physiol.,* 1988, vol. 91, 440-444
- **FROMM et al.** *Biotechnology,* 1990, vol. 8, 833-839
- Direct DNA Transfer into Intact Plant Cells via Microprojectile Bombardment. **TOMES et al.** Plant Cell, Tissue, and Organ Culture: Fundamental Methods. Springer-Verlag, 1995
- **HOOYKAAS-VAN SLOGTEREN et al.** *Nature,* 1984, vol. 311, 763-764
- **WEISSBACH ; WEISSBACH.** Methods for Plant Molecular Biology. Academic, 1988
- **SAMBROOK et al.** Molecular Cloning: A Laboratory Manual. Cold Spring Harbor, 1989
- **MALIGA et al.** Methods in Plant Molecular Biology. Cold Spring Harbor, 1995
- **BIRREN et al.** Genome Analysis: Detecting Genes. Cold Spring Harbor, 1998, vol. 1
- **BIRREN et al.** Genome Analysis: Analyzing DNA. Cold Spring Harbor, 1998, vol. 2
- Plant Molecular Biology: A Laboratory Manual. Springer, 1997
- **SOUTHERN.** *J. Mol. Biol.,* 1975, vol. 98, 503
- **KROCZEK.** *J. Chromatogr. Biomed. Appl.,* 1993, vol. 618 (1-2), 133-145
- **KLEIN et al.** *Nature,* 1987, vol. 327, 70
- **SCHMIDT et al.** *Cell Biology and Morphogenesis,* 2005, vol. 24, 393
- **CHU et al.** *Sci. Sin. Peking,* 1975, vol. 18, 659-668
- **ODELL et al.** *Nature,* 1985, vol. 313, 810-812
- **KLEIN et al.** *Nature,* 1987, vol. 327, 70-73
- **FROMM et al.** *Bio/Technology,* 1990, vol. 8, 833-839

- **MAYONADO et al.** *Pestic. Biochem. Physiol.,* 1989, vol. 35, 138-145
- **SCHULTZ et al.** *FEBS lett.,* 1993, vol. 318, 162-166
- **SECOR.** *Plant Phys.,* 1994, vol. 106, 1429-1433
- **MORAN GR.** *Archives of biochemistry and biophysics,* 1995, vol. 433, 117-28
- **GARCIA,I., RODGERS et al.** *Plant Physiol.,* 1999, vol. 119, 1507-1516
- **KEON J ; HARGREAVES J.** *FEMS Microbiol Lett.,* 15 April 1998, vol. 161 (2), 337-43
- **AWATA H ; ENDO F ; MATSUDA I.** *Genomics,* October 1994, vol. 23 (3), 534-9
- **WYCKOFF EE ; PISHKO EJ ; KIRKLAND TN ; COLE GT.** *Gene,* 08 August 1995, vol. 161 (1), 107-11
- **BONVIN J et al.** *Protein Sci.,* 2006, vol. 15 (6), 1417-32
- Biosynthesis of Tryptophan, Tyrosine and Phenylalanine from Chorismate. **SIEHL, DL.** Plant Amino Acids: Biochemistry and Biotechnology. Marcel Dekker, 1999
- **NORRIS et al.** *Plant Cell,* 1995, vol. 7, 2139-2149
- **KIM SK et al.** *J Bacteriol.,* 2006, vol. 188 (24), 8638-48
- **XU S et al.** *Protein Expr Purif.,* 2006, vol. 49 (2), 151-8
- **MOK, D. W. ; MOK, M. C.** *Annu. Rev. Plant Physiol. Plant Mol. Biol.,* 2001, vol. 52, 89-1181
- **TAYA, Y. ; TANAKA, Y. ; NISHIMURA, S.** *Nature,* 1978, vol. 271, 545-547
- **CHEN, C.-M. ; KRISTOPEIT, S. M.** *Plant Physiol.,* 1981, vol. 67, 494-498
- **KASAHARA et al.** *J. Biol. Chem.,* 2004, vol. 279, 14049-14054
- **ALTINCICEK B ; KOLLAS A ; EBERL M ; WIESNER J ; SANDERBRAND S ; HINTZ M ; BECK E ; JOMAA H.** LytB, a novel gene of the 2-C-methyl-D-erythritol 4-phosphate pathway of isoprenoid biosynthesis in Escherichia coli. *FEBS Lett,* 2001, vol. 499, 37-40
- **HECHT S ; EISENREICH W ; ADAM P ; AMSLINGER S ; KIS K ; BACHER A ; ARIGONI D ; ROHDICH F.** Studies on the nonmevalonate pathway to terpenes: the role of the GcpE (IspG) protein. *Proc Natl Acad Sci USA,* 2001, vol. 98, 14837-14842
- **TAKEI et al.** *J. Biol. Chem.,* 2001, vol. 276, 26405-26410
- **KAKIMOTO.** *Plant Cell Physiol.,* 2001, vol. 42, 677-685
- **SAKAMOTO et al.** *Plant Physiol,* September 2006, vol. 142 (1), 54-62
- **SUGAWARA et al.** *Proc Natl Acead Sci USA,* 19 February 2008, vol. 105 (7), 2734-9
- **CAMPOS et al.** *Biochem J,* 01 January 2001, vol. 353, 59-67
- **LU et al.** *Mol Biol Rep,* 26 May 2007
- **KIM et al.** *Planta,* January 2008, vol. 227 (2), 287-98
- **ROHDICH F ; HECHT S ; GÄRTNER K ; ADAM P ; KRIEGER C ; AMSLINGER S ; ARIGONI D ; BACHER A ; EISENREICH W.** Studies on the nonmevalonate terpene biosynthetic pathway: metabolic role of IspH (LytB) protein. *Proc Natl Acad Sci USA,* 2002, vol. 99, 1158-1163
- **KAKIMOTO, T.** *Plant Cell Physiol.,* 2001, vol. 42, 677-685
- **TAKEI et al.** *J. Plant Res.,* 2003, vol. 116, 265-269
- **J. GREEN.** *Weed technology,* 2006, vol. 21, 547-558